# EUROPEAN PATENT APPLICATION

(11) **EP 4 183 415 A1**
(43) Date of publication of application: **24.05.2023**
(21) Application number: 21842049.5
(22) Date of filing: 16.07.2021
(51) Int. Cl.: A61K 45/00, A61K 31/4709, A61K 31/497, A61K 31/5377, A61K 39/395, A61K 48/00, A61P 27/02, A61P 27/06, G01N 33/50

(54) **TRPV4 INHIBITOR AS THERAPEUTIC DRUG FOR EYE DISEASE**

(30) Priority: 16.07.2020 US 202063052572 P
(71) Applicant: RaQualia Pharma Inc., Nagoya-shi Aichi 450-0003 (JP)
(72) Inventor: OHARA Kentaro, Nagoya-shi, Aichi 450-0003 (JP); WATANABE Shuzo, Nagoya-shi, Aichi 450-0003 (JP); HARA Hideaki, Nara-shi, Nara 630-8002 (JP); SHIMAZAWA Masamitsu, Gifu-shi, Gifu 502-0816 (JP); SUGARU Eiji, Nagoya-shi, Aichi 450-0003 (JP)
(74) Representative: Lederer & Keller Patentanwälte Partnerschaft mbB
(86) International application number: PCT/JP2021/026829
(87) International publication number: WO 2022/014707

(57) **Abstract**

The present invention relates to a use of compounds having TRPV4 inhibitory activity or pharmaceutically acceptable salts thereof, or pharmaceutical compositions containing them for the manufacture of a medicament for preventing or treating a retinal disease accompanied with blood flow disorder or cell disorder. It also relates to a method for preventing or treating the disease, wherein the method comprises administering the compounds or the pharmaceutical compositions containing them to humans or animals. The compounds, the pharmaceutically acceptable salts thereof, or the pharmaceutical compositions containing them may be used in combination with one or more second active agents. The present invention relates to a pharmaceutical composition and a kit containing a compound having TRPV4 inhibitory activity or a pharmaceutically acceptable salt thereof, which is used for preventing or treating the disease. Furthermore, it relates to a marker for diagnosing a disease for which treatment with the compound is useful, and a screening method for drugs in preventing or treating the disease.

## Description

### [Technical Field]

The present invention relates to providing an agent for preventing or treating a retinal disease accompanied with blood flow disorder or cell disorder in animals including humans. More specifically, it relates to a use of compounds having TRPV4 (transient receptor potential vanilloid 4) inhibitory activity, or pharmaceutically acceptable salts thereof, or pharmaceutical compositions containing them for the manufacture of a medicament for preventing or treating a retinal disease. It also relates to a method for preventing or treating the disease, wherein the method comprises administering the compounds or the pharmaceutical compositions containing them to humans or animals. The compounds, the pharmaceutically acceptable salts thereof, or the pharmaceutical compositions containing them may be used in combination with one or more second active agents. The present invention relates to a pharmaceutical composition and a kit containing a compound having TRPV4 inhibitory activity or pharmaceutically acceptable salt thereof, which is used for preventing or treating the diseases. Furthermore, it relates to a marker for diagnosing a disease for which treatment with the compound is useful, and a screening method for drugs in preventing or treating the disease.

### [Background Art]

A retinal disease accompanied with blood flow disorder or cell disorder are thought to be caused mainly by aging, hypertension, cardiovascular disease, dyslipidemia, diabetes, arteriosclerosis, smoking, and the like.

A retinal disease accompanied with blood flow disorder or cell disorder includes, but is not limited to, retinal vein occlusion, retinal artery occlusion, wet age-related macular degeneration, retinitis pigmentosa, diabetic retinopathy, ischemic optic neuropathy and glaucoma. In addition, a retinal disease accompanied with blood flow disorder includes hypertensive retinopathy and amaurosis, and a retinal disease accompanied with cell disorder include hereditary optic neuropathy, retinal detachment, choroidal metastases, choroidal melanoma and choroidal hemangioma.

In addition, a retinal disease accompanied with blood flow disorder or cell disorder is extensively associated with symptoms of retinal damage such as blood flow disorder, cell disorder, retinal edema and pathological angiogenesis.

Retinal Vein Occlusion (RVO) is a retinal vascular disease in which a retinal vein is compressed by an artery in the retina or at the arteriovenous crossing on the surface of the optic disc, resulting in occlusion of retinal vein vessels. In the retina of patients with RVO, edema and nonperfusion area are formed, leading to rapid decreased visual acuity and visual field defect. Depending on where the venous occlusion occurs, a difference in medical conditions is observed. The optic disc is a point where the optic nerves connecting the photoreceptor cells to the brain converge toward the back of the eyeball to form a single thick bundle that penetrates the wall of the eye (sclera) to go to the brain. The veins of the retina are branched and spread all over the retina, and are gathered at the optic disc behind the eyeball to form a central retinal vein. When a branch of a vein in the retina is occluded, it is called Branch Retinal Vein Occlusion (BRVO), and when the base of the vein is occluded, it is called Central Retinal Vein Occlusion (CRVO).

Branch retinal vein occlusion (BRVO) occurs when blood flow is disrupted, primarily by thrombi at the arteriovenous crossing in the retina. When a vein becomes occluded, blood flows out of the peripheral veins (those farther from the heart) due to vascular occlusion, congestion, or blockage of the blood supply. The overflowing blood causes ocular fundus hemorrhage that spreads within the retina, and retinal edema (swelling of the retina), in which blood components seeping from blood vessels accumulate in the retina and cause the retina to swell.

Central retinal vein occlusion (CRVO) affects the entire retina because the base of the vein is occluded. Hemorrhage and edema spread all over the ocular fundus, and severe bleeding and edema also occur in the macula which is the most sensitive part of visual acuity located almost in the center of the retina. Symptoms are generally more severe the closer the occluding site is to the optic disc.

The most common cause of visual impairment in retinal vein occlusion (RVO) is macular edema, where fluid accumulates in the extracellular space within the retina, disrupting normal anatomy and potentially causing retinal neurodegeneration due to mechanical pressure. In addition, when capillaries on the peripheral side from the occluding site become occluded and the retina becomes ischemic, inappropriate neovascular vessel growth is caused to deliver oxygen and nutrients to the ischemic area. These neovascular vessels are fragile and bleed easily, often leading to vision-threatening complications such as neovascular glaucoma, vitreous hemorrhage, and fractional retinal detachment. In addition, loss of visual acuity occurs when capillaries at the occluding site disappear, blood flow to the macula does not resume, and photoreceptor cells disappear.

Retinal vein occlusion (RVO) is the second most common cause of loss of visual acuity resulting from a retinal vascular disease. Especially when there is hemorrhage or edema in the macula, which is the most sensitive part of visual acuity located almost in the center of the retina, retinal edema dramatically reduces visual acuity. According to the report by Rogers *et al.,* RVO affects 16.4 million adults worldwide. Inadequate treatment of macular edema associated with RVO can lead to significant loss of visual acuity and eventual blindness (Non-Patent Literature 1).

Retinal artery occlusion is a disease in which an artery supplying blood to the retina is occluded by an embolus or a thrombus, thereby interrupting the blood flow to the cells of the retina. Since oxygen and nutrients necessary for cell activity are supplied by blood, if the blood flow is interrupted, the cells of the retina from the interrupted site forward are exposed to the danger of necrosis. Occlusion of the blood vessel may occur in the central artery or in its branches, and is therefore also called central retinal artery occlusion or branch retinal artery occlusion. If the artery is not recanalized in time for the retinal nerve cells to tolerate the ischemic condition, the nerve cells will not function even if the blood flow is subsequently restored. Therefore, treatment is urgent.

Hypertensive retinopathy is a disease in which high blood pressure causes damage to the retina. When high blood pressure damages the capillaries of the retina, the walls of the blood vessels thicken and the amount of blood flowing through them decreases. As a result, the amount of blood supplied to the retina is reduced and damage to the retina occurs where the blood supply is inadequate. When hypertensive retinopathy progresses, blood components leaked from blood vessels may ooze into the retina, and accumulate in the retina to cause retinal edema. When edema occurs in the macula, visual acuity is severely impaired. In addition, a portion of the retina where blood flow is interrupted (ischemic region) is formed, and neovascular vessels grow (angiogenesis) to deliver oxygen and nutrients to the region. These blood vessels are very fragile and easily rupture, and vitreous hemorrhage, in which hemorrhage spreads into the vitreous body, or retinal detachment from hemorrhage may occur, which leads to a high possibility that severe visual impairment will remain. The course of progression of symptoms is almost the same as the course after proliferative retinopathy in diabetes, which is described later. Once hypertensive retinopathy becomes proliferative retinopathy, it progresses independently as a retinal disease, irrespective of blood pressure control.

Amaurosis causes temporary loss of visual acuity in one eye, resulting in symptoms such as "blackness". But after a while, usually a few seconds to a few minutes, it becomes visible again. Even if you lose your sight temporarily in this way, the symptoms soon improve. Thus, the condition is called "transient amaurosis" since the "blood flow was temporarily impaired" and then returns to normal. The blood vessels themselves that affect vision are not occluded, and in order to prevent recurrence, carotid artery stenosis due to arteriosclerosis, etc., or transient ischemic attack should be suspected.

On the other hand, if the state of complete blindness continues, the state is considered to be a condition in which the occlusion of ocular artery has already been completed, and falls under the aforementioned retinal artery occlusion.

Wet age-related macular degeneration is a disease that occurs in the macula with aging. There are two types of age-related macular degeneration, which are classified into exudative (wet) type and atrophic (dry) type depending on the presence or absence of choroidal neovascularization. Wet age-related macular degeneration is a type in which abnormal blood vessels called neovascular vessel proliferate from the choroid in the macula and extend toward the retina. Retinal cells repeat metabolism, and waste products generated by metabolism are digested and eliminated in the retinal pigment epithelium between the retina and the choroid. As the function of the retinal pigment epithelium declines with aging, undigested waste products accumulate in Bruch's membrane which lies beneath the retinal pigment epithelium and borders the choroid. Chronic weak inflammation occurs against waste products (white masses called drusen), and vascular endothelial growth factor (VEGF) is released to mitigate the inflammation, resulting in the growth of neovascular vessels from the choroid. When neovascular vessels penetrate Bruch's membrane and proliferate below or above the retinal pigment epithelium, hemorrhage and exudation (leakage) become severe, resulting in edema of the macula and impairment of macular function. In dry age-related macular degeneration, retinal pigment epithelial cells or choroidal capillary plate atrophies, resulting in damage to the retina.

Retinitis pigmentosa is a rare disease that causes progressive degeneration of the retina. It is a bilateral disease in which photoreceptor cells age prematurely and cease to function. In retinitis pigmentosa, narrowing of retinal arteries and atrophy of the optic nerves are observed, and areas where photoreceptor cells have stopped functioning cannot perceive light and cannot produce images. This is a genetically related disease.

Diabetic retinopathy is a disease in which diabetes causes damage to the retina. When the blood sugar level is high, blood vessels are heavily burdened and blood flow is poor, and thus the retina, which is densely packed with fine blood vessels, is very susceptible to the effects of the high blood sugar level. In the stage of simple retinopathy, the blood flow in the retina begins to deteriorate, and capillary aneurysm, intraretinal hemorrhage and exudation in which blood and plasma components seep out from the walls of blood vessels appear. Diabetic retinopathy progresses through the following stages. In the stage of preproliferative retinopathy, there appear macular ischemia, in which blood vessels are occluded and a part of the retina has an ischemic area where blood does not flow; venous anomaly, in which the veins are abnormally swollen; and retinal edema and macular edema, in which blood components exuding from blood vessels accumulate in the retina and swell the retina. In the stage of proliferative retinopathy, inappropriate neovascular vessel growth occurs to deliver oxygen and nutrients to the ischemic area. The neovascular vessel is very fragile and can bleed, form scars, or rupture and spread vitreous hemorrhages, greatly affecting visual acuity. In addition, a thin membranous proliferative membrane is formed by stimulating the components exuded from the neovascular vessel, and the proliferative membrane pulls the retina, resulting in retinal detachment. Diabetic macular edema is also the most common cause of moderate loss of visual acuity in diabetic patients and is a symptom of diabetic retinopathy.

Ischemic optic neuropathy is a disease in which the blood supply to the optic nerve is interrupted, resulting in infarction and damage to the optic nerve. There are two types of optic nerve infarction, non-arteritic form and arteritic form. Obstruction can occur without inflammation of the arteries (non-arteritic form) or with inflammation of the arteries (arteritic form). When the obstruction is accompanied with stenosis of the internal carotid artery, repeated bouts of the "transient amaurosis" mentioned above may occur before onset.

The non-arteritic form occurs more frequently, typically in patients about 50 years of age or older. The arteritic form tends to cause more severe visual impairment and usually occurs in older populations, typically over 70 years of age. The optic nerve disc swells and bulges, and the swollen optic nerve fibers obscure the fine blood vessels on the surface of the optic nerve. Often hemorrhages are seen around the optic disc. The optic disc may be pale white in the arteritic form and hyperemic in the non-arteritic form.

Glaucoma is a disease in which the optic disc is crushed from the inside of the eyeball, resulting in progressive depression of the optic disc and a decrease in the number of normally functioning optic nerves. Once the optic nerve is lost, it cannot be restored, and in the worst case, it is a disease that leads to blindness. A major cause of optic nerve damage is high intraocular pressure. When the fluid in the eye that nourishes the eye (aqueous humor) does not drain normally, such as when the drainage duct (angle) between the iris and cornea is occluded, causing excess aqueous humor to build up, optic nerve damage occurs. At that time, the intraocular pressure rises, and if the intraocular pressure exceeds the limit that the optic nerve can withstand, the optic nerve is damaged. In addition, it is not only intraocular pressure that is related to glaucoma. Even in normal-tension glaucoma with normal intraocular pressure, depression of the optic disc may progress. It is thought that the cause is that the structure of the optic disc is relatively weakened comparing to the intraocular pressure due to optic nerve circulation disorder (poor blood flow). Neovascular glaucoma, where vascular endothelial growth factor (VEGF) is produced due to retinal ischemia, such as retinal vein occlusion and diabetic retinopathy mentioned above, and neovascular vessels are formed to compensate for the ischemic condition, is also known.

Hereditary optic neuropathy is caused by a genetic disorder that damages the optic nerve and sometimes causes cardiac or nervous system abnormalities in addition to visual impairment. Optic nerve damage is usually permanent and may be progressive. By the time optic nerve atrophy is observed, considerable optic nerve damage has already occurred. This is a genetically related disease.

Retinal detachment is the separation (detaching) of the light-sensitive layer of the sensory retina from the underlying retinal pigment epithelium layer. There are three types of retinal detachment: fractional retinal detachment, exudative (serous) retinal detachment and rhegmatogenous retinal detachment (with retinal tear). Tractional retinal detachment occurs in vascular occlusive diseases of the retina (ischemic disease), such as diabetic retinopathy, where a proliferative membrane forms between the retina and the vitreous body due to newly arising neovascular vessels, causing strong adhesion between the two. As a result, the proliferative membrane contracts and the retina is pulled and detached. Exudative retinal detachment is a type of retinal detachment in which exudate accumulates under the sensory retina, causing it to lift and detach. Causes include inflammation of the ocular fundus, severe uveitis, choroidal hemangioma, and primary or metastatic choroidal carcinoma. When the vitreous body and retina are strongly adherent or when the retina is weakened, the retina may tear as it is pulled by the contracting vitreous body, creating a fissure or hole, namely retinal tear. Risk factors include myopia, previous cataract surgery, ocular trauma and retinal lattice degeneration, etc. If rhegmatogenous retinal detachment is acute and threatens central visual acuity, treatment is urgent.

Choroidal metastases tend not to present symptoms until later stages, but choroidal damage caused by cancer includes symptoms of visual impairment and retinal detachment. Since the choroid contains many blood vessels and half of the retinal blood supply depends on the choroid, the frequency of choroidal metastases is high. Cancers that affect the retina usually originate in the choroid.

Choroidal melanoma arises from melanocytes in the choroid and is the most common cancer occurring in the eye. Symptoms tend not to appear until later stages, but symptoms such as visual impairment and retinal detachment are present.

Choroidal hemangioma is a benign tumor arising in the choroid. There are two types in choroidal hemangioma: an independent type, in which the tumor occurs only in the choroid, and a diffuse type, which is associated with Sturge-Weber syndrome, in which hemangiomas also occur in the face or brain. When retinal detachment occurs at the site where the tumor is formed, subjective symptoms such as visual field defect and decreased visual acuity may appear.

Currently, anti-VEGF agents (bevacizumab, aflibercept, pegaptanib, or ranibizumab) with pharmacological effects to suppress Vascular Endothelial Growth Factor (VEGF) are used for retinal diseases as effective therapeutic agents for angiogenesis and macular edema. However, since the effect is temporary, repeated administration is necessary depending on the condition.

Vascular endothelial growth factor (VEGF) is an inflammatory, angiogenic, and permeability factor produced by many cells including vascular endothelial cells, Müller cells, astrocytes, and retinal ganglion cells. Overproduction of VEGF further increases vascular permeability and exacerbates retinal and macular edema (Non-Patent Literature 2).

Also in diabetes, retinal Müller cells may have an adaptive response to protect tissues from damage, and VEGF is one of the factors released by activated Müller cells. Low concentrations of VEGF have pro-survival effects on vascular endothelial cells and retinal neurons, but overproduction of VEGF may exacerbate retinal disease progression by inducing vascular leakage and angiogenesis. High concentrations of VEGF cause endothelial cell hyperplasia, cause capillary nonperfusion, and induce vascular function characteristic of diabetic retinopathy including vascular dilation and tortuosity, vascular leakage, focal hemorrhages, microaneurysms, and preretinal angiogenesis (Non-Patent Literature 3).

In addition, anti-VEGF drugs and other agents may not improve visual function or have only a limited effect, despite the disappearance of macular edema. It has been suggested that pathologies other than macular edema are involved in such visual deterioration, and it has been found that photoreceptor cells in the central fossa are damaged and the retinal nonperfusion area (NPA) in the paracentral fossa is enlarged (Non-Patent Literature 4).

In clinical trials of anti-VEGF drugs, it has been reported that only 50% to 60% of patients achieved an improvement in visual acuity of 15 letters or more on the ETDRS visual acuity chart, and that the effect of overall average improvement in visual acuity was also limited (VIBRANT study and GALILEO study).

Therefore, there exists an unmet need for treating ocular diseases, and agents with novel mechanisms of action that provide better efficacy are required.

TRPV4 (transient receptor potential vanilloid 4) is a member of the TRPV (vanilloid) family which is one of the seven major subfamilies of the TRP family of ion channels, and is a Ca(2+) and Mg(2+) permeable cation channel with six transmembrane regions. It is called a cell osmotic sensor because it is activated when hypotonic cells swell. TRPV4 is also thermosensitive and reacts with not only moderate heat (24 to 27 °C), but also reacts with some endogenous substances such as phorbol esters (4α-PDD), arachidonic acid (AA) and epoxyeicosatrienoic acid, which is a P450 cytochrome metabolite of endocannabinoids and arachidonic acid. TRPV4 is distributed in a variety of tissues and is activated by a wide range of stimuli including physical (such as cell swelling, heat and mechanical stimuli) and chemical (such as phospholipase A2 (PLA2) and its metabolite, arachidonic acid, etc.) (Non-Patent Literatures 5-8).

Results of animal studies suggest that TRPV4 inhibitory effects are associated with pulmonary edema, cerebral edema, pain, itching, gastrointestinal disorder, vascular tone, hypertension and diabetes due to endothelial dysfunction, heart failure, and diseases of respiratory function such as bronchoconstriction, pulmonary hypertension, acute lung injury, cystic fibrosis, chronic obstructive pulmonary disease, acute respiratory distress syndrome, and cough (Non-Patent Literatures 8 and 9).

In the vasculature, TRPV4 is a regulator of vascular tone and is implicated in hypertension and diabetes due to endothelial dysfunction. TRPV4 is a key regulator of epithelial and endothelial barrier function, and signaling to TRPV4 and release of signaling may disrupt these important protective barriers (Non-Patent Literature 8).

Sustained exposure to TRPV4 agonists reduces the survival rate of mouse retinal ganglion cells (RGCs) by triggering an apoptotic process. This is consistent with reports that even low levels of Ca(2+) are toxic to retinal ganglion cells when sustained for long periods of time (Non-Patent Literature 10).

In the currently known prior art related to TRPV4 on ocular diseases, there is a report that a specific TRPV4 inhibitor exhibits efficacy in a glaucoma model caused by intraocular pressure loading (Patent Literature 1).

There exist two kinds of reports that a single administration of GSK2193874, a TRPV4 inhibitor, in the vitreous body of diabetic model mice suppresses retinal edema (Patent Literature 2) and that it does not (Non-Patent Literature 11). Therefore, the technical common knowledge has not yet been established. It has also been reported that retinal thinning is not suppressed (Non-Patent Literature 11).

### [Prior Art Literature]

### [Patent Literature]

[Patent Literature 1] WO2013169396
[Patent Literature 2] MX2017011166

### [Non-Patent Literature]

[Non-Patent Literature 1] Ophthalmology, 2010, 117: 313-319
[Non-Patent Literature 2] Survey of ophthalmology, 2018, 63: 816-850
[Non-Patent Literature 3] Prog Retin Eye Res, 2009, 28: 423-451
[Non-Patent Literature 4] RETINA, 2018, 38: 272-282
[Non-Patent Literature 5] Pflugers Arch, 2005, 451: 1-10
[Non-Patent Literature 6] Handbook of Experimental Pharmacology; Transient Receptor Potential (TRP) Channels, 2007, 179: 189-205
[Non-Patent Literature 7] Exp Eye Res, 2011, 93: 710-719
[Non-Patent Literature 8] Int J Biochem Cell Biol, 2016, 78: 217-228
[Non-Patent Literature 9] Pharmacol Ther, 2017, 177: 9-22
[Non-Patent Literature 10] J Neurosci, 2011, 31: 7089-7101
[Non-Patent Literature 11] PLoS ONE, 2019, 14(5): e0212158

### [Summary of Invention]

### [Technical Problem]

There is an unmet need for existing therapeutic agents and therapeutic methods for treating a retinal disease accompanied with blood flow disorder or cell disorder, and agents and therapies with novel mechanisms of action that provide better efficacy are required.

In RVO, retinal capillary perfusion is reduced, which can cause retinal ischemia and hypoxia. Retinal hypoxia causes loss of retinal capillary pericytes and damage to vascular endothelial cells. Retinal ischemia damages various layers of the neurosensory retina, causing the death of cells including ganglion and Müller cells by necrosis and/or apoptosis, resulting in loss of visual function (Survey of ophthalmology, 2018, 63: 816-850).

Various retinal diseases such as retinal detachment and wet age-related macular degeneration are thought to cause degeneration of photoreceptor cells, resulting in visual impairment. It is known that inflammatory cytokines (such as TNFα) are involved in this degeneration of photoreceptor cells (Invest Ophthalmol Vis Sci, 2011, 52: 1384-1391).

Therefore, if a substance that improves retinal nonperfusion and protects the retina can be found, it may become a therapeutic agent for a retinal disease accompanied with blood flow disorder or cell disorder represented by retinal vein occlusion, retinal detachment, and wet age-related macular degeneration. As a result of intensive studies, the inventors of the present application surprisingly found that compounds exhibiting TRPV4 inhibitory activity are useful for a retinal disease accompanied with blood flow disorder or cell disorder, and completed the present invention after further investigation.

An object of the present invention is to provide a novel therapeutic agent and a therapeutic method for a retinal disease accompanied with blood flow disorder or cell disorder.

A further object of the present invention is to focus on the relationship between retinal diseases and TRPV4 channels, and to provide a disease marker useful for diagnosis and treatment of the diseases, and a method for screening drugs effective for preventing or treating the diseases.

### [Solution to Problem]

In order to solve the above problems, the present invention includes the following inventions. The compounds for preventing or treating a retinal disease accompanied with blood flow disorder or cell disorder may be compounds with TRPV4 inhibitory activity already known, and may be compounds with TRPV4 inhibitory activity to be discovered in the future.
[1] The present invention provides a use of a compound having TRPV4 inhibitory activity or a pharmaceutically acceptable salt thereof for the manufacture of a medicament that prevents or treats a retinal disease accompanied with blood flow disorder or cell disorder in humans or animals.
[2] The present invention provides the use according to [1], wherein the retinal disease is selected from the group consisting of hypertensive retinopathy and amaurosis, which are retinal diseases accompanied with blood flow disorder; selected from the group consisting of hereditary optic neuropathy, retinal detachment, choroidal metastases, choroidal melanoma and choroidal hemangioma, which are retinal diseases accompanied with cell disorder; or selected from the group consisting of retinal vein occlusion, retinal artery occlusion, wet age-related macular degeneration, retinitis pigmentosa, diabetic retinopathy, ischemic optic neuropathy, and glaucoma, which are retinal diseases accompanied with both blood flow disorder and cell disorder.
[3] The present invention provides the use according to [1], wherein the retinal disease is retinal vein occlusion or wet age-related macular degeneration.
[4] The present invention provides the use according to [1], wherein the retinal disease is branch retinal vein occlusion or central retinal vein occlusion.
[5] Compounds for preventing or treating the retinal disease of the present invention includes a compound with TRPV4 inhibitory activity or a pharmaceutically acceptable salt thereof already known, and a compound with TRPV4 inhibitory activity or a pharmaceutically acceptable salt thereof to be discovered in the future. The list includes the following.

Examples of known compounds having TRPV4 inhibitory activity include the following:
compounds disclosed in WO2013012500 represented by GSK2798745: (1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile);
compounds disclosed in WO2011119704 represented by GSK2193874: (3-(1,4'-bipiperidin-1'-ylmethyl)-7-bromo-N-(1-phenylcyclopropyl)-2-[3-(trifluoromethyl)phenyl]-4-quinolinecarboxamide);
compounds disclosed in WO2013169396 represented by HC-067047: (N-(3-(trifluoromethyl)phenyl)-2-methyl-1-(3-morpholinopropyl)-5-phenyl-1H-pyrrole-3-carboxamide);
GSK3395879; GSK3527497; GSK205; GSK3491943; RN-1734; RN-1747; RN-9893; PF-05214030;
rosmarinic acid derivatives disclosed in WO2019244937;
pyrrolidine sulfonamide derivatives disclosed in WO2018055527, WO2018055526 and WO2018055524;
1-(((5S,7R)-3-(5-cyclopropylpyrazin-2-yl)-7-hydroxy-2-oxo-1-oxa-3-azaspiro[4.5]decane-7-yl)methyl)-1H-benzo[d]imidazole-6-carbonitrile disclosed in WO2017199199;
spirocarbamate derivatives disclosed in WO2013012500, WO2012174342 and WO2012174340;
quinoline derivatives disclosed in WO2011119701, WO2011119704 and WO2011119693;
naphthyridine derivatives disclosed in WO2011119694;
indole or benzothiophene derivatives disclosed in WO2011091410;
diazabicyclo[2.2.1]hept-2-yl derivatives disclosed in WO2009111680, WO2009146177, WO2009146182, WO2010011912, WO2010011914 and WO2011091407;
substituted piperidine derivatives disclosed in WO2007098393;
substituted pyrrolidine derivatives disclosed in WO2007082262;
aromatic heterocyclic amine derivatives disclosed in WO2015046193;
aromatic five-membered heterocyclic derivatives disclosed in WO2013146754;
aromatic heterocyclic derivatives disclosed in WO2012144661;
6-membered ring derivatives disclosed in WO2015199206;
phenylacetic acid-based anti-inflammatory agents disclosed in JP-A-2009-084209;
a TRPV4 inhibitor as described in Bioorg Med Chem Lett., 2020, 30(8): 127022;
a TRPV4 inhibitor as described in Bioorg Med Chem., 2019, 27: 3775-3 7 8 7;
following compounds described in US63017891 (application number) or PCT/JP2021/17277 (application number) disclosed by RaQualia Pharma Inc. :
   (R)-1-(3'-(1-(6-(2-(3-chlorophenyl)-2-hydroxyacetyl)-4-oxo-4, 5, 6, 7, 8,9-hexahydro-3H-pyrimido[5,4-c]azepin-2-yl)cyclopropyl)-[1,1'-biph enyl]-4-yl)cyclopropane-1-carbonitrile;
   (R)-2-(1-(3-(benzo[b]thiophen-3-yl)phenyl)cyclopropyl)-6-(2-hydroxy -2-(3-(trifluoromethyl)phenyl)acetyl)-3,5,6,7,8,9-hexahydro-4H-pyri mido[5,4-c]azepin-4-one;
   (R)-2-(1-(3-(benzofuran-3-yl)phenyl)cyclopropyl)-6-(2-(3-chlorophen yl)-2-hydroxyacetyl)-3,5,6,7,8,9-hexahydro-4H-pyrimido[5,4-c]azepin -4-one;
   (R)-2-(1-(3-chlorophenyl)cyclopropyl)-6-(2-hydroxy-2-(3-(trifluorom ethyl)phenyl)acetyl)-3,5,6,7,8,9-hexahydro-4H-pyrimido[5,4-c]azepin -4-one;
   (R)-2-(1-(4-bromothiophen-2-yl)cyclopropyl)-6-(2-(3-chlorophenyl)-2 -hydroxyacetyl)-5,6,7,8-tetrahydropyrido[4,3-d]pyrimidin-4(3H)-one;
   (R)-2-(1-(5-Cyclohexylpyridin-3-yl)cyclopropyl)-6-(2-hydroxy-2-(3-( trifluoromethyl)phenyl)acetyl)-3,5,6,7,8,9-hexahydro-4H-pyrimido[5, 4-c]azepin-4-one;
   (R)-5-(3-(1-(6-(2-hydroxy-2-(3-(trifluoromethyl)phenyl)acetyl)-4-ox o-4,5,6,7,8,9)-hexahydro-3H-pyrimido[5,4-c]azepin-2-yl)cyclopropyl) phenyl)nicotinonitrile;
   (R)-6-(2-(2,3-difluorophenyl)-2-hydroxyacetyl)-2-(1-phenylcycloprop yl)-3,5,6,7,8,9-hexahydro-4H-pyrimido[5,4-c]azepin-4-one;
   (R)-6-(2-(2,3-difluorophenyl)-2-hydroxyacetyl)-2-(1-phenylcycloprop yl)-5,6,7,8-tetrahydropyrido[4,3-d]pyrimidin-4 (3H)-one;
   (R)-6-(2-(3-(benzofuran-2-yl)phenyl)-2-hydroxyacetyl)-2-(1-phenylcy clopropyl)-3,5,6,7,8,9-hexahydro-4H-pyrimido[5,4-c]azepin-4-one;
   (R)-6-(2-(3-(benzofuran-2-yl)phenyl)-2-hydroxyacetyl)-2-(1-phenylcy clopropyl)-3,5,6,7,8,9-hexahydro-4H-pyrimido[5,4-c]azepin-4-one;
   (R)-6-(2-(3-(benzofuran-2-yl)phenyl)-2-hydroxyacetyl)-2-(1-phenylcy clopropyl)-5,6,7,8-tetrahydropyrido[4,3-d]pyrimidin-4(3H)-one;
   (R)-6-(2-(3'-(tert-butyl)-[1,1'-biphenyl]-3-yl)-2-hydroxyacetyl)-2-(1-(4-isopropylthiophene-2-yl)cyclopropyl)-3,5,6,7,8,9-hexahydro-4H -pyrimido[5,4-c]azepin-4-one;
   (R)-6-(2-(3'-(tert-butyl)-[1,1'-biphenyl]-3-yl)-2-hydroxyacetyl)-2-(1-(4-phenylthiophene-2-yl)cyclopropyl)-3,5,6,7,8,9-hexahydro-4H-py rimido[5,4-c]azepin-4-one;
   (R)-6-(2-(3'-chloro-[1,1'-biphenyl]-3-yl)-2-hydroxyacetyl)-2-(1-phe nylcyclopropyl)-3,5,6,7,8,9-hexahydro-4H-pyrimido[5,4-c]azepin-4-on e;
   (R)-6-(2-(3-chlorophenyl)-2-hydroxyacetyl)-2-(1-(3-(1-methyl-1H-ind azol-4-yl)phenyl)cyclopropyl)-3,5,6,7,8,9-hexahydro-4H-pyrimido[5,4 -c]azepin-4-one;
   (R)-6-(2-(3-chlorophenyl)-2-hydroxyacetyl)-2-(1-(3-(1-methyl-1H-ind azol-5-yl)phenyl)cyclopropyl)-3,5,6,7,8,9-hexahydro-4H-pyrimido[5,4 -c]azepin-4-one;
   (R)-6-(2-(3-chlorophenyl)-2-hydroxyacetyl)-2-(1-(3-(2-methoxypyridi n-4-yl)phenyl)cyclopropyl)-3,5,6,7,8,9-hexahydro-4H-pyrimido[5,4-c] azepin-4-one;
   (R)-6-(2-(3-chlorophenyl)-2-hydroxyacetyl)-2-(1-(3-chlorophenyl)cyc lopropyl)-5,6,7,8-tetrahydropyrido[4,3-d]pyrimidin-4 (3H)-one;
   (R)-6-(2-(3-chlorophenyl)-2-hydroxyacetyl)-2-(1-(3-fluorophenyl)cyc lopropyl)-5,6,7,8-tetrahydropyrido[4,3-d]pyrimidin-4 (3H)-one;
   (R)-6-(2-(3-chlorophenyl)-2-hydroxyacetyl)-2-(1-(4-chlorophenyl)cyc lopropyl)-5,6,7,8-tetrahydropyrido[4,3-d]pyrimidin-4 (3H)-one;
   (R)-6-(2-(3-chlorophenyl)-2-hydroxyacetyl)-2-(1-(4-isopropylthiophe n-2-yl)cyclopropyl)-3,5,6,7,8,9-hexahydro-4H-pyrimido[5,4-c]azepin-4-one;
   (R)-6-(2-(3-chlorophenyl)-2-hydroxyacetyl)-2-(1-(4-phenylthiophen-2 -yl)cyclopropyl)-5,6,7,8-tetrahydropyrido[4,3-d]pyrimidin-4(3H)-one ;
   (R)-6-(2-(3-chlorophenyl)-2-hydroxyacetyl)-2-(1-(thiophen-2-yl)cycl opropyl)-5,6,7,8-tetrahydropyrido[4,3-d]pyrimidin-4(3H)-one;
   (R)-6-(2-(3-chlorophenyl)-2-hydroxyacetyl)-2-(1-phenylcyclopropyl)-3,5,6,7,8,9-hexahydro-4H-pyrimido[5,4-c]azepin-4-one;
   (R)-6-(2-(3-chlorophenyl)-2-hydroxyacetyl)-2-(1-phenylcyclopropyl)-5,6,7,8-tetrahydropyrido[4,3-d]pyrimidine-4 (3H)-one;
   (R)-6-(2-(4'-(benzyloxy)-[1,1'-biphenyl]-3-yl)-2-hydroxyacetyl)-2-( 1-phenylcyclopropyl)-5,6,7,8-tetrahydropyrido[4,3-d]pyrimidin-4(3H) -one;
   (R)-6-(2-Hydroxy-2-(3-(2-(trifluoromethyl)pyridin-4-yl)phenyl)acety 1)-2-(1-(3-isopropylphenyl)cyclopropyl)-3,5,6,7,8,9-hexahydro-4H-py rimido[5,4-c]azepin-4-one;
   (R)-6-(2-hydroxy-2-(3-(2-(trifluoromethyl)pyridin-4-yl)phenyl)acety 1)-2-(1-(4-isopropylthiophen-2-yl)cyclopropyl)-3,5,6,7,8,9-hexahydr o-4H-pyrimido[5,4-c]azepin-4-one;
   (R)-6-(2-hydroxy-2-(3-(6-(trifluoromethyl)pyridin-2-yl)phenyl)acety 1)-2-(1-phenylcyclopropyl)-3,5,6,7,8,9-hexahydro-4H-pyrimido[5,4-c] azepin-4-one;
   (R)-6-(2-hydroxy-2-(3-(6-(trifluoromethyl)pyridin-2-yl)phenyl)acety 1)-2-(1-phenylcyclopropyl)-5,6,7,8-tetrahydropyrido[4,3-d]pyrimidin -4 (3H)-one;
   (R)-6-(2-hydroxy-2-(3-(quinolin-3-yl)phenyl)acetyl)-2-(1-phenylcycl opropyl)-5,6,7,8-tetrahydropyrido[4,3-d]pyrimidin-4(3H)-one;
   (R)-6-(2-hydroxy-2-(3'-(trifluoromethyl)-[1,1'-biphenyl]-3-yl)acety 1)-2-(1-(3-isopropylphenyl)cyclopropyl)-3,5,6,7,8,9-hexahydro-4H-py rimido[5,4-c]azepin-4-one;
   (R)-6-(2-hydroxy-2-(3'-(trifluoromethyl)-[1,1'-biphenyl]-3-yl)acety 1)-2-(1-phenylcyclopropyl)-3,5,6,7,8,9-hexahydro-4H-pyrimido[5,4-c] azepin-4-one;
   (R)-6-(2-hydroxy-2-(3-(trifluoromethyl)phenyl)acetyl)-2-(1-(3-(isot hiazol-4-yl)phenyl)cyclopropyl)-3,5,6,7,8,9-hexahydro-4H-pyrimido[5 ,4-c]azepin-4-one;
   (R)-6-(2-hydroxy-2-(3-(trifluoromethyl)phenyl)acetyl)-2-(1-(3-(isot hiazol-4-yl)phenyl)cyclopropyl)-5,6,7,8-tetrahydropyrido[4,3-d]pyri midin-4(3H)-one;
   (R)-6-(2-hydroxy-2-(3-(trifluoromethyl)phenyl)acetyl)-2-(1-(3-(prop -1-en-2-yl)phenyl)cyclopropyl)-3,5,6,7,8,9-hexahydro-4H-pyrimido[5, 4-c]azepin-4-one;
   (R)-6-(2-hydroxy-2-(3-(trifluoromethyl)phenyl)acetyl)-2-(1-(3-(pyri din-3-yl)phenyl)cyclopropyl)-3,5,6,7,8,9-hexahydro-4H-pyrimido[5,4-c]azepin-4-one;
   (R)-6-(2-hydroxy-2-(3-(trifluoromethyl)phenyl)acetyl)-2-(1-(3-isopr opylphenyl)cyclopropyl)-3,5,6,7,8,9-hexahydro-4H-pyrimido[5,4-c]aze pin-4-one;
   (R)-6-(2-hydroxy-2-(3-(trifluoromethyl)phenyl)acetyl)-2-(1-(4-isopr opylthiophen-2-yl)cyclopropyl)-3,5,6,7,8,9-hexahydro-4H-pyrimido[5, 4-c]azepin-4-one;
   (R)-6-(2-hydroxy-2-(3-(trifluoromethyl)phenyl)acetyl)-2-(1-(4-isopr opylthiophen-2-yl)cyclopropyl)-5,6,7,8-tetrahydropyrido[4,3-d]pyrim idin-4(3H)-one;
   (R)-6-(2-hydroxy-2-(3-(trifluoromethyl)phenyl)acetyl)-2-(1-(4-isopr opylthiophen-2-yl)cyclopropyl)-5,6,7,8-tetrahydropyrido[4,3-d]pyrim idin-4(3H)-one;
   (R)-6-(2-hydroxy-2-(3-(trifluoromethyl)phenyl)acetyl)-2-(1-(5-isopr opylpyridin-3-yl)cyclopropyl)-3,5,6,7,8,9-hexahydro-4H-pyrimido[5,4 -c]azepin-4-one;
   (R)-6-(2-hydroxy-2-(3-(trifluoromethyl)phenyl)acetyl)-2-(1-(5-pheny lpyridin-3-yl)cyclopropyl)-3,5,6,7,8,9-hexahydro-4H-pyrimido[5,4-c] azepin-4-one;
   (R)-6-(2-hydroxy-2-(3-(trifluoromethyl)phenyl)acetyl)-2-(1-phenylcy clopropyl)-5,6,7,8-tetrahydropyrido[4,3-d]pyrimidin-4(3H)-one;
   2-(1-(3-chlorophenyl)cyclopropyl)-6-(2-(4'-(trifluoromethoxy)-[1,1' -biphenyl]-3-yl)acetyl)-3,5,6,7,8,9-hexahydro-4H-pyrimido[5,4-c]aze pin-4-one;
   2-(1-(3-chlorophenyl)cyclopropyl)-6-(2-hydroxy-2-(3'-(trifluorometh oxy)-[1,1'-biphenyl]-3-yl)acetyl)-3,5,6,7,8,9-hexahydro-4H-pyrimido [5,4-c]azepin-4-one;
   2-(1-(5-phenylpyridin-3-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
   3'-(2-(2-(1-(3-chlorophenyl)cyclopropyl)-4-oxo-3,4,5,7,8,9-hexahydr o-6H-pyrimido[5,4-c]azepine-6-yl)-2-oxoethyl)-[1,1'-biphenyl]-4-car bonitrile;
   5-chloro-3'-(2-oxo-2-(4-oxo-2-(1-phenylcyclopropyl)-3,4,5,7,8,9-hex ahydro-6H-pyrimido[5,4-c]azepin-6-yl)ethyl)-[1,1'-biphenyl]-3-carbo nitrile;
   6-(2-(2-(benzyloxy)phenyl)acetyl)-2-(1-phenylcyclopropyl)-3,5,6,7,8 ,9-hexahydro-4H-pyrimido[5,4-c]azepin-4-one;
   6-(2-(3-(benzyloxy)phenyl)acetyl)-2-(1-phenylcyclopropyl)-3,5,6,7,8 , 9-hexahydro-4H-pyrimido[5,4-c]azepin-4-one;
   6-(2-(3'-(tert-butyl)-[1,1'-biphenyl]-3-yl)-2,2-difluoroacetyl)-2-( 1-phenylcyclopropyl)-3,5,6,7,8,9-hexahydro-4H-pyrimido[5,4-c]azepin -4-one;
   6-(2-(3'-chloro-[1,1'-biphenyl]-3-yl)acetyl)-2-(1-(3-chlorophenyl)c yclopropyl)-3,5,6,7,8,9-hexahydro-4H-pyrimido[5,4-c]azepin-4-one; 6-(2-(3'-chloro-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
   6-(2-(3'-chloro-[1,1'-biphenyl]-3-yl)acetyl)-2-(1-phenylcyclopropyl )-3,5,6,7,8,9-hexahydro-4H-pyrimido[5,4-c]azepin-4-one; 6-(2-(3-chloro-2-fluorophenyl)-2-hydroxyacetyl)-2-(1-(4-isopropylth iophen-2-yl)cyclopropyl)-3,5,6,7,8,9-hexahydro-4H-pyrimido[5,4-c]az epin-4-one;
   6-(2-(3'-chloro-5'-fluoro-[1,1'-biphenyl]-3-yl)acetyl)-2-(1-(3-chlo rophenyl)cyclopropyl)-3,5,6,7,8,9-hexahydro-4H-pyrimido[5,4-c]azepi n-4-one;
   6-(2-(3'-cyclopropyl-[1,1'-biphenyl]-3-yl)-2,2-difluoroacetyl)-2-(1 -phenylcyclopropyl)-3,5,6,7,8,9-hexahydro-4H-pyrimido[5,4-c]azepin-4-one;
   6-(2-(4'-chloro-[1,1'-biphenyl]-3-yl)-2-hydroxyacetyl)-2-(1-phenylc yclopropyl)-5,6,7,8-tetrahydropyrido[4,3-d]pyrimidin-4(3H)-one; 6-(2,2-difluoro-2-(3'-(trifluoromethoxy)-[1,1'-biphenyl]-3-yl)acety 1)-2-(1-phenylcyclopropyl)-3,5,6,7,8,9-hexahydro-4H-pyrimido[5,4-c] azepin-4-one;
   6-(2,2-difluoro-2-(3'-(trifluoromethyl)-[1,1'-biphenyl]-3-yl)acetyl )-2-(1-phenylcyclopropyl)-3,5,6,7,8,9-hexahydro-4H-pyrimido[5,4-c]a zepin-4-one;
   6-(2,2-difluoro-2-(3-(trifluoromethyl)phenyl)acetyl)-2-(1-(5-phenyl pyridin-3-yl)cyclopropyl)-3,5,6,7,8,9-hexahydro-4H-pyrimido[5,4-c]a zepin-4-one;
   6-(2,2-difluoro-2-(3'-isopropoxy-[1,1'-biphenyl]-3-yl)acetyl)-2-(1-phenylcyclopropyl)-3,5,6,7,8,9-hexahydro-4H-pyrimido[5,4-c]azepin-4 -one; and
   6-(2-hydroxy-2-(3'-(trifluoromethoxy)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one, and the like. The compounds described in the above literatures mean all the compounds recited in each claim of the above document. In addition, all the descriptions of the above literatures are incorporated into the description of the present specification.

Preferably, the compound of the present invention is a compound represented by formula (1), (2), (3), or (4) below, or a pharmaceutically acceptable salt thereof. The compound of the invention includes a solvate, a complex, a polymorph, an isomer, and an isotopically-labeled compound thereof described below.

That is, the present invention includes a compound represented by the following formula (1), (2), (3), or (4) or a pharmaceutically acceptable salt thereof (hereinafter it may be referred to as "the compound(s) of the present invention") for use in preventing or treating ocular diseases in animals including humans.
[5-a] Compounds disclosed in US63017891 (application number) or PCT/JP2021/17277 (application number), i.e., a compound represented by the following formula (1).
"A compound of formula (1)": 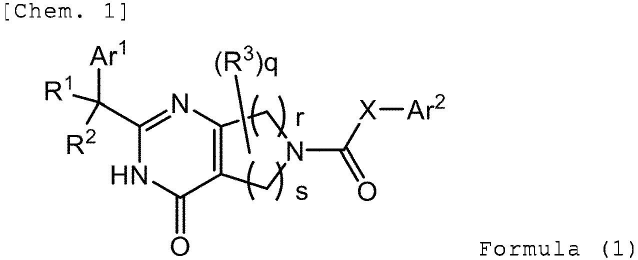 wherein
R¹ and R² are independently selected from the group consisting of: hydrogen, hydroxyl, halogen, (C₁-C₆) alkyl, (C₁-C₆) haloalkyl, (C₁-C₆) alkoxy, (C₁-C₆) haloalkoxy, (C₃-C₇) cycloalkyl, and phenyl(C₀-C₄)alkyl; wherein the said (C₃-C₇)cycloalkyl or phenyl(C₀-C₄)alkyl is optionally substituted with 1 to 4 substituents independently selected from the group consisting of: halogen, hydroxyl, (C₁-C₆) alkyl, (C₁-C₆) haloalkyl, (C₁-C₆) alkoxy, and (C₁-C₆)haloalkoxy; or alternatively R¹ and R², together with the atom to which they are attached, may form a 3 to 8 membered ring which may contain 0 to 4 heteroatoms independently selected from oxygen, sulfur, and nitrogen; wherein the said 3 to 8 membered ring is optionally substituted with 1 to 6 substituents independently selected from the group consisting of: halogen, hydroxyl, (C₁-C₆) alkyl, (C₁-C₆) haloalkyl, -SO₂(C₁-C₆) alkyl, -SO₂(C₁-C₆) haloalkyl, - C(=O)(C₁-C₆) alkyl, and -C(=O)(C₁-C₆) haloalkyl; R³ is independently selected from the group consisting of: hydrogen, fluoride, methyl, ethyl, and (C₁-C₆) haloalkyl;
X is selected from the group consisting of: a chemical bond, -N(R⁴)-, -(C(R⁵)(R⁶))ₙ-, -(C₃-C₆)cycloalkyl-, -C(R⁵)=C(R⁶)-, -C(=O) -, -CR⁴(N(R⁵)(R⁶))-, -[(C(R⁵)(R⁶))ₙO]-, -[(C(R⁵)(R⁶))ₙN(R⁴)]-, - [(C(R⁵)(R⁶))ₙS]-, and -[N(R⁴)(C(R⁵)(R⁶))ₙ]-;
R⁴ is hydrogen or (C₁-C₆) alkyl;
R⁵ and R⁶ are independently selected from the group consisting of: hydrogen, halogen, hydroxyl, (C₁-C₆)alkyl, (C₁-C₆) alkoxy, hydroxyl (C₁-C₆) alkyl, (C₁-C₆) alkoxy (C₁-C₆) alkyl, (C₁-C₆)haloalkyl, and (C₁-C₆)haloalkoxy;
   when R⁵ is two or more than two, R⁵ is same or different;
   when R⁶ is two or more than two, R⁶ is same or different;
n is 1, 2, 3, or 4;
q is 1, 2, 3, or 4;
r is 1, 2, 3, or 4;
s is 1, 2, 3, or 4;
Ar¹ is selected from aryl and heteroaryl which are optionally substituted with 1 to 6 substituents independently selected from the group consisting of: hydrogen, halogen, cyano, nitro, hydroxyl, -C(=O)R⁷, -C(=O)NR⁷R⁸, -NHSO₂R⁷, -SO₂NR⁷R⁸, (C₁-C₆) alkylthio-, (C₁-C₆)haloalkylthio-, (C₁-C₆)alkylsulfinyl, (C₁-C₆)alkylsulfonyl, -NR⁷R⁸, tri (C₁-C₆)alkylsilyl, (C₁-C₆)alkyl, (C₃-C₇)cycloalkyl, (C₃-C₇) cycloalkoxy, (C₁-C₆)alkoxy, (C₁-C₆)alkenyl, aryl (C₀-C₄)alkyl, heteroaryl (C₀-C₄)alkyl, aryl (C₀-C₆)alkoxy, heterocyclyl(C₀-C₄)alkyl, heterocyclyl(C₀-C₆)alkoxy, heteroaryl(C₀-C₆)alkoxy, and substituent group Q;
wherein the said (C₁-C₆)alkyl, (C₃-C₇)cycloalkyl, (C₃-C₇)cycloalkoxy, (C₁-C₆)alkoxy, (C₁-C₆)alkenyl, aryl (C₀-C₄)alkyl, heteroaryl(Co-C₄)alkyl, aryl(C₀-C₆)alkoxy, heterocyclyl(C₀-C₄)alkyl, heterocyclyl(C₀-C₆)alkoxy, or heteroaryl(C₀-C₆)alkoxy is optionally substituted with 1 to 6 substituents independently selected from the group consisting of: halogen, hydroxyl, cyano, (C₁-C₆)alkyl, (C₃-C₇) cycloalkyl, (C₁-C₆)haloalkyl, (C₁-C₆)alkoxy, (C₁-C₆)alkoxy (C₁-C₆)alkyl, (C₁-C₆) alkoxy (C₁-C₆) alkoxy, (C₁-C₆) haloalkoxy, phenyl, - NR^{a}R^{b}, R^{a}R^{b}N (C₁-C₆) alkyl, R^{a}R^{b}N (C₁-C₆)alkoxy, -C(=O)NR^{a}R^{b}, -C(=O)R^{a}, - SO₂(C₁-C₆)alkyl, -SO₂NR^{a}R^{b}, and R^{a}R^{b}NC(=O)(C₁-C₆) alkoxy; wherein the said (C₃-C₇)cycloalkyl is optionally substituted with hydroxyl or cyano;
wherein the substituent group Q is 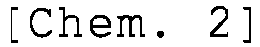 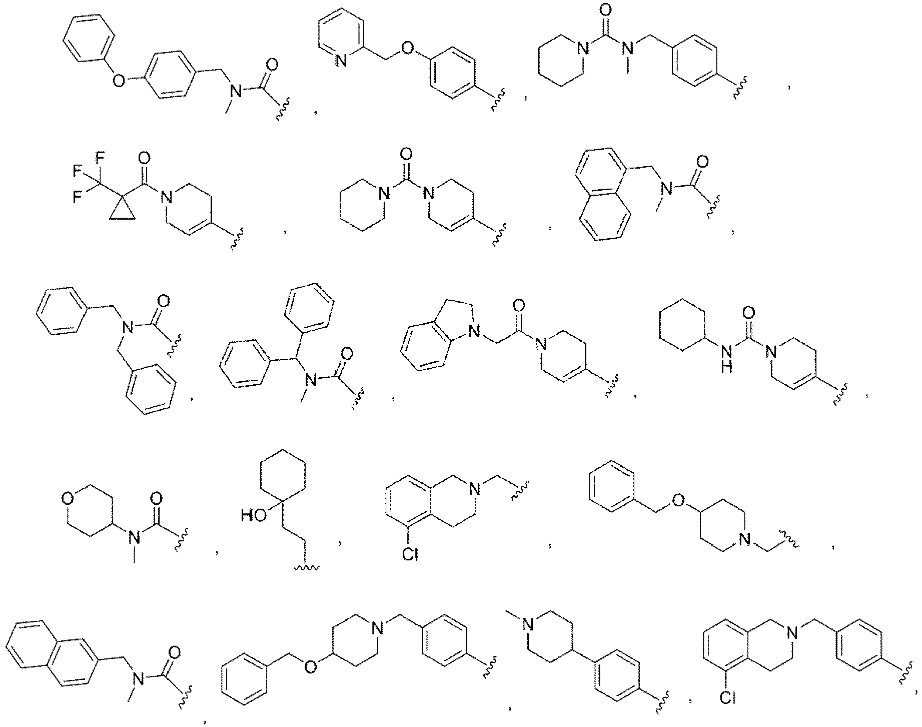 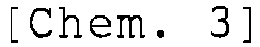 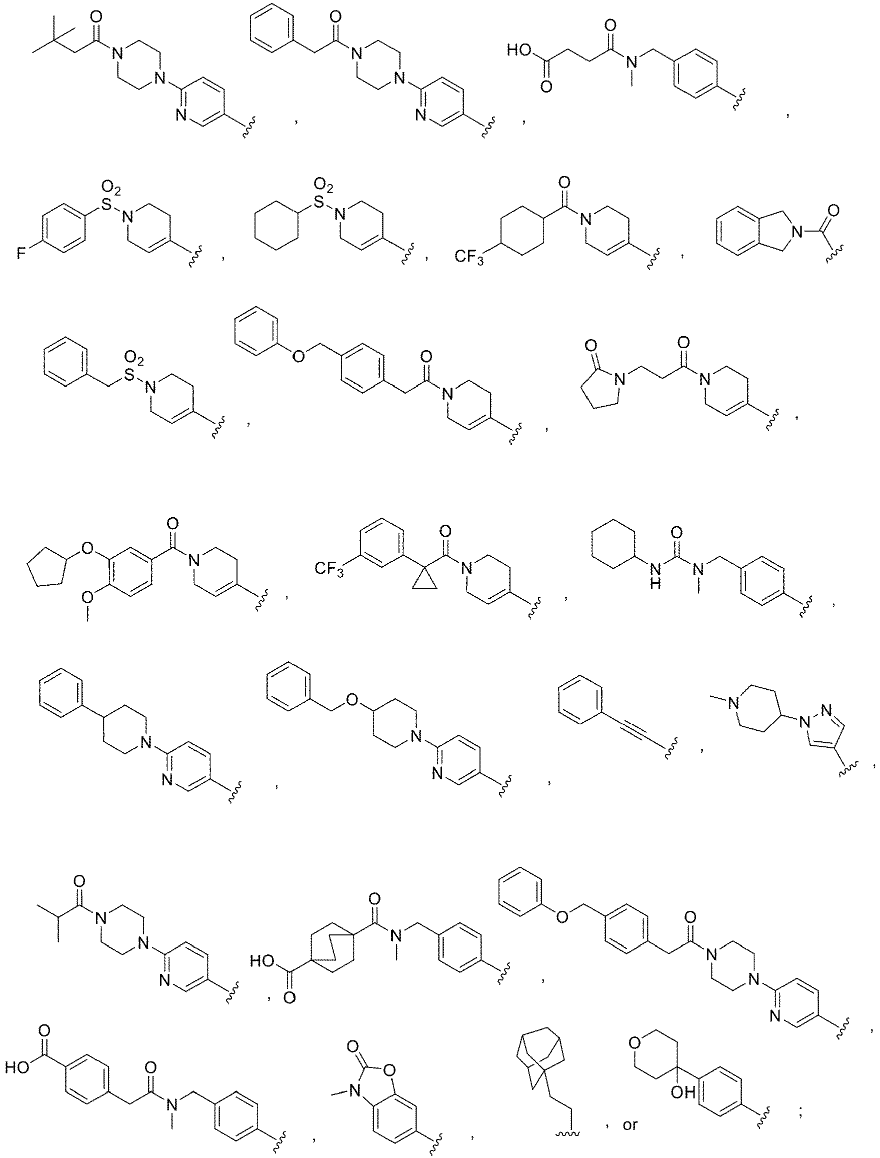 in which the substituent group Q may be optionally substituted with halogen, hydroxyl, or (C₁-C₆)alkyl;
R⁷ and R⁸ are independently selected from the group consisting of: hydrogen, hydroxyl, (C₁-C₆)alkyl, (C₁-C₆)alkoxy, (C₁-C₆) haloalkyl, (C₁-C₆)haloalkoxy, (C₃-C₇) cycloalkyl, heterocyclyl, hydroxy (C₁-C₆)alkyl, (C₁-C₆)alkoxy (C₁-C₆)alkyl, (C₁-C₆)haloalkoxy (C₁-C₆) alkyl, benzyl, H₂N (C₁-C₆)alkyl, (C₁-C₆)alkylNH (C₁-C₆)alkyl, and [(C₁-C₆)alkyl]₂N(C₁-C₆)alkyl; or R⁷ and R⁸, together with nitrogen atom to which they are attached, may form a 3 to 10 membered ring which may contain a heteroatom selected from oxygen, sulfur, and nitrogen; wherein the said 3 to 10 membered ring is optionally substituted with 1 to 6 substituents independently selected from the group consisting of: halogen, hydroxyl, oxo, (C₁-C₆)alkyl, (C₁-C₆)alkoxy, (C₁-C₆)haloalkyl, -SO₂(C₁-C₆) alkyl, -SO₂(C₁-C₆)haloalkyl, - C(=O)(C₁-C₆)alkyl, and -C(=O) (C₁-C₆)haloalkyl;
R^{a} and R^{b} are independently selected from the group consisting of: hydrogen, (C₁-C₆)alkyl, (C₁-C₆)haloalkyl, hydroxy(C₁-C₆) alkyl, (C₁-C₆) alkoxy (C₁-C₆) alkyl, (C₃-C₇) cycloalkyl, phenyl(C₀-C₆)alkyl, (C₁-C₆)haloalkoxy(C₁-C₆)alkyl, H₂N(C₁-C₆)alkyl, (C₁-C₆)alkylNH(C₁-C₆)alkyl, and [(C₁-C₆)alkyl]₂N(C₁-C₆)alkyl; or R^{a} and R^{b}, together with nitrogen atom to which they are attached, may form a 3 to 7 membered ring which may contain a heteroatom selected from oxygen, sulfur, and nitrogen; wherein the said 3 to 7 membered ring is optionally substituted with 1 to 3 substituents independently selected from (C₁-C₆) alkyl;
Ar² is selected from aryl and heteroaryl which are optionally substituted with 1 to 6 substituents independently selected from the group consisting of: hydrogen, halogen, cyano, nitro, hydroxyl, -COR⁹, -CONR⁹R¹⁰, -NHSO₂R⁹, -SO₂NR⁹R¹⁰, (C₁-C₆) alkylthio-, (C₁-C₆) alkylsulfinyl, (C₁-C₆) alkylsulfonyl, -NR⁹R¹⁰, tri (C₁-C₆) alkylsilyl, (C₁-C₆)haloalkylthio-, -SF₅, (C₁-C₆) alkyl, (C₃-C₇)cycloalkyl, (C₃-C₇) cycloalkoxy, (C₁-C₆) alkoxy, aryl (C₀-C₄)alkyl, heteroaryl (C₀-C₄) alkyl, aryl(C₀-C₆)alkoxy, phenoxy, heteroaryl (C₀-C₆) alkoxy, heterocyclyl(C₀-C₄)alkyl, and heterocyclyl (C₀-C₆)alkoxy; wherein the said (C₁-C₆)alkyl, (C₃-C₇) cycloalkyl, (C₃-C₇) cycloalkoxy, (C₁-C₆) alkoxy, aryl (C₀-C₄)alkyl, heteroaryl (Cₒ-C₄)alkyl, aryl (C₀-C₆) alkoxy, phenoxy, heteroaryl (C₀-C₆)alkoxy, heterocyclyl (C₀-C₄)alkyl, or heterocyclyl(C₀-C₆)alkoxy is optionally substituted with 1 to 6 substituents independently selected from the group consisting of: halogen, hydroxyl, (C₁-C₆) alkyl, (C₃-C₇) cycloalkyl (C₁-C₆) haloalkyl, (C₁-C₆) alkoxy, (C₁-C₆)alkoxy (C₁-C₆) alkoxy, (C₁-C₆)haloalkoxy, -NR^{c}R^{d}, R^{c}R^{d}N(C₁-C₆) alkyl, R^{c}R^{d}N(C₁-C₆) alkoxy, - C(=O)NR^{c}R^{d}, R^{c}R^{d}NC(=O)(C₁-C₆) alkoxy, benzyloxy, and cyano;
R⁹ and R¹⁰ are independently selected from the group consisting of: hydrogen, (C₁-C₆)alkyl, (C₁-C₆)haloalkyl, hydroxy(C₁-C₆)alkyl, (C₁-C₆)alkoxy (C₁-C₆)alkyl, (C₁-C₆)haloalkoxy (C₁-C₆)alkyl, H₂N(C₁-C₆)alkyl, (C₁-C₆)alkylNH(C₁-C₆)alkyl, and [(C₁-C₆)alkyl]₂N(C₁-C₆)alkyl; or R⁹ and R¹⁰, together with nitrogen atom to which they are attached, may form a 3 to 10 membered ring which may contain a heteroatom selected from oxygen, sulfur, and nitrogen; wherein the said 3 to 10 membered ring is optionally substituted with 1 to 6 substituents independently selected from the group consisting of: halogen, hydroxyl, oxo, (C₁-C₆) alkyl, (C₁-C₆) alkoxy, (C₁-C₆)haloalkyl, -SO₂(C₁-C₆)alkyl, -SO₂(C₁-C₆)haloalkyl, -C(=O)(C₁-C₆)alkyl, and -C(=O)(C₁-C₆)haloalkyl; and R^{c} and R^{d} are independently selected from the group consisting of: hydrogen, (C₁-C₆)alkyl, (C₁-C₆)haloalkyl, hydroxy(C₁-C₆)alkyl, (C₁-C₆)alkoxy(C₁-C₆)alkyl, (C₁-C₆)haloalkoxy(C₁-C₆)alkyl, H₂N(C₁-C₆)alkyl, (C₁-C₆)alkylNH (C₁-C₆) alkyl, and [(C₁-C₆)alkyl]₂N(C₁-C₆)alkyl; or R^{c} and R^{d}, together with nitrogen atom to which they are attached, may form a 3 to 7 membered ring which may contain a heteroatom selected from oxygen, sulfur, and nitrogen; or a pharmaceutically acceptable salt thereof.

[5-b] A compound of the following formula (1):
"A compound of formula (1)" 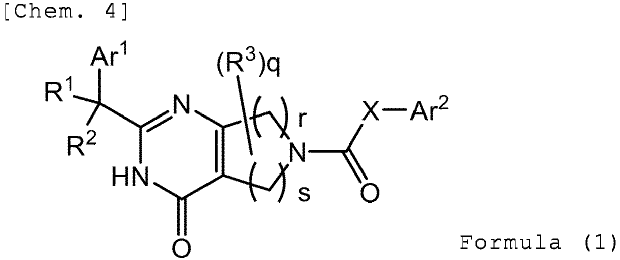 wherein
R¹ and R² are independently selected from the group consisting of: hydrogen, hydroxyl, halogen, (C₁-C₆)alkyl, (C₁-C₆) haloalkyl, (C₁-C₆)alkoxy, (C₁-C₆)haloalkoxy, (C₃-C₇) cycloalkyl, and phenyl(C₀-C₄)alkyl; wherein the said (C₃-C₇)cycloalkyl or phenyl(C₀-C₄)alkyl is optionally substituted with 1 to 4 substituents independently selected from halogen, hydroxyl, (C₁-C₆) alkyl, (C₁-C₆) haloalkyl, (C₁-C₆) alkoxy and (C₁-C₆)haloalkoxy; or alternatively R¹ and R², together with the atom to which they are attached, may form a 3 to 8 membered ring which may contain 0 to 4 heteroatoms independently selected from oxygen, sulfur, and nitrogen; wherein the said 3 to 8 membered ring is optionally substituted with 1 to 6 substituents independently selected from halogen, hydroxyl, (C₁-C₆)alkyl, (C₁-C₆)haloalkyl, -SO₂(C₁-C₆) alkyl, -SO₂(C₁-C₆)haloalkyl, -C(=O)( C₁-C₆)alkyl, and -C(=O) (C₁-C₆)haloalkyl;
R³ is independently selected from the group consisting of: hydrogen, fluoride, methyl, ethyl, and (C₁-C₆)haloalkyl;
X is selected from the group consisting of: a chemical bond, -N(R⁴)-, -(C(R⁵)(R⁶))ₙ-, -(C₃-C₈)cycloalkyl-, -C(R⁵)=C(R⁶)-, - [(C(R⁵)(R⁶))ₙO]-, -[(C(R⁵)(R⁶))ₙN(R⁴)]-, -[(C(R⁵)(R⁶))ₙS]-, and - [N(R⁴)(C(R⁵)(R⁶))ₙ]-;
R⁴ is hydrogen or (C₁-C₆) alkyl;
R⁵ and R⁶ are independently selected from the group consisting of: hydrogen, halogen, hydroxyl, (C₁-C₆)alkyl, (C₁-C₆) alkoxy, hydroxyl (C₁-C₆) alkyl, (C₁-C₆) alkoxy (C₁-C₆) alkyl, (C₁-C₆) haloalkyl, and (C₁-C₆)haloalkoxy;
n is 1, 2, 3, or 4;
q is 1, 2, 3, or 4;
r is 1, 2, 3, or 4;
s is 1, 2, 3, or 4;
Ar¹ is selected from aryl and heteroaryl which are optionally substituted with 1 to 6 substituents independently selected from hydrogen, halogen, cyano, nitro, hydroxyl, -COR⁷, - CONR⁷R⁸, -NHSO₂R⁷, -SO₂NR⁷R⁸, (C₁-C₆)alkylthio, (C₁-C₆)alkylsulfinyl, (C₁-C₆)alkylsulfonyl, -NR⁷R⁸, tri (C₁-C₆) alkylsilyl, (C₁-C₆) alkyl, (C₃-C₇)cycloalkyl, (C₃-C₇) cycloalkoxy, (C₁-C₆) alkoxy, aryl (Cₒ-C₄)alkyl, heteroaryl(C₀-C₄)alkyl, aryl(C₀-C₆)alkoxy, heterocyclyl(C₀-C₄)alkyl, heterocyclyl(C₀-C₆)alkoxy, and heteroaryl(C₀-C₆)alkoxy;
wherein the said (C₁-C₆)alkyl, (C₃-C₇)cycloalkyl, (C₃-C₇)cycloalkoxy, (C₁-C₆) alkoxy, aryl (Cₒ-C₄)alkyl, heteroaryl (C₀-C₄)alkyl, aryl (C₀-C₆)alkoxy, heterocyclyl (Cₒ-C₄)alkyl, heterocyclyl(C₀-C₆)alkoxy, or heteroaryl(C₀-C₆)alkoxy is optionally substituted with 1 to 6 substituents independently selected from halogen, hydroxyl, (C₁-C₆)alkyl, (C₃-C₇) cycloalkyl (C₁-C₆)haloalkyl, (C₁-C₆) alkoxy, (C₁-C₆) haloalkoxy, R^{a}R^{b}N-, R^{a}R^{b}N (C₁-C₆) alkyl, R^{a}R^{b}N (C₁-C₆)alkoxy, -C(=O)NR^{a}R^{b}, and R^{a}R^{b}NC(=O)(C₁-C₆) alkoxy;
R⁷ and R⁸ are independently selected from hydrogen, (C₁-C₆)alkyl, (C₁-C₆)haloalkyl, hydroxy(C₁-C₆)alkyl, (C₁-C₆)alkoxy(C₁-C₆)alkyl, (C₁-C₆) haloalkoxy (C₁-C₆) alkyl, H₂N(C₁-C₆) alkyl, (C₁-C₆)alkylNH(C₁-C₆)alkyl, and [(C₁-C₆)alkyl]₂N(C₁-C₆)alkyl; or R⁷ and R⁸, together with nitrogen atom to which they are attached, may form a 3 to 10 membered ring which may contain a heteroatom selected from oxygen, sulfur, and nitrogen; wherein the said 3 to 10 membered ring is optionally substituted with 1 to 6 substituents independently selected from halogen, hydroxyl, oxo, (C₁-C₆)alkyl, (C₁-C₆) alkoxy, (C₁-C₆) haloalkyl, -SO₂(C₁-C₆)alkyl, -SO₂(C₁-C₆)haloalkyl, -C(=O)( C₁-C₆)alkyl, and -C(=O) (C₁-C₆)haloalkyl;
R^{a} and R^{b} are independently selected from hydrogen, (C₁-C₆)alkyl, (C₁-C₆)haloalkyl, hydroxy(C₁-C₆)alkyl, (C₁-C₆)alkoxy(C₁-C₆)alkyl, (C₁-C₆) haloalkoxy (C₁-C₆) alkyl, H₂N(C₁-C₆) alkyl, (C₁-C₆)alkylNH(C₁-C₆)alkyl, and [(C₁-C₆)alkyl]₂N(C₁-C₆)alkyl; or R^{a} and R^{b}, together with nitrogen atom to which they are attached, may form a 3 to 7 membered ring which may contain a heteroatom selected from oxygen, sulfur, and nitrogen;
Ar² is selected from aryl and heteroaryl which are optionally substituted with 1 to 6 substituents independently selected from hydrogen, halogen, cyano, nitro, hydroxyl, -COR⁹, - CONR⁹R¹⁰, -NHSO₂R⁹, -SO₂NR⁹R¹⁰, (C₁-C₆)alkylthio, (C₁-C₆)alkylsulfinyl, (C₁-C₆) alkylsulfonyl, -NR⁹R¹⁰, tri (C₁-C₆) alkylsilyl, (C₁-C₆) alkyl, (C₃-C₇)cycloalkyl, (C₃-C₇) cycloalkoxy, (C₁-C₆) alkoxy, aryl (C₀-C₄)alkyl, heteroaryl (C₀-C₄)alkyl, aryl (C₀-C₆)alkoxy, heteroaryl (C₀-C₆)alkoxy, heterocyclyl(C₀-C₄)alkyl, and heterocyclyl(C₀-C₆)alkoxy; wherein the said (C₁-C₆) alkyl, (C₃-C₇) cycloalkyl, (C₁-C₆) alkoxy, aryl(C₀-C₄)alkyl, heteroaryl (C₀-C₄)alkyl, aryl (C₀-C₆)alkoxy, heteroaryl (C₀-C₆)alkoxy, heterocyclyl(C₀-C₄)alkyl, or heterocyclyl(C₀-C₆)alkoxy is optionally substituted with 1 to 6 substituents independently selected from halogen, hydroxyl, (C₁-C₆)alkyl, (C₃-C₇) cycloalkyl (C₁-C₆)haloalkyl, (C₁-C₆) alkoxy, (C₁-C₆)haloalkoxy, R^{c}R^{d}N-, R^{c}R^{d}N(C₁-C₆) alkyl, R^{c}R^{d}N(C₁-C₆) alkoxy, -C(=O)NR^{c}R^{d}, R^{c}R^{d}NC(=O)(C₁-C₆)alkoxy, and cyano;
R⁹ and R¹⁰ are independently selected from hydrogen, (C₁-C₆)alkyl, (C₁-C₆) haloalkyl, hydroxy (C₁-C₆) alkyl, (C₁-C₆) alkoxy (C₁-C₆)alkyl, (C₁-C₆) haloalkoxy (C₁-C₆) alkyl, H₂N(C₁-C₆)alkyl, (C₁-C₆)alkylNH (C₁-C₆)alkyl, and [(C₁-C₆)alkyl]₂N(C₁-C₆)alkyl; or R⁹ and R¹⁰, together with nitrogen atom to which they are attached, may form a 3 to 10 membered ring which may contain a heteroatom selected from oxygen, sulfur, and nitrogen;
wherein the said 3 to 10 membered ring is optionally substituted with 1 to 6 substituents independently selected from halogen, hydroxyl, oxo, (C₁-C₆)alkyl, (C₁-C₆)alkoxy, (C₁-C₆)haloalkyl, -SO₂(C₁-C₆)alkyl, -SO₂(C₁-C₆)haloalkyl, -C(=O)( C₁-C₆)alkyl, and -C(=O) (C₁-Cₛ)haloalkyl; and
R^{c} and R^{d} are independently selected from hydrogen, (C₁-C₆)alkyl, (C₁-C₆)haloalkyl, hydroxy(C₁-C₆)alkyl, (C₁-C₆)alkoxy(C₁-C₆)alkyl, (C₁-C₆)haloalkoxy(C₁-C₆)alkyl, H₂N(C₁-C₆)alkyl, (C₁-C₆) alkylNH (C₁-C₆) alkyl, and [(C₁-C₆)alkyl]₂N(C₁-C₆)alkyl; or
R^{c} and R^{d}, together with nitrogen atom to which they are attached, may form a 3 to 7 membered ring which may contain a heteroatom selected from oxygen, sulfur, and nitrogen; or a pharmaceutically acceptable salt thereof or a prodrug thereof.

[5-c] In the compound of formula (1), more preferably the compound described in the above "compound of formula (1)", which is selected from the following:
(R)-2-(1-phenylcyclopropyl)-6-(2-phenylpropanoyl)-5,6,7,8-tetrahydropyrido[4,3-d]pyrimidin-4(3H)-one;
(S)-2-(1-phenylcyclopropyl)-6-(2-phenylpropanoyl)-5,6,7,8-tetrahydropyrido[4,3-d]pyrimidin-4(3H)-one;
(R)-6-(2-(4-isobutylphenyl)propanoyl)-2-(1-phenylcyclopropyl)-5,6,7,8-tetrahydropyrido[4,3-d]pyrimidin-4(3H)-one;
(S)-6-(2-(4-isobutylphenyl)propanoyl)-2-(1-phenylcyclopropyl)-5,6,7,8-tetrahydropyrido[4,3-d]pyrimidin-4(3H)-one;
(R)-2-(1-(4-fluorophenyl)cyclopropyl)-6-(2-(4-isobutylphenyl)propanoyl)-5,6,7,8-tetrahydropyrido[4,3-d]pyrimidin-4(3H)-one;
(R)-2-(1-(4-chlorophenyl)cyclopropyl)-6-(2-(4-isobutylphenyl)propanoyl)-5,6,7,8-tetrahydropyrido[4,3-d]pyrimidin-4(3H)-one;
6-(2-(3,5-difluorophenyl)propanoyl)-2-(1-phenylcyclopropyl)-5,6,7,8-tetrahydropyrido[4,3-d]pyrimidin-4(3H)-one;
6-(2-(4-chlorophenyl)propanoyl)-2-(1-phenylcyclopropyl)-5,6,7,8-tetrahydropyrido[4,3-d]pyrimidin-4(3H)-one;
6-(2-(3,5-difluorophenyl)acetyl)-2-(1-phenylcyclopropyl)-5,6,7,8-tetrahydropyrido[4,3-d]pyrimidin-4(3H)-one;
2-(1-phenylcyclopropyl)-6-(2-(4-(trifluoromethyl)phenyl)propanoyl)-5,6,7,8-tetrahydropyrido[4,3-d]pyrimidin-4(3H)-one;
6-(2-(4-fluorophenyl)propanoyl)-2-(1-phenylcyclopropyl)-5,6,7,8-tetrahydropyrido[4,3-d]pyrimidin-4(3H)-one;
6-(2-(4-isopropylphenyl)acetyl)-2-(1-phenylcyclopropyl)-5,6,7,8-tetrahydropyrido[4,3-d]pyrimidin-4(3H)-one;
6-(2-(3,5-difluorophenyl)propanoyl)-2-(1-(4-fluorophenyl)cyclopropyl)-5,6,7,8-tetrahydropyrido[4,3-d]pyrimidin-4(3H)-one;
6-(2-(4-(tert-butyl)phenoxy)acetyl)-2-(1-phenylcyclopropyl)-5,6,7,8-tetrahydropyrido[4,3-d]pyrimidin-4(3H)-one;
6-(2-(1H-indol-3-yl)acetyl)-2-(1-phenylcyclopropyl)-5,6,7,8-tetrahydropyrido[4,3-d]pyrimidin-4(3H)-one;
6-(2-(5-fluoro-1H-indol-3-yl)acetyl)-2-(1-phenylcyclopropyl)-5,6,7,8-tetrahydropyrido[4,3-d]pyrimidin-4(3H)-one;
6-(2-(benzo[d]isoxazol-3-yl)acetyl)-2-(1-phenylcyclopropyl)-5,6,7,8-tetrahydropyrido[4,3-d]pyrimidin-4(3H)-one;
6-(2-(5-methyl-2-phenyloxazol-4-yl)acetyl)-2-(1-phenylcyclopropyl)-5,6,7,8-tetrahydropyrido[4,3-d]pyrimidin-4(3H)-one;
6-(3-(3-phenyl-1,2,4-oxadiazol-5-yl)propanoyl)-2-(1-phenylcyclopropyl)-5,6,7,8-tetrahydropyrido[4,3-d]pyrimidin-4(3H)-one;
2-(1-phenylcyclopropyl)-6-(2-(quinoxalin-6-yl)acetyl)-5,6,7,8-tetrahydropyrido[4,3-d]pyrimidin-4(3H)-one;
6-(2,2-difluoro-2-phenylacetyl)-2-(1-phenylcyclopropyl)-5,6,7,8-tetrahydropyrido[4,3-d]pyrimidin-4(3H)-one;
6-(2-fluoro-2-phenylacetyl)-2-(1-phenylcyclopropyl)-5,6,7,8-tetrahydropyrido[4,3-d]pyrimidin-4(3H)-one;
6-(2-(5-chloro-1H-benzo[d]imidazol-2-yl)acetyl)-2-(1-phenylcyclopropyl)-5,6,7,8-tetrahydropyrido[4,3-d]pyrimidin-4(3H)-one;
6-(2-(2-hydroxyphenyl)acetyl)-2-(1-phenylcyclopropyl)-5,6,7,8-tetrahydropyrido[4,3-d]pyrimidin-4(3H)-one;
6-(2-(2-methoxyphenyl)acetyl)-2-(1-phenylcyclopropyl)-5,6,7,8-tetrahydropyrido[4,3-d]pyrimidin-4(3H)-one;
6-(3-(1H-indol-3-yl)propanoyl)-2-(1-phenylcyclopropyl)-5,6,7,8-tetrahydropyrido[4,3-d]pyrimidin-4(3H)-one;
6-(3-(3-(4-methoxyphenyl)-1,2,4-oxadiazol-5-yl)propanoyl)-2-(1-phenylcyclopropyl)-5,6,7,8-tetrahydropyrido[4,3-d]pyrimidin-4(3H)-one;
6-(2-(1H-indazol-1-yl)acetyl)-2-(1-phenylcyclopropyl)-5,6,7,8-tetrahydropyrido[4,3-d]pyrimidin-4(3H)-one;
4-(2-oxo-2-(4-oxo-2-(1-phenylcyclopropyl)-3,5,7,8-tetrahydropyrido[4,3-d]pyrimidin-6(4H)-yl)ethyl)-2H-benzo[b][1,4]oxazin-3(4H)-one;
(E)-6-(3-(3-chlorophenyl)acryloyl)-2-(1-phenylcyclopropyl)-5,6,7,8-tetrahydropyrido[4,3-d]pyrimidin-4(3H)-one;
6-((1R*,2R*)-2-phenylcyclopropane-1-carbonyl)-2-(1-phenylcyclopropyl)-5,6,7,8-tetrahydropyrido[4,3-d]pyrimidin-4(3H)-one;
6-((1R*,2R*)-2-(2,5-difluorophenyl)cyclopropane-1-carbonyl)-2-(1-phenylcyclopropyl)-5,6,7,8-tetrahydropyrido[4,3-d]pyrimidin-4(3H)-one;
(R)-6-(2-hydroxy-2-phenylacetyl)-2-(1-phenylcyclopropyl)-5,6,7,8-tetrahydropyrido[4,3-d]pyrimidin-4(3H)-one;
(R)-6-(2-(3-chlorophenyl)-2-hydroxyacetyl)-2-(1-phenylcyclopropyl)-5,6,7,8-tetrahydropyrido[4,3-d]pyrimidin-4(3H)-one;
(R)-(-)-6-(2-(2,3-difluorophenyl)-2-hydroxyacetyl)-2-(1-phenylcyclopropyl)-5,6,7,8-tetrahydropyrido[4,3-d]pyrimidin-4(3H)-one;
(S)-(+)-6-(2-(2,3-difluorophenyl)-2-hydroxyacetyl)-2-(1-phenylcyclopropyl)-5,6,7,8-tetrahydropyrido[4,3-d]pyrimidin-4(3H)-one;
(R)-(-)-6-(2-(2,3-difluorophenyl)-2-hydroxyacetyl)-2-(1-(4-fluorophenyl)cyclopropyl)-5,6,7,8-tetrahydropyrido[4,3-d]pyrimidin-4(3H)-one;
(S)-(+)-6-(2-(2,3-difluorophenyl)-2-hydroxyacetyl)-2-(1-(4-fluorophenyl)cyclopropyl)-5,6,7,8-tetrahydropyrido[4,3-d]pyrimidin-4(3H)-one;
(R)-(-)-2-(1-(4-chlorophenyl)cyclopropyl)-6-(2-(2,3-difluorophenyl)-2-hydroxyacetyl)-5,6,7,8-tetrahydropyrido[4,3-d]pyrimidin-4(3H)-one;
(R)-(-)-6-(2-(2,3-difluorophenyl)-2-hydroxyacetyl)-2-(1-(3-fluorophenyl)cyclopropyl)-5,6,7,8-tetrahydropyrido[4,3-d]pyrimidin-4(3H)-one;
6-(2-(3,5-difluorophenyl)-2-hydroxyacetyl)-2-(1-(3-fluorophenyl)cyclopropyl)-5,6,7,8-tetrahydropyrido[4,3-d]pyrimidin-4(3H)-one;
(-)-2-(1-(4-fluorophenyl)cyclopropyl)-6-(2-hydroxy-2-(m-tolyl)acetyl)-5,6,7,8-tetrahydropyrido[4,3-d]pyrimidin-4(3H)-one;
(-)-2-(1-(4-chlorophenyl)cyclopropyl)-6-(2-hydroxy-2-(m-tolyl)acetyl)-5,6,7,8-tetrahydropyrido[4,3-d]pyrimidin-4(3H)-one;
(S)-6-(2-hydroxy-2-phenylacetyl)-2-(1-phenylcyclopropyl)-5,6,7,8-tetrahydropyrido[4,3-d]pyrimidin-4(3H)-one;
6-(2-(2-chlorophenyl)-2-hydroxyacetyl)-2-(1-phenylcyclopropyl)-5,6,7,8-tetrahydropyrido[4,3-d]pyrimidin-4(3H)-one;
6-(2-(4-chlorophenyl)-2-hydroxyacetyl)-2-(1-phenylcyclopropyl)-5,6,7,8-tetrahydropyrido[4,3-d]pyrimidin-4(3H)-one;
(S)-6-(2-(2-chlorophenyl)-2-hydroxyacetyl)-2-(1-phenylcyclopropyl)-5,6,7,8-tetrahydropyrido[4,3-d]pyrimidin-4(3H)-one;
(R)-6-(2-(2-chlorophenyl)-2-hydroxyacetyl)-2-(1-phenylcyclopropyl)-5,6,7,8-tetrahydropyrido[4,3-d]pyrimidin-4(3H)-one;
6-(2-(2,3-difluorophenyl)-2-hydroxyacetyl)-2-(1-phenylcyclopropyl)-5,6,7,8-tetrahydropyrido[4,3-d]pyrimidin-4(3H)-one;
6-(2-(3,4-difluorophenyl)-2-hydroxyacetyl)-2-(1-phenylcyclopropyl)-5,6,7,8-tetrahydropyrido[4,3-d]pyrimidin-4(3H)-one;
6-(2-(2,4-difluorophenyl)-2-hydroxyacetyl)-2-(1-phenylcyclopropyl)-5,6,7,8-tetrahydropyrido[4,3-d]pyrimidin-4(3H)-one;
6-(2-(2,6-difluorophenyl)-2-hydroxyacetyl)-2-(1-phenylcyclopropyl)-5,6,7,8-tetrahydropyrido[4,3-d]pyrimidin-4(3H)-one;
(R)-6-(2-hydroxy-3-phenylpropanoyl)-2-(1-phenylcyclopropyl)-5,6,7,8-tetrahydropyrido[4,3-d]pyrimidin-4(3H)-one;
(R)-6-(2-hydroxy-4-phenylbutanoyl)-2-(1-phenylcyclopropyl)-5,6,7,8-tetrahydropyrido[4,3-d]pyrimidin-4(3H)-one;
6-(2-(3-fluoro-4-methoxyphenyl)-2-hydroxyacetyl)-2-(1-phenylcyclopropyl)-5,6,7,8-tetrahydropyrido[4,3-d]pyrimidin-4(3H)-one;
6-(2-(3-chlorophenyl)-2-hydroxyacetyl)-2-(1-phenylcyclopropyl)-5,6,7,8-tetrahydropyrido[4,3-d]pyrimidin-4(3H)-one;
6-(2-hydroxy-2-(6-methylpyridin-2-yl)acetyl)-2-(1-phenylcyclopropyl)-5,6,7,8-tetrahydropyrido[4,3-d]pyrimidin-4(3H)-one;
6-(2-(3-fluorophenyl)-2-hydroxyacetyl)-2-(1-phenylcyclopropyl)-5,6,7,8-tetrahydropyrido[4,3-d]pyrimidin-4(3H)-one;
6-(2-(3-fluorophenyl)-2-hydroxyacetyl)-2-(1-(4-fluorophenyl)cyclopropyl)-5,6,7,8-tetrahydropyrido[4,3-d]pyrimidin-4(3H)-one;
6-(2-(3-fluoro-4-methoxyphenyl)-2-hydroxyacetyl)-2-(1-(4-fluorophenyl)cyclopropyl)-5,6,7,8-tetrahydropyrido[4,3-d]pyrimidin-4(3H)-one;
2-(1-(4-chlorophenyl)cyclopropyl)-6-(2-(2,3-difluorophenyl)-2-hydroxyacetyl)-5,6,7,8-tetrahydropyrido[4,3-d]pyrimidin-4(3H)-one;
(R)-6-(2-(2-chlorophenyl)-2-hydroxyacetyl)-2-(1-(4-chlorophenyl)cyclopropyl)-5,6,7,8-tetrahydropyrido[4,3-d]pyrimidin-4(3H)-one;
2-(1-(4-chlorophenyl)cyclopropyl)-6-(2-(3,5-difluorophenyl)-2-hydroxyacetyl)-5,6,7,8-tetrahydropyrido[4,3-d]pyrimidin-4(3H)-one;
(R)-6-(2-(3-chlorophenyl)-2-hydroxyacetyl)-2-(1-(4-chlorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-fluorophenyl)cyclopropyl)-5,6,7,8-tetrahydropyrido[4,3-d]pyrimidin-4(3H)-one;
6-(2-(2,3-difluorophenyl)-2-hydroxyacetyl)-2-(1-(4-fluorophenyl)cyclopropyl)-5,6,7,8-tetrahydropyrido[4,3-d]pyrimidin-4(3H)-one;
(R)-6-(2-(2-chlorophenyl)-2-hydroxyacetyl)-2-(1-(4-fluorophenyl)cyclopropyl)-5,6,7,8-tetrahydropyrido[4,3-d]pyrimidin-4(3H)-one;
6-(2-(3,5-difluorophenyl)-2-hydroxyacetyl)-2-(1-(4-fluorophenyl)cyclopropyl)-5,6,7,8-tetrahydropyrido[4,3-d]pyrimidin-4(3H)-one;
6-(2-hydroxy-2-(4-(trifluoromethyl)phenyl)acetyl)-2-(1-phenylcyclopropyl)-5,6,7,8-tetrahydropyrido[4,3-d]pyrimidin-4(3H)-one;
6-(2-(2,5-difluorophenyl)-2-hydroxyacetyl)-2-(1-phenylcyclopropyl)-5,6,7,8-tetrahydropyrido[4,3-d]pyrimidin-4(3H)-one;
6-(2-(2,3-difluorophenyl)-2-hydroxyacetyl)-2-(1-(3-fluorophenyl)cyclopropyl)-5,6,7,8-tetrahydropyrido[4,3-d]pyrimidin-4(3H)-one;
6-(2-(3,5-difluorophenyl)-2-hydroxyacetyl)-2-(1-(3-fluorophenyl)cyclopropyl)-5,6,7,8-tetrahydropyrido[4,3-d]pyrimidin-4(3H)-one;
(R)-6-(2-(2-chlorophenyl)-2-hydroxyacetyl)-2-(1-(3-fluorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-fluorophenyl)cyclopropyl)-5,6,7,8-tetrahydropyrido[4,3-d]pyrimidin-4(3H)-one;
6-(2-(2,3-difluorophenyl)-2-hydroxyacetyl)-2-(1-(4-(trifluoromethyl)phenyl)cyclopropyl)-5,6,7,8-tetrahydropyrido[4,3-d]pyrimidin-4(3H)-one;
6-(2-(3,5-difluorophenyl)-2-hydroxyacetyl)-2-(1-(4-(trifluoromethyl)phenyl)cyclopropyl)-5,6,7,8-tetrahydropyrido[4,3-d]pyrimidin-4(3H)-one;
(R)-6-(2-(2-chlorophenyl)-2-hydroxyacetyl)-2-(1-(4-(trifluoromethyl)phenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-(trifluoromethyl)phenyl)cyclopropyl)-5,6,7,8-tetrahydropyrido[4,3-d]pyrimidin-4(3H)-one;
(R)-6-(2-hydroxy-2-(naphthalen-2-yl)acetyl)-2-(1-phenylcyclopropyl)-5,6,7,8-tetrahydropyrido[4,3-d]pyrimidin-4(3H)-one;
6-(2-hydroxy-2-(3-methoxyphenyl)acetyl)-2-(1-phenylcyclopropyl)-5,6,7,8-tetrahydropyrido[4,3-d]pyrimidin-4(3H)-one;
6-(2-hydroxy-2-(m-tolyl)acetyl)-2-(1-phenylcyclopropyl)-5,6,7,8-tetrahydropyrido[4,3-d]pyrimidin-4(3H)-one;
(R)-4-(1-(6-(2-(3-chlorophenyl)-2-hydroxyacetyl)-4-oxo-3,4,5,6,7,8-hexahydropyrido[4,3-d]pyrimidin-2-yl)cyclopropyl)benzonitrile;
4-(1-(6-(2-(2,3-difluorophenyl)-2-hydroxyacetyl)-4-oxo-3,4,5,6,7,8-hexahydropyrido[4,3-d]pyrimidin-2-yl)cyclopropyl)benzonitrile;
(S)-2-(1-(4-chlorophenyl)cyclopropyl)-6-(2-(2,3-difluorophenyl)-2-hydroxyacetyl)-5,6,7,8-tetrahydropyrido[4,3-d]pyrimidin-4(3H)-one;
(S)-6-(2-(2,3-difluorophenyl)-2-hydroxyacetyl)-2-(1-(3-fluorophenyl)cyclopropyl)-5,6,7,8-tetrahydropyrido[4,3-d]pyrimidin-4(3H)-one;
6-(2-hydroxy-2-(3-hydroxyphenyl)acetyl)-2-(1-phenylcyclopropyl)-5,6,7,8-tetrahydropyrido[4,3-d]pyrimidin-4(3H)-one;
6-(2-(3-bromophenyl)-2-hydroxyacetyl)-2-(1-phenylcyclopropyl)-5,6,7,8-tetrahydropyrido[4,3-d]pyrimidin-4(3H)-one;
(R)-6-(2-(3-bromophenyl)-2-hydroxyacetyl)-2-(1-phenylcyclopropyl)-5,6,7,8-tetrahydropyrido[4,3-d]pyrimidin-4(3H)-one;
(R)-6-(2-(3-bromophenyl)-2-hydroxyacetyl)-2-(1-(3-fluorophenyl)cyclopropyl)-5,6,7,8-tetrahydropyrido[4,3-d]pyrimidin-4(3H)-one;
6-(2-(3-bromo-2-fluorophenyl)-2-hydroxyacetyl)-2-(1-phenylcyclopropyl)-5,6,7,8-tetrahydropyrido[4,3-d]pyrimidin-4(3H)-one;
(R)-2-(1-(3-chlorophenyl)cyclopropyl)-6-(2-(2,3-difluorophenyl)-2-hydroxyacetyl)-5,6,7,8-tetrahydropyrido[4,3-d]pyrimidin-4(3H)-one;
(S)-2-(1-(3-chlorophenyl)cyclopropyl)-6-(2-(2,3-difluorophenyl)-2-hydroxyacetyl)-5,6,7,8-tetrahydropyrido[4,3-d]pyrimidin-4(3H)-one; (R)-6-(2-(3-chlorophenyl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(p-tolyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(p-tolyl)cyclopropyl)-5,6,7,8-tetrahydropyrido[4,3-d]pyrimidin-4(3H)-one;
6-(2-hydroxy-2-(3-isopropoxyphenyl)acetyl)-2-(1-phenylcyclopropyl)-5,6,7,8-tetrahydropyrido[4,3-d]pyrimidin-4(3H)-one;
(R)-2-(1-(2-chlorophenyl)cyclopropyl)-6-(2-(2,3-difluorophenyl)-2-hydroxyacetyl)-5,6,7,8-tetrahydropyrido[4,3-d]pyrimidin-4(3H)-one;
(R)-6-(2-(3-chlorophenyl)-2-hydroxyacetyl)-2-(1-(2-chlorophenyl)cyclopropyl)-5,6,7,8-tetrahydropyrido[4,3-d]pyrimidin-4(3H)-one;
6-(2-(3-(cyclopentyloxy)phenyl)-2-hydroxyacetyl)-2-(1-phenylcyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(3,5-difluorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3,5-difluorophenyl)cyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(3,4-difluorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3,4-difluorophenyl)cyclopropyl)-5,6,7,8-tetrahydropyrido[4,3-d]pyrimidin-4(3H)-one;
6-(2-hydroxy-2-(3-(trifluoromethoxy)phenyl)acetyl)-2-(1-phenylcyclopropyl)-5,6,7,8-tetrahydropyrido[4,3-d]pyrimidin-4(3H)-one;
6-(2-(3,5-dichlorophenyl)-2-hydroxyacetyl)-2-(1-phenylcyclopropyl)-5,6,7,8-tetrahydropyrido[4,3-d]pyrimidin-4(3H)-one;
6-(2-(2,3-dichlorophenyl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(2,2,2-trifluoroethoxy)phenyl)acetyl)-2-(1-phenylcyclopropyl)-5,6,7,8-tetrahydropyrido[4,3-dJpyrimidin-4(3H)-one;
6-(2-(2-fluoro-3-methoxyphenyl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(trifluoromethyl)phenyl)acetyl)-2-(1-phenylcyclopropyl)-5,6,7,8-tetrahydropyrido[4,3-d]pyrimidin-4(3H)-one;
2-(1-(4-fluorophenyl)cyclopropyl)-6-(2-hydroxy-2-(3-(trifluoromethyl)phenyl)acetyl)-5,6,7,8-tetrahydropyrido[4,3-d]pyrimidin-4(3H)-one;
6-(2-(3-chloro-2-fluorophenyl)-2-hydroxyacetyl)-2-(1-phenylcyclopropyl)-5,6,7 ,8-tetrahydropyrido[4,3-d]pyrimidin-4(3H) - one;
(R)-6-(2-hydroxy-2-(3-(trifluoromethyl)phenyl)acetyl)-2-(1-phenylcyclopropyl)-5,6,7,8-tetrahydropyrido[4,3-d]pyrimidin-4(3H)-one;
(R)-2-(1-(3-chlorophenyl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-2-(1-(3-fluorophenyl)cyclopropyl)-6-(2-hydroxy-2-(3-(trifluoromethyl)phenyl)acetyl)-5,6,7,8-tetrahydropyrido[4,3-d]pyrimidin-4(3H)-one;
6-(2-hydroxy-2-(m-tolyl)acetyl)-2-(1-phenylcyclopropyl)-5,6,7,8-tetrahydropyrido[4,3-d]pyrimidin-4(3H)-one;
6-(2-(3-cyclopropylphenyl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(6-methylpyridin-3-yl)phenyl)acetyl)-2-(1-phenylcyclopropyl)-5,6,7,8-tetrahydropyrido[4,3-d]pyrimidin-4(3H)-one;
6-(2-hydroxy-2-(3-(oxazol-5-yl)phenyl)acetyl)-2-(1-phenylcyclopropyl)-5,6,7,8-tetrahydropyrido[4,3-d]pyrimidin-4(3H)-one;
3-(1-hydroxy-2-oxo-2-(4-oxo-2-(1-phenylcyclopropyl)-3,5,7,8-tetrahydropyrido[4,3-d]pyrimidin-6(4H)-yl)ethyl)benzonitrile;
6-(2-hydroxy-2-(3-(pyrimidin-5-yl)phenyl)acetyl)-2-(1-phenylcyclopropyl)-5,6,7,8-tetrahydropyrido[4,3-d]pyrimidin-4(3H)-one;
6-(2-hydroxy-2-(3-(2-methoxypyrimidin-5-yl)phenyl)acetyl)-2-(1-phenylcyclopropyl)-5,6,7,8-tetrahydropyrido[4,3-d]pyrimidin-4(3H)-one;
6-(2-(3-(1,3-dimethyl-1H-pyrazol-4-yl)phenyl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(pyridin-3-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(oxazol-5-yl)phenyl)acetyl)-2-(1-phenylcyclopropyl)-5,6,7,8-tetrahydropyrido[4,3-d]pyrimidin-4(3H)-one;
(R)-2-(1-(3-fluorophenyl)cyclopropyl)-6-(2-hydroxy-2-(3-(pyridin-3-yl)phenyl)acetyl)-5,6,7,8-tetrahydropyrido[4,3-d]pyrimidin-4(3H)-one;
(R)-2-(1-(3-fluorophenyl)cyclopropyl)-6-(2-hydroxy-2-(3-(oxazol-5-yl)phenyl)acetyl)-5,6,7,8-tetrahydropyrido[4,3-d]pyrimidin-4(3H)-one;
2-fluoro-3-(1-hydroxy-2-oxo-2-(4-oxo-2-(1-phenylcyclopropyl)-3,5,7,8-tetrahydropyrido[4,3-d]pyrimidin-6(4H)-yl)ethyl)benzonitrile;
6-(2-(3,5-difluorophenyl)-2-hydroxyacetyl)-2-(1-phenylcyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(thiophen-2-yl)cyclopropyl)-5,6,7,8-tetrahydropyrido[4,3-d]pyrimidin-4(3H)-one;
(S)-6-(2-(2,3-difluorophenyl)-2-hydroxyacetyl)-2-(1-(thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(pyridin-2-yl)cyclopropyl)-5,6,7,8-tetrahydropyrido[4,3-d]pyrimidin-4(3H)-one;
6-(2-(2,3-difluorophenyl)-2-hydroxyacetyl)-2-(1-(pyridin-3-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(pyridin-3-yl)cyclopropyl)-5,6,7,8-tetrahydropyrido[4,3-d]pyrimidin-4(3H)-one;
6-(2-(2,3-difluorophenyl)-2-hydroxyacetyl)-2-(1-(thiophen-2-yl)cyclopropyl)-5,6,7,8-tetrahydropyrido[4,3-d]pyrimidin-4(3H)-one;
(R)-6-(2-(2-chlorophenyl)-2-hydroxyacetyl)-2-(1-(thiophen-2-yl)cyclopropyl)-5,6,7,8-tetrahydropyrido[4,3-d]pyrimidin-4(3H)-one;
6-(2-(3,5-difluorophenyl)-2-hydroxyacetyl)-2-(1-(thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(thiophen-2-yl)cyclopropyl)-5,6,7,8-tetrahydropyrido[4,3-d]pyrimidin-4(3H)-one;
(R)-2-(1-benzylcyclopropyl)-6-(2-(3-chlorophenyl)-2-hydroxyacetyl)-5,6,7,8-tetrahydropyrido[4,3-d]pyrimidin-4(3H)-one;
2-(1-benzylcyclopropyl)-6-(2-(2,3-difluorophenyl)-2-hydroxyacetyl)-5,6,7,8-tetrahydropyrido[4,3-d]pyrimidin-4(3H)-one;
6-(2-hydroxy-2-(m-tolyl)acetyl)-2-(1-(thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(2-methylthiazol-4-yl)cyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(thiazol-4-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(thiazol-4-yl)cyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(thiazol-5-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(thiazol-5-yl)cyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(5-methylthiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(5-methylthiophen-2-yl)cyclopropyl)-5,6,7,8-tetrahydropyrido[4,3-d]pyrimidin-4(3H)-one;
(R)-2-(1-(4-bromothiophen-2-yl)cyclopropyl)-6-(2-(3-chlorophenyl)-2-hydroxyacetyl)-5,6,7,8-tetrahydropyrido[4,3-d]pyrimidin-4(3H)-one;
(R)-6-(2-(3-chlorophenyl)-2-hydroxyacetyl)-2-(1-(4-methylthiazol-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-methylthiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-phenylthiophen-2-yl)cyclopropyl)-5,6,7,8-tetrahydropyrido[4,3-d]pyrimidin-4(3H)-one;
6-((R)-2-(3-chlorophenyl)-2-hydroxyacetyl)-2-((R/S)-1-phenylethyl)-5,6,7,8-tetrahydropyrido[4,3-d]pyrimidin-4(3H)-one;
(R)-6-(2-(3-chlorophenyl)-2-hydroxyacetyl)-2-(2-phenylpropan-2-yl)-5,6,7,8-tetrahydropyrido[4,3-d]pyrimidin-4(3H)-one;
6-(2-(2,3-difluorophenyl)-2-hydroxyacetyl)-2-(2-phenylpropan-2-yl)-5,6,7,8-tetrahydropyrido[4,3-d]pyrimidin-4(3H)-one;
6-(2-(3,5-difluorophenyl)-2-hydroxyacetyl)-2-(2-phenylpropan-2-yl)-5,6,7,8-tetrahydropyrido[4,3-d]pyrimidin-4(3H)-one;
(R)-6-(2-(2-chlorophenyl)-2-hydroxyacetyl)-2-(2-phenylpropan-2-yl)-5,6,7,8-tetrahydropyrido[4,3-d]pyrimidin-4(3H)-one;
6-(2-(2,3-difluorophenyl)-2-hydroxyacetyl)-2-(1-phenylcyclobutyl)-5,6,7,8-tetrahydropyrido[4,3-d]pyrimidin-4(3H)-one;
(R)-6-(2-(2-chlorophenyl)-2-hydroxyacetyl)-2-(1-phenylcyclobutyl)-5,6,7,8-tetrahydropyrido[4,3-d]pyrimidin-4(3H)-one;
6-(2-(3,5-difluorophenyl)-2-hydroxyacetyl)-2-(1-phenylcyclobutyl)-5,6,7,8-tetrahydropyrido[4,3-d]pyrimidin-4(3H)-one;
(R)-6-(2-(3-chlorophenyl)-2-hydroxyacetyl)-2-(1-phenylcyclobutyl)-5,6,7,8-tetrahydropyrido[4,3-d]pyrimidin-4(3H)-one;
7-(2-(2,3-difluorophenyl)-2-hydroxyacetyl)-2-(1-phenylcyclopropyl)-3, 5,6,7,8,9-hexahydro-4H-pyrimido[4,5-d]azepin-4-one;
(R)-7-(2-(3-chlorophenyl)-2-hydroxyacetyl)-2-(1-phenylcyclopropyl)-3,5, 6, 7, 8, 9-hexahydro-4H-pyrimido[4,5-d]azepin-4-one;
(R)-6-(2-(2-chlorophenyl)-2-hydroxyacetyl)-2-(1-phenylcyclopropyl)-3,5,6,7-tetrahydro-4H-pyrrolo[3,4-d]pyrimidin-4-one;
(R)-7-(2-(3-chlorophenyl)-2-hydroxyacetyl)-2-(1-phenylcyclopropyl)-5, 6, 7, 8-tetrahydropyrido[3,4-d]pyrimidin-4(3H)-one;
7-(2-(2,3-difluorophenyl)-2-hydroxyacetyl)-2-(1-phenylcyclopropyl)-5,6,7,8-tetrahydropyrido[3,4-d]pyrimidin-4(3H)-one;
(R)-6-(2-(2,3-difluorophenyl)-2-hydroxyacetyl)-2-(1-phenylcyclopropyl)-3,5,6,7,8,9-hexahydro-4H-pyrimido[5,4-c]azepin-4-one;
(R)-6-(2-(3-chlorophenyl)-2-hydroxyacetyl)-2-(1-phenylcyclopropyl)-3,5, 6, 7, 8, 9-hexahydro-4H-pyrimido[5,4-c]azepin-4-one;
(R)-8-(2-(2,3-difluorophenyl)-2-hydroxyacetyl)-2-(1-phenylcyclopropyl)-3,5,6,7,8,9-hexahydro-4H-pyrimido[4,5-c]azepin-4-one;
(R)-8-(2-(3-chlorophenyl)-2-hydroxyacetyl)-2-(1-phenylcyclopropyl)-3,5,6,7,8,9-hexahydro-4H-pyrimido[4,5-c]azepin-4-one;
6-(2-hydroxy-2-(2-(pyrrolidin-1-yl)pyridin-4-yl)acetyl)-2-(1-phenylcyclopropyl)-5,6,7,8-tetrahydropyrido[4,3-d]pyrimidin-4(3H)-one;
6-(2-hydroxy-2-(2-(piperidin-1-yl)pyridin-4-yl)acetyl)-2-(1-phenylcyclopropyl)-5,6,7,8-tetrahydropyrido[4,3-d]pyrimidin-4(3H)-one;
6-(2-(2-(azepan-1-yl)pyridin-4-yl)-2-hydroxyacetyl)-2-(1-phenylcyclopropyl)-5,6,7,8-tetrahydropyrido[4,3-d]pyrimidin-4(3H)-one;
(R)-5-(1-(6-(2-(3-chlorophenyl)-2-hydroxyacetyl)-4-oxo-3,4,5,6,7,8-hexahydropyrido[4,3-d]pyrimidin-2-yl)cyclopropyl)thiophene-3-carbonitrile;
4-oxo-2-(1-phenylcyclopropyl)-N-(3-(trifluoromethyl)phenyl)-3,5,7,8-tetrahydropyrido[4,3-d]pyrimidine-6(4H)-carboxamide;
6-(1H-benzo[d]imidazole-2-carbonyl)-2-(1-phenylcyclopropyl)-5,6,7,8-tetrahydropyrido[4,3-d]pyrimidin-4(3H)-one;
6-(5,6-difluoro-1H-benzo[d]imidazole-2-carbonyl)-2-(1-phenylcyclopropyl)-5,6,7,8-tetrahydropyrido[4,3-d]pyrimidin-4(3H)-one;
6-(5,6-difluoro-1H-benzo[d]imidazole-2-carbonyl)-2-(2-phenylpropan-2-yl)-5,6,7,8-tetrahydropyrido[4,3-d]pyrimidin-4(3H)-one;
2-benzyl-6-(5,6-difluoro-1H-benzo[d]imidazole-2-carbonyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(thiophen-2-yl)cyclopropyl)-5,6,7,8-tetrahydropyrido[4,3-d]pyrimidin-4(3H)-one;
(R)-2-(1-(4-bromothiophen-2-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-phenylcyclopropyl)-3,5,6,7,8,9-hexahydro-4H-pyrimido[5,4-c]azepin-4-one;
6-(2-(5-chloro-2-fluorophenyl)-2-hydroxyacetyl)-2-(1-phenylcyclopropyl)-5,6,7,8-tetrahydropyrido[4,3-d]pyrimidin-4(3H)-one;
6-(2-(3-chloro-5-fluorophenyl)-2-hydroxyacetyl)-2-(1-phenylcyclopropyl)-5,6,7,8-tetrahydropyrido[4,3-d]pyrimidin-4(3H)-one;
6-(2-hydroxy-2-(3-isopropylphenyl)acetyl)-2-(1-phenylcyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-cyclohexylthiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-(pyridin-4-yl)thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-(pyridin-3-yl)thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-isopropylthiophen-2-yl)cyclopropyl)-5,6,7,8-tetrahydropyrido[4,3-d]pyrimidin-4(3H)-one;
6-(2-(3-(tert-butyl)phenyl)-2-hydroxyacetyl)-2-(1-phenylcyclopropyl)-5,6,7,8-tetrahydropyrido[4,3-d]pyrimidin-4(3H)-one;
6-(2-(3-(tert-butyl)phenyl)-2-hydroxyacetyl)-2-(1-(3-fluorophenyl)cyclopropyl)-5,6,7,8-tetrahydropyrido[4,3-d]pyrimidin-4(3H)-one;
6-(2-(3-(tert-butyl)phenyl)-2-hydroxyacetyl)-2-(1-(thiophen-2-yl)cyclopropyl)-5,6,7,8-tetrahydropyrido[4,3-d]pyrimidin-4(3H)-one;
6-(2-hydroxy-2-(3-((trifluoromethyl)thio)phenyl)acetyl)-2-(1-phenylcyclopropyl)-5,6,7,8-tetrahydropyrido[4,3-d]pyrimidin-4(3H)-one;
6-(2-(3-bromophenyl)-2-hydroxyacetyl)-2-(1-phenylcyclopropyl)-5,6,7,8-tetrahydropyrido[4,3-d]pyrimidin-4(3H)-one;
6-(2-(2-chloro-3-fluorophenyl)-2-hydroxyacetyl)-2-(1-phenylcyclopropyl)-5,6,7,8-tetrahydropyrido[4,3-d]pyrimidin-4(3H)-one;
(R)-2-(1-(4-(benzyloxy)phenyl)cyclopropyl)-6-(2-(3-chlorophenyl)-2-hydroxyacetyl)-5,6,7,8-tetrahydropyrido[4,3-d]pyrimidin-4(3H)-one;
(R)-6-(2-(3-chlorophenyl)-2-hydroxyacetyl)-2-(1-(4-(tetrahydro-2H-pyran-4-yl)thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-fluorophenyl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-phenylcyclopropyl)-3,5,6,7,8,9-hexahydro-4H-pyrimido[5,4-c]azepin-4-one;
(R)-2-(1-(3-fluorophenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-(3-chlorophenyl)-2-hydroxyacetyl)-2-(1-(4-(1-methyl-1H-pyrazol-4-yl)thiophen-2-yl)cyclopropyl)-5,6,7,8-tetrahydropyrido[4,3-d]pyrimidin-4(3H)-one;
6-(4-hydroxy-2-naphthoyl)-2-(1-phenylcyclopropyl)-5,6,7,8-tetrahydropyrido[4,3-d]pyrimidin-4(3H)-one;
6-(2-hydroxy-2-(2-(trifluoromethyl)phenyl)acetyl)-2-(1-phenylcyclopropyl)-5,6,7,8-tetrahydropyrido[4,3-d]pyrimidin-4(3H)-one;
6-(2-(2,3-dihydrobenzofuran-7-yl)-2-hydroxyacetyl)-2-(1-phenylcyclopropyl)-5,6,7,8-tetrahydropyrido[4,3-d]pyrimidin-4(3H)-one;
6-(2-(2-fluoro-3-methylphenyl)-2-hydroxyacetyl)-2-(1-phenylcyclopropyl)-5,6,7,8-tetrahydropyrido[4,3-d]pyrimidin-4(3H)-one;
6-(2-(3-chloro-4-fluorophenyl)-2-hydroxyacetyl)-2-(1-phenylcyclopropyl)-5,6,7,8-tetrahydropyrido[4,3-d]pyrimidin-4(3H)-one;
6-(2-(3-chloro-2,4-difluorophenyl)-2-hydroxyacetyl)-2-(1-phenylcyclopropyl)-5,6,7,8-tetrahydropyrido[4,3-d]pyrimidin-4(3H)-one;
6-(3-(1H-indol-1-yl)propanoyl)-2-(1-phenylcyclopropyl)-5,6,7,8-tetrahydropyrido[4,3-d]pyrimidin-4(3H)-one;
6-(2-(5-chlorothiophen-2-yl)-2-hydroxyacetyl)-2-(1-phenylcyclopropyl)-5,6,7,8-tetrahydropyrido[4,3-d]pyrimidin-4(3H)-one;
6-(2-(4-cyclopropylphenyl)acetyl)-2-(1-phenylcyclopropyl)-5,6,7,8-tetrahydropyrido[4,3-d]pyrimidin-4(3H)-one;
2-(1-phenylcyclopropyl)-6-(2-(4-(trifluoromethyl)phenyl)acetyl)-5,6,7,8-tetrahydropyrido[4,3-d]pyrimidin-4(3H)-one;
6-(2-(4-chlorophenyl)acetyl)-2-(1-phenylcyclopropyl)-5,6,7,8-tetrahydropyrido[4,3-d]pyrimidin-4(3H)-one;
6-(2-(3-chlorophenyl)acetyl)-2-(1-phenylcyclopropyl)-5,6,7,8-tetrahydropyrido[4,3-d]pyrimidin-4(3H)-one;
2-(1-phenylcyclopropyl)-6-(2-(3-(trifluoromethyl)phenyl)acetyl)-5,6,7,8-tetrahydropyrido[4,3-d]pyrimidin-4(3H)-one;
(R)-6-(3-hydroxy-3-phenylpropanoyl)-2-(1-phenylcyclopropyl)-5,6,7,8-tetrahydropyrido[4,3-d]pyrimidin-4(3H)-one;
6-(2-(3-(difluoromethyl)phenyl)-2-hydroxyacetyl)-2-(1-phenylcyclopropyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-phenylcyclopropyl)-5,6,7,8-tetrahydropyrido[4,3-d]pyrimidin-4(3H)-one;
6-(2-(4-(tert-butyl)phenyl)acetyl)-2-(1-phenylcyclopropyl)-5,6,7,8-tetrahydropyrido[4,3-d]pyrimidin-4(3H)-one;
(R)-6-(2-(2,3-difluorophenyl)-2-hydroxyacetyl)-2-(1-(3-fluorophenyl)cyclopropyl)-3,5,6,7,8,9-hexahydro-4H-pyrimido[5,4-c]azepin-4-one;
6-(2-(3,5-dichlorophenyl)-2-hydroxyacetyl)-2-(1-phenylcyclopropyl)-3,5, 6, 7, 8, 9-hexahydro-4H-pyrimido[5,4-c]azepin-4-one;
(R)-2-(1-(4-(1H-pyrazol-4-yl)thiophen-2-yl)cyclopropyl)-6-(2-(3-chlorophenyl)-2-hydroxyacetyl)-5,6,7,8-tetrahydropyrido[4,3-d]pyrimidin-4(3H)-one;
(R)-6-(2-(3-chlorophenyl)-2-hydroxyacetyl)-2-(1-(thiophen-3-yl)cyclopropyl)-5,6,7,8-tetrahydropyrido[4,3-d]pyrimidin-4(3H)-one;
6-(2-(3-chlorophenyl)-2,2-difluoroacetyl)-2-(1-phenylcyclopropyl)-5,6,7,8-tetrahydropyrido[4,3-d]pyrimidin-4(3H)-one;
6-(2-(4-chloro-2-fluorophenyl)-2-hydroxyacetyl)-2-(1-phenylcyclopropyl)-5,6,7,8-tetrahydropyrido[4,3-d]pyrimidin-4(3H)-one;
6-(2-hydroxy-2-(5-(trifluoromethyl)pyridin-3-yl)acetyl)-2-(1-phenylcyclopropyl)-5,6,7,8-tetrahydropyrido[4,3-d]pyrimidin-4(3H)-one;
6-(2-(2-fluoro-5-(trifluoromethyl)phenyl)-2-hydroxyacetyl)-2-(1-phenylcyclopropyl)-5,6,7,8-tetrahydropyrido[4,3-d]pyrimidin-4(3H)-one;
(R)-6-(2-(3-chlorophenyl)-2-hydroxyacetyl)-2-(1-(naphthalen-1-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(naphthalen-1-ylmethyl)-5,6,7,8-tetrahydropyrido[4,3-d]pyrimidin-4 (3H)-one;
(R)-6-(2-(3-chlorophenyl)-2-hydroxyacetyl)-2-(1-(naphthalen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(thiophen-2-yl)cyclopropyl)-3,5,6,7,8,9-hexahydro-4H-pyrimido[5,4-c] azepin-4-one;
(R)-8-(2-(3-chlorophenyl)-2-hydroxyacetyl)-2-(1-(thiophen-2-yl)cyclopropyl)-3,5,6,7,8,9-hexahydro-4H-pyrimido[4,5-c]azepin-4-one;
(R)-8-(2-hydroxy-2-(3-(trifluoromethyl)phenyl)acetyl)-2-(1-phenylcyclopropyl)-3,5,6,7,8,9-hexahydro-4H-pyrimido[4,5-c]azepin-4-one;
(R)-6-(2-(2,3-difluorophenyl)-2-hydroxyacetyl)-2-(1-(thiophen-2-yl)cyclopropyl)-3,5,6,7,8,9-hexahydro-4H-pyrimido[5,4-c]azepin-4-one;
(R)-6-(2-(2-chlorophenyl)-2-hydroxyacetyl)-2-(1-phenylcyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(thiophen-2-yl)cyclopropyl)-3,5,6,7,8,9-hexahydro-4H-pyrimido[5,4-c]azepin-4-one;
6-(2-hydroxy-2-(6-(trifluoromethyl)pyridin-2-yl)acetyl)-2-(1-phenylcyclopropyl)-5,6,7,8-tetrahydropyrido[4,3-d]pyrimidin-4(3H)-one;
6-(2-hydroxy-2-(2-(trifluoromethyl)pyridin-4-yl)acetyl)-2-(1-phenylcyclopropyl)-5,6,7,8-tetrahydropyrido[4,3-d]pyrimidin-4(3H)-one;
6-(6-chloro-1H-indole-2-carbonyl)-2-(1-phenylcyclopropyl)-5,6,7,8-tetrahydropyrido[4,3-d]pyrimidin-4(3H)-one;
2-(1-phenylcyclopropyl)-6-(5-(trifluoromethyl)-1H-benzo[d]imidazole-2-carbonyl)-5,6,7,8-tetrahydropyrido[4,3-d] pyrimidin-4(3H)-one;
6-(3-(2-fluorophenyl)-1H-pyrazole-5-carbonyl)-2-(1-phenylcyclopropyl)-5,6,7,8-tetrahydropyrido[4,3-d]pyrimidin-4(3H)-one;
6-(2-(2-chlorophenyl)acetyl)-2-(1-phenylcyclopropyl)-5,6,7,8-tetrahydropyrido[4,3-d]pyrimidin-4(3H)-one;
6-(3-(2-chlorophenyl)propanoyl)-2-(1-phenylcyclopropyl)-5,6,7,8-tetrahydropyrido[4,3-d]pyrimidin-4(3H)-one;
6-(2-(2-chlorophenoxy)acetyl)-2-(1-phenylcyclopropyl)-5,6,7,8-tetrahydropyrido[4,3-d]pyrimidin-4(3H)-one;
2-(1-phenylcyclopropyl)-6-(2-(2-(trifluoromethyl)phenyl)acetyl)-5,6,7,8-tetrahydropyrido[4,3-d]pyrimidin-4(3H)-one;
6-(3-(3-chlorophenyl)propanoyl)-2-(1-phenylcyclopropyl)-5,6,7,8-tetrahydropyrido[4,3-d]pyrimidin-4(3H)-one;
6-(2-(3-chlorophenoxy)acetyl)-2-(1-phenylcyclopropyl)-5,6,7,8-tetrahydropyrido[4,3-d]pyrimidin-4(3H)-one;
6-(2-hydroxy-2-(3-(pentafluoro-(lambda)⁶-sulfaneyl)phenyl)acetyl)-2-(1-phenylcyclopropyl)-5,6,7,8-tetrahydropyrido[4,3-d]pyrimidin-4(3H)-one;
6-(2-(4-chloro-3-fluorophenyl)-2-hydroxyacetyl)-2-(1-phenylcyclopropyl)-5,6,7,8-tetrahydropyrido[4,3-d]pyrimidin-4(3H)-one;
6-(2-hydroxy-3-(o-tolyloxy)propanoyl)-2-(1-phenylcyclopropyl)-5,6,7,8-tetrahydropyrido[4,3-d]pyrimidin-4(3H)-one;
6-(2-(3,4-dichlorophenyl)-2-hydroxyacetyl)-2-(1-phenylcyclopropyl)-5,6,7,8-tetrahydropyrido[4,3-d]pyrimidin-4(3H)-one;
6-(2-(3-acetylphenyl)-2-hydroxyacetyl)-2-(1-phenylcyclopropyl)-5,6,7,8-tetrahydropyrido[4,3-d]pyrimidin-4(3H)-one;
6-(2-(3-ethylphenyl)-2-hydroxyacetyl)-2-(1-phenylcyclopropyl)-5,6,7,8-tetrahydropyrido[4,3-d]pyrimidin-4(3H)-one;
(R)-2-(1-(3-bromothiophen-2-yl)cyclopropyl)-6-(2-(3-chlorophenyl)-2-hydroxyacetyl)-5,6,7,8-tetrahydropyrido[4,3-d]pyrimidin-4(3H)-one;
(R)-6-(2-(3-chlorophenyl)-2-hydroxyacetyl)-2-(1-(6-phenylpyridin-3-yl)cyclopropyl)-5,6,7,8-tetrahydropyrido[4,3-d]pyrimidin-4(3H)-one;
6-(2-(3-chloro-2-fluorophenyl)-2-hydroxyacetyl)-2-(1-phenylcyclopropyl)-3,5,6,7,8,9-hexahydro-4H-pyrimido[5,4-c]azepin-4-one;
6-(2-(2-fluoro-3-methylphenyl)-2-hydroxyacetyl)-2-(1-phenylcyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(thiophen-2-yl)cyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(3-chlorophenyl)cyclopropyl)-5,6,7,8-tetrahydropyrido[4,3-d]pyrimidin-4(3H)-one;
(R)-2-(1-(3-bromophenyl)cyclopropyl)-6-(2-(3-chlorophenyl)-2-hydroxyacetyl)-5,6,7,8-tetrahydropyrido[4,3-d]pyrimidin-4(3H)-one;
6-(2-(2,5-dichlorophenyl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(p-tolyl)acetyl)-2-(1-phenylcyclopropyl)-5,6,7,8-tetrahydropyrido[4,3-d]pyrimidin-4(3H)-one;
6-(2-hydroxy-2-(3-isopropylphenyl)acetyl)-2-(1-phenylcyclopropyl)-3,5,6, 7,8,9-hexahydro-4H-pyrimido[5,4-c]azepin-4-one;
6-(2-(3-(difluoromethyl)phenyl)-2-hydroxyacetyl)-2-(1-phenylcyclopropyl)-3,5,6,7,8,9-hexahydro-4H-pyrimido[5,4-c]azepin-4-one;
6-(2-(2-fluoro-3-(trifluoromethyl)phenyl)-2-hydroxyacetyl)-2-(1-phenylcyclopropyl)-3,5,6,7,8,9-hexahydro-4H-pyrimido[5,4-c]azepin-4-one;
2-(1-phenylcyclopropyl)-6-(2-(4-(trifluoromethoxy)phenyl)acetyl)-5,6,7,8-tetrahydropyrido[4,3-d]pyrimidin-4(3H)-one;
6-(2-(4-phenoxyphenyl)acetyl)-2-(1-phenylcyclopropyl)-5,6,7,8-tetrahydropyrido[4,3-d]pyrimidin-4(3H)-one;
6-(2-(3-chloro-2-methoxyphenyl)-2-hydroxyacetyl)-2-(1-phenylcyclopropyl)-5,6,7,8-tetrahydropyrido[4,3-d]pyrimidin-4(3H)-one;
(R)-6-(2-(3-chlorophenyl)-2-hydroxyacetyl)-2-(1-(5-phenylpyridin-3-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-chlorothiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(5-chlorothiophen-2-yl)cyclopropyl)-5,6,7,8-tetrahydropyrido[4,3-d]pyrimidin-4(3H)-one;
(R)-2-(1-([1,1'-biphenyl]-3-yl)cyclopropyl)-6-(2-(3-chlorophenyl)-2-hydroxyacetyl)-5,6,7,8-tetrahydropyrido[4,3-d]pyrimidin-4(3H)-one;
(R)-2-(1-(4-bromophenyl)cyclopropyl)-6-(2-(3-chlorophenyl)-2-hydroxyacetyl)-5,6,7,8-tetrahydropyrido[4,3-d]pyrimidin-4(3H)-one;
(R)-2-(1-([1,1'-biphenyl]-4-yl)cyclopropyl)-6-(2-(3-chlorophenyl)-2-hydroxyacetyl)-5,6,7,8-tetrahydropyrido[4,3-d]pyrimidin-4(3H)-one;
6-(2-hydroxy-2-(naphthalen-1-yl)acetyl)-2-(1-phenylcyclopropyl)-5,6,7,8-tetrahydropyrido[4,3-d]pyrimidin-4(3H)-one;
6-(2-(3-chloro-2-methylphenyl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(2-methylpyridin-3-yl)phenyl)acetyl)-2-(1-phenylcyclopropyl)-5,6,7,8-tetrahydropyrido[4,3-d]pyrimidin-4(3H)-one;
2-(1-phenylcyclopropyl)-6-(2-(quinolin-3-yl)acetyl)-5,6,7,8-tetrahydropyrido[4,3-d]pyrimidin-4(3H)-one;
2-(1-phenylcyclopropyl)-6-(2-(4-(pyridin-2-yl)phenyl)acetyl)-5,6,7,8-tetrahydropyrido[4,3-d]pyrimidin-4(3H)-one;
2-(1-phenylcyclopropyl)-6-(2-(3-(trifluoromethoxy)phenyl)acetyl)-5,6,7,8-tetrahydropyrido[4,3-d]pyrimidin-4(3H)-one;
6-(2-([1,1'-biphenyl]-3-yl)acetyl)-2-(1-phenylcyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-phenoxyphenyl)cyclopropyl)-5,6,7,8-tetrahydropyrido[4,3-d]pyrimidin-4(3H)-one;
6-(2-hydroxy-2-(3-(5-methylpyridin-3-yl)phenyl)acetyl)-2-(1-phenylcyclopropyl)-5,6,7,8-tetrahydropyrido[4,3-d]pyrimidin-4(3H)-one;
6-(2-hydroxy-2-(3-(4-methylpyridin-3-yl)phenyl)acetyl)-2-(1-phenylcyclopropyl)-5,6,7,8-tetrahydropyrido[4,3-d]pyrimidin-4(3H)-one;
6-(2-hydroxy-2-(3-(6-methoxypyridin-3-yl)phenyl)acetyl)-2-(1-phenylcyclopropyl)-5,6,7,8-tetrahydropyrido[4,3-d]pyrimidin-4(3H)-one;
6-(2-hydroxy-2-(4'-methyl-[1,1'-biphenyl]-3-yl)acetyl)-2-(1-phenylcyclopropyl)-5,6,7,8-tetrahydropyrido[4,3-d]pyrimidin-4(3H)-one;
6-(2-(4'-chloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(2-methylpyridin-4-yl)phenyl)acetyl)-2-(1-phenylcyclopropyl)-5,6,7,8-tetrahydropyrido[4,3-d]pyrimidin-4(3H)-one;
6-(2-hydroxy-2-(3-(2-methylpyrimidin-5-yl)phenyl)acetyl)-2-(1-phenylcyclopropyl)-5,6,7,8-tetrahydropyrido[4,3-d]pyrimidin-4(3H)-one;
6-(2-(3-(6-(dimethylamino)pyridin-3-yl)phenyl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(6-(trifluoromethyl)pyridin-3-yl)phenyl)acetyl)-2-(1-phenylcyclopropyl)-5,6,7,8-tetrahydropyrido[4,3-d]pyrimidin-4(3H)-one;
6-(2-hydroxy-2-(3-(2-(trifluoromethyl)pyridin-4-yl)phenyl)acetyl)-2-(1-phenylcyclopropyl)-5,6,7,8-tetrahydropyrido[4,3-d]pyrimidin-4(3H)-one;
6-(2-(3-(1-cyclopropyl-1H-pyrazol-4-yl)phenyl)-2-hydroxyacetyl)-2-(1-phenylcyclopropyl)-5,6,7,8-tetrahydropyrido[4,3-d]pyrimidin-4(3H)-one;
6-(2-(3-(5-chlorothiophen-2-yl)phenyl)-2-hydroxyacetyl)-2-(1-phenylcyclopropyl)-5,6,7,8-tetrahydropyrido[4,3-d]pyrimidin-4(3H)-one;
2-(1-(3-fluorophenyl)cyclopropyl)-6-(2-hydroxy-2-(3-isopropylphenyl)acetyl)-5,6,7,8-tetrahydropyrido[4,3-d]pyrimidin-4(3H)-one;
2-(1-(3-chlorophenyl)cyclopropyl)-6-(2-hydroxy-2-(3-isopropylphenyl)acetyl)-5,6,7,8-tetrahydropyrido[4,3-d]pyrimidin-4(3H)-one;
6-(2-hydroxy-2-(3-isopropylphenyl)acetyl)-2-(1-(thiophen-2-yl)cyclopropyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-(3-fluorophenyl)cyclopropyl)-5,6,7,8-tetrahydropyrido[4,3-d]pyrimidin-4(3H)-one;
2-(1-(3-chlorophenyl)cyclopropyl)-6-(2-(2-fluoro-3-(trifluoromethyl)phenyl)-2-hydroxyacetyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-(thiophen-2-yl)cyclopropyl)-5,6,7,8-tetrahydropyrido[4,3-d]pyrimidin-4(3H)-one;
2-(1-(3-fluorophenyl)cyclopropyl)-6-(2-hydroxy-2-(3-((trifluoromethyl)thio)phenyl)acetyl)-5,6,7,8-tetrahydropyrido[4,3-d]pyrimidin-4(3H)-one;
2-(1-(3-chlorophenyl)cyclopropyl)-6-(2-hydroxy-2-(3-((trifluoromethyl)thio)phenyl)acetyl)-5,6,7,8-tetrahydropyrido[4,3-d]pyrimidin-4(3H)-one;
6-(2-hydroxy-2-(3-((trifluoromethyl)thio)phenyl)acetyl)-2-(1-(thiophen-2-yl)cyclopropyl)-5,6,7,8-tetrahydropyrido[4,3-d]pyrimidin-4(3H)-one;
6-(2-hydroxy-2-(3-((trifluoromethyl)thio)phenyl)acetyl)-2-(1-phenylcyclopropyl)-3,5,6,7,8,9-hexahydro-4H-pyrimido[5,4-c]azepin-4-one;
2-(1-(3-fluorophenyl)cyclopropyl)-6-(2-hydroxy-2-(3-(pentafluoro-(lambda)⁶-sulfaneyl)phenyl)acetyl)-5,6,7,8-tetrahydropyrido[4,3-d]pyrimidin-4(3H)-one;
2-(1-(3-chlorophenyl)cyclopropyl)-6-(2-hydroxy-2-(3-(pentafluoro-(lambda)⁶-sulfaneyl)phenyl)acetyl)-5,6,7,8-tetrahydropyrido[4,3-d]pyrimidin-4(3H)-one;
6-(2-hydroxy-2-(3-(pentafluoro-(lambda)⁶-sulfaneyl)phenyl)acetyl)-2-(1-(thiophen-2-yl)cyclopropyl)-5,6,7,8-tetrahydropyrido[4,3-d]pyrimidin-4(3H)-one;
6-(2-hydroxy-2-(3-(pentafluoro-(lambda)⁶-sulfaneyl)phenyl)acetyl)-2-(1-phenylcyclopropyl)-3,5,6,7,8,9-hexahydro-4H-pyrimido[5,4-c]azepin-4-one;
6-(2-hydroxy-2-(2-phenylpyridin-4-yl)acetyl)-2-(1-phenylcyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-isopropylphenyl)cyclopropyl)-5,6,7,8-tetrahydropyrido[4,3-d] pyrimidin-4(3H)-one;
(R)-6-(2-(3-chlorophenyl)-2-hydroxyacetyl)-2-(1-(3-cyclohexylphenyl)cyclopropyl)-5,6,7,8-tetrahydropyrido[4,3-d]pyrimidin-4(3H)-one;
6-(2-(2-fluoro-3-(trifluoromethyl)phenyl)acetyl)-2-(1-phenylcyclopropyl)-5,6,7,8-tetrahydropyrido[4,3-d]pyrimidin-4(3H)-one;
6-(2-(3-cyclopropylphenyl)acetyl)-2-(1-phenylcyclopropyl)-5,6,7,8-tetrahydropyrido[4,3-d]pyrimidin-4(3H)-one;
6-(2-hydroxy-2-(1-methyl-1H-indol-3-yl)acetyl)-2-(1-phenylcyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-isopropylthiophen-2-yl)cyclopropyl)-5,6,7,8-tetrahydropyrido[4,3-d]pyrimidin-4(3H)-one;
(R)-2-(1-(4-ethylthiophen-2-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-2-(1-(4-cyclopentylthiophen-2-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-isopropylthiophen-2-yl)cyclopropyl)-3,5,6,7,8,9-hexahydro-4H-pyrimido[5,4-c]azepin-4-one;
6-(2-hydroxy-2-(2-methyl-3-(trifluoromethyl)phenyl)acetyl)-2-(1-phenylcyclopropyl)-5,6,7,8-tetrahydropyrido[4,3-d]pyrimidin-4(3H)-one;
6-(2-(2-chloro-3-(trifluoromethyl)phenyl)-2-hydroxyacetyl)-2-(1-phenylcyclopropyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-(thiophen-2-yl)cyclopropyl)-3,5,6,7,8,9-hexahydro-4H-pyrimido[5,4-c]azepin-4-one;
6-(2-hydroxy-2-(3-(pentafluoro-(lambda)⁶-sulfaneyl)phenyl)acetyl)-2-(1-(thiophen-2-yl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-(3-fluorophenyl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-(thiophen-2-yl)cyclopropyl)-3,5,6,7,8,9-hexahydro-4H-pyrimido[5,4-c]azepin-4-one;
6-(2-(3-fluoro-5-(trifluoromethyl)phenyl)-2-hydroxyacetyl)-2-(1-phenylcyclopropyl)-5,6,7,8-tetrahydropyrido[4,3-d]pyrimidin-4(3H)-one;
6-(2-(3-chloro-5-(trifluoromethyl)phenyl)-2-hydroxyacetyl)-2-(1-phenylcyclopropyl)-5,6,7,8-tetrahydropyrido[4,3-d]pyrimidin-4(3H)-one;
2-(1-phenylcyclopropyl)-6-(2-(3-(trifluoromethyl)phenyl)acetyl)-3,5,6,7,8,9-hexahydro-4H-pyrimido[5,4-c]azepin-4-one;
6-(2-(3-chlorophenyl)-2,2-difluoroacetyl)-2-(1-phenylcyclopropyl)-3,5,6,7,8,9-hexahydro-4H-pyrimido[5,4-c]azepin-4-one;
6-(2-hydroxy-2-(3-((trifluoromethyl)thio)phenyl)acetyl)-2-(1-(thiophen-2-yl)cyclopropyl)-3,5,6,7,8,9-hexahydro-4H-pyrimido[5,4-c] azepin-4-one;
(R)-6-(2-hydroxy-2-(3-(trifluoromethyl)phenyl)acetyl)-2-(1-(3-phenoxyphenyl)cyclopropyl)-5,6,7,8-tetrahydropyrido[4,3-d] pyrimidin-4(3H)-one;
(R)-2-(1-(4-cycloheptylthiophen-2-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-2-(1-(4-cyclohexylthiophen-2-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
6-(2-hydroxy-2-(2'-(trifluoromethyl)-[2,4'-bipyridin]-4-yl)acetyl)-2-(1-phenylcyclopropyl)-5,6,7,8-tetrahydropyrido[4,3-d]pyrimidin-4(3H)-one;
6-(2-(3-(tert-butyl)phenyl)-2-hydroxyacetyl)-2-(1-(3-chlorophenyl)cyclopropyl)-5,6,7,8-tetrahydropyrido[4,3-d]pyrimidin-4(3H)-one;
6-(2-(4'-fluoro-[1,1'-biphenyl]-3-yl)-2-hydroxyacetyl)-2-(1-phenylcyclopropyl)-5,6,7,8-tetrahydropyrido[4,3-dJpyrimidin-4(3H)-one;
6-(2-hydroxy-2-(4'-(trifluoromethyl)-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(4'-(trifluoromethoxy)-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(3'-(trifluoromethyl)-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(3'-(trifluoromethoxy)-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(3'-methyl-[1,1'-biphenyl]-3-yl)acetyl)-2-(1-phenylcyclopropyl)-5,6,7,8-tetrahydropyrido[4,3-d]pyrimidin-4(3H)-one;
6-(2-(3'-chloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(4'-(tert-butyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-2-(1-(4-cyclohexylthiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
6-(2-(3'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(quinolin-3-yl)phenyl)acetyl)-2-(1-phenylcyclopropyl)-5,6,7,8-tetrahydropyrido[4,3-d]pyrimidin-4(3H)-one;
6-(2-(3-(benzo[b]thiophen-2-yl)phenyl)-2-hydroxyacetyl)-2-(1-phenylcyclopropyl)-5,6,7,8-tetrahydropyrido[4,3-d]pyrimidin-4(3H)-one;
6-(2-(3-(5-chlorothiophen-3-yl)phenyl)-2-hydroxyacetyl)-2-(1-phenylcyclopropyl)-5,6,7,8-tetrahydropyrido[4,3-d]pyrimidin-4(3H)-one;
6-(2-hydroxy-2-(3-methyl-5-(trifluoromethyl)phenyl)acetyl)-2-(1-phenylcyclopropyl)-5,6,7,8-tetrahydropyrido[4,3-d]pyrimidin-4(3H)-one;
2-(1-phenylcyclopropyl)-6-(2-(3-(trifluoromethyl)phenyl)propanoyl)-3,5,6,7,8,9-hexahydro-4H-pyrimido[5,4-c]azepin-4-one;
6-(2-hydroxy-2-(3'-methoxy-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(4'-methoxy-[1,1'-biphenyl]-3-yl)acetyl)-2-(1-phenylcyclopropyl)-5,6,7,8-tetrahydropyrido[4,3-d]pyrimidin-4(3H)-one;
6-(2-(3'-(tert-butyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
3'-(1-hydroxy-2-oxo-2-(4-oxo-2-(1-phenylcyclopropyl)-3,5,7,8-tetrahydropyrido[4,3-d]pyrimidin-6(4H)-yl)ethyl)-[1,1'-biphenyl]-3-carbonitrile;
6-(2-(4'-ethoxy-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-hydroxy-2-(4'-isopropoxy-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(2'-(trifluoromethyl)-[1,1'-biphenyl]-3-yl)acetyl)-2-(1-phenylcyclopropyl)-5,6,7,8-tetrahydropyrido[4,3-d]pyrimidin-4(3H)-one;
6-(2-(3-(benzo[b]thiophen-3-yl)phenyl)-2-hydroxyacetyl)-2-(1-phenylcyclopropyl)-5,6,7,8-tetrahydropyrido[4,3-d]pyrimidin-4(3H)-one;
6-(2-(3-(5,6-dimethylpyridin-3-yl)phenyl)-2-hydroxyacetyl)-2-(1-phenylcyclopropyl)-5,6,7,8-tetrahydropyrido[4,3-d]pyrimidin-4(3H)-one;
6-(2-(3'-chloro-4'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3'-chloro-5'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(4'-(difluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3'-chloro-4'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3'-chloro-5'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(4'-chloro-3'-(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-isopropylpyridin-3-yl)cyclopropyl)-3,5,6,7,8,9-hexahydro-4H-pyrimido[5,4-c]azepin-4-one;
(R)-2-(1-(5-cyclohexylpyridin-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(5-bromopyridin-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(4-bromothiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(prop-1-en-2-yl)phenyl)cyclopropyl)-3,5,6,7,8,9-hexahydro-4H-pyrimido[5,4-c]azepin-4-one;
(R)-2-(1-(3-cyclohexylphenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
6-(2-(3-chlorophenyl)-2,2-difluoroacetyl)-2-(1-(thiophen-2-yl)cyclopropyl)-3,5,6,7,8,9-hexahydro-4H-pyrimido[5,4-c]azepin-4-one;
6-(2-(2-fluoro-3-(trifluoromethyl)phenyl)acetyl)-2-(1-phenylcyclopropyl)-3,5,6,7,8,9-hexahydro-4H-pyrimido[5,4-c]azepin-4-one;
6-(2,2-difluoro-2-(3-(trifluoromethyl)phenyl)acetyl)-2-(1-phenylcyclopropyl)-5,6,7,8-tetrahydropyrido[4,3-d]pyrimidin-4(3H)-one;
6-(2,2-difluoro-2-(3-(trifluoromethyl)phenyl)acetyl)-2-(1-phenylcyclopropyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(thiophen-2-yl)cyclopropyl)-3,5,6,7,8,9-hexahydro-4H-pyrimido[5,4-c]azepin-4-one;
6-(2-(2-fluoro-3-(trifluoromethyl)phenyl)-2-hydroxyacetyl)-2-(1-(3-fluorophenyl)cyclopropyl)-3,5,6,7,8,9-hexahydro-4H-pyrimido[5,4-c]azepin-4-one;
2-(1-(3-fluorophenyl)cyclopropyl)-6-(2-hydroxy-2-(3-((trifluoromethyl)thio)phenyl)acetyl)-3,5,6,7,8,9-hexahydro-4H-pyrimido[5,4-c]azepin-4-one;
2-(1-(3-fluorophenyl)cyclopropyl)-6-(2-hydroxy-2-(3-(pentafluoro-(lambda)⁶-sulfaneyl)phenyl)acetyl)-3,5,6,7,8,9-hexahydro-4H-pyrimido[5,4-c]azepin-4-one;
6-(2-(3-chlorophenyl)-2,2-difluoroacetyl)-2-(1-(3-chlorophenyl)cyclopropyl)-5,6,7,8-tetrahydropyrido[4,3-d]pyrimidin-4(3H)-one;
6-(2-(3-chlorophenyl)-2,2-difluoroacetyl)-2-(1-(3-fluorophenyl)cyclopropyl)-3,5,6,7,8,9-hexahydro-4H-pyrimido[5,4-c]azepin-4-one;
2-(1-(3-chlorophenyl)cyclopropyl)-6-(2,2-difluoro-2-(3-(trifluoromethyl)phenyl)acetyl)-5,6,7,8-tetrahydropyrido[4,3-d]pyrimidin-4(3H)-one;
6-(2,2-difluoro-2-(3-(trifluoromethyl)phenyl)acetyl)-2-(1-(3-fluorophenyl)cyclopropyl)-3,5,6,7,8,9-hexahydro-4H-pyrimido[5,4-c]azepin-4-one;
2-(1-(3-chlorophenyl)cyclopropyl)-6-(2-(3-cyclopropylphenyl)-2-hydroxyacetyl)-5,6,7,8-tetrahydropyrido[4,3-d]pyrimidin-4(3H)-one;
6-(2-(3-cyclopropylphenyl)-2-hydroxyacetyl)-2-(1-phenylcyclopropyl)-3,5,6,7,8,9-hexahydro-4H-pyrimido[5,4-c]azepin-4-one;
6-(2-(3-cyclopropylphenyl)-2-hydroxyacetyl)-2-(1-(thiophen-2-yl)cyclopropyl)-3,5,6,7,8,9-hexahydro-4H-pyrimido[5,4-c]azepin-4-one;
6-(2-(3-cyclopropylphenyl)-2-hydroxyacetyl)-2-(1-(3-fluorophenyl)cyclopropyl)-3,5,6,7,8,9-hexahydro-4H-pyrimido[5,4-c]azepin-4-one;
(R)-2-(1-(4-ethylthiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(4-cyclopentylthiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
2-(1-(3-chlorophenyl)cyclopropyl)-6-(2-(2-fluoro-3-(trifluoromethyl)phenyl)-2-hydroxyacetyl)-3,5,6,7,8,9-hexahydro-4H-pyrimido[5,4-c]azepin-4-one;
2-(1-(3-chlorophenyl)cyclopropyl)-6-(2-hydroxy-2-(3-((trifluoromethyl)thio)phenyl)acetyl)-3,5,6,7,8,9-hexahydro-4H-pyrimido[5,4-c]azepin-4-one;
2-(1-(3-chlorophenyl)cyclopropyl)-6-(2-hydroxy-2-(3-(pentafluoro-(lambda)⁶-sulfaneyl)phenyl)acetyl)-3,5,6,7,8,9-hexahydro-4H-pyrimido[5,4-c]azepin-4-one;
6-(2-(3-chlorophenyl)-2,2-difluoroacetyl)-2-(1-(3-chlorophenyl)cyclopropyl)-3,5,6,7,8,9-hexahydro-4H-pyrimido[5,4-c]azepin-4-one;
2-(1-(3-chlorophenyl)cyclopropyl)-6-(2,2-difluoro-2-(3-(trifluoromethyl)phenyl)acetyl)-3,5,6,7,8,9-hexahydro-4H-pyrimido[5,4-c]azepin-4-one;
(R)-2-(1-(4-(1-acetylpiperidin-4-yl)thiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-isopropylphenyl)cyclopropyl)-3,5,6,7,8,9-hexahydro-4H-pyrimido[5,4-c]azepin-4-one;
(R)-2-(1-(3-(4,4-difluorocyclohexyl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
6-(2-(6-chloropyridin-2-yl)-2-hydroxyacetyl)-2-(1-phenylcyclopropyl)-5,6,7,8-tetrahydropyrido[4,3-d]pyrimidin-4(3H)-one;
6-(2-hydroxy-2-(6-(3-(trifluoromethyl)phenyl)pyridin-2-yl)acetyl)-2-(1-phenylcyclopropyl)-5,6,7,8-tetrahydropyrido[4,3-d]pyrimidin-4(3H)-one;
6-(2-hydroxy-2-(2'-(trifluoromethyl)-[2,4'-bipyridin]-6-yl)acetyl)-2-(1-phenylcyclopropyl)-5,6,7,8-tetrahydropyrido[4,3-d]pyrimidin-4(3H)-one;
6-(2-(6-(3-chlorophenyl)pyridin-2-yl)-2-hydroxyacetyl)-2-(1-phenylcyclopropyl)-5,6,7,8-tetrahydropyrido[4,3-d]pyrimidin-4(3H)-one;
6-(2-hydroxy-2-(6-(3-(trifluoromethoxy)phenyl)pyridin-2-yl)acetyl)-2-(1-phenylcyclopropyl)-5,6,7,8-tetrahydropyrido[4,3-d]pyrimidin-4(3H)-one;
(R)-2-(1-(3-chlorophenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-(3-chlorophenyl)-2-hydroxyacetyl)-2-(1-(3-chlorophenyl)cyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(3-chlorophenyl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(piperidin-4-yl)thiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(1-methylpiperidin-4-yl)thiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(2-isopropylpyridin-4-yl)cyclopropyl)-3,5,6,7,8,9-hexahydro-4H-pyrimido[5,4-c]azepin-4-one;
2-(1-(3-chlorophenyl)cyclopropyl)-6-(2-(3-cyclopropylphenyl)-2-hydroxyacetyl)-3,5,6,7,8,9-hexahydro-4H-pyrimido[5,4-c]azepin-4-one;
6-(2-(2'-chloro-5'-(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3',5'-bis(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3'-((dimethylamino)methyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-2-(1-(3-acetylphenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-methoxyphenyl)cyclopropyl)-3,5,6,7,8,9-hexahydro-4H-pyrimido[5,4-c]azepin-4-one;
6-(1H-benzo[d]imidazole-2-carbonyl)-2-(1-(3-chlorophenyl)cyclopropyl)-3,5,6,7,8,9-hexahydro-4H-pyrimido[5,4-c]azepin-4-one;
6-(2-(6-(cyclohex-1-en-1-yl)pyridin-2-yl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(2,2,2-trifluoroethoxy)phenyl)acetyl)-2-(1-phenylcyclopropyl)-3,5,6,7,8,9-hexahydro-4H-pyrimido[5,4-c]azepin-4-one;
6-(2-hydroxy-2-(1'-methyl-1',2',3',6'-tetrahydro-[2,4'-bipyridin]-6-yl)acetyl)-2-(1-phenylcyclopropyl)-5,6,7,8-tetrahydropyrido[4,3-d] pyrimidin-4(3H)-one;
(R)-2-(1-(3-chlorophenyl)cyclopropyl)-6-(2-(2,3-difluorophenyl)-2-hydroxyacetyl)-3,5,6,7,8,9-hexahydro-4H-pyrimido[5,4-c]azepin-4-one;
6-(2-(6-cyclohexylpyridin-2-yl)-2-hydroxyacetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-hydroxy-2-(3-(2,2,2-trifluoroethoxy)phenyl)acetyl)-3,5,6,7,8,9-hexahydro-4H-pyrimido[5,4-c]azepin-4-one;
6-(2-(2'-chloro-3'-(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3'-chloro-5'-(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3',5'-dichloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(6-isopropylpyridin-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-phenylthiophen-2-yl)cyclopropyl)-3,5,6,7,8,9-hexahydro-4H-pyrimido[5,4-c]azepin-4-one;
2-(1-phenylcyclopropyl)-6-(2-(3-((trifluoromethyl)thio)phenyl)acetyl)-3,5,6,7,8,9-hexahydro-4H-pyrimido[5,4-c]azepin-4-one;
6-(2-(3-(pentafluoro-(lambda)⁶-sulfaneyl)phenyl)acetyl)-2-(1-phenylcyclopropyl)-3,5,6,7,8,9-hexahydro-4H-pyrimido[5,4-c]azepin-4-one;
6-(2-amino-2-(3-(trifluoromethyl)phenyl)acetyl)-2-(1-phenylcyclopropyl)-3,5,6,7,8,9-hexahydro-4H-pyrimido[5,4-c]azepin-4-one;
2-(1-(3-chlorophenyl)cyclopropyl)-6-(2-hydroxy-2-(2-(trifluoromethyl)phenyl)acetyl)-3,5,6,7,8,9-hexahydro-4H-pyrimido[5,4-c]azepin-4-one;
2-(1-(3-chlorophenyl)cyclopropyl)-6-(2-hydroxy-2-(4-(trifluoromethyl)phenyl)acetyl)-3,5,6,7,8,9-hexahydro-4H-pyrimido[5,4-c]azepin-4-one;
6-(2-methyl-2-(3-(trifluoromethyl)phenyl)propanoyl)-2-(1-phenylcyclopropyl)-3,5,6,7,8,9-hexahydro-4H-pyrimido[5,4-c]azepin-4-one;
6-(2-hydroxy-2-(3'-hydroxy-5'-(trifluoromethyl)-[1,1'-biphenyl]-3-yl)acetyl)-2-(1-phenylcyclopropyl)-5,6,7,8-tetrahydropyrido[4,3-d]pyrimidin-4(3H)-one;
6-(2-(3'-cyclopropyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-hydroxy-2-(3'-((trifluoromethyl)thio)-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(2'-(trifluoromethoxy)-[1,1'-biphenyl]-3-yl)acetyl)-2-(1-phenylcyclopropyl)-5,6,7,8-tetrahydropyrido[4,3-d]pyrimidin-4(3H)-one;
(R)-2-(1-([1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(thiazol-5-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(3-methoxypropyl)thiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-phenethylthiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
6-(2-(6-(3-(tert-butyl)phenyl)pyridin-2-yl)-2-hydroxyacetyl)-2-(1-phenylcyclopropyl)-5,6,7,8-tetrahydropyrido[4,3-d]pyrimidin-4(3H)-one;
6-(2-(6-(3-cyclopropylphenyl)pyridin-2-yl)-2-hydroxyacetyl)-2-(1-phenylcyclopropyl)-5,6,7,8-tetrahydropyrido[4,3-d]pyrimidin-4(3H)-one;
6-(2-(3-bromophenyl)-2-hydroxyacetyl)-2-(1-phenylcyclopropyl)-3,5,6,7,8,9-hexahydro-4H-pyrimido[5,4-c]azepin-4-one;
2-(1-phenylcyclopropyl)-6-(1-(3-(trifluoromethyl)phenyl)cyclopropane-1-carbonyl)-3,5,6,7,8,9-hexahydro-4H-pyrimido[5,4-c]azepin-4-one;
6-(2-hydroxy-2-(3-(trifluoromethyl)phenyl)propanoyl)-2-(1-phenylcyclopropyl)-3,5,6,7,8,9-hexahydro-4H-pyrimido[5,4-c]azepin-4-one;
6-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
6-(2-hydroxy-2-(3-(2-(trifluoromethyl)pyridin-4-yl)phenyl)acetyl)-2-(1-phenylcyclopropyl)-3,5,6,7,8,9-hexahydro-4H-pyrimido[5,4-c]azepin-4-one;
6-(2-hydroxy-2-(3'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3'-(tert-butyl)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
6-(2-(3'-chloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
6-(2-hydroxy-2-(4'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3'-fluoro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(2-(tetrahydro-2H-pyran-4-yl)ethyl)thiophen-2-yl)cyclopropyl)-3,5,6,7,8,9-hexahydro-4H-pyrimido[5,4-c]azepin-4-one;
(R)-2-(1-(4-(2-cyclopropylethyl)thiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(pyrimidin-5-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-methyl-1H-pyrazol-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(isothiazol-4-yl)phenyl)cyclopropyl)-3,5,6,7,8,9-hexahydro-4H-pyrimido[5,4-c]azepin-4-one;
6-((R)-2-hydroxy-2-(3-(trifluoromethyl)phenyl)acetyl)-2-(1-(4'-(trifluoromethyl)-2',3',4',5'-tetrahydro-[1,1'-biphenyl]-3-yl)cyclopropyl)-3,5,6,7,8,9-hexahydro-4H-pyrimido[5,4-c]azepin-4-one;
6-((R)-2-hydroxy-2-(3-(trifluoromethyl)phenyl)acetyl)-2-(1-(3-(1,1,1-trifluoropropan-2-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-isobutylphenyl)cyclopropyl)-3,5,6,7,8,9-hexahydro-4H-pyrimido[5,4-c]azepin-4-one;
(R)-2-(1-(3-(4,4-dimethylcyclohexyl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(3-hydroxy-3-methylbutyl)thiophen-2-yl)cyclopropyl)-3,5,6,7,8,9-hexahydro-4H-pyrimido[5,4-c]azepin-4-one;
(R)-2-(1-(4-(3,3-dimethylbutyl)thiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-N-benzyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)thiophene-3-carboxamide;
(R)-N-benzyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methylthiophene-3-carboxamide;
(R)-5-(1-(6-(2-hydroxy-2-(3-(trifluoromethyl)phenyl)acetyl)-4-oxo-4, 5,6,7,8,9-hexahydro-3H-pyrimido[5,4-c]azepin-2-yl)cyclopropyl)-N,N-dimethylthiophene-3-carboxamide;
1-(2-(1-(3-chlorophenyl)cyclopropyl)-4-oxo-3,5,7,8-tetrahydropyrido[4,3-d]pyrimidin-6(4H)-yl)-2-(3-(trifluoromethyl)phenyl)ethane-1,2-dione;
(R)-6-(2-hydroxy-2-(3-(trifluoromethyl)phenyl)acetyl)-2-(1-(3-(hydroxymethyl)phenyl)cyclopropyl)-3,5,6,7,8,9-hexahydro-4H-pyrimido[5,4-c]azepin-4-one;
6-(2-(3'-chloro-5'-fluoro-[1,1'-biphenyl]-3-yl)-2-hydroxyacetyl)-2-(1-phenylcyclopropyl)-3,5,6,7,8,9-hexahydro-4H-pyrimido[5,4-c] azepin-4-one;
6-(2-(3',5'-bis(trifluoromethyl)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
6-(2-(3',4'-difluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3',5'-difluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(2',5'-difluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3'-fluoro-[1,1'-biphenyl]-3-yl)-2-hydroxyacetyl)-2-(1-(3-fluorophenyl)cyclopropyl)-5,6,7,8-tetrahydropyrido[4,3-d]pyrimidin-4(3H)-one;
2-(1-(3-chlorophenyl)cyclopropyl)-6-(2-(3'-fluoro-[1,1'-biphenyl]-3-yl)-2-hydroxyacetyl)-5,6,7,8-tetrahydropyrido[4,3-d]pyrimidin-4(3H)-one;
6-(2-(3'-fluoro-[1,1'-biphenyl]-3-yl)-2-hydroxyacetyl)-2-(1-(thiophen-2-yl)cyclopropyl)-5,6,7,8-tetrahydropyrido[4,3-d]pyrimidin-4(3H)-one;
6-(2-(3'-fluoro-[1,1'-biphenyl]-3-yl)-2-hydroxyacetyl)-2-(1-(3-fluorophenyl)cyclopropyl)-3,5,6,7,8,9-hexahydro-4H-pyrimido[5,4-c]azepin-4-one;
2-(1-(3-chlorophenyl)cyclopropyl)-6-(2-(3'-fluoro-[1,1'-biphenyl]-3-yl)-2-hydroxyacetyl)-3,5,6,7,8,9-hexahydro-4H-pyrimido[5,4-c]azepin-4-one;
6-(2-hydroxy-2-(3-phenoxyphenyl)acetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-hydroxy-2-(3-phenoxyphenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(2-(oxetan-3-yl)ethyl)thiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(trifluoromethyl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(piperidin-1-ylmethyl)phenyl)cyclopropyl)-3,5,6,7,8,9-hexahydro-4H-pyrimido[5,4-c]azepin-4-one;
(R)-5-(3-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)phenyl)nicotinonitrile;
(R)-2-(1-(3-(6-(dimethylamino)pyridin-3-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N,N-dimethyl-[1,1'-biphenyl]-3-carboxamide;
6-(2-(3-chlorophenyl)-2,2-difluoroacetyl)-2-(1-(5-isopropylpyridin-3-yl)cyclopropyl)-3,5,6,7,8,9-hexahydro-4H-pyrimido[5,4-c]azepin-4-one;
6-(2-(3'-fluoro-4'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(3'-fluoro-5'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(5'-fluoro-2'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(2',3'-difluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(4'-chloro-3'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(2'-chloro-5'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-( [1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(2'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-hydroxy-2-(3-(5-(trifluoromethyl)pyridin-3-yl)phenyl)acetyl)-2-(1-phenylcyclopropyl)-5,6,7,8-tetrahydropyrido[4,3-d]pyrimidin-4(3H)-one;
6-(2-(2',4'-difluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(5-bromopyridin-3-yl)-2-hydroxyacetyl)-2-(1-phenylcyclopropyl)-5,6,7,8-tetrahydropyrido[4,3-d]pyrimidin-4(3H)-one;
6-(2-hydroxy-2-(5-(3-(trifluoromethyl)phenyl)pyridin-3-yl)acetyl)-2-(1-phenylcyclopropyl)-5,6,7,8-tetrahydropyrido[4,3-d]pyrimidin-4(3H)-one;
6-(2-(5-(3-chlorophenyl)pyridin-3-yl)-2-hydroxyacetyl)-2-(1-phenylcyclopropyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-(5-isopropylpyridin-3-yl)cyclopropyl)-3,5,6,7,8,9-hexahydro-4H-pyrimido[5,4-c]azepin-4-one;
2-(1-(4-(2-( (3S,5S)-adamantan-1-yl)ethyl)thiophen-2-yl)cyclopropyl)-6-((RS)-2-hydroxy-2-(3-(trifluoromethyl)phenyl)acetyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(5-isopropylpyridin-3-yl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-(5-isopropylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(trifluoromethoxy)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(2-methoxypyridin-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-methyl-6-oxo-1,6-dihydropyridin-3-yl)phenyl)cyclopropyl)-3,5,6,7,8,9-hexahydro-4H-pyrimido[5,4-c]azepin-4-one;
(R)-2-(1-(3'-((dimethylamino)methyl)-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
6-(2-(5-(3-fluorophenyl)pyridin-3-yl)-2-hydroxyacetyl)-2-(1-phenylcyclopropyl)-5,6,7,8-tetrahydropyrido[4,3-d]pyrimidin-4(3H)-one;
6-(2-hydroxy-2-(2'-(trifluoromethyl)-[3,4'-bipyridin]-5-yl)acetyl)-2-(1-phenylcyclopropyl)-5,6,7,8-tetrahydropyrido[4,3-d]pyrimidin-4(3H)-one;
6-(2-(6-(3-fluorophenyl)pyridin-2-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(1-(2,2,2-trifluoroethyl)piperidin-4-yl)phenyl)cyclopropyl)-3,5,6,7,8,9-hexahydro-4H-pyrimido[5,4-c]azepin-4-one;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-3-sulfonamide;
(R)-6-(2-hydroxy-2-(3-(trifluoromethyl)phenyl)acetyl)-2-(1-(4'-(4-hydroxytetrahydro-2H-pyran-4-yl)-[1,1'-biphenyl]-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-methyl-1H-pyrazol-5-yl)phenyl)cyclopropyl)-3,5,6,7,8,9-hexahydro-4H-pyrimido[5,4-c]azepin-4-one;
(R)-2-(1-(3-(1-cyclopropyl-1H-pyrazol-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(1-(difluoromethyl)-1H-pyrazol-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-methyl-1H-pyrazol-3-yl)phenyl)cyclopropyl)-3,5,6,7,8,9-hexahydro-4H-pyrimido[5,4-c]azepin-4-one;
(R)-2-(1-(3-(1-cyclobutyl-1H-pyrazol-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(2-methoxyethyl)-1H-pyrazol-4-yl)phenyl)cyclopropyl)-3,5,6,7,8,9-hexahydro-4H-pyrimido[5,4-c]azepin-4-one;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-3-carbonitrile;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N,N-dimethyl-[1,1'-biphenyl]-4-sulfonamide;
6-(2-(4'-chloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(oxazol-5-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3'-(methylsulfonyl)-[1,1'-biphenyl]-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(pyridin-4-yl)phenyl)cyclopropyl)-3,5,6,7,8,9-hexahydro-4H-pyrimido[5,4-c]azepin-4-one;
6-(2-hydroxy-2-(3'-(trifluoromethoxy)-[1,1'-biphenyl]-3-yl)acetyl)-2-(1-(5-isopropylpyridin-3-yl)cyclopropyl)-3,5,6,7,8,9-hexahydro-4H-pyrimido[5,4-c]azepin-4-one;
6-(2-hydroxy-2-(3'-(trifluoromethyl)-[1,1'-biphenyl]-3-yl)acetyl)-2-(1-(5-isopropylpyridin-3-yl)cyclopropyl)-3,5,6,7,8,9-hexahydro-4H-pyrimido[5,4-c]azepin-4-one;
6-(2-(3'-(tert-butyl)-[1,1'-biphenyl]-3-yl)-2-hydroxyacetyl)-2-(1-(5-isopropylpyridin-3-yl)cyclopropyl)-3,5,6,7,8,9-hexahydro-4H-pyrimido[5,4-c]azepin-4-one;
(R)-N-cyclohexyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methylthiophene-3-carboxamide;
(R)-N-benzyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-isopropylthiophene-3-carboxamide;
(R)-6-(2-hydroxy-2-(3-(trifluoromethyl)phenyl)acetyl)-2-(1-(4-(isoindoline-2-carbonyl)thiophen-2-yl)cyclopropyl)-3,5,6,7,8,9-hexahydro-4H-pyrimido[5,4-c]azepin-4-one;
6-(2-(4'-chloro-[1,1'-biphenyl]-3-yl)-2-hydroxyacetyl)-2-(1-(3-fluorophenyl)cyclopropyl)-5,6,7,8-tetrahydropyrido[4,3-d]pyrimidin-4(3H)-one;
6-(2-(4'-chloro-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
6-(2-(4'-chloro-[1,1'-biphenyl]-3-yl)-2-hydroxyacetyl)-2-(1-(thiophen-2-yl)cyclopropyl)-5,6,7,8-tetrahydropyrido[4,3-d]pyrimidin-4(3H)-one;
6-(2-(4'-chloro-[1,1'-biphenyl]-3-yl)-2-hydroxyacetyl)-2-(1-(3-fluorophenyl)cyclopropyl)-3,5,6,7,8,9-hexahydro-4H-pyrimido[5,4-c]azepin-4-one;
6-(2-(4'-chloro-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
6-(2-(4'-chloro-[1,1'-biphenyl]-3-yl)-2-hydroxyacetyl)-2-(1-(thiophen-2-yl)cyclopropyl)-3,5,6,7,8,9-hexahydro-4H-pyrimido[5,4-c] azepin-4-one;
6-(2-(4"-chloro-[1,1':4',1"-terphenyl]-3-yl)-2-hydroxyacetyl)-2-(1-phenylcyclopropyl)-5,6,7,8-tetrahydropyrido[4,3-d]pyrimidin-4(3H)-one;
6-(2-(2-fluoro-3'-(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(2,3'-difluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2-(2-fluoro-3'-(trifluoromethoxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(2-(methylamino)pyridin-4-yl)phenyl)cyclopropyl)-3,5,6,7,8,9-hexahydro-4H-pyrimido[5,4-c]azepin-4-one;
(R)-2-(1-(4'-(dimethylamino)-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(2-(pyrrolidin-1-yl)pyrimidin-5-yl)phenyl)cyclopropyl)-3,5,6,7,8,9-hexahydro-4H-pyrimido[5,4-c]azepin-4-one;
(R)-2-(1-(5-cyclohexylpyridin-3-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(3-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)phenyl)-3-methylbenzo[d]oxazol-2(3H)-one;
(R)-6-(2-hydroxy-2-(3-(trifluoromethyl)phenyl)acetyl)-2-(1-(3'-(piperidine-1-carbonyl)-[1,1'-biphenyl]-3-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(5-isopropylpyridin-3-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-isopropylpyridin-3-yl)cyclopropyl)-5,6,7,8-tetrahydropyrido[4,3-d]pyrimidin-4(3H)-one;
6-(2-hydroxy-2-(3'-(2-methoxyethoxy)-[1,1'-biphenyl]-3-yl)acetyl)-2-(1-phenylcyclopropyl)-5,6,7,8-tetrahydropyrido[4,3-d]pyrimidin-4(3H)-one;
6-(2-hydroxy-2-(3-(6-(trifluoromethyl)pyridin-2-yl)phenyl)acetyl)-2-(1-phenylcyclopropyl)-5,6,7,8-tetrahydropyrido[4,3-d]pyrimidin-4(3H)-one;
6-(2-(3'-(benzyloxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
2-(1-(3-fluorophenyl)cyclopropyl)-6-(2-hydroxy-2-(3'-(trifluoromethyl)-[1,1'-biphenyl]-3-yl)acetyl)-5,6,7,8-tetrahydropyrido[4,3-d]pyrimidin-4(3H)-one;
2-(1-(3-chlorophenyl)cyclopropyl)-6-(2-hydroxy-2-(3'-(trifluoromethyl)-[1,1'-biphenyl]-3-yl)acetyl)-5,6,7,8-tetrahydropyrido[4,3-d]pyrimidin-4(3H)-one;
6-(2-hydroxy-2-(3'-(trifluoromethyl)-[1,1'-biphenyl]-3-yl)acetyl)-2-(1-(thiophen-2-yl)cyclopropyl)-5,6,7,8-tetrahydropyrido[4,3-d]pyrimidin-4(3H)-one;
2-(1-(3-fluorophenyl)cyclopropyl)-6-(2-hydroxy-2-(3'-(trifluoromethyl)-[1,1'-biphenyl]-3-yl)acetyl)-3,5,6,7,8,9-hexahydro-4H-pyrimido[5,4-c]azepin-4-one;
2-(1-(3-chlorophenyl)cyclopropyl)-6-(2-hydroxy-2-(3'-(trifluoromethyl)-[1,1'-biphenyl]-3-yl)acetyl)-3,5,6,7,8,9-hexahydro-4H-pyrimido[5,4-c]azepin-4-one;
2-(1-(3-fluorophenyl)cyclopropyl)-6-(2-hydroxy-2-(3'-(trifluoromethoxy)-[1,1'-biphenyl]-3-yl)acetyl)-5,6,7,8-tetrahydropyrido[4,3-d]pyrimidin-4(3H)-one;
2-(1-(3-chlorophenyl)cyclopropyl)-6-(2-hydroxy-2-(3'-(trifluoromethoxy)-[1,1'-biphenyl]-3-yl)acetyl)-5,6,7,8-tetrahydropyrido[4,3-d]pyrimidin-4(3H)-one;
6-(2-hydroxy-2-(3'-(trifluoromethoxy)-[1,1'-biphenyl]-3-yl)acetyl)-2-(1-(thiophen-2-yl)cyclopropyl)-5,6,7,8-tetrahydropyrido[4,3-d]pyrimidin-4(3H)-one;
2-(1-(3-chlorophenyl)cyclopropyl)-6-(2-hydroxy-2-(3-phenoxyphenyl)acetyl)-5,6,7,8-tetrahydropyrido[4,3-d]pyrimidin-4(3H)-one;
6-(2-hydroxy-2-(3-phenoxyphenyl)acetyl)-2-(1-phenylcyclopropyl)-3,5,6,7,8,9-hexahydro-4H-pyrimido[5,4-c]azepin-4-one;
2-(1-(3-fluorophenyl)cyclopropyl)-6-(2-hydroxy-2-(3-phenoxyphenyl)acetyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-(5-phenylpyridin-3-yl)cyclopropyl)-3,5,6,7,8,9-hexahydro-4H-pyrimido[5,4-c]azepin-4-one;
6-(2-(2-fluoro-3-(trifluoromethyl)phenyl)-2-hydroxyacetyl)-2-(1-(5-phenylpyridin-3-yl)cyclopropyl)-3,5,6,7,8,9-hexahydro-4H-pyrimido[5,4-c]azepin-4-one;
6-(2-hydroxy-2-(3'-(trifluoromethoxy)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
6-(2-hydroxy-2-(3'-(trifluoromethyl)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
6-(2-(3'-(tert-butyl)-[1,1'-biphenyl]-3-yl)-2-hydroxyacetyl)-2-(1-(5-phenylpyridin-3-yl)cyclopropyl)-3,5,6,7,8,9-hexahydro-4H-pyrimido[5,4-c]azepin-4-one;
6-(2-(2-fluoro-3-(2-(trifluoromethyl)pyridin-4-yl)phenyl)-2-hydroxyacetyl)-2-(1-phenylcyclopropyl)-5,6,7,8-tetrahydropyrido[4,3-d]pyrimidin-4(3H)-one;
(R)-6-(2-(3-chlorophenyl)-2-hydroxyacetyl)-2-(1-(5-cyclohexylpyridin-3-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3'-(2-methoxyethoxy)-[1,1'-biphenyl]-3-yl)cyclopropyl)-3,5,6,7,8,9-hexahydro-4H-pyrimido[5,4-c]azepin-4-one;
2-(1-(3-fluorophenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
2-(1-(3-chlorophenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(6-(4-methylpiperidin-1-yl)pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(6-(4-methylpiperazin-1-yl)pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4'-((4-methylpiperazin-1-yl)methyl)-[1,1'-biphenyl]-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(6-methoxypyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(1-methylpiperidin-4-yl)-1H-pyrazol-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4'-(1-methylpiperidin-4-yl)-[1,1'-biphenyl]-3-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(5-cyclopentylpyridin-3-yl)cyclopropyl)-5,6,7,8-tetrahydropyrido[4,3-d]pyrimidin-4(3H)-one;
(R)-2-(1-(5-cyclopentylpyridin-3-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-2-(1-(3-cyclopentylphenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(3-isopropylphenyl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-(3-isopropylphenyl)cyclopropyl)-3,5,6,7,8,9-hexahydro-4H-pyrimido[5,4-c]azepin-4-one;
6-(2-(2-fluoro-3-(trifluoromethyl)phenyl)-2-hydroxyacetyl)-2-(1-(3-isopropylphenyl)cyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(3-isopropylphenyl)cyclopropyl)-3,5,6,7,8,9-hexahydro-4H-pyrimido[5,4-c]azepin-4-one;
6-(2-hydroxy-2-(3-(pentafluoro-(lambda)⁶-sulfaneyl)phenyl)acetyl)-2-(1-(3-isopropylphenyl)cyclopropyl)-3,5,6,7,8,9-hexahydro-4H-pyrimido[5,4-c]azepin-4-one;
6-(2-hydroxy-2-(3-((trifluoromethyl)thio)phenyl)acetyl)-2-(1-(3-isopropylphenyl)cyclopropyl)-3,5,6,7,8,9-hexahydro-4H-pyrimido[5,4-c]azepin-4-one;
6-(2-hydroxy-2-(3'-(trifluoromethoxy)-[1,1'-biphenyl]-3-yl)acetyl)-2-(1-(3-isopropylphenyl)cyclopropyl)-3,5,6,7,8,9-hexahydro-4H-pyrimido[5,4-c]azepin-4-one;
6-(2-hydroxy-2-(3'-(trifluoromethyl)-[1,1'-biphenyl]-3-yl)acetyl)-2-(1-(3-isopropylphenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-(thiazol-5-yl)thiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-phenylthiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-(pyridin-3-yl)thiophen-2-yl)cyclopropyl)-5,6,7,8-tetrahydropyrido[4,3-d]pyrimidin-4(3H)-one;
6-(2-hydroxy-2-(3-(2-(trifluoromethyl)pyridin-4-yl)phenyl)acetyl)-2-(1-(3-isopropylphenyl)cyclopropyl)-3,5,6,7,8,9-hexahydro-4H-pyrimido[5,4-c]azepin-4-one;
(R)-2-(1-([3,3'-bipyridin]-5-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-(isothiazol-4-yl)pyridin-3-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-phenylpyridin-3-yl)cyclopropyl)-5,6,7,8-tetrahydropyrido[4,3-d]pyrimidin-4(3H)-one;
6-(2-hydroxy-2-(2-phenoxyphenyl)acetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-hydroxy-2-(2-phenoxyphenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(2-phenylacetyl)piperidin-4-yl)phenyl)cyclopropyl)-3,5,6,7,8,9-hexahydro-4H-pyrimido[5,4-c]azepin-4-one;
(R)-2-(1-(3-(1-(3,3-dimethylbutanoyl)piperidin-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(1-benzoylpiperidin-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(3-phenylpropanoyl)piperidin-4-yl)phenyl)cyclopropyl)-3,5,6,7,8,9-hexahydro-4H-pyrimido[5,4-c]azepin-4-one;
2-(1-(3-chlorophenyl)cyclopropyl)-6-(2-hydroxy-2-(4-phenoxyphenyl)acetyl)-3,5,6,7,8,9-hexahydro-4H-pyrimido[5,4-c]azepin-4-one;
6-(2-(3-(benzyloxy)phenyl)-2-hydroxyacetyl)-2-(1-phenylcyclopropyl)-5,6,7,8-tetrahydropyrido[4,3-d]pyrimidin-4(3H)-one;
6-(2-(3-(benzyloxy)phenyl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-phenylthiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-(pyridin-3-yl)thiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-(isothiazol-4-yl)thiophen-2-yl)cyclopropyl)-3,5,6,7,8,9-hexahydro-4H-pyrimido[5,4-c]azepin-4-one;
(R)-2-(1-(3-bromophenyl)cyclopropyl)-6-(2-(3-chlorophenyl)-2-hydroxyacetyl)-3,5,6,7,8,9-hexahydro-4H-pyrimido[5,4-c]azepin-4-one;
(R)-2-(1-([1,1'-biphenyl]-3-yl)cyclopropyl)-6-(2-(3-chlorophenyl)-2-hydroxyacetyl)-3,5,6,7,8,9-hexahydro-4H-pyrimido[5,4-c]azepin-4-one;
(R)-6-(2-(3-chlorophenyl)-2-hydroxyacetyl)-2-(1-(3-(isothiazol-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(pyridin-3-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(2',3',4',5'-tetrahydro-[1,1'-biphenyl]-3-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(prop-1-en-2-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(5-(tetrahydro-2H-pyran-4-yl)pyridin-3-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-(tetrahydro-2H-pyran-4-yl)pyridin-3-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(tetrahydro-2H-pyran-4-yl)phenyl)cyclopropyl)-3,5,6,7,8,9-hexahydro-4H-pyrimido[5,4-c]azepin-4-one;
(R)-2-(1-([3,3'-bipyridin]-5-yl)cyclopropyl)-6-(2-(3-chlorophenyl)-2-hydroxyacetyl)-5,6,7,8-tetrahydropyrido[4,3-d]pyrimidin-4(3H)-one;
(R)-6-(2-(3-chlorophenyl)-2-hydroxyacetyl)-2-(1-(5-(isothiazol-4-yl)pyridin-3-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(6-(methylamino)pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(6-(pyrrolidin-1-yl)pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4'-(pyrrolidin-1-ylmethyl)-[1,1'-biphenyl]-3-yl)cyclopropyl)-3,5,6,7,8,9-hexahydro-4H-pyrimido[5,4-c]azepin-4-one;
6-(2-hydroxy-2-(3-(3-(trifluoromethyl)phenoxy)phenyl)acetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-hydroxy-2-(3-(3-(trifluoromethyl)phenoxy)phenyl)acetyl)-3,5,6,7,8,9-hexahydro-4H-pyrimido[5,4-c]azepin-4-one;
(R)-6-(2-(3-chlorophenyl)-2-hydroxyacetyl)-2-(1-(4-(4,4-difluorocyclohexyl)thiophen-2-yl)cyclopropyl)-5,6,7,8-tetrahydropyrido[4,3-d]pyrimidin-4(3H)-one;
(R)-6 - (2-(3-chlorophenyl)-2-hydroxyacetyl)-2-(1-(4-cyclopentylthiophen-2-yl)cyclopropyl)-5,6,7,8-tetrahydropyrido[4,3-d]pyrimidin-4(3H)-one;
(R)-2-(1-(4-(4,4-difluorocyclohexyl)thiophen-2-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-(tetrahydro-2H-pyran-4-yl)thiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-(pyridin-3-yl)thiophen-2-yl)cyclopropyl)-3,5,6,7,8,9-hexahydro-4H-pyrimido[5,4-c]azepin-4-one;
(R)-2-(1-(4-(4,4-difluorocyclohexyl)thiophen-2-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(tetrahydro-2H-pyran-4-yl)thiophen-2-yl)cyclopropyl)-3,5,6,7,8,9-hexahydro-4H-pyrimido[5,4-c]azepin-4-one;
6-(2-(3-chlorophenyl)-2,2-difluoroacetyl)-2-(1-(5-phenylpyridin-3-yl)cyclopropyl)-3,5,6,7,8,9-hexahydro-4H-pyrimido[5,4-c]azepin-4-one;
6-(2-(3-chlorophenyl)-2,2-difluoroacetyl)-2-(1-(3-isopropylphenyl)cyclopropyl)-3,5,6,7,8,9-hexahydro-4H-pyrimido[5,4-c]azepin-4-one;
(R)-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methyl-N-(tetrahydro-2H-pyran-4-yl)thiophene-3-carboxamide;
(R)-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-isopropyl-N-methylthiophene-3-carboxamide;
(R)-6-(2-hydroxy-2-(3-(trifluoromethyl)phenyl)acetyl)-2-(1-(4-(piperidine-1-carbonyl)thiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(2-(1-hydroxycyclohexyl)ethyl)thiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2 -(1-(3-isopropylphenyl)cyclopropyl)-3,5,6,7,8,9-hexahydro-4H-pyrimido[5,4-c]azepin-4-one;
2-(1-(3-chlorophenyl)cyclopropyl)-6-(2-hydroxy-2-(3-(quinolin-3-yl)phenyl)acetyl)-5,6,7,8-tetrahydropyrido[4,3-d]pyrimidin-4(3H)-one;
6-(2-hydroxy-2-(3-(quinolin-3-yl)phenyl)acetyl)-2-(1-phenylcyclopropyl)-3,5,6,7,8,9-hexahydro-4H-pyrimido[5,4-c]azepin-4-one;
2-(1-(3-chlorophenyl)cyclopropyl)-6-(2-hydroxy-2-(3-(quinolin-3-yl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-methyl-1H-indazol-5-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-methyl-1H-indazol-4-yl)phenyl)cyclopropyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(benzo[b]thiophen-2-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-(3-chlorophenyl)-2-hydroxyacetyl)-2-(1-(5-(4,4-difluorocyclohexyl)pyridin-3-yl)cyclopropyl)-5,6,7,8-tetrahydropyrido[4,3-d]pyrimidin-4(3H)-one;
(R)-2-(1-(5-(4,4-difluorocyclohexyl)pyridin-3-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(2-(3'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3'-(trifluoromethyl)-[1,1'-biphenyl]-3-yl)acetyl)-2-(1-phenylcyclopropyl)-5,6,7,8-tetrahydropyrido[4,3-d]pyrimidin-4(3H)-one;
(R)-6-(2-hydroxy-2-(3'-(trifluoromethoxy)-[1,1'-biphenyl]-3-yl)acetyl)-2-(1-phenylcyclopropyl)-5,6,7,8-tetrahydropyrido[4,3-d]pyrimidin-4(3H)-one;
(R)-6-(2-(3'-chloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3'-(tert-butyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(S)-6-(2-(3'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(S)-6-(2-hydroxy-2-(3'-(trifluoromethyl)-[1,1'-biphenyl]-3-yl)acetyl)-2-(1-phenylcyclopropyl)-5,6,7,8-tetrahydropyrido[4,3-d]pyrimidin-4(3H)-one;
(R)-6-(2-(3'-chloro-5'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(6-(trifluoromethyl)pyridin-2-yl)phenyl)acetyl)-2-(1-phenylcyclopropyl)-5,6,7,8-tetrahydropyrido[4,3-d]pyrimidin-4(3H)-one;
(R)-6-(2-(3'-fluoro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-hydroxy-2-(3'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-phenylcyclopropyl)-3,5,6,7,8,9-hexahydro-4H-pyrimido[5,4-c] azepin-4-one;
(R)-6-(2-(3-chlorophenyl)-2-hydroxyacetyl)-2-(1-(3-cyclopentylphenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-(pyridin-3-yl)phenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-(isothiazol-4-yl)phenyl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-isopropylphenyl)cyclopropyl)-5,6,7,8-tetrahydropyrido[4,3-d]pyrimidin-4(3H)-one;
(R)-2-(1-(3-cyclopentylphenyl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(tetrahydro-2H-pyran-4-yl)phenyl)cyclopropyl)-5,6,7,8-tetrahydropyrido[4,3-d]pyrimidin-4(3H)-one;
(R)-2-(1-([1,1'-biphenyl]-3-yl)cyclopropyl)-6-(2-hydroxy-2-(3-(trifluoromethyl)phenyl)acetyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(pyridin-3-yl)phenyl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(isothiazol-4-yl)phenyl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-phenoxyphenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-phenoxyphenyl)cyclopropyl)-3,5,6,7,8,9-hexahydro-4H-pyrimido[5,4-c]azepin-4-one;
6-(2-(3-(tert-butyl)phenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
6-(2-(2-fluoro-3-(trifluoromethyl)phenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
6-(2-(3-chlorophenyl)-2,2-difluoroacetyl)-2-(1-(4-isopropylthiophen-2-yl)cyclopropyl)-3,5,6,7,8,9-hexahydro-4H-pyrimido[5,4-c]azepin-4-one;
6-(2-hydroxy-2-(3-(pentafluoro-(lambda)⁶-sulfaneyl)phenyl)acetyl)-2-(1-(4-isopropylthiophen-2-yl)cyclopropyl)-3,5,6,7,8,9-hexahydro-4H-pyrimido[5,4-c]azepin-4-one;
6-(2-hydroxy-2-(3-((trifluoromethyl)thio)phenyl)acetyl)-2-(1-(4-isopropylthiophen-2-yl)cyclopropyl)-3,5,6,7,8,9-hexahydro-4H-pyrimido[5,4-c]azepin-4-one;
6-(2-hydroxy-2-(3'-(trifluoromethoxy)-[1,1'-biphenyl]-3-yl)acetyl)-2-(1-(4-isopropylthiophen-2-yl)cyclopropyl)-3,5,6,7,8,9-hexahydro-4H-pyrimido[5,4-c]azepin-4-one;
6-(2-hydroxy-2-(3'-(trifluoromethyl)-[1,1'-biphenyl]-3-yl)acetyl)-2-(1-(4-isopropylthiophen-2-yl)cyclopropyl)-3,5,6,7,8,9-hexahydro-4H-pyrimido[5,4-c]azepin-4-one;
6-(2-hydroxy-2-(4-phenoxyphenyl)acetyl)-2-(1-phenylcyclopropyl)-5,6,7,8-tetrahydropyrido[4,3-d]pyrimidin-4(3H)-one;
(R)-6-(2-hydroxy-2-(3-(2-(trifluoromethyl)pyridin-4-yl)phenyl)acetyl)-2-(1-phenylcyclopropyl)-5,6,7,8-tetrahydropyrido[4,3-d]pyrimidin-4(3H)-one;
(S)-6-(2-hydroxy-2-(3-(2-(trifluoromethyl)pyridin-4-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(quinolin-3-yl)phenyl)acetyl)-2-(1-phenylcyclopropyl)-5,6,7,8-tetrahydropyrido[4,3-d]pyrimidin-4(3H)-one;
(R)-6-(2-(3'-chloro-4'-fluoro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(2-(trifluoromethyl)pyridin-4-yl)phenyl)acetyl)-2-(1-phenylcyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(2-phenoxypyridin-4-yl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(4-isopropylthiophen-2-yl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(4-isopropylthiophen-2-yl)cyclopropyl)-3,5,6,7,8,9-hexahydro-4H-pyrimido[5,4-c]azepin-4-one;
(R)-6-(2-hydroxy-2-(3-(2-(trifluoromethyl)pyridin-4-yl)phenyl)acetyl)-2-(1-(4-isopropylthiophen-2-yl)cyclopropyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(4-isopropylthiophen-2-yl)cyclopropyl)-5,6,7,8-tetrahydropyrido[4,3-d]pyrimidin-4(3H)-one;
6-(2-(3-(tert-butyl)phenyl)-2-hydroxyacetyl)-2-(1-(4-isopropylthiophen-2-yl)cyclopropyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-(4-isopropylthiophen-2-yl)cyclopropyl)-5,6,7,8-tetrahydropyrido[4,3-d]pyrimidin-4(3H)-one;
6-(2-(3-chloro-2-fluorophenyl)-2-hydroxyacetyl)-2-(1-(4-isopropylthiophen-2-yl)cyclopropyl)-5,6,7,8-tetrahydropyrido[4,3-d]pyrimidin-4(3H)-one;
6-(2-(3-chlorophenyl)-2,2-difluoroacetyl)-2-(1-(4-isopropylthiophen-2-yl)cyclopropyl)-5,6,7,8-tetrahydropyrido[4,3-d]pyrimidin-4(3H)-one;
6-(2-hydroxy-2-(3-(pentafluoro-(lambda)⁶-sulfaneyl)phenyl)acetyl)-2-(1-(4-isopropylthiophen-2-yl)cyclopropyl)-5,6,7,8-tetrahydropyrido[4,3-d]pyrimidin-4(3H)-one;
(S)-6-(2-(3'-fluoro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(S)-6-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-(4'-chloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(4'-chloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(3-(5-(trifluoromethyl)pyridin-3-yl)phenyl)acetyl)-2-(1-phenylcyclopropyl)-3,5,6,7,8,9-hexahydro-4H-pyrimido[5,4-c]azepin-4-one;
(R)-6-(2-hydroxy-2-(3-(6-(trifluoromethyl)pyridin-2-yl)phenyl)acetyl)-2-(1-phenylcyclopropyl)-3,5,6,7,8,9-hexahydro-4H-pyrimido[5,4-c]azepin-4-one;
(R)-6-(2-hydroxy-2-(3-(quinolin-6-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(isoquinolin-6-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(quinolin-7-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(quinolin-4-yl)phenyl)acetyl)-2-(1-phenylcyclopropyl)-5,6,7,8-tetrahydropyrido[4,3-d]pyrimidin-4(3H)-one;
(R)-6-(2-(3-(benzofuran-2-yl)phenyl)-2-hydroxyacetyl)-2-(1-phenylcyclopropyl)-5,6,7,8-tetrahydropyrido[4,3-d]pyrimidin-4(3H)-one;
(R)-6-(2-(3-(2,2-difluorobenzo[d][1,3]dioxol-5-yl)phenyl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3-(5-(trifluoromethyl)pyridin-3-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(4'-(pyridin-2-ylmethoxy)-[1,1'-biphenyl]-3-yl)acetyl)-2-(1-phenylcyclopropyl)-5,6,7,8-tetrahydropyrido[4,3-d]pyrimidin-4(3H)-one;
(R)-6-(2-(3-bromophenyl)-2-hydroxyacetyl)-2-(1-phenylcyclopropyl)-3,5, 6, 7, 8, 9-hexahydro-4H-pyrimido[5,4-c]azepin-4-one;
(R)-6-(2-(3-chlorophenyl)-2-hydroxyacetyl)-2-(1-(3-cyclohexylphenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-cyclopentylphenyl)cyclopropyl)-3,5,6,7,8,9-hexahydro-4H-pyrimido[5,4-c]azepin-4-one;
(R)-6-(2-hydroxy-2-(3'-(trifluoromethoxy)-[1,1'-biphenyl]-3-yl)acetyl)-2-(1-(4-isopropylthiophen-2-yl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-(4-isopropylthiophen-2-yl)cyclopropyl)-3,5,6,7,8,9-hexahydro-4H-pyrimido[5,4-c]azepin-4-one;
(R)-6-(2-(3'-fluoro-[1,1'-biphenyl]-3-yl)-2-hydroxyacetyl)-2-(1-(4-isopropylthiophen-2-yl)cyclopropyl)-3,5,6,7,8,9-hexahydro-4H-pyrimido[5,4-c]azepin-4-one;
(R)-6-(2-(3-(6-(benzyloxy)pyridin-3-yl)phenyl)-2-hydroxyacetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-hydroxy-2-(3-(3-(trifluoromethyl)phenoxy)phenyl)acetyl)-5,6,7,8-tetrahydropyrido[4,3-d]pyrimidin-4(3H)-one;
6-(2-hydroxy-2-(3-(3-(trifluoromethyl)phenoxy)phenyl)acetyl)-2-(1-phenylcyclopropyl)-3,5,6,7,8,9-hexahydro-4H-pyrimido[5,4-c]azepin-4-one;
6-(2-(3-(benzyloxy)phenyl)-2-hydroxyacetyl)-2-(1-phenylcyclopropyl)-3,5,6,7,8,9-hexahydro-4H-pyrimido[5,4-c]azepin-4-one;
(R)-5-(3-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)phenyl)-N,N-dimethylnicotinamide;
(R)-6-(2-hydroxy-2-(3-(trifluoromethyl)phenyl)acetyl)-2-(1-(3-(1-(1-(trifluoromethyl)cyclopropane-1-carbonyl)-1,2,3,6-tetrahydropyridin-4-yl)phenyl)cyclopropyl)-3,5,6,7,8,9-hexahydro-4H-pyrimido[5,4-c]azepin-4-one;
(R)-6-(2-(3'-fluoro-[1,1'-biphenyl]-3-yl)-2-hydroxyacetyl)-2-(1-(3-isopropylphenyl)cyclopropyl)-5,6,7,8-tetrahydropyrido[4,3-d]pyrimidin-4(3H)-one;
(R)-6-(2-hydroxy-2-(3'-(trifluoromethyl)-[1,1'-biphenyl]-3-yl)acetyl)-2-(1-(3-isopropylphenyl)cyclopropyl)-5,6,7,8-tetrahydropyrido[4,3-d]pyrimidin-4(3H)-one;
(R)-6-(2-hydroxy-2-(3'-(trifluoromethoxy)-[1,1'-biphenyl]-3-yl)acetyl)-2-(1-(3-isopropylphenyl)cyclopropyl)-5,6,7,8-tetrahydropyrido[4,3-d]pyrimidin-4(3H)-one;
(R)-2-(1-(4'-(tert-butyl)-[1,1'-biphenyl]-3-yl)cyclopropyl)-6-(2-(3-chlorophenyl)-2-hydroxyacetyl)-3,5,6,7,8,9-hexahydro-4H-pyrimido[5,4-c]azepin-4-one;
(R)-6-(2-(3-chlorophenyl)-2-hydroxyacetyl)-2-(1-(4'-fluoro-[1,1'-biphenyl]-3-yl)cyclopropyl)-3,5,6,7,8,9-hexahydro-4H-pyrimido[5,4-c]azepin-4-one;
(R)-2-(1-(4'-chloro-[1,1'-biphenyl]-3-yl)cyclopropyl)-6-(2-(3-chlorophenyl)-2-hydroxyacetyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-cyclopropyl-1H-pyrazol-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(2-methoxypyridin-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4'-(trifluoromethyl)-[1,1'-biphenyl]-3-yl)cyclopropyl)-3,5,6,7,8,9-hexahydro-4H-pyrimido[5,4-c]azepin-4-one;
8-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[4,5-c]azepin-4-one;
(R)-6-(2-(3-chlorophenyl)-2-hydroxyacetyl)-2-(1-(4-cyclohexylthiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-(tetrahydro-2H-pyran-4-yl)thiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-cyclopentylthiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
6-(2-(3-(benzyloxy)phenyl)-2-hydroxyacetyl)-2-(1-(3-chlorophenyl)cyclopropyl)-5,6,7,8-tetrahydropyrido[4,3-d]pyrimidin-4(3H)-one;
(R)-2-(1-(3'-chloro-[1,1'-biphenyl]-3-yl)cyclopropyl)-6-(2-(3-chlorophenyl)-2-hydroxyacetyl)-3,5,6,7,8,9-hexahydro-4H-pyrimido[5,4-c]azepin-4-one;
(R)-6-(2-(3-chlorophenyl)-2-hydroxyacetyl)-2-(1-(3'-(2-methoxyethoxy)-[1,1'-biphenyl]-3-yl)cyclopropyl)-3,5,6,7,8,9-hexahydro-4H-pyrimido[5,4-c]azepin-4-one;
(R)-3'-(1-(6-(2-(3-chlorophenyl)-2-hydroxyacetyl)-4-oxo-4,5,6,7,8,9-hexahydro-3H-pyrimido[5,4-c]azepin-2-yl)cyclopropyl)-[1,1'-biphenyl]-3-carbonitrile;
2-(1-(3-chlorophenyl)cyclopropyl)-6-(2-(3-(trifluoromethyl)phenyl)propanoyl)-3,5,6,7,8,9-hexahydro-4H-pyrimido[5,4-c]azepin-4-one;
2-(1-(3-fluorophenyl)cyclopropyl)-6-(2-(3-(trifluoromethyl)phenyl)propanoyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(6-(4-isobutyrylpiperazin-1-yl)pyridin-3-yl)phenyl)cyclopropyl)-3,5,6,7,8,9-hexahydro-4H-pyrimido[5,4-c]azepin-4-one;
(R)-2-(1-(3-(6-(4-(3,3-dimethylbutanoyl)piperazin-1-yl)pyridin-3-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(6-(4-(2-phenylacetyl)piperazin-1-yl)pyridin-3-yl)phenyl)cyclopropyl)-3,5,6,7,8,9-hexahydro-4H-pyrimido[5,4-c]azepin-4-one;
(R)-5-(3-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)phenyl)picolinonitrile;
(R)-2-(1-(4'-((dimethylamino)methyl)-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4'-(pyridin-2-ylmethoxy)-[1,1'-biphenyl]-3-yl)cyclopropyl)-3,5, 6, 7, 8, 9-hexahydro-4H-pyrimido[5,4-c]azepin-4-one;
(R)-6-(2-hydroxy-2-(4'-(trifluoromethoxy)-[1,1'-biphenyl]-3-yl)acetyl)-2-(1-(5-isopropylpyridin-3-yl)cyclopropyl)-5,6,7,8-tetrahydropyrido[4,3-d]pyrimidin-4(3H)-one;
(R)-6-(2-hydroxy-2-(4'-(trifluoromethyl)-[1,1'-biphenyl]-3-yl)acetyl)-2-(1-(5-isopropylpyridin-3-yl)cyclopropyl)-5,6,7,8-tetrahydropyrido[4,3-d]pyrimidin-4(3H)-one;
(R)-6-(2-(4'-(tert-butyl)-[1,1'-biphenyl]-3-yl)-2-hydroxyacetyl)-2-(1-(5-isopropylpyridin-3-yl)cyclopropyl)-5,6,7,8-tetrahydropyrido[4,3-d]pyrimidin-4(3H)-one;
(R)-6-(2-(4'-fluoro-[1,1'-biphenyl]-3-yl)-2-hydroxyacetyl)-2-(1-(5-isopropylpyridin-3-yl)cyclopropyl)-5,6,7,8-tetrahydropyrido[4,3-d]pyrimidin-4(3H)-one;
(R)-6-(2-(4'-chloro-[1,1'-biphenyl]-3-yl)-2-hydroxyacetyl)-2-(1-(5-isopropylpyridin-3-yl)cyclopropyl)-5,6,7,8-tetrahydropyrido[4,3-d]pyrimidin-4(3H)-one;
(R)-6-(2-hydroxy-2-(3-(6-(trifluoromethyl)pyridin-3-yl)phenyl)acetyl)-2-(1-(5-isopropylpyridin-3-yl)cyclopropyl)-5,6,7,8-tetrahydropyrido[4,3-d]pyrimidin-4(3H)-one;
(R)-6-(2-hydroxy-2-(3'-(trifluoromethoxy)-[1,1'-biphenyl]-3-yl)acetyl)-2-(1-(5-isopropylpyridin-3-yl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-(5-isopropylpyridin-3-yl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(5-isopropylpyridin-3-yl)cyclopropyl)-3,5,6,7,8,9-hexahydro-4H-pyrimido[5,4-c]azepin-4-one;
(R)-6-(2-(3'-fluoro-[1,1'-biphenyl]-3-yl)-2-hydroxyacetyl)-2-(1-(5-isopropylpyridin-3-yl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(5-isopropylpyridin-3-yl)cyclopropyl)-3,5,6,7,8,9-hexahydro-4H-pyrimido[5,4-c]azepin-4-one;
(R)-6-(2-hydroxy-2-(3-(2-(trifluoromethyl)pyridin-4-yl)phenyl)acetyl)-2-(1-(5-isopropylpyridin-3-yl)cyclopropyl)-3,5,6,7,8,9-hexahydro-4H-pyrimido[5,4-c]azepin-4-one;
6-(2-(2-(benzyloxy)phenyl)acetyl)-2-(1-(3-chlorophenyl)cyclopropyl)-3,5,6,7,8,9-hexahydro-4H-pyrimido[5,4-c]azepin-4-one;
6-(2-(4-(benzyloxy)phenyl)acetyl)-2-(1-(3-chlorophenyl)cyclopropyl)-3,5,6,7,8,9-hexahydro-4H-pyrimido[5,4-c]azepin-4-one;
6-(2-([1,1'-biphenyl]-3-yl)acetyl)-2-(1-(3-chlorophenyl)cyclopropyl)-3,5,6,7,8,9-hexahydro-4H-pyrimido[5,4-c] azepin-4-one;
6-(2,2-difluoro-2-(3-(trifluoromethyl)phenyl)acetyl)-2-(1-(3-isopropylphenyl)cyclopropyl)-5,6,7,8-tetrahydropyrido[4,3-d] pyrimidin-4(3H)-one;
6-(2-(3-(tert-butyl)phenyl)-2-hydroxyacetyl)-2-(1-(3-isopropylphenyl)cyclopropyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-(3-isopropylphenyl)cyclopropyl)-5,6,7,8-tetrahydropyrido[4,3-d]pyrimidin-4(3H)-one;
6-(2-(3-chloro-2-fluorophenyl)-2-hydroxyacetyl)-2-(1-(3-isopropylphenyl)cyclopropyl)-5,6,7,8-tetrahydropyrido[4,3-d]pyrimidin-4(3H)-one;
6-(2-(3-chlorophenyl)-2,2-difluoroacetyl)-2-(1-(3-isopropylphenyl)cyclopropyl)-5,6,7,8-tetrahydropyrido[4,3-d]pyrimidin-4(3H)-one;
6-(2-(2-fluoro-3-(trifluoromethyl)phenyl)-2-hydroxyacetyl)-2-(1-(5-phenylpyridin-3-yl)cyclopropyl)-5,6,7,8-tetrahydropyrido[4,3-d]pyrimidin-4(3H)-one;
6-(2-(3-chlorophenyl)-2,2-difluoroacetyl)-2-(1-(5-phenylpyridin-3-yl)cyclopropyl)-5,6,7,8-tetrahydropyrido[4,3-d]pyrimidin-4(3H)-one;
6-(2,2-difluoro-2-(3-(trifluoromethyl)phenyl)acetyl)-2-(1-(5-isopropylpyridin-3-yl)cyclopropyl)-5,6,7,8-tetrahydropyrido[4,3-d]pyrimidin-4(3H)-one;
6-(2-(3-chlorophenyl)-2,2-difluoroacetyl)-2-(1-(5-isopropylpyridin-3-yl)cyclopropyl)-5,6,7,8-tetrahydropyrido[4,3-d]pyrimidin-4(3H)-one;
(R)-6-(2-(3'-chloro-[1,1'-biphenyl]-3-yl)-2-hydroxyacetyl)-2-(1-(3-isopropylphenyl)cyclopropyl)-5,6,7,8-tetrahydropyrido[4,3-d]pyrimidin-4(3H)-one;
(R)-6-(2-(3'-(tert-butyl)-[1,1'-biphenyl]-3-yl)-2-hydroxyacetyl)-2-(1-(3-isopropylphenyl)cyclopropyl)-5,6,7,8-tetrahydropyrido[4,3-d]pyrimidin-4(3H)-one;
(R)-6-(2-hydroxy-2-(3-(2-(trifluoromethyl)pyridin-4-yl)phenyl)acetyl)-2-(1-(3-isopropylphenyl)cyclopropyl)-5,6,7,8-tetrahydropyrido[4,3-d]pyrimidin-4(3H)-one;
6-(2,2-difluoro-2-(3-(trifluoromethyl)phenyl)acetyl)-2-(1-(5-phenylpyridin-3-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(1-methyl-1H-indazol-4-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(1-methyl-1H-indazol-4-yl)cyclopropyl)-3,5,6,7,8,9-hexahydro-4H-pyrimido[5,4-c]azepin-4-one;
(S)-6-(2-hydroxy-2-(3-(2-(trifluoromethyl)pyridin-4-yl) phenyl)acetyl)-2-(1-phenylcyclopropyl)-3,5,6,7,8,9-hexahydro-4H-pyrimido[5,4-c]azepin-4-one;
6-(2-([1,1'-biphenyl]-2-yl)acetyl)-2-(1-(3-chlorophenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-methyl-1H-indazol-4-yl)phenyl)cyclopropyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-(3-chlorophenyl)-2-hydroxyacetyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-methyl-1H-indazol-5-yl)phenyl)cyclopropyl)-3,5,6,7,8,9-hexahydro-4H-pyrimido[5,4-c]azepin-4-one;
(R)-1-(3'-(1-(6-(2-(3-chlorophenyl)-2-hydroxyacetyl)-4-oxo-4,5,6,7,8,9-hexahydro-3H-pyrimido[5,4-c]azepin-2-yl)cyclopropyl)-[1,1'-biphenyl]-4-yl)cyclopropane-1-carbonitrile;
(R)-3'-(1-(6-(2-(3-chlorophenyl)-2-hydroxyacetyl)-4-oxo-4,5,6,7,8,9-hexahydro-3H-pyrimido[5,4-c]azepin-2-yl)cyclopropyl)-[1,1'-biphenyl]-3-sulfonamide;
(R)-6-(2-(3-chlorophenyl)-2-hydroxyacetyl)-2-(1-(3-(4,4-difluorocyclohexyl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(2-(indolin-1-yl)acetyl)-1,2,3,6-tetrahydropyridin-4-yl)phenyl)cyclopropyl)-3,5,6,7,8,9-hexahydro-4H-pyrimido[5,4-c]azepin-4-one;
(R)-6-(2-(3'-fluoro-[1,1'-biphenyl]-3-yl)-2-hydroxyacetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3'-(trifluoromethoxy)-[1,1'-biphenyl]-3-yl)acetyl)-2-(1-(3-isopropylphenyl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(3-isopropylphenyl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3-(2-(trifluoromethyl)pyridin-4-yl)phenyl)acetyl)-2-(1-(3-isopropylphenyl)cyclopropyl)-3,5,6,7,8,9-hexahydro-4H-pyrimido[5,4-c]azepin-4-one;
(R)-4-(((3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-4-yl)methyl)(methyl)amino)-4-oxobutanoic acid;
(R)-6-(2-(3-chlorophenyl)-2-hydroxyacetyl)-2-(1-(2-phenoxypyridin-4-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-(isothiazol-4-yl)thiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(5-(isothiazol-4-yl)pyridin-3-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(5-(isothiazol-4-yl)pyridin-3-yl)cyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(5-phenylpyridin-3-yl)cyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(5-isopropylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(2-(trifluoromethyl)pyridin-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(6-(trifluoromethyl)pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(5-(trifluoromethyl)pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(piperidine-1-carbonyl)-1,2,3,6-tetrahydropyridin-4-yl)phenyl)cyclopropyl)-3,5,6,7,8,9-hexahydro-4H-pyrimido[5,4-c]azepin-4-one;
(R)-N-((3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-4-yl)methyl)-N-methylpiperidine-1-carboxamide;
(R)-6-(2-(3-(cyclopent-1-en-1-yl)phenyl)-2-hydroxyacetyl)-2-(1-(3-isopropylphenyl)cyclopropyl)-3,5,6,7,8,9-hexahydro-4H-pyrimido[5,4-c] azepin-4-one;
(R)-6-(2-hydroxy-2-(3-(trifluoromethyl)phenyl)acetyl)-2-(1-(3-(naphthalen-1-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(naphthalen-2-yl)phenyl)cyclopropyl)-3,5,6,7,8,9-hexahydro-4H-pyrimido[5,4-c]azepin-4-one;
(R)-6-(2-(3'-chloro-5'-(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3',5'-dichloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3'-chloro-5'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(4'-chloro-3'-(trifluoromethyl)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3',4'-dichloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3'-chloro-5'-(trifluoromethoxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(4'-chloro-3'-(trifluoromethoxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3'-chloro-4'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3'-(benzyloxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3-(benzo[b]thiophen-2-yl)phenyl)-2-hydroxyacetyl)-2-(1-phenylcyclopropyl)-5,6,7,8-tetrahydropyrido[4,3-d]pyrimidin-4(3H)-one;
8,8-difluoro-2-(1-phenylcyclopropyl)-6-(2-(3-(trifluoromethyl)phenyl)acetyl)-5,6,7,8-tetrahydropyrido[4,3-d]pyrimidin-4(3H)-one;
(R)-N,N-dibenzyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)thiophene-3-carboxamide;
(R)-N-benzhydryl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methylthiophene-3-carboxamide;
(R)-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methyl-N-(4-phenoxybenzyl)thiophene-3-carboxamide;
(R)-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methyl-N-(naphthalen-1-ylmethyl)thiophene-3-carboxamide;
(R)-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N,N-diisopropylthiophene-3-carboxamide;
6-((R)-2-hydroxy-2-(3-(trifluoromethyl)phenyl)acetyl)-2-(1-(3-(1-((trans)-4-(trifluoromethyl)cyclohexane-1-carbonyl)-1,2,3,6-tetrahydropyridin-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(2-(4-(phenoxymethyl)phenyl)acetyl)-1,2,3,6-tetrahydropyridin-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-methylindolin-5-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(3-(2-oxopyrrolidin-1-yl)propanoyl)-1,2,3,6-tetrahydropyridin-4-yl)phenyl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(3-(1-(2,2,2-trifluoroethyl)-1,2,3,6-tetrahydropyridin-4-yl)phenyl)cyclopropyl)-3,5,6,7,8,9-hexahydro-4H-pyrimido[5,4-c]azepin-4-one;
(R)-2-(1-(3-(1-(benzylsulfonyl)-1,2,3,6-tetrahydropyridin-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(1-(cyclohexylsulfonyl)-1,2,3,6-tetrahydropyridin-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(1-((4-fluorophenyl)sulfonyl)-1,2,3,6-tetrahydropyridin-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-(2',3'-dichloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(2',5'-dichloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-5-chloro-3'-(1-hydroxy-2-oxo-2-(4-oxo-2-(1-phenylcyclopropyl)-3,5,7,8-tetrahydropyrido[4,3-d]pyrimidin-6(4H)-yl)ethyl)-[1,1'-biphenyl]-3-carbonitrile;
(R)-6-(2-hydroxy-2-(3'-isopropoxy-[1,1'-biphenyl]-3-yl)acetyl)-2-(1-phenylcyclopropyl)-5,6,7,8-tetrahydropyrido[4,3-d]pyrimidin-4(3H)-one;
(R)-6-(2-(3',5'-di-tert-butyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(4'-(benzyloxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3-(3,4-dihydro-2H-benzo[b][1,4]dioxepin-7-yl)phenyl)-2-hydroxyacetyl)-2-(1-phenylcyclopropyl)-5,6,7,8-tetrahydropyrido[4,3-d]pyrimidin-4(3H)-one;
(R)-6-(2-(3'-cyclopropyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-2-(1-(3-chlorophenyl)cyclopropyl)-6-(2-hydroxy-2-(3'-(trifluoromethyl)-[1,1'-biphenyl]-3-yl)acetyl)-3,5,6,7,8,9-hexahydro-4H-pyrimido[5,4-c]azepin-4-one;
(R)-6-(2-(3-(benzo[b]thiophen-3-yl)phenyl)-2-hydroxyacetyl)-2-(1-(3-chlorophenyl)cyclopropyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
(R)-6-(2-(3-(benzo[b]thiophen-2-yl)phenyl)-2-hydroxyacetyl)-2-(1-(3-chlorophenyl)cyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(3-phenoxyphenyl)cyclopropyl)-3,5,6,7,8,9-hexahydro-4H-pyrimido[5,4-c]azepin-4-one;
6-(2-(3-chloro-2-fluorophenyl)-2,2-difluoroacetyl)-2-(1-(4-isopropylthiophen-2-yl)cyclopropyl)-3,5,6,7,8,9-hexahydro-4H-pyrimido[5,4-c]azepin-4-one;
6-(2-(3-chloro-2-fluorophenyl)-2,2-difluoroacetyl)-2-(1-(5-isopropylpyridin-3-yl)cyclopropyl)-3,5,6,7,8,9-hexahydro-4H-pyrimido[5,4-c]azepin-4-one;
6-(2-(3-chloro-2-fluorophenyl)-2,2-difluoroacetyl)-2-(1-(5-phenylpyridin-3-yl)cyclopropyl)-3,5,6,7,8,9-hexahydro-4H-pyrimido[5,4-c]azepin-4-one;
(R)-2-(1-(3-(benzofuran-2-yl)phenyl)cyclopropyl)-6-(2-(3-chlorophenyl)-2-hydroxyacetyl)-3,5,6,7,8,9-hexahydro-4H-pyrimido[5,4-c]azepin-4-one;
(R)-2-(1-(3-(benzo[b]thiophen-2-yl)phenyl)cyclopropyl)-6-(2-(3-chlorophenyl)-2-hydroxyacetyl)-3,5,6,7,8,9-hexahydro-4H-pyrimido[5,4-c]azepin-4-one;
(R)-2-(1-(3-(benzofuran-3-yl)phenyl)cyclopropyl)-6-(2-(3-chlorophenyl)-2-hydroxyacetyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(3-(benzofuran-3-yl)phenyl)-2-hydroxyacetyl)-2-(1-(3-chlorophenyl)cyclopropyl)-3,5,6,7,8,9-hexahydro-4H-pyrimido[5,4-c]azepin-4-one;
(R)-2-(1-(3-chlorophenyl)cyclopropyl)-6-(2-(3'-fluoro-[1,1'-biphenyl]-3-yl)-2-hydroxyacetyl)-3,5,6,7,8,9-hexahydro-4H-pyrimido[5,4-c]azepin-4-one;
(R)-6-(2-(4'-chloro-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(3-(benzofuran-3-yl)phenyl)-2-hydroxyacetyl)-2-(1-phenylcyclopropyl)-5,6,7,8-tetrahydropyrido[4,3-d]pyrimidin-4(3H)-one;
(R)-6-(2-(2'-(benzyloxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3',4'-dimethyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3',5'-dimethyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-(3-(2,3-dihydrobenzofuran-5-yl)phenyl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3'-isopropyl-[1,1'-biphenyl]-3-yl)acetyl)-2-(1-phenylcyclopropyl)-5,6,7,8-tetrahydropyrido[4,3-d]pyrimidin-4(3H)-one;
(R)-6-(2-(4'-chloro-3'-methyl-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-hydroxy-2-(3'-(trifluoromethoxy)-[1,1'-biphenyl]-3-yl)acetyl)-2-(1-(4-phenylthiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(1-(1-(3-(trifluoromethyl)phenyl)cyclopropane-1-carbonyl)-1,2,3,6-tetrahydropyridin-4-yl)phenyl)cyclopropyl)-3,5,6,7,8,9-hexahydro-4H-pyrimido[5,4-c]azepin-4-one;
(R)-2-(1-(3-(1-(3-(cyclopentyloxy)-4-methoxybenzoyl)-1,2,3,6-tetrahydropyridin-4-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-N-cyclohexyl-4-(3-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)phenyl)-3,6-dihydropyridine-1(2H)-carboxamide;
(R)-3-cyclohexyl-1-((3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-4-yl)methyl)-1-methylurea;
(R)-6-(2-hydroxy-2-(3-(trifluoromethyl)phenyl)acetyl)-2-(1-(3-(6-(4-phenylpiperidin-1-yl)pyridin-3-yl)phenyl)cyclopropyl)-3,5,6,7,8,9-hexahydro-4H-pyrimido[5,4-c]azepin-4-one;
(R)-2-(1-(3-(6-(4-(benzyloxy)piperidin-1-yl)pyridin-3-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-4-(2-(((3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-4-yl)methyl)(methyl)amino)-2-oxoethyl)benzoic acid;
(R)-4-(((3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-4-yl)methyl)(methyl)carbamoyl)bicyclo[2.2.2]octane-1-carboxylic acid;
(R)-6-(2-hydroxy-2-(3-(trifluoromethyl)phenyl)acetyl)-2-(1-(3-(6-(4-(2-(4-(phenoxymethyl)phenyl)acetyl)piperazin-1-yl)pyridin-3-yl)phenyl)cyclopropyl)-3,5,6,7,8,9-hexahydro-4H-pyrimido[5,4-c]azepin-4-one;
(R)-2-(1-(3-chlorophenyl)cyclopropyl)-6-(2-(3'-cyclopropyl-[1,1'-biphenyl]-3-yl)-2-hydroxyacetyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3-(2-(trifluoromethyl)pyridin-4-yl)phenyl)acetyl)-3,5,6,7,8,9-hexahydro-4H-pyrimido[5,4-c]azepin-4-one;
(R)-6-(2-(3'-(benzyloxy)-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3'-isopropyl-[1,1'-biphenyl]-3-yl)acetyl)-3,5,6,7,8,9-hexahydro-4H-pyrimido[5,4-c]azepin-4-one;
(R)-6-(2-(3'-chloro-5'-fluoro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3'-chloro-5'-methyl-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzo[b]thiophen-3-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3-(6-(benzyloxy)pyridin-3-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3'-chloro-4'-fluoro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzo[b]thiophen-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(4'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-(3-(2,2-difluorobenzo[d][1,3]dioxol-5-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3'-chloro-5'-(trifluoromethyl)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzofuran-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(4'-chloro-3'-(trifluoromethyl)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3',5'-bis(trifluoromethyl)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3'-chloro-5'-(trifluoromethoxy)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3'-chloro-4'-methyl-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3',5'-dichloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3'-(benzyloxy)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(5-(trifluoromethyl)pyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(2-phenyloxazol-5-yl)cyclopropyl)-3,5,6,7,8,9-hexahydro-4H-pyrimido[5,4-c]azepin-4-one;
(R)-N-cyclohexyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-isopropylthiophene-3-carboxamide;
(R)-N-benzyl-N-cyclohexyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)thiophene-3-carboxamide;
(R)-N,N-dicyclohexyl-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)thiophene-3-carboxamide;
(R)-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methyl-N-(naphthalen-2-ylmethyl)thiophene-3-carboxamide;
(R)-N-(tert-butyl)-5-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-N-methylthiophene-3-carboxamide;
6-(2-(3'-fluoro-[1,1'-biphenyl]-3-yl)acetyl)-2-(1-phenylcyclopropyl)-3,5,6,7,8,9-hexahydro-4H-pyrimido[5,4-c]azepin-4-one;
2-(1-phenylcyclopropyl)-6-(2-(3'-(trifluoromethoxy)-[1,1'-biphenyl]-3-yl)acetyl)-3,5,6,7,8,9-hexahydro-4H-pyrimido[5,4-c]azepin-4-one;
2-(1-phenylcyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
6-(2-(3'-(tert-butyl)-[1,1'-biphenyl]-3-yl)acetyl)-2-(1-phenylcyclopropyl)-3,5,6,7,8,9-hexahydro-4H-pyrimido[5,4-c]azepin-4-one;
(R)-2-(1-(3-cyclohexylphenyl)cyclopropyl)-6-(2-(3'-fluoro-[1,1'-biphenyl]-3-yl)-2-hydroxyacetyl)-3,5,6,7,8,9-hexahydro-4H-pyrimido[5,4-c]azepin-4-one;
(R)-2-(1-(3-cyclohexylphenyl)cyclopropyl)-6-(2-hydroxy-2-(3'-(trifluoromethyl)-[1,1'-biphenyl]-3-yl)acetyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(3-cyclohexylphenyl)cyclopropyl)-3,5,6,7,8,9-hexahydro-4H-pyrimido[5,4-c]azepin-4-one;
(R)-2-(1-(3-cyclohexylphenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
(R)-2-(1-(3-(benzyloxy)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(benzyloxy)phenyl)cyclopropyl)-6-(2-(3-chlorophenyl)-2-hydroxyacetyl)-3,5,6,7,8,9-hexahydro-4H-pyrimido[5,4-c]azepin-4-one;
(S)-6-(2-hydroxy-2-(3-(trifluoromethyl)phenyl)acetyl)-2-(1-phenylcyclopropyl)-5,6,7,8-tetrahydropyrido[4,3-d]pyrimidin-4(3H)-one;
(S)-6-(2-hydroxy-2-(3-(trifluoromethyl)phenyl)acetyl)-2-(1-phenylcyclopropyl)-3,5,6,7,8,9-hexahydro-4H-pyrimido[5,4-c]azepin-4-one;
(S)-2-(1-(3-chlorophenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-(3',4'-dichloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(4'-chloro-3'-(trifluoromethoxy)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(2',3'-dichloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(2',5'-dichloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-5-chloro-3'-(1-hydroxy-2-oxo-2-(4-oxo-2-(1-phenylcyclopropyl)-3,4,5,7,8,9-hexahydro-6H-pyrimido[5,4-c]azepin-6-yl)ethyl)-[1,1'-biphenyl]-3-carbonitrile;
(R)-6-(2-hydroxy-2-(3'-isopropoxy-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-(3-(benzofuran-3-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3'-cyclopropyl-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3',4'-dimethyl-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3',5'-dimethyl-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(3'-isopropyl-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-(4'-chloro-3'-methyl-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(2'-chloro-5'-(trifluoromethyl)-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-hydroxy-2-(3'-((trifluoromethyl)thio)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(5-phenylpyridin-3-yl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
(R)-6-(2-hydroxy-2-(3'-(trifluoromethoxy)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
(R)-8-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[4,5-c]azepin-4-one;
(R)-8-(2-(3'-(tert-butyl)-[1,1'-biphenyl]-3-yl)-2-hydroxyacetyl)-2-(1-phenylcyclopropyl)-3,5,6,7,8,9-hexahydro-4H-pyrimido[4,5-c]azepin-4-one;
(R)-8-(2-hydroxy-2-(3'-(trifluoromethoxy)-[1,1'-biphenyl]-3-yl)acetyl)-2-(1-phenylcyclopropyl)-3,5,6,7,8,9-hexahydro-4H-pyrimido[4,5-c]azepin-4-one;
(R)-8-(2-hydroxy-2-(3'-isopropyl-[1,1'-biphenyl]-3-yl)acetyl)-2-(1-phenylcyclopropyl)-3,5,6,7,8,9-hexahydro-4H-pyrimido[4,5-c]azepin-4-one;
(R)-2-(1-(4'-((5-chloro-3,4-dihydroisoquinolin-2(1H)-yl)methyl)-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(4'-((4-(benzyloxy)piperidin-1-yl)methyl)-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(4-phenylthiophen-2-yl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(4-phenylthiophen-2-yl)cyclopropyl)-3,5,6,7,8,9-hexahydro-4H-pyrimido[5,4-c]azepin-4-one;
(R)-6-(2-(3-(benzofuran-3-yl)phenyl)-2-hydroxyacetyl)-2-(1-(3-cyclohexylphenyl)cyclopropyl)-3,5,6,7,8,9-hexahydro-4H-pyrimido[5,4-c]azepin-4-one;
(R)-6-(2-(3-(benzo[b]thiophen-3-yl)phenyl)-2-hydroxyacetyl)-2-(1-(3-cyclohexylphenyl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(3-cyclohexylphenyl)cyclopropyl)-3,5,6,7,8,9-hexahydro-4H-pyrimido[5,4-c]azepin-4-one;
(R)-2-(1-(3-(benzo[b]thiophen-5-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(benzo[d]thiazol-6-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(benzo[d]thiazol-2-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(2-phenyloxazol-5-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(5-phenylthiazol-2-yl)phenyl)cyclopropyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3-(6-(trifluoromethyl)pyridin-2-yl)phenyl)acetyl)-5,6,7,8-tetrahydropyrido[4,3-d]pyrimidin-4(3H)-one;
(R)-5-chloro-3'-(2-(2-(1-(3-chlorophenyl)cyclopropyl)-4-oxo-3,5,7,8-tetrahydropyrido[4,3-d]pyrimidin-6(4H)-yl)-1-hydroxy-2-oxoethyl)-[1,1'-biphenyl]-3-carbonitrile;
(R)-2-(1-(3-chlorophenyl)cyclopropyl)-6-(2-hydroxy-2-(3'-(trifluoromethoxy)-[1,1'-biphenyl]-3-yl)acetyl)-5,6,7,8-tetrahydropyrido[4,3-d]pyrimidin-4(3H)-one;
(R)-6-(2-(3'-chloro-4'-fluoro-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(4'-chloro-3'-(trifluoromethyl)-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
(R)-2-(1-(3-chlorophenyl)cyclopropyl)-6-(2-hydroxy-2-(3'-(trifluoromethyl)-[1,1'-biphenyl]-3-yl)acetyl)-5,6,7,8-tetrahydropyrido[4,3-d]pyrimidin-4(3H)-one;
(R)-6-(2-(3'-chloro-5'-(trifluoromethoxy)-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(3'-(tert-butyl)-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(3'-chloro-5'-(trifluoromethyl)-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(3'-chloro-5'-fluoro-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(3'-chloro-[1,1'-biphenyl]-3-yl)-2-hydroxyacetyl)-2-(1-(3-chlorophenyl)cyclopropyl)-5,6,7,8-tetrahydropyrido[4,3-d]pyrimidin-4(3H)-one;
6-(2,2-difluoro-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
6-(2,2-difluoro-2-(3'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3'-(tert-butyl)-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
6-(2,2-difluoro-2-(3'-isopropoxy-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3'-cyclopropyl-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
(R)-2-(1-(2'-fluoro-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3'-fluoro-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(4'-fluoro-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-2-carbonitrile;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-4-carbonitrile;
(R)-6-(2-hydroxy-2-(3-(trifluoromethyl)phenyl)acetyl)-2-(1-(2'-methyl-[1,1'-biphenyl]-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3'-methyl-[1,1'-biphenyl]-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4'-methyl-[1,1'-biphenyl]-3-yl)cyclopropyl)-3,5,6,7,8,9-hexahydro-4H-pyrimido[5,4-c]azepin-4-one;
(R)-2-(1-(3',4'-difluoro-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3',5'-difluoro-[1,1'-biphenyl]-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-(thiophen-2-yl)cyclopropyl)-3,5,6,7,8,9-hexahydro-4H-pyrimido[5,4-c]azepin-4-one;
(R)-6-(2-hydroxy-2-(3'-(trifluoromethoxy)-[1,1'-biphenyl]-3-yl)acetyl)-2-(1-(thiophen-2-yl)cyclopropyl)-3,5,6,7,8,9-hexahydro-4H-pyrimido[5,4-c]azepin-4-one;
(R)-6-(2-hydroxy-2-(3'-isopropyl-[1,1'-biphenyl]-3-yl)acetyl)-2-(1-(thiophen-2-yl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(thiophen-2-yl)cyclopropyl)-3,5,6,7,8,9-hexahydro-4H-pyrimido[5,4-c]azepin-4-one;
(R)-6-(2-(3'-fluoro-[1,1'-biphenyl]-3-yl)-2-hydroxyacetyl)-2-(1-(thiophen-2-yl)cyclopropyl)-3,5,6,7,8,9-hexahydro-4H-pyrimido[5,4-c]azepin-4-one;
(R)-6-(2-(3'-cyclopropyl-[1,1'-biphenyl]-3-yl)-2-hydroxyacetyl)-2-(1-(thiophen-2-yl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-(3-phenoxyphenyl)cyclopropyl)-3,5,6,7,8,9-hexahydro-4H-pyrimido[5,4-c]azepin-4-one;
(R)-4-fluoro-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-3-carbonitrile;
(R)-3-fluoro-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-4-carbonitrile;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-3,4-dicarbonitrile;
2-(1-(3-chlorophenyl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
6-(2-(3'-chloro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3'-isopropoxy-[1,1'-biphenyl]-3-yl)acetyl)-3,5,6,7,8,9-hexahydro-4H-pyrimido[5,4-c]azepin-4-one;
6-(2-(3'-chloro-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
2-(1-(5-phenylpyridin-3-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
2-(1-(2',3',4',5'-tetrahydro-[1,1'-biphenyl]-3-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
2-(1-(3-isopropylphenyl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
2-(1-(3-cyclohexylphenyl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
(R)-5-fluoro-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-3-carbonitrile;
(R)-3'-(1-(6-(2-hydroxy-2-(3-(trifluoromethyl)phenyl)acetyl)-4-oxo-4,5,6,7,8,9-hexahydro-3H-pyrimido[5,4-c]azepin-2-yl)cyclopropyl)-[1,1'-biphenyl]-3,5-dicarbonitrile;
(R)-2-(1-(4-cyclohexylthiophen-2-yl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
(R)-2-(1-(4-cyclohexylthiophen-2-yl)cyclopropyl)-6-(2-hydroxy-2-(3'-(trifluoromethyl)-[1,1'-biphenyl]-3-yl)acetyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(4-cyclohexylthiophen-2-yl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(4-cyclohexylthiophen-2-yl)cyclopropyl)-3,5,6,7,8,9-hexahydro-4H-pyrimido[5,4-c]azepin-4-one;
(R)-6-(2-(3-(benzo[b]thiophen-3-yl)phenyl)-2-hydroxyacetyl)-2-(1-(4-cyclohexylthiophen-2-yl)cyclopropyl)-3,5,6,7,8,9-hexahydro-4H-pyrimido[5,4-c]azepin-4-one;
2-(1-(3-chlorophenyl)cyclopropyl)-6-(2-(3'-(trifluoromethoxy)-[1,1'-biphenyl]-3-yl)acetyl)-3,5,6,7,8,9-hexahydro-4H-pyrimido[5,4-c]azepin-4-one;
6-(2-(3'-(tert-butyl)-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
2-(1-(3-chlorophenyl)cyclopropyl)-6-(2-(3'-isopropyl-[1,1'-biphenyl]-3-yl)acetyl)-3,5,6,7,8,9-hexahydro-4H-pyrimido[5,4-c]azepin-4-one;
2-(1-(3-chlorophenyl)cyclopropyl)-6-(2-(3-(6-(trifluoromethyl)pyridin-2-yl)phenyl)acetyl)-3,5,6,7,8,9-hexahydro-4H-pyrimido[5,4-c]azepin-4-one;
6-(2-(3'-chloro-5'-fluoro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
6-(2-([1,1'-biphenyl]-4-yl)acetyl)-2-(1-(3-chlorophenyl)cyclopropyl)-3,5,6,7,8,9-hexahydro-4H-pyrimido[5,4-c]azepin-4-one;
(R)-2-(1-(5-cyclohexylpyridin-3-yl)cyclopropyl)-6-(2-hydroxy-2-(3'-(trifluoromethyl)-[1,1'-biphenyl]-3-yl)acetyl)-3,5,6,7,8,9-hexahydro-4H-pyrimido[5,4-c]azepin-4-one;
(R)-2-(1-(5-cyclohexylpyridin-3-yl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(5-cyclohexylpyridin-3-yl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-(5-cyclohexylpyridin-3-yl)cyclopropyl)-3,5,6,7,8,9-hexahydro-4H-pyrimido[5,4-c]azepin-4-one;
(R)-2-(1-(5-cyclohexylpyridin-3-yl)cyclopropyl)-6-(2-(3'-fluoro-[1,1'-biphenyl]-3-yl)-2-hydroxyacetyl)-3,5,6,7,8,9-hexahydro-4H-pyrimido[5,4-c]azepin-4-one;
(R)-6-(2-(3'-chloro-5'-fluoro-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(3'-chloro-4'-fluoro-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(4'-chloro-3'-methyl-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(3'-chloro-5'-(trifluoromethyl)-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(4'-chloro-3'-(trifluoromethyl)-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-6-(2-(3'-chloro-5'-(trifluoromethoxy)-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
(R)-2-(1-(3-chlorophenyl)cyclopropyl)-6-(2-(3-(2,2-difluorobenzo[d][1,3]dioxol-5-yl)phenyl)-2-hydroxyacetyl)-3,5, 6, 7, 8, 9-hexahydro-4H-pyrimido[5,4-c]azepin-4-one;
(R)-3'-(2-(2-(1-(3-chlorophenyl)cyclopropyl)-4-oxo-3,4,5,7,8,9-hexahydro-6H-pyrimido[5,4-c]azepin-6-yl)-1-hydroxy-2-oxoethyl)-[1,1'-biphenyl]-3-carbonitrile;
(R)-3'-(2-(2-(1-(3-chlorophenyl)cyclopropyl)-4-oxo-3,4,5,7,8,9-hexahydro-6H-pyrimido[5,4-c]azepin-6-yl)-1-hydroxy-2-oxoethyl)-[1,1'-biphenyl]-4-carbonitrile;
(R)-2-(1-(3-chlorophenyl)cyclopropyl)-6-(2-hydroxy-2-(4'-(trifluoromethyl)-[1,1'-biphenyl]-3-yl)acetyl)-3,5,6,7,8,9-hexahydro-4H-pyrimido[5,4-c]azepin-4-one;
(R)-2-(1-(3-chlorophenyl)cyclopropyl)-6-(2-hydroxy-2-(4'-(trifluoromethoxy)-[1,1'-biphenyl]-3-yl)acetyl)-3,5,6,7,8,9-hexahydro-4H-pyrimido[5,4-c]azepin-4-one;
(R)-6-(2-(4'-(tert-butyl)-[1,1'-biphenyl]-3-yl)-2-hydroxyacetyl)-2-(1-(3-chlorophenyl)cyclopropyl)-3,5,6,7,8,9-hexahydro-4H-pyrimido[5,4-c]azepin-4-one;
6-(2-([1,1'-biphenyl]-4-yloxy)acetyl)-2-(1-(3-chlorophenyl)cyclopropyl)-3,5,6,7,8,9-hexahydro-4H-pyrimido[5,4-c]azepin-4-one;
6-([1,1'-biphenyl]-3-carbonyl)-2-(1-(3-chlorophenyl)cyclopropyl)-3,5, 6, 7, 8, 9-hexahydro-4H-pyrimido[5,4-c]azepin-4-one;
6-([1,1'-biphenyl]-4-carbonyl)-2-(1-(3-chlorophenyl)cyclopropyl)-3,5,6,7,8,9-hexahydro-4H-pyrimido[5,4-c]azepin-4-one;
6-(2-([1,1'-biphenyl]-3-yloxy)acetyl)-2-(1-(3-chlorophenyl)cyclopropyl)-3,5,6,7,8,9-hexahydro-4H-pyrimido[5,4-c]azepin-4-one;
2-(1-(3-chlorophenyl)cyclopropyl)-6-(3-phenoxybenzoyl)-3,5,6,7,8,9-hexahydro-4H-pyrimido[5,4-c]azepin-4-one;
2-(1-(3-chlorophenyl)cyclopropyl)-6-(4-phenoxybenzoyl)-3,5,6,7,8,9-hexahydro-4H-pyrimido[5,4-c]azepin-4-one;
6-(2-(3'-chloro-[1,1'-biphenyl]-3-yl)acetyl)-2-(1-(4-phenylthiophen-2-yl)cyclopropyl)-3,5,6,7,8,9-hexahydro-4H-pyrimido[5,4-c]azepin-4-one;
2-(1-(4-phenylthiophen-2-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
6-(2-(3'-chloro-[1,1'-biphenyl]-3-yl)acetyl)-2-(1-(3-isopropylphenyl)cyclopropyl)-3,5,6,7,8,9-hexahydro-4H-pyrimido[5,4-c] azepin-4-one;
8-(2-(3'-chloro-[1,1'-biphenyl]-3-yl)acetyl)-2-(1-phenylcyclopropyl)-3,5,6,7,8,9-hexahydro-4H-pyrimido[4,5-c]azepin-4-one;
2-(1-phenylcyclopropyl)-8-(2-(3'-(trifluoromethyl)-[1,1'-biphenyl]-3-yl)acetyl)-3,5,6,7,8,9-hexahydro-4H-pyrimido[4,5-c]azepin-4-one;
2-(1-(4-isopropylthiophen-2-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
6-(2-(3'-chloro-[1,1'-biphenyl]-3-yl)acetyl)-2-(1-(4-isopropylthiophen-2-yl)cyclopropyl)-3,5,6,7,8,9-hexahydro-4H-pyrimido[5,4-c]azepin-4-one;
2-(1-(3-cyclohexylphenyl)cyclopropyl)-6-(2-(3'-(trifluoromethyl)-[1,1'-biphenyl]-3-yl)acetyl)-5,6,7,8-tetrahydropyrido[4,3-d]pyrimidin-4(3H)-one;
6-(2-(3'-chloro-[1,1'-biphenyl]-3-yl)acetyl)-2-(1-(3-isopropylphenyl)cyclopropyl)-5,6,7,8-tetrahydropyrido[4,3-d]pyrimidin-4(3H)-one;
2-(1-(3-isopropylphenyl)cyclopropyl)-6-(2-(3'-(trifluoromethyl)-[1,1'-biphenyl]-3-yl)acetyl)-5,6,7,8-tetrahydropyrido[4,3-d]pyrimidin-4(3H)-one;
2-(1-(3-chlorophenyl)cyclopropyl)-6-(2-(3-(trifluoromethyl)phenyl)acetyl)-3,5,6,7,8,9-hexahydro-4H-pyrimido[5,4-c]azepin-4-one;
2-(1-(3-chlorophenyl)cyclopropyl)-6-(2-(2-fluoro-3-(trifluoromethyl)phenyl)acetyl)-3,5,6,7,8,9-hexahydro-4H-pyrimido[5,4-c]azepin-4-one;
2-(1-(3-chlorophenyl)cyclopropyl)-6-(2-(3-(trifluoromethoxy)phenyl)acetyl)-3,5,6,7,8,9-hexahydro-4H-pyrimido[5,4-c]azepin-4-one;
2-(1-(3-chlorophenyl)cyclopropyl)-6-(2-(3-((trifluoromethyl)thio)phenyl)acetyl)-3,5,6,7,8,9-hexahydro-4H-pyrimido[5,4-c]azepin-4-one;
2-(1-(3-chlorophenyl)cyclopropyl)-6-(2-(3-(pentafluoro-(lambda)⁶-sulfaneyl)phenyl)acetyl)-3,5,6,7,8,9-hexahydro-4H-pyrimido[5,4-c]azepin-4-one;
2-(1-(3-chlorophenyl)cyclopropyl)-6-(2-(naphthalen-2-yl)acetyl)-3,5,6,7,8,9-hexahydro-4H-pyrimido[5,4-c]azepin-4-one;
2-(1-(3-chlorophenyl)cyclopropyl)-6-(2-(3-cyclopropylphenyl)acetyl)-3,5,6,7,8,9-hexahydro-4H-pyrimido[5,4-c]azepin-4-one;
(E)-2-(1-(3-chlorophenyl)cyclopropyl)-6-(3-(3-(trifluoromethyl)phenyl)acryloyl)-3,5,6,7,8,9-hexahydro-4H-pyrimido[5,4-c]azepin-4-one;
6-(2-(3'-chloro-[1,1'-biphenyl]-3-yl)acetyl)-2-(1-(3-cyclohexylphenyl)cyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-(3-cyclohexylphenyl)cyclopropyl)-5,6,7,8-tetrahydropyrido[4,3-d]pyrimidin-4(3H)-one;
6-(2-(3-(benzofuran-3-yl)phenyl)acetyl)-2-(1-(3-chlorophenyl)cyclopropyl)-3,5,6,7,8,9-hexahydro-4H-pyrimido[5,4-c]azepin-4-one;
5-chloro-3'-(2-(2-(1-(3-chlorophenyl)cyclopropyl)-4-oxo-3,4,5,7,8,9-hexahydro-6H-pyrimido[5,4-c]azepin-6-yl)-2-oxoethyl)-[1,1'-biphenyl]-3-carbonitrile;
2-(1-(3-chlorophenyl)cyclopropyl)-6-(2-(naphthalen-1-yl)acetyl)-3,5, 6, 7, 8, 9-hexahydro-4H-pyrimido[5,4-c]azepin-4-one;
2-(1-(3-chlorophenyl)cyclopropyl)-6-(3-(3-(trifluoromethyl)phenyl)propanoyl)-3,5,6,7,8,9-hexahydro-4H-pyrimido[5,4-c]azepin-4-one;
6-(2-(3-(benzyloxy)phenyl)acetyl)-2-(1-(3-chlorophenyl)cyclopropyl)-3,5,6,7,8,9-hexahydro-4H-pyrimido[5,4-c]azepin-4-one;
2-(1-(3-(benzo[b]thiophen-3-yl)phenyl)cyclopropyl)-6-(2-(3'-chloro-[1,1'-biphenyl]-3-yl)acetyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-(3-bromophenyl)-2-hydroxyacetyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-hydroxy-2-(3'-(trifluoromethyl)-[1,1'-biphenyl]-3-yl)acetyl)-3,5, 6, 7, 8, 9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-(4-phenylthiophen-2-yl)cyclopropyl)-3,5,6,7,8,9-hexahydro-4H-pyrimido[5,4-c]azepin-4-one;
2-(1-(3-(benzo[b]thiophen-3-yl)phenyl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
2-(1-(3-(benzo[b]thiophen-3-yl)phenyl)cyclopropyl)-6-(2,2-difluoro-2-(3-(trifluoromethyl)phenyl)acetyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-(3'-chloro-[1,1'-biphenyl]-3-yl)-2-hydroxyacetyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-(3'-(tert-butyl)-[1,1'-biphenyl]-3-yl)-2-hydroxyacetyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-hydroxy-2-(3'-isopropyl-[1,1'-biphenyl]-3-yl)acetyl)-3,5,6,7,8,9-hexahydro-4H-pyrimido[5,4-c]azepin-4-one;
(R)-2-(1-(3-(benzo[b]thiophen-3-yl)phenyl)cyclopropyl)-6-(2-(3'-chloro-5'-fluoro-[1,1'-biphenyl]-3-yl)-2-hydroxyacetyl)-3,5, 6, 7, 8, 9-hexahydro-4H-pyrimido[5,4-c]azepin-4-one;
(R)-2-(1-(3-(benzo[b]thiophen-3-yl)phenyl)cyclopropyl)-6-(2-(3'-cyclopropyl-[1,1'-biphenyl]-3-yl)-2-hydroxyacetyl)-3,5,6,7,8,9-hexahydro-4H-pyrimido[5,4-c]azepin-4-one;
3'-(2-(2-(1-(3-chlorophenyl)cyclopropyl)-4-oxo-3,4,5,7,8,9-hexahydro-6H-pyrimido[5,4-c]azepin-6-yl)-2-oxoethyl)-[1,1'-biphenyl]-4-carbonitrile;
6-(2-(4'-(tert-butyl)-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
2-(1-(3-chlorophenyl)cyclopropyl)-6-(2-(4'-(trifluoromethyl)-[1,1'-biphenyl]-3-yl)acetyl)-3,5,6,7,8,9-hexahydro-4H-pyrimido[5,4-c]azepin-4-one;
2-(1-(3-chlorophenyl)cyclopropyl)-6-(2-(4'-(trifluoromethoxy)-[1,1'-biphenyl]-3-yl)acetyl)-3,5,6,7,8,9-hexahydro-4H-pyrimido[5,4-c]azepin-4-one;
6-(2-(4'-chloro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
3'-(2-oxo-2-(4-oxo-2-(1-phenylcyclopropyl)-3,4,5,7,8,9-hexahydro-6H-pyrimido[5,4-c]azepin-6-yl)ethyl)-[1,1'-biphenyl]-4-carbonitrile;
6-(2-(4'-(tert-butyl)-[1,1'-biphenyl]-3-yl)acetyl)-2-(1-phenylcyclopropyl)-3,5,6,7,8,9-hexahydro-4H-pyrimido[5,4-c]azepin-4-one;
2-(1-phenylcyclopropyl)-6-(2-(4'-(trifluoromethyl)-[1,1'-biphenyl]-3-yl)acetyl)-3,5,6,7,8,9-hexahydro-4H-pyrimido[5,4-c]azepin-4-one;
2-(1-phenylcyclopropyl)-6-(2-(4'-(trifluoromethoxy)-[1,1'-biphenyl]-3-yl)acetyl)-3,5,6,7,8,9-hexahydro-4H-pyrimido[5,4-c]azepin-4-one;
6-(2-(4'-chloro-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3-(benzofuran-3-yl)phenyl)acetyl)-2-(1-phenylcyclopropyl)-3,5,6,7,8,9-hexahydro-4H-pyrimido[5,4-c]azepin-4-one;
5-chloro-3'-(2-oxo-2-(4-oxo-2-(1-phenylcyclopropyl)-3,4,5,7,8,9-hexahydro-6H-pyrimido[5,4-c]azepin-6-yl)ethyl)-[1,1'-biphenyl]-3-carbonitrile;
2-(1-(3-chlorophenyl)cyclopropyl)-6-(2-(2-phenoxyphenyl)acetyl)-3,5,6,7,8,9-hexahydro-4H-pyrimido[5,4-c]azepin-4-one;
2-(1-(3-chlorophenyl)cyclopropyl)-6-(2-(3-phenoxyphenyl)acetyl)-3,5, 6, 7, 8, 9-hexahydro-4H-pyrimido[5,4-c]azepin-4-one;
6-(2-(3-(tert-butyl)phenyl)acetyl)-2-(1-(3-chlorophenyl)cyclopropyl)-3,5,6,7,8,9-hexahydro-4H-pyrimido[5,4-c]azepin-4-one;
6-(2-(3-(tert-butyl)phenyl)acetyl)-2-(1-phenylcyclopropyl)-3,5, 6, 7, 8, 9-hexahydro-4H-pyrimido[5,4-c]azepin-4-one;
6-(2-(3'-chloro-[1,1'-biphenyl]-3-yl)acetyl)-2-(1-(3-chlorophenyl)cyclopropyl)-5,6,7,8-tetrahydropyrido[4,3-d]pyrimidin-4(3H)-one;
2-(1-(3-chlorophenyl)cyclopropyl)-6-(2-(3'-(trifluoromethoxy)-[1,1'-biphenyl]-3-yl)acetyl)-5,6,7,8-tetrahydropyrido[4,3-d]pyrimidin-4(3H)-one;
6-(2-(3'-(tert-butyl)-[1,1'-biphenyl]-3-yl)acetyl)-2-(1-(3-chlorophenyl)cyclopropyl)-5,6,7,8-tetrahydropyrido[4,3-d]pyrimidin-4(3H)-one;
3'-(2-(2-(1-(3-chlorophenyl)cyclopropyl)-4-oxo-3,5,7,8-tetrahydropyrido[4,3-d]pyrimidin-6(4H)-yl)-2-oxoethyl)-[1,1'-biphenyl]-4-carbonitrile;
6-(2-(4'-(tert-butyl)-[1,1'-biphenyl]-3-yl)acetyl)-2-(1-(3-chlorophenyl)cyclopropyl)-5,6,7,8-tetrahydropyrido[4,3-d]pyrimidin-4(3H)-one;
2-(1-(3-chlorophenyl)cyclopropyl)-6-(2-(4'-(trifluoromethyl)-[1,1'-biphenyl]-3-yl)acetyl)-5,6,7,8-tetrahydropyrido[4,3-d]pyrimidin-4(3H)-one;
2-(1-(3-chlorophenyl)cyclopropyl)-6-(2-(4'-(trifluoromethoxy)-[1,1'-biphenyl]-3-yl)acetyl)-5,6,7,8-tetrahydropyrido[4,3-d]pyrimidin-4(3H)-one;
6-(2-(4'-chloro-[1,1'-biphenyl]-3-yl)acetyl)-2-(1-(3-chlorophenyl)cyclopropyl)-5,6,7,8-tetrahydropyrido[4,3-d]pyrimidin-4(3H)-one;
6-(2-(3-(benzofuran-3-yl)phenyl)acetyl)-2-(1-(3-chlorophenyl)cyclopropyl)-5,6,7,8-tetrahydropyrido[4,3-d]pyrimidin-4(3H)-one;
5-chloro-3'-(2-(2-(1-(3-chlorophenyl)cyclopropyl)-4-oxo-3,5,7,8-tetrahydropyrido[4,3-d]pyrimidin-6(4H)-yl)-2-oxoethyl)-[1,1'-biphenyl]-3-carbonitrile;
6-(2-(2-phenoxyphenyl)acetyl)-2-(1-phenylcyclopropyl)-3,5,6,7,8,9-hexahydro-4H-pyrimido[5,4-c]azepin-4-one;
6-(2-(3-phenoxyphenyl)acetyl)-2-(1-phenylcyclopropyl)-3,5,6,7,8,9-hexahydro-4H-pyrimido[5,4-c]azepin-4-one;
6-(2-(2-(benzyloxy)phenyl)acetyl)-2-(1-phenylcyclopropyl)-3,5,6,7,8,9-hexahydro-4H-pyrimido[5,4-c]azepin-4-one;
6-(2-(3-(benzyloxy)phenyl)acetyl)-2-(1-phenylcyclopropyl)-3,5,6,7,8,9-hexahydro-4H-pyrimido[5,4-c]azepin-4-one;
2-(1-(3-chlorophenyl)cyclopropyl)-6-(2-(3'-(trifluoromethyl)-[1,1'-biphenyl]-3-yl)acetyl)-5,6,7,8-tetrahydropyrido[4,3-d]pyrimidin-4(3H)-one;
2-(1-(3-chlorophenyl)cyclopropyl)-6-(2-(3-(trifluoromethyl)phenoxy)acetyl)-3,5,6,7,8,9-hexahydro-4H-pyrimido[5,4-c]azepin-4-one; and
(R)-6-(2-hydroxy-2-(3-(trifluoromethyl)phenyl)acetyl)-2-(1-(4-(phenylethynyl)thiophen-2-yl)cyclopropyl)-3,5,6,7,8,9-hexahydro-4H-pyrimido[5,4-c]azepin-4-one;
or a pharmaceutically acceptable salt thereof.

[5-d] In the compound of formula (1), most preferably the compound described in the above "compound of formula (1)", which is selected from the following:
(R)-1-(3'-(1-(6-(2-(3-chlorophenyl)-2-hydroxyacetyl)-4-oxo-4,5,6,7,8,9-hexahydro-3H-pyrimido[5,4-c]azepin-2-yl)cyclopropyl)-[1,1'-biphenyl]-4-yl)cyclopropane-1-carbonitrile;
(R)-2-(1-(3-(benzo[b]thiophen-3-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(benzofuran-3-yl)phenyl)cyclopropyl)-6-(2-(3-chlorophenyl)-2-hydroxyacetyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(4-bromothiophen-2-yl)cyclopropyl)-6-(2-(3-chlorophenyl)-2-hydroxyacetyl)-5,6,7,8-tetrahydropyrido[4,3-d]pyrimidin-4(3H)-one;
(R)-2-(1-(5-Cyclohexylpyridin-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-5-(3-(1-(6-(2-hydroxy-2-(3-(trifluoromethyl)phenyl)acetyl)-4-oxo-4,5,6,7,8,9)-hexahydro-3H-pyrimido[5,4-c]azepin-2-yl)cyclopropyl)phenyl)nicotinonitrile;
(R)-6-(2-(2,3-difluorophenyl)-2-hydroxyacetyl)-2-(1-phenylcyclopropyl)-3,5,6,7,8,9-hexahydro-4H-pyrimido[5,4-c]azepin-4-one;
(R)-6-(2-(2,3-difluorophenyl)-2-hydroxyacetyl)-2-(1-phenylcyclopropyl)-5,6,7,8-tetrahydropyrido[4,3-d]pyrimidin-4(3H)-one;
(R)-6-(2-(3-(benzofuran-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzofuran-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzofuran-2-yl)phenyl)-2-hydroxyacetyl)-2-(1-phenylcyclopropyl)-5,6,7,8-tetrahydropyrido[4,3-d]pyrimidin-4(3H)-one;
(R)-6-(2-(3'-(tert-butyl)-[1,1'-biphenyl]-3-yl)-2-hydroxyacetyl)-2-(1-(4-isopropylthiophene-2-yl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(4-phenylthiophene-2-yl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-methyl-1H-indazol-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-methyl-1H-indazol-5-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(2-methoxypyridin-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-fluorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-chlorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-phenylthiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-phenylcyclopropyl)-3,5,6,7,8,9-hexahydro-4H-pyrimido[5,4-c]azepin-4-one;
(R)-6-(2-(3-chlorophenyl)-2-hydroxyacetyl)-2-(1-phenylcyclopropyl)-5,6,7,8-tetrahydropyrido[4,3-d]pyrimidine-4(3H)-one;
(R)-6-(2-(4'-(benzyloxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-Hydroxy-2-(3-(2-(trifluoromethyl)pyridin-4-yl)phenyl)acetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3-(2-(trifluoromethyl)pyridin-4-yl)phenyl)acetyl)-2-(1-(4-isopropylthiophen-2-yl)cyclopropyl)-3,5,6,7,8,9-hexahydro-4H-pyrimido[5,4-c]azepin-4-one;
(R)-6-(2-hydroxy-2-(3-(6-(trifluoromethyl)pyridin-2-yl)phenyl)acetyl)-2-(1-phenylcyclopropyl)-3,5,6,7,8,9-hexahydro-4H-pyrimido[5,4-c]azepin-4-one;
(R)-6-(2-hydroxy-2-(3-(6-(trifluoromethyl)pyridin-2-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(quinolin-3-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3'-(trifluoromethyl)-[1,1'-biphenyl]-3-yl)acetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(isothiazol-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(isothiazol-4-yl)phenyl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(prop-1-en-2-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-isopropylphenyl)cyclopropyl)-3,5,6,7,8,9-hexahydro-4H-pyrimido[5,4-c] azepin-4-one;
(R)-6-(2-hydroxy-2-(3-(trifluoromethyl)phenyl)acetyl)-2-(1-(4-isopropylthiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-isopropylthiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-isopropylthiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-isopropylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-(4'-(trifluoromethoxy)-[1,1'-biphenyl]-3-yl)acetyl)-3,5,6,7,8,9-hexahydro-4H-pyrimido[5,4-c]azepin-4-one;
2-(1-(3-chlorophenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
2-(1-(5-phenylpyridin-3-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
3'-(2-(2-(1-(3-chlorophenyl)cyclopropyl)-4-oxo-3,4,5,7,8,9-hexahydro-6H-pyrimido[5,4-c]azepine-6-yl)-2-oxoethyl)-[1,1'-biphenyl]-4-carbonitrile;
5-chloro-3'-(2-oxo-2-(4-oxo-2-(1-phenylcyclopropyl)-3,4,5,7,8,9-hexahydro-6H-pyrimido[5,4-c]azepin-6-yl)ethyl)-[1,1'-biphenyl]-3-carbonitrile;
6-(2-(2-(benzyloxy)phenyl)acetyl)-2-(1-phenylcyclopropyl)-3,5,6,7,8,9-hexahydro-4H-pyrimido[5,4-c]azepin-4-one;
6-(2-(3-(benzyloxy)phenyl)acetyl)-2-(1-phenylcyclopropyl)-3,5,6,7,8,9-hexahydro-4H-pyrimido[5,4-c]azepin-4-one;
6-(2-(3'-(tert-butyl)-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
6-(2-(3'-chloro-5'-fluoro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
6-(2-(3'-cyclopropyl-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
6-(2-(4'-chloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2,2-difluoro-2-(3'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
6-(2,2-difluoro-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
6-(2,2-difluoro-2-(3'-isopropoxy-[1,1'-biphenyl]-3-yl)acetyl)-2-(1-phenylcyclopropyl)-3,5,6,7,8,9-hexahydro-4H-pyrimido[5,4-c]azepin-4-one; and
6-(2-hydroxy-2-(3'-(trifluoromethoxy)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
or a pharmaceutically acceptable salt thereof.

[5-e] A compound disclosed in WO2013012500 and represented by the following formula (2).
"A compound of formula (2)": 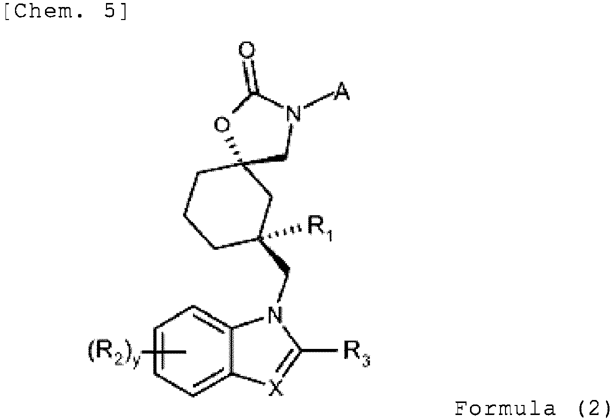 wherein:
R₁ is hydrogen, C₁₋₃alkyl, CH₂OH, CH₂-O-CH₃, CH₂OCH₂Ph, CH₂CN, CN, halo or C(O)OCH₃;
R₂ is independently hydrogen, CN, CF₃, halo, SO₂C₁₋₃alkyl, C₁₋₃alkyl or C ≡ CH;
R₃ is hydrogen, C₁₋₂alkyl, CF₃ or OH;
R₄ is hydrogen, halo or C₁₋₃alkyl;
X is CR₄ or N;
A is (CH₂)ₙ-Het;
or A is (CH₂)ₙ-(CRₐR_{b})-(CH₂)ₘ-Het;
Rₐ is hydrogen or C₁₋₃alkyl, wherein the C₁₋₃alkyl may be further substituted with one or more halos;
R_{b} is C₁₋₃alkyl;
or Rₐ and R_{b} together with the carbon atom they are attached form a C₃₋₆cycloalkyl group;
or one of the carbon atoms in the C₃₋₆cycloalkyl group formed by Rₐ and R_{b} may be replaced with oxygen to form an oxetane, tetrahydrofuryl or tetrahydropyranyl group;
or one of the carbon atoms in the C₃₋₆cycloalkyl group formed by Rₐ and R_{b} may be replaced with nitrogen to form a pyrrolidinyl or piperidinyl group;
Het is: 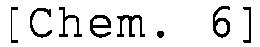 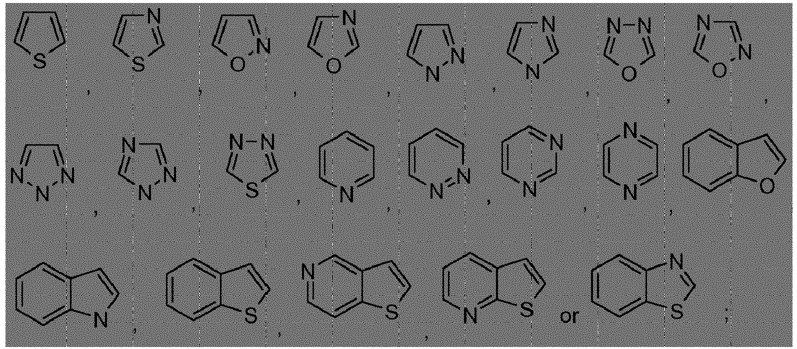
wherein Het may be substituted by one, two or three substituents chosen from: halo, C₁₋₅alkyl, CN, CH₂F, CHF₂, CF₃, C₃₋₆cycloalkyl, (CH₂)ₙ-O-C₁₋₃alkyl, (CH₂)ₙ-phenyl, (CH₂)ₙ-pyridyl, pyrimidinyl, pyrazinyl, CH(CH₃)-O-C₁₋₃alkyl, CH(OH)-C₁₋₅alkyl, C(CH₃)₂-R₅, C(O)N(CH₃)ₚ, N(C₁₋₃alkyl)ₚ, NH₂, C(O)NH₂, oxetane, oxetane-CH₃, tetrahydrofuryl, tetrahydropyranyl, morpholinyl, or pyrazolyl;
wherein the phenyl, pyrazolyl, and pyridyl substituent on the Het may be further substituted by one or two substituents chosen from: halo, CN, OCH₃, C₁₋₃alkyl or CF₃;
and the C₁₋₅alkyl and C₃₋₆cycloalkyl substituent on the Het may be further substituted by CN or OH;
R₅ is CN, O-C₁₋₄alkyl, (CH₂)ₘ-OH, (CH₂)_{P}-O-C(O)-O-C₁₋₅alkyl, or O-(CH₂)ₚ-O-R₆;
R₆ is C₁₋₄alkyl or P(O)₂(CH₃)₂;
n is independently 0, 1 or 2;
m is independently 0, 1 or 2;
p is independently 1 or 2; and
y is 1, 2 or 3;
or a pharmaceutically acceptable salt thereof.

[5-f] In the compound of formula (2), more preferably the compound described in the above "compound of formula (2)", which is selected from the following:
1-({(5S,7S)-3-[3-(1,1-dimethylethyl)-5-isoxazolyl]-7-methyl-2-oxo-1-oxa-3-azaspiro[4.5]dec-7-yl}methyl)-1H-benzimidazole-6-carbonitrile;
1-(((5S,7S)-3-(3-(2-cyanopropan-2-yl)isoxazol-5-yl)-7-methyl-2-oxo-1-oxa-3-azaspiro[4.5]decan-7-yl)methyl)-1H-benzo[d]imidazole-6-carbonitrile;
1-({(5S,7S)-3-[5-(1,1-dimethylethyl)-3-isoxazolyl]-7-methyl-2-oxo-1-oxa-3-azaspiro[4.5]dec-7-yl}methyl)-1H-benzimidazole-6-carbonitrile;
1-{[(5S,7S)-7-methyl-2-oxo-3-(2-pyridinylmethyl)-1-oxa-3-azaspiro[4.5]dec-7-yl]methyl}-1H-benzimidazole-6-carbonitrile;
1-({(5S,7S)-3-[2-methyl-2-(5-phenyl-1,3,4-oxadiazol-2-yl)propyl]-2-oxo-1-oxa-3-azaspiro[4.5]dec-7-yl}methyl)-1H-benzimidazole-6-carbonitrile;
1-({(5S,7S)-3-[2-(3-ethyl-1,2,4-oxadiazol-5-yl)-2-methylpropyl]-2-oxo-1-oxa-3-azaspiro[4.5]dec-7-yl}methyl)-1H-benzimidazole-6-carbonitrile;
1-[((5S,7S)-3-{[3-methyl-1-(2-pyrimidinyl)-3-pyrrolidinyl]methyl}-2-oxo-1-oxa-3-azaspiro[4.5]dec-7-yl)methyl]-1H-benzimidazole-6-carbonitrile;
1- ({ (5S, 7S) -7-methyl-2-oxo-3- [(1-phenyl-1H-1, 2, 3-triazol-4-yl)methyl]-1-oxa-3-azaspiro[4.5]dec-7-yl}methyl)-1H-benzimidazole-6-carbonitrile;
1-{[(5S,7S)-2-oxo-3-({1-[5-(trifluoromethyl)-3-pyridinyl]-1H-1,2,3-triazol-4-yl}methyl)-1-oxa-3-azaspiro[4.5]dec-7-yl]methyl}-1H-benzimidazole-6-carbonitrile;
1-({ (5S, 7S)-3-[4-chloro-3-(1,1-dimethylethyl)-5-isoxazolyl]-7-methyl-2-oxo-1-oxa-3-azaspiro[4.5]dec-7-yl}methyl)-1H-benzimidazole-6-carbonitrile;
1-({(5S, 7S)-7-methyl-2-oxo-3-[5-(trifluoromethyl)-2-pyridinyl]-1-oxa-3-azaspiro[4.5]dec-7-yl}methyl)-1H-benzimidazole-6-carbonitrile;
1-({(5S,7S)-3-[6-(ethyloxy)-3-pyridinyl]-7-methyl-2-oxo-1-oxa-3-azaspiro[4.5]dec-7-yl}methyl)-1H-benzimidazole-6-carbonitrile;
1-(((5S,7S)-3-(6-methoxypyridin-3-yl)-7-methyl-2-oxo-1-oxa-3-azaspiro[4.5]decan-7-yl)methyl)-1H-benzo[d]imidazole-6-carbonitrile;
1-(((5S,7S)-3-(5-ethoxypyrazin-2-yl)-7-methyl-2-oxo-1-oxa-3-azaspiro[4.5]decan-7-yl)methyl)-1H-benzo[d]imidazole-6-carbonitrile;
1-(((5S,7S)-3-(6-methoxy-4-methylpyridin-3-yl)-7-methyl-2-oxo-1-oxa-3-azaspiro[4.5]decane-7-yl)methyl)-1H-benzo[d]imidazole-6-carbonitrile;
1-(((5S,7S)-3-(1-ethyl-5-methyl-1H-pyrazol-4-yl)-7-methyl-2-oxo-1-oxa-3-azaspiro[4.5]decan-7-yl)methyl)-1H-benzo[d]imidazole-6-carbonitrile;
1-(((7S)-3-(5-methoxypyrazin-2-yl)-7-methyl-2-oxo-1-oxa-3-azaspiro[4.5]decan-7-yl)methyl)-1H-benzo[d]imidazole-6-carbonitrile;
1-(((5S,7S)-3-(6-chloropyridin-3-yl)-7-methyl-2-oxo-1-oxa-3-azaspiro[4.5]decan-7-yl)methyl)-1H-benzo[d]imidazole-6-carbonitrile;
1-(((5S,7S)-3-(6-(dimethylamino)pyridin-3-yl)-7-methyl-2-oxo-1-oxa-3-azaspiro[4.5]decane-7-yl)methyl)-1H-benzo[d]imidazole-6-carbonitrile;
(2-(5-((5S,7S)-7-((6-cyano-1H-benzo[d]imidazol-1-yl)methyl)-7-methyl-2-oxo-1-oxa-3-azaspiro[4.5]decan-3-yl)isoxazol-3-yl)-2-methylpropyl)tert-butyl carbonate;
1-(((5S,7S)-3-(3-(1-hydroxy-2-methylpropan-2-yl)isoxazol-5-yl)-7-methyl-2-oxo-1-oxa-3-azaspiro[4.5]decan-7-yl)methyl)-1H-benzo[d]imidazole-6-carbonitrile;
1-(((5S,7S)-3-(4,6-dimethoxypyridin-3-yl)-7-methyl-2-oxo-1-oxa-3-azaspiro[4.5]decan-7-yl)methyl)-1H-benzo[d]imidazole-6-carbonitrile;
1-(((5S,7S)-3-(6-ethoxy-4-methylpyridin-3-yl)-2-oxo-1-oxa-3-azaspiro[4.5]decan-7-yl)methyl)-1H-benzo[d]imidazole-6-carbonitrile;
1-(((5S,7S)-3-(6-ethoxy-4-methylpyridazin-3-yl)-7-methyl-2-oxo-1-oxa-3-azaspiro[4.5]decane-7-yl)methyl)-1H-benzo[d]imidazole-6-carbonitrile;
1-(((5S,7S)-7-methyl-2-oxo-3-(2-(trifluoromethyl)pyrimidin-5-yl)-1-oxa-3-azaspiro[4.5]decane-7-yl)methyl)-1H-benzo[d]imidazole-6-carbonitrile;
1-(((5S,7S)-7-methyl-2-oxo-3-(3-(trifluoromethyl)pyridin-2-yl)-1-oxa-3-azaspiro[4.5]decane-7-yl)methyl)-1H-benzo[d]imidazole-6-carbonitrile;
1-({(5S,7S)-3-[(5-chloro-1-benzothien-3-yl)methyl]-2-oxo-1-oxa-3-azaspiro[4.5]dec-7-yl}methyl)-1H-benzimidazole-6-carbonitrile;
1-{[(5S,7S)-3-(2-{3-[1-(ethyloxy)ethyl]-1,2,4-oxadiazol-5-yl}-2-methylpropyl)-2-oxo-1-oxa-3-azaspiro[4.5]dec-7-yl]methyl}-1H-benzimidazole-6-carbonitrile;
1-{ [(5S,7S)-7-methyl-3-(5-methyl-2-pyridinyl)-2-oxo-1-oxa-3-azaspiro[4.5]dec-7-yl]methyl}-1H-benzimidazole-6-carbonitrile;
1-({(5S,7S)-7-methyl-3-[3-(1-methylethyl)-5-isoxazolyl]-2-oxo-1-oxa-3-azaspiro[4.5]dec-7-yl}methyl)-1H-benzimidazole-6-carbonitrile;
1-({(5S,7S)-7-methyl-3-[3-(2-methylpropyl)-5-isoxazolyl]-2-oxo-1-oxa-3-azaspiro[4.5]dec-7-yl}methyl)-1H-benzimidazole-6-carbonitrile;
1-{[(5S,7S)-3-(1-tert-butyl-1H-pyrazol-4-yl)-7-methyl-2-oxo-1-oxa-3-azaspiro[4.5]dec-7-yl]methyl}-1H-benzimidazole-6-carbonitrile;
1-{[(5S,7S)-3-(3-ethyl-5-isoxazolyl)-7-methyl-2-oxo-1-oxa-3-azaspiro[4.5]dec-7-yl]methyl}-1H-benzimidazole-6-carbonitrile;
1-{[(5S,7S)-3-(3-cyclopropyl-5-isoxazolyl)-7-methyl-2-oxo-1-oxa-3-azaspiro[4.5]dec-7-yl]methyl}-1H-benzimidazole-6-carbonitrile;
1-{[(5S,7S)-7-methyl-2-oxo-3-(3-phenyl-5-isoxazolyl)-1-oxa-3-azaspiro[4.5]dec-7-yl]methyl}-1H-benzimidazole-6-carbonitrile;
1-({(5S,7S)-3-[3-(1,1-dimethylethyl)-1-methyl-1H-pyrazol-5-yl]-7-methyl-2-oxo-1-oxa-3-azaspiro[4.5]dec-7-yl}methyl)-1H-benzimidazole-6-carbonitrile;
1-({(5S,7S)-7-methyl-2-oxo-3-[3-(trifluoromethyl)-5-isoxazolyl]-1-oxa-3-azaspiro[4.5]dec-7-yl}methyl)-1H-benzimidazole-6-carbonitrile;
1-({(5S,7S)-3-[3-(1-cyanocyclopropyl)-5-isoxazolyl]-7-methyl-2-oxo-1-oxa-3-azaspiro[4.5]dec-7-yl}methyl)-1H-benzimidazole-6-carbonitrile;
1-(((5S,7S)-3-(3-(2-fluoropropan-2-yl)isoxazol-5-yl)-7-methyl-2-oxo-1-oxa-3-azaspiro[4.5]decan-7-yl)methyl)-1H-benzo[d]imidazole-6-carbonitrile;
1-(((5S,7S)-3-(3-cyclobutylisoxazol-5-yl)-7-methyl-2-oxo-1-oxa-3-azaspiro[4.5]decane-7-yl)methyl)-1H-benzo[d]imidazole-6-carbonitrile;
1-(((5S,7S)-3-(3-(tert-butyl)-4-methylisoxazol-5-yl)-7-methyl-2-oxo-1-oxa-3-azaspiro[4.5]decan-7-yl)methyl)-1H-benzo[d]imidazole-6-carbonitrile;
1-(((5S,7S)-3-(4-(tert-butyl)oxazol-2-yl)-7-methyl-2-oxo-1-oxa-3-azaspiro[4.5]decane-7-yl)methyl)-1H-benzo[d]imidazole-6-carbonitrile;
1-(((5S,7S)-3-(1-(tert-butyl)-1H-pyrazol-4-yl)-7-methyl-2-oxo-1-oxa-3-azaspiro[4.5]decan-7-yl)methyl)-1H-benzo[d]imidazole-6-carbonitrile;
1-(((5S,7S)-3-(3-(tert-butyl)-4-fluoroisoxazol-5-yl)-7-methyl-2-oxo-1-oxa-3-azaspiro[4.5]decan-7-yl)methyl)-1H-benzo[d]imidazole-6-carbonitrile;
1-(((5S,7S)-3-(3-(1,1-difluoroethyl)isoxazol-5-yl)-7-methyl-2-oxo-1-oxa-3-azaspiro[4.5]decan-7-yl)methyl)-1H-benzo[d]imidazole-6-carbonitrile;
1-(((5S,7S)-3-(3-(tert-butyl)-4-methylisoxazol-5-yl)-2-oxo-1-oxa-3-azaspiro[4.5]decane-7-yl)methyl)-1H-benzo[d]imidazole-6-carbonitrile;
1-(((5S,7S)-3-(3-(tert-butyl)-4-fluoroisoxazol-5-yl)-2-oxo-1-oxa-3-azaspiro[4.5]decane-7-yl)methyl)-1H-benzo[d]imidazole-6-carbonitrile;
1-(((5S,7S)-3-(6-(tert-butyl)pyridazin-3-yl)-7-methyl-2-oxo-1-oxa-3-azaspiro[4.5]decane-7-yl)methyl)-1H-benzo[d]imidazole-6-carbonitrile;
1-(((5S,7S)-3-(5-(tert-butyl)pyrimidin-2-yl)-7-methyl-2-oxo-1-oxa-3-azaspiro[4.5]decane-7-yl)methyl)-1H-benzo[d]imidazole-6-carbonitrile;
1-(((5S,7S)-3-(2-(tert-butyl)-2H-1,2,3-triazol-4-yl)-7-methyl-2-oxo-1-oxa-3-azaspiro[4.5]decan-7-yl)methyl)-1H-benzo[d]imidazole-6-carbonitrile;
1-(((5S,7S)-7-methyl-3-(3-(1-methyl-1H-pyrazol-3-yl)isoxazol-5-yl)-2-oxo-1-oxa-3-azaspiro[4.5]decane-7-yl)methyl)-1H-benzo[d]imidazole-6-carbonitrile;
1-(((5S,7S)-3-(1-(tert-butyl)-1H-1,2,3-triazol-4-yl)-7-methyl-2-oxo-1-oxa-3-azaspiro[4.5]decan-7-yl)methyl)-1H-benzo[d]imidazole-6-carbonitrile;
1-(((5S,7S)-3-(5-(tert-butyl)pyrazin-2-yl)-7-methyl-2-oxo-1-oxa-3-azaspiro[4.5]decane-7-yl)methyl)-1H-benzo[d]imidazole-6-carbonitrile;
1-(((5S,7S)-7-methyl-2-oxo-3-(5-(trifluoromethyl)pyrimidin-2-yl)-1-oxa-3-azaspiro[4.5]decane-7-yl)methyl)-1H-benzo[d]imidazole-6-carbonitrile;
1-(((5S,7S)-3-(6-(2-methoxypropan-2-yl)pyridin-3-yl)-7-methyl-2-oxo-1-oxa-3-azaspiro[4.5]decan-7-yl)methyl)-1H-benzo[d]imidazole-6-carbonitrile;
1-(((5S,7S)-7-methyl-2-oxo-3-(3-(prop-1-en-2-yl)isoxazol-5-yl)-1-oxa-3-azaspiro[4.5]decan-7-yl)methyl)-1H-benzo[d]imidazole-6-carbonitrile;
1-(((5S,7S)-3-(3,4-dimethylisoxazol-5-yl)-7-methyl-2-oxo-1-oxa-3-azaspiro[4.5]decane-7-yl)methyl)-1H-benzo[d]imidazole-6-carbonitrile;
1-(((5S,7S)-3-(3,4-dimethylisoxazol-5-yl)-2-oxo-1-oxa-3-azaspiro[4.5]decan-7-yl)methyl)-1H-benzo[d]imidazole-6-carbonitrile;
1-({3-[(5-chloro-1-benzothien-3-yl)methyl]-2-oxo-1-oxa-3-azaspiro[4.5]dec-7-yl}methyl)-1H-indole-6-carbonitrile;
1-({3-[(5-chloro-1-benzothien-3-yl)methyl]-2-oxo-1-oxa-3-azaspiro[4.5]dec-7-yl}methyl)-2-(trifluoromethyl)-1H-benzimidazole-6-carbonitrile;
1-({3-[(5-chloro-1-benzothien-3-yl)methyl]-2-oxo-1-oxa-3-azaspiro[4.5]dec-7-yl}methyl)-1H-benzimidazole-6-carbonitrile;
1-{[3-(1-benzothien-3-ylmethyl)-2-oxo-1-oxa-3-azaspiro[4.5]dec-7-yl]methyl}-1H-benzimidazole-6-carbonitrile;
1-({(5S,7S)-2-oxo-3-[(2-phenyl-1,3-thiazol-4-yl)methyl]-1-oxa-3-azaspiro[4.5]dec-7-yl}methyl)-1H-benzimidazole-6-carbonitrile;
1-({(5S,7S)-2-oxo-3-[(6-phenyl-2-pyridinyl)methyl]-1-oxa-3-azaspiro[4.5]dec-7-yl}methyl)-1H-benzimidazole-6-carbonitrile;
1-[((5S,7S)-3-{2-methyl-2-[3-(tetrahydro-2H-pyran-4-yl)-1,2,4-oxadiazol-5-yl]propyl}-2-oxo-1-oxa-3-azaspiro[4.5]dec-7-yl)methyl]-1H-benzimidazole-6-carbonitrile;
1-({(5S,7S)-2-oxo-3-[(4-phenyl-1,3-thiazol-2-yl)methyl]-1-oxa-3-azaspiro[4.5]dec-7-yl}methyl)-1H-benzimidazole-6-carbonitrile;
1-[((5S,7S)-3-{[3-(4-chlorophenyl)-5-isoxazolyl]methyl}-2-oxo-1-oxa-3-azaspiro[4.5]dec-7-yl)methyl]-1H-benzimidazole-6-carbonitrile;
1-({(5S,7S)-2-oxo-3-[(3-phenyl-5-isoxazolyl)methyl]-1-oxa-3-azaspiro[4.5]dec-7-yl}methyl)-1H-benzimidazole-6-carbonitrile;
1-({(5S,7S)-3-[(1-methyl-5-phenyl-1H-pyrazol-3-yl)methyl]-2-oxo-1-oxa-3-azaspiro[4.5]dec-7-yl}methyl)-1H-benzimidazole-6-carbonitrile;
1-{[(5S,7S)-3-({4-[3-methyl-4-(methyloxy)phenyl]-1,3-thiazol-2-yl}methyl)-2-oxo-1-oxa-3-azaspiro[4.5]dec-7-yl]methyl}-1H-benzimidazole-6-carbonitrile;
1-({(5S,7S)-2-oxo-3-[(3-phenyl-1H-1,2,4-triazol-5-yl)methyl]-1-oxa-3-azaspiro[4.5]dec-7-yl}methyl)-1H-benzimidazole-6-carbonitrile;
1-({(5S,7S)-2-oxo-3-[(5-phenyl-3-pyridinyl)methyl]-1-oxa-3-azaspiro[4.5]dec-7-yl}methyl)-1H-benzimidazole-6-carbonitriletrifluoro acetate;
4-chloro-1-({ (5S,7S)-3-[2-methyl-2-(3-phenyl-1,2,4-oxadiazol-5-yl)propyl]-2-oxo-1-oxa-3-azaspiro[4.5]dec-7-yl}methyl)-1H-benzimidazole-6-carbonitrile;
1-({(5S,7S)-3-[2-methyl-2-(3-methyl-1,2,4-oxadiazol-5-yl)propyl]-2-oxo-1-oxa-3-azaspiro[4.5]dec-7-yl}methyl)-1H-benzimidazole-6-carbonitrile;
1-({(5S,7S)-3-[2-methyl-2-(3-phenyl-1,2,4-oxadiazol-5-yl)propyl]-2-oxo-1-oxa-3-azaspiro[4.5]dec-7-yl}methyl)-1H-benzimidazole-6-carbonitrile;
1-[((5S,7S)-3-{2-methyl-2-[3-(1-methylethyl)-1,2,4-oxadiazol-5-yl]propyl}-2-oxo-1-oxa-3-azaspiro[4.5]dec-7-yl)methyl]-1H-benzimidazole-6-carbonitrile;
1-({ (5S,7S)-3-[2-(3-cyclopentyl-1,2,4-oxadiazol-5-yl)-2-methylpropyl]-2-oxo-1-oxa-3-azaspiro[4.5]dec-7-yl}methyl)-1H-benzimidazole-6-carbonitrile;
1-[((5S,7S)-3-{2-methyl-2-[3-(5-pyrimidinyl)-1,2,4-oxadiazol-5-yl]propyl}-2-oxo-1-oxa-3-azaspiro[4.5]dec-7-yl)methyl]-1H-benzimidazole-6-carbonitrile;
1-[((5S,7S)-3-{2-[3-(1,1-dimethylethyl)-1,2,4-oxadiazol-5-yl]-2-methylpropyl}-2-oxo-1-oxa-3-azaspiro[4.5]dec-7-yl)methyl]-1H-benzimidazole-6-carbonitrile;
1-[((5S,7S)-3-{2-methyl-2-[3-(trifluoromethyl)-1,2,4-oxadiazol-5-yl]propyl}-2-oxo-1-oxa-3-azaspiro[4.5]dec-7-yl)methyl]-1H-benzimidazole-6-carbonitrile;
1-{[(5S,7S)-3-(2-methyl-2-{3-[(methyloxy)methyl]-1,2,4-oxadiazol-5-yl}propyl)-2-oxo-1-oxa-3-azaspiro[4.5]dec-7-yl]methyl}-1H-benzimidazole-6-carbonitrile;
1-[((5S,7S)-3-{2-methyl-2-[3-(2-methylpropyl)-1,2,4-oxadiazol-5-yl]propyl}-2-oxo-1-oxa-3-azaspiro[4.5]dec-7-yl)methyl]-1H-benzimidazole-6-carbonitrile;
1-({(5S,7S)-3-[2-methyl-2-(3-{[(1-methylethyl)oxy]methyl}-1,2,4-oxadiazol-5-yl)propyl]-2-oxo-1-oxa-3-azaspiro[4.5]dec-7-yl}methyl)-1H-benzimidazole-6-carbonitrile;
4-chloro-1-[((5S,7S)-2-oxo-3-{[4-(3-phenyl-1,2,4-oxadiazol-5-yl)tetrahydro-2H-pyran-4-yl]methyl}-1-oxa-3-azaspiro[4.5]dec-7-yl)methyl]-1H-benzimidazole-6-carbonitrile;
4-chloro-1-[((5S,7S)-3-{[4-(3-cyclopentyl-1,2,4-oxadiazol-5-yl)tetrahydro-2H-pyran-4-yl]methyl}-2-oxo-1-oxa-3-azaspiro[4.5]dec-7-yl)methyl]-1H-benzimidazole-6-carbonitrile;
1-[((5S,7S)-2-oxo-3-{[1-(2-pyridinyl)-3-pyrrolidinyl]methyl}-1-oxa-3-azaspiro[4.5]dec-7-yl)methyl]-1H-benzimidazole-6-carbonitrile;
1-({(5S,7S)-2-oxo-3-[(1-phenyl-1H-1,2,3-triazol-4-yl)methyl]-1-oxa-3-azaspiro[4.5]dec-7-yl}methyl)-1H-benzimidazole-6-carbonitrile;
1-[((5S,7S)-3-{[1-(4-cyanophenyl)-1H-1,2,3-triazol-4-yl]methyl}-2-oxo-1-oxa-3-azaspiro[4.5]dec-7-yl)methyl]-1H-benzimidazole-6-carbonitrile;
1-[((5S,7S)-3-{[1-(4-chlorophenyl)-1H-1,2,3-triazol-4-yl]methyl}-2-oxo-1-oxa-3-azaspiro[4.5]dec-7-yl)methyl]-1H-benzimidazole-6-carbonitrile;
1-[((5S,7S)-3-{[1-(3-chlorophenyl)-1H-1,2,3-triazol-4-yl]methyl}-2-oxo-1-oxa-3-azaspiro[4.5]dec-7-yl)methyl]-1H-benzimidazole-6-carbonitrile;
1-[((5S,7S)-3-{[1-(4-methylphenyl)-1H-1,2,3-triazol-4-yl]methyl}-2-oxo-1-oxa-3-azaspiro[4.5]dec-7-yl)methyl]-1H-benzimidazole-6-carbonitrile;
1-[((5S,7S)-3-{[1-(3-cyanophenyl)-1H-1,2,3-triazol-4-yl]methyl}-2-oxo-1-oxa-3-azaspiro[4.5]dec-7-yl)methyl]-1H-benzimidazole-6-carbonitrile;
1-{[(5S,7S)-2-oxo-3-({1-[3-(trifluoromethyl)phenyl]-1H-1,2,3-triazol-4-yl}methyl)-1-oxa-3-azaspiro[4.5]dec-7-yl]methyl}-1H-benzimidazole-6-carbonitrile;
1-[((5S,7S)-3-{[1-(3-fluorophenyl)-1H-1,2,3-triazol-4-yl]methyl}-2-oxo-1-oxa-3-azaspiro[4.5]dec-7-yl)methyl]-1H-benzimidazole-6-carbonitrile;
1-{[(5S,7S)-3-({1-[3-(methyloxy)phenyl]-1H-1,2,3-triazol-4-yl}methyl)-2-oxo-1-oxa-3-azaspiro[4.5]dec-7-yl]methyl}-1H-benzimidazole-6-carbonitrile;
1-[((5S,7S)-3-{[1-(3-methylphenyl)-1H-1,2,3-triazol-4-yl]methyl}-2-oxo-1-oxa-3-azaspiro[4.5]dec-7-yl)methyl]-1H-benzimidazole-6-carbonitrile;
1-({(5S,7S)-3-[(1-cyclohexanyl-1H-1,2,3-triazol-4-yl)methyl]-2-oxo-1-oxa-3-azaspiro[4.5]dec-7-yl}methyl)-1H-benzimidazole-6-carbonitrile;
1-{[(5S,7S)-3-({1-[4-cyano-3-(trifluoromethyl)phenyl]-1H-1,2,3-triazol-4-yl}methyl)-2-oxo-1-oxa-3-azaspiro[4.5]dec-7-yl]methyl}-1H-benzimidazole-6-carbonitrile;
1-[((5S,7S)-3-{[1-(3-chloro-5-cyanophenyl)-1H-1,2,3-triazol-4-yl]methyl}-2-oxo-1-oxa-3-azaspiro[4.5]dec-7-yl)methyl]-1H-benzimidazole-6-carbonitrile;
1-{[(5S,7S)-2-oxo-3-({1-[2-(trifluoromethyl)-4-pyridinyl]-1H-1,2,3-triazol-4-yl}methyl)-1-oxa-3-azaspiro[4.5]dec-7-yl]methyl}-1H-benzimidazole-6-carbonitrile;
1-[((5S,7S)-3-{[1-(3,5-difluorophenyl)-1H-1,2,3-triazol-4-yl]methyl}-2-oxo-1-oxa-3-azaspiro[4.5]dec-7-yl)methyl]-1H-benzimidazole-6-carbonitrile;
1-{[(5S,7S)-2-oxo-3-({1-[4-(trifluoromethyl)phenyl]-1H-1,2,3-triazol-4-yl}methyl)-1-oxa-3-azaspiro[4.5]dec-7-yl]methyl}-1H-benzimidazole-6-carbonitrile;
1-[((5S,7S)-3-{[1-(3-cyano-5-fluorophenyl)-1H-1,2,3-triazol-4-yl]methyl}-2-oxo-1-oxa-3-azaspiro[4.5]dec-7-yl)methyl]-1H-benzimidazole-6-carbonitrile;
1-[((5S,7S)-7-methyl-3-{[1-(1-methylethyl)-1H-1,2,3-triazol-4-yl]methyl}-2-oxo-1-oxa-3-azaspiro[4.5]dec-7-yl)methyl]-1H-benzimidazole-6-carbonitrile;
1-({(5R,7S)-7-methyl-2-oxo-3-[(1-phenyl-1H-1,2,3-triazol-4-yl)methyl]-1-oxa-3-azaspiro[4.5]dec-7-yl}methyl)-1H-benzimidazole-6-carbonitrile;
1-[((5S,7S)-3-{[1-(5-chloro-3-pyridinyl)-1H-1,2,3-triazol-4-yl]methyl}-2-oxo-1-oxa-3-azaspiro[4.5]dec-7-yl)methyl]-1H-benzimidazole-6-carbonitrile;
1-(((5S,7S)-3-(4-chloro-3-(2-cyanopropan-2-yl)isoxazol-5-yl)-7-methyl-2-oxo-1-oxa-3-azaspiro[4.5]decan-7-yl)methyl)-1H-benzo[d]imidazole-6-carbonitrile;
1-({(5S,7S)-3-[4-bromo-3-(1,1-dimethylethyl)-5-isoxazolyl]-7-methyl-2-oxo-1-oxa-3-azaspiro[4.5]dec-7-yl}methyl)-1H-benzimidazole-6-carbonitrile;
1-({(5S,7S)-7-methyl-3-[2-(methyloxy)-3-pyridinyl]-2-oxo-1-oxa-3-azaspiro[4.5]dec-7-yl}methyl)-1H-benzimidazole-6-carbonitrile; 1-({(5S,7S)-3-[2,6-bis(methyloxy)-3-pyridinyl]-7-methyl-2-oxo-1-oxa-3-azaspiro[4.5]dec-7-yl}methyl)-1H-benzimidazole-6-carbonitrile;
1-({(5S,7S)-3-[4-methyl-6-(methyloxy)-3-pyridinyl]-2-oxo-1-oxa-3-azaspiro[4.5]dec-7-yl}methyl)-1H-benzimidazole-6-carbonitrile;
1-(((7S)-3-(3,5-dichloropyridin-2-yl)-7-methyl-2-oxo-1-oxa-3-azaspiro[4.5]decan-7-yl)methyl)-1H-benzo[d]imidazole-6-carbonitrile;
1-(((5S,7S)-3-(2-ethoxypyrimidin-5-yl)-7-methyl-2-oxo-1-oxa-3-azaspiro[4.5]decan-7-yl)methyl)-1H-benzo[d]imidazole-6-carbonitrile;
1-(((5S,7S)-3-(5-chloro-3-fluoropyridin-2-yl)-7-methyl-2-oxo-1-oxa-3-azaspiro[4.5]decane-7-yl)methyl)-1H-benzo[d]imidazole-6-carbonitrile;
1-(((5S,7S)-7-methyl-2-oxo-3-(5-(trifluoromethyl)pyrazin-2-yl)-1-oxa-3-azaspiro[4.5]decane-7-yl)methyl)-1H-benzo[d]imidazole-6-carbonitrile;
1-(((5S,7S)-3-(2-(tert-butyl)pyrimidin-5-yl)-2-oxo-1-oxa-3-azaspiro[4.5]decan-7-yl)methyl)-1H-benzo[d]imidazole-6-carbonitrile;
1-(((5S,7S)-7-methyl-3-(5-methylpyrazin-2-yl)-2-oxo-1-oxa-3-azaspiro[4.5]decan-7-yl)methyl)-1H-benzo[d]imidazole-6-carbonitrile;
1-(((5S,7S)-3-(6-ethoxy-4-methylpyridin-3-yl)-7-methyl-2-oxo-1-oxa-3-azaspiro[4.5]decane-7-yl)methyl)-1H-benzo[d]imidazole-6-carbonitrile;
1-(((5S,7S)-3-(6-chloro-4-methylpyridin-3-yl)-7-methyl-2-oxo-1-oxa-3-azaspiro[4.5]decane-7-yl)methyl)-1H-benzo[d]imidazole-6-carbonitrile;
1-(((5S,7S)-3-(6-chloro-4-methylpyridin-3-yl)-2-oxo-1-oxa-3-azaspiro[4.5]decan-7-yl)methyl)-1H-benzo[d]imidazole-6-carbonitrile;
1-(((5S,7S)-3-(5-chloro-3-methylpyridin-2-yl)-7-methyl-2-oxo-1-oxa-3-azaspiro[4.5]decane-7-yl)methyl)-1H-benzo[d]imidazole-6-carbonitrile;
1-(((5S,7S)-3-(6-chloro-4-methoxypyridin-3-yl)-7-methyl-2-oxo-1-oxa-3-azaspiro[4.5]decane-7-yl)methyl)-1H-benzo[d]imidazole-6-carbonitrile;
1-(((5S,7S)-3-(6-chloro-4-methoxypyridin-3-yl)-2-oxo-1-oxa-3-azaspiro[4.5]decan-7-yl)methyl)-1H-benzo[d]imidazole-6-carbonitrile;
1-(((5S,7S)-3-(4-methyl-2-(trifluoromethyl)pyrimidin-5-yl)-2-oxo-1-oxa-3-azaspiro[4.5]decane-7-yl)methyl)-1H-benzo[d]imidazole-6-carbonitrile;
1-(((5S,7S)-3-(6-ethoxy-4-methylpyridin-3-yl)-2-oxo-1-oxa-3-azaspiro[4.5]decan-7-yl)methyl)-5-fluoro-1H-benzo[d]imidazole-6-carbonitrile;
1-(((5S,7S)-3-(2-methoxy-6-methylpyridin-3-yl)-7-methyl-2-oxo-1-oxa-3-azaspiro[4.5]decane-7-yl)methyl)-1H-benzo[d]imidazole-6-carbonitrile;
1-(((5S,7S)-3-(3-methoxy-5-methylpyridin-2-yl)-7-methyl-2-oxo-1-oxa-3-azaspiro[4.5]decane-7-yl)methyl)-1H-benzo[d]imidazole-6-carbonitrile;
1-(((5S,7S)-3-(3-chloro-5-methylpyridin-2-yl)-7-methyl-2-oxo-1-oxa-3-azaspiro[4.5]decane-7-yl)methyl)-1H-benzo[d]imidazole-6-carbonitrile;
1-(((5S,7S)-3-(3-ethylpyridin-2-yl)-7-methyl-2-oxo-1-oxa-3-azaspiro[4.5]decan-7-yl)methyl)-1H-benzo[d]imidazole-6-carbonitrile;
1-(((5S,7S)-3-(3,5-dimethylpyrazin-2-yl)-7-methyl-2-oxo-1-oxa-3-azaspiro[4.5]decan-7-yl)methyl)-1H-benzo[d]imidazole-6-carbonitrile;
1-(((5S,7S)-3-(3-methyl-5-(trifluoromethyl)pyrazin-2-yl)-2-oxo-1-oxa-3-azaspiro[4.5]decane-7-yl)methyl)-1H-benzo[d]imidazole-6-carbonitrile;
1-(((5S,7S)-3-(6-methoxy-5-methylpyridin-3-yl)-7-methyl-2-oxo-1-oxa-3-azaspiro[4.5]decane-7-yl)methyl)-1H-benzo[d]imidazole-6-carbonitrile;
1-(((5S,7S)-3-(6-(2-cyanopropan-2-yl)pyridin-3-yl)-7-methyl-2-oxo-1-oxa-3-azaspiro[4.5]decan-7-yl)methyl)-1H-benzo[d]imidazole-6-carbonitrile;
1-(((5S,7S)-3-(2-(tert-butyl)pyrimidin-5-yl)-7-methyl-2-oxo-1-oxa-3-azaspiro[4.5]decane-7-yl)methyl)-1H-benzo[d]imidazole-6-carbonitrile;
1-({(5S,7S)-7-methyl-3-[5-(methyloxy)-2-pyridinyl]-2-oxo-1-oxa-3-azaspiro[4.5]dec-7-yl}methyl)-1H-benzimidazole-6-carbonitrile;
1-({(5S,7S)-3-[6-(ethyloxy)-3-pyridazinyl]-7-methyl-2-oxo-1-oxa-3-azaspiro[4.5]dec-7-yl}methyl)-1H-benzimidazole-6-carbonitrile;
1-{[(5S,7S)-3-(3-chloro-2-pyridinyl)-7-methyl-2-oxo-1-oxa-3-azaspiro[4.5]dec-7-yl]methyl}-1H-benzimidazole-6-carbonitrile;
1-({(5S,7S)-7-methyl-3-[3-(methyloxy)-2-pyridinyl]-2-oxo-1-oxa-3-azaspiro[4.5]dec-7-yl}methyl)-1H-benzimidazole-6-carbonitrile;
1-({(5S, 7S)-7-methyl-2-oxo-3-[6-(trifluoromethyl)-3-pyridinyl]-1-oxa-3-azaspiro[4.5]dec-7-yl}methyl)-1H-benzimidazole-6-carbonitrile;
1-(((7S)-7-methyl-3-(6-methylpyridin-3-yl)-2-oxo-1-oxa-3-azaspiro[4.5]decan-7-yl)methyl)-1H-benzo[d]imidazole-6-carbonitrile;
1-({(5S,7S)-3-[4,6-bis(methyloxy)-3-pyridinyl]-2-oxo-1-oxa-3-azaspiro[4.5]dec-7-yl}methyl)-1H-benzimidazole-6-carbonitrile;
1-({(5S,7S)-3-[6-(1-methylethyl)-3-pyridinyl]-2-oxo-1-oxa-3-azaspiro[4.5]dec-7-yl}methyl)-1H-benzimidazole-6-carbonitrile;
1-((7-(hydroxymethyl)-3-(6-methoxy-4-methylpyridin-3-yl)-2-oxo-1-oxa-3-azaspiro[4.5]decan-7-yl)methyl)-1H-benzo[d]imidazole-6-carbonitrile;
1-(((5S,7S)-7-methyl-2-oxo-3-(4-(trifluoromethyl)pyridin-3-yl)-1-oxa-3-azaspiro[4.5]decane-7-yl)methyl)-1H-benzo[d]imidazole-6-carbonitrile;
1-(((5S,7S)-3-(2-(dimethylamino)pyrimidin-5-yl)-7-methyl-2-oxo-1-oxa-3-azaspiro[4.5]decane-7-yl)methyl)-1H-benzo[d]imidazole-6-carbonitrile;
1-(((5S,7S)-7-methyl-3-(4-methylpyridin-3-yl)-2-oxo-1-oxa-3-azaspiro[4.5]decan-7-yl)methyl)-1H-benzo[d]imidazole-6-carbonitrile;
1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile;
1-(((5S,7S)-3-(2-(2-hydroxypropan-2-yl)pyrimidin-5-yl)-7-methyl-2-oxo-1-oxa-3-azaspiro[4.5]decan-7-yl)methyl)-1H-benzo[d]imidazole-6-carbonitrile;
1-(((5S,7S)-3-(4-methoxypyridin-3-yl)-7-methyl-2-oxo-1-oxa-3-azaspiro[4.5]decan-7-yl)methyl)-1H-benzo[d]imidazole-6-carbonitrile;
1-(((5S,7S)-3-(5-(dimethylamino)pyrazin-2-yl)-7-methyl-2-oxo-1-oxa-3-azaspiro[4.5]decane-7-yl)methyl)-1H-benzo[d]imidazole-6-carbonitrile;
1-{ [(5S,7S)-2-oxo-3-(thieno[2,3-b]pyridin-3-ylmethyl)-1-oxa-3-azaspiro[4.5]dec-7-yl]methyl}-1H-benzimidazole-6-carbonitrile;
1-{[trans)-7-methyl-2-oxo-3-(thieno[2,3-b]pyridin-3-ylmethyl)-1-oxa-3-azaspiro[4.5]dec-7-yl]methyl}-1H-benzimidazole-6-carbonitrile;
1-({(5S,7S)-3-[(3-bromothieno[2,3-b]pyridin-2-yl)methyl]-2-oxo-1-oxa-3-azaspiro[4.5]dec-7-yl}methyl)-1H-benzimidazole-6-carbonitrile;
1-{[(5S,7S)-3-(2-methyl-2-{3-[1-(methyloxy)ethyl]-1,2,4-oxadiazol-5-yl}propyl)-2-oxo-1-oxa-3-azaspiro[4.5]dec-7-yl]methyl}-1H-benzimidazole-6-carbonitrile;
1-(((5S,7S)-3-((5-ethoxypyrazin-2-yl)methyl)-7-methyl-2-oxo-1-oxa-3-azaspiro[4.5]decane-7-yl)methyl)-1H-benzo[d]imidazole-6-carbonitrile;
1-(((5S,7S)-3-((4-ethoxypyridin-2-yl)methyl)-7-methyl-2-oxo-1-oxa-3-azaspiro[4.5]decane-7-yl)methyl)-1H-benzo[d]imidazole-6-carbonitrile;
1-(((5S,7S)-3-((5-ethoxypyridin-2-yl)methyl)-7-methyl-2-oxo-1-oxa-3-azaspiro[4.5]decane-7-yl)methyl)-1H-benzo[d]imidazole-6-carbonitrile;
1-(((5S,7S)-3-((4-fluoropyridin-2-yl)methyl)-7-methyl-2-oxo-1-oxa-3-azaspiro[4.5]decane-7-yl)methyl)-1H-benzo[d]imidazole-6-carbonitrile;
1-(((5S,7S)-3-((5-fluoropyridin-2-yl)methyl)-7-methyl-2-oxo-1-oxa-3-azaspiro[4.5]decane-7-yl)methyl)-1H-benzo[d]imidazole-6-carbonitrile;
1-(((5S,7S)-7-methyl-3-((4-(1-methyl-1H-pyrazol-4-yl)pyridin-2-yl)methyl)-2-oxo-1-oxa-3-azaspiro[4.5]decan-7-yl)methyl)-1H-benzo[d]imidazole-6-carbonitrile;
1-(((5S,7S)-7-methyl-3-((6-(1-methyl-1H-pyrazol-4-yl)pyridin-2-yl)methyl)-2-oxo-1-oxa-3-azaspiro[4.5]decan-7-yl)methyl)-1H-benzo[d]imidazole-6-carbonitrile;
1-(((5S,7S)-3-(5-(2-methoxypropan-2-yl)pyrazin-2-yl)-7-methyl-2-oxo-1-oxa-3-azaspiro[4.5]decan-7-yl)methyl)-1H-benzo[d]imidazole-6-carbonitrile;
1-(((5S,7S)-3-(5-(2-(2-methoxyethoxy)propan-2-yl)pyrazin-2-yl)-7-methyl-2-oxo-1-oxa-3-azaspiro[4.5]decan-7-yl)methyl)-1H-benzo[d]imidazole-6-carbonitrile;
2-((2-(5-((5S,7S)-7-((6-cyano-1H-benzo[d]imidazol-1-yl)methyl)-7-methyl-2-oxo-1-oxa-3-azaspiro[4.5]decan-3-yl)pyrazin-2-yl)propan-2-yl)oxy)ethyldimethylphosphinic acid;
1-(((5S,7S)-3-(5'-fluoro-4-methyl-[2,2'-bipyridin]-5-yl)-7-methyl-2-oxo-1-oxa-3-azaspiro[4.5]decan-7-yl)methyl)-1H-benzo[d]imidazole-6-carbonitrile;
1-(((5S,7S)-7-methyl-3-(4-methyl-6-morpholinopyridin-3-yl)-2-oxo-1-oxa-3-azaspiro[4.5]decane-7-yl)methyl)-1H-benzo[d]imidazole-6-carbonitrile;
1-(((5S,7S)-3-(5-(2-hydroxypropan-2-yl)pyridin-2-yl)-7-methyl-2-oxo-1-oxa-3-azaspiro[4.5]decan-7-yl)methyl)-1H-benzo[d]imidazole-6-carbonitrile;
1-(((5S,7S)-3-(5-(1-hydroxy-2-methylpropan-2-yl)pyrazin-2-yl)-7-methyl-2-oxo-1-oxa-3-azaspiro[4.5]decan-7-yl)methyl)-1H-benzo[d]imidazole-6-carbonitrile; and
1-(((5S,7S)-3-(6-cyclopropyl-4-methoxypyridin-3-yl)-7-methyl-2-oxo-1-oxa-3-azaspiro[4.5]decane-7-yl)methyl)-1H-benzo[d]imidazole-6-carbonitrile;
or a pharmaceutically acceptable salt thereof:

[5-g] In the compound of formula (2), most preferably 1-(((5S,7S)-3-(5-(2-hydroxypropan-2-yl)pyrazin-2-yl)-7-methyl-2-oxo-1-oxa-3-azaspiro[4.5]decane-7-yl)methyl)-1H-benzo[d]imidazole-6-carbonitrile
or a pharmaceutically acceptable salt thereof.

[5-h] A compound disclosed in WO2011119704 and represented by the following formula (3).
"A compound of formula (3)": 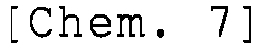 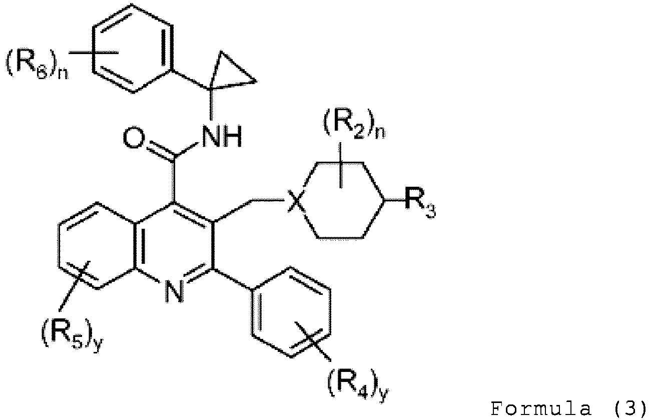 wherein:
R₁ is independently C₁₋₆ alkyl or C₃₋₆ cycloalkyl;
R₂ is independently OH, OC₁₋₄ alkyl, C₁₋₄ alkyl, CH₂OH, F, CH₂OC₁₋₄ alkyl, CF₃, or CF₂H;
R₃ is morpholinyl, piperidinyl, pyrrolidinyl, or hexahydroazepinyl, all of which may be unsubstituted or substituted by one or two R₂; or R₃ is N(C₁₋₆ alkyl)₂, wherein C₁₋₆ alkyl may be unsubstituted or substituted by OH or -OCH₃;
R₄ is independently CF₃, halo, C₁₋₃ alkyl or OC₁₋₃ alkyl;
R₅ is independently 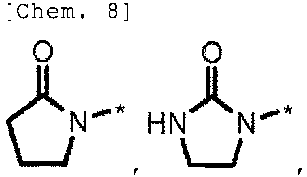 SO₂R₁, NH₂, NHSO₂R₁, NR₁SO₂R₁, C(O)NH₂, C(O)NHR₁, C(O)NHR₂, halo, cyano, CF₃, C₁₋₆ alkyl, C₂₋₄ alkenyl, pyrrolidinyl, morpholinyl, piperidinyl, phenyl, pyridyl, pyrazolyl, oxazolyl, tetrazolyl, pyrrolyl, piperazinyl, pyrimidinyl, OH, OCH₂CH₂OH, OCH₂CH₂OR₁, OCF₃, OCH₂CF₃, OCH₂CN, OR₁ or CH₂R₇;
wherein 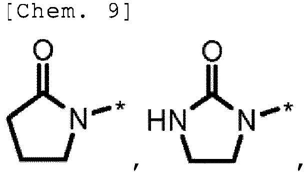 pyrrolidinyl, morpholinyl, piperidinyl, phenyl, pyridyl, pyrazolyl, oxazolyl, tetrazolyl, pyrrolyl, piperazinyl or pyrimidinyl may be unsubstituted or substituted with one or two halo, OH, OR₁ or R_{1;} or two adjacent R₅ groups may be combined to form 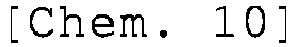 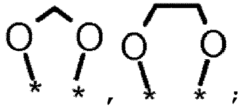
R₆ is independently halo, methyl, or OMe;
R₇ is pyrrolidinyl, morpholinyl, or piperidinyl;
n is independently 0, 1, or 2;
X is N or C; and
y is independently 0, 1 or 2;
   or a pharmaceutically acceptable salt thereof.

[5-i] In the compound of the formula (3), more preferably the compound of the above "compound of the formula (3)", which is selected from the following,
3-(1,4'-bipiperidin-1'-ylmethyl)-7-bromo-N-(1-phenylcyclopropyl)-2-[3-(trifluoromethyl)phenyl]-4-quinolinecarboxamide;
3-(1,4'-bipiperidin-1'-ylmethyl)-2-phenyl-N-(1-phenylcyclopropyl)-4-quinolinecarboxamide;
3-(1,4'-bipiperidin-1'-ylmethyl)-N-(1-phenylcyclopropyl)-2-[3-(trifluoromethyl)phenyl]-4-quinolinecarboxamide;
3-(1,4'-bipiperidin-1'-ylmethyl)-2-(3-chlorophenyl)-N-(1-phenylcyclopropyl)-4-quinolinecarboxamide trifluoroacetate;
3-(1,4'-bipiperidin-1'-ylmethyl)-8-bromo-N-(1-phenylcyclopropyl)-2-[3-(trifluoromethyl)phenyl]-4-quinolinecarboxamide;
3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-7-bromo-8-fluoro-N-(1-phenylcyclopropyl)-2-[3-(trifluoromethyl)phenyl]-4-quinolinecarboxamide;
7-bromo-8-fluoro-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-7-(ethyloxy)-N-(1-phenylcyclopropyl)-2-[3-(trifluoromethyl)phenyl]-4-quinolinecarboxamide;
7-(ethyloxy)-3-{[4-(4-morpholinyl)-1-piperidinyl]methyl)-N-(1-phenylcyclopropyl)-2-[3-(trifluoromethyl)phenyl]-4-quinolinecarboxamide;
6-Chloro-3-{[4-(4-morpholinyl)-1-piperidinyl]methyl}-N-(1-phenylcyclopropyl)-2-[3-(trifluoromethyl)phenyl]-4-quinolinecarboxamide;
6-chloro-2-[4-chloro-3-(trifluoromethyl)phenyl]-3-{[4-(4-morpholinyl)-1-piperidinyl]methyl)-N-(1-phenylcyclopropyl)-4-quinolinecarboxamide;
3-(1,4'-bipiperidin-1'-ylmethyl)-N-(1-phenylcyclopropyl)-6-[(trifluoromethyl)oxy)-2-[3-(trifluoromethyl)phenyl]-4-quinolinecarboxamide;
3-{[4-(4-morpholinyl)-1-piperidinyl]methyl)-N-(1-phenylcyclopropyl)-6-[(trifluoromethyl)oxy)-2-[3-(trifluoromethylphenyl]-4-quinolinecarboxamide;
3-(1,4'-bipiperidin-1'-ylmethyl)-N-(1-phenylcyclopropyl)-7-[(trifluoromethyl)oxy)-2-[3-(trifluoromethylphenyl]-4-quinolinecarboxamide;
3-{[4-(4-morpholinyl)-1-piperidinyl]methyl-N-(1-phenylcyclopropyl)-7-[(trifluoromethyl)oxyl-2-[3-(trifluoromethylphenyl]-4-quinolinecarboxamide;
3-(1,4'-bipiperidin-1'-ylmethyl)-7-chloro-6-(methyloxy)-N-(1-phenylcyclopropyl)-2-[3-(trifluoromethylphenyl]-4-quinolinecarboxamide;
7-chloro-6-(methyloxy)-3-{[4-(4-morpholinyl)-1-piperidinyl]methyl)-N-(1-phenylcyclopropyl)-2-[3-(trifluoromethylphenyl]-4-quinolinecarboxamide;
3-(1,4'-bipiperidin-1'-ylmethyl)-N-1-phenylcyclopropyl)-8-[(trifluoromethyl)oxyl-2-[3-(trifluoromethyl)phenyl]-4-quinolinecarboxamide;
3-{[4-(4-morpholinyl)-1-piperidinyl)methyl)-N-(1-phenylcyclopropyl)-8-[(trifluoromethyl)oxy]-2-[3-(trifluoromethylphenyl]-4-quinolinecarboxamide;
6-(ethyloxy)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-6-(ethyloxy)-N-(1-phenylcyclopropyl)-2-[3-(trifluoromethyl)phenyl]-4-quinolinecarboxamide;
3-(1,4'-bipiperidin-1-ylmethyl)-6-bromo-N-(1-phenylcyclopropyl)-2-[3-(trifluoromethyl)phenyl)-4-quinolinecarboxamide;
3-{[4-(4-morpholinyl)-1-piperidinyl]methyl}-N-(1-phenylcyclopropyl)-6-(trifluoromethyl)-2-[3-(trifluoromethylphenyl]-4-quinolinecarboxamide;
3-(1,4'-bipiperidin-1'-ylmethyl)-N-(1-phenylcyclopropyl)-6-(trifluoromethyl)-2-[3-(trifluoromethyl)phenyl]-4-quinolinecarboxamide;
6, 8-Dichloro-3-{ [4 - (4-morpholinyl)-1-piperidinyl]methyl}-N-(1 - phenylcyclopropyl)-2-[3-(trifluoromethyl)phenyl]-4-quinolinecarboxamide;
8-{[4-(4-morpholinyl)-1-piperidinyl]methyl)-N-(1-phenylcyclopropyl)-7-(3-(trifluoromethyl)phenyl]-2,3-dihydro[1,4]dioxino[2,3-9]quinoline-9-carboxamide;
8-(1,4-bipiperidin-1'-ylmethyl)-N-(1-phenylcyclopropyl)-7-[3-(trifluoromethyl)phenyl]-2,3-dihydro[1,4]dioxino[2,3-g]quinoline-9-carboxamide;
3-(1,4'-bipiperidin-1'-ylmethyl)-5-chloro-N-(1-phenylcyclopropyl)-2-[3-(trifluoromethyl)phenyl]-4-quinolinecarboxamide;
5-chloro-3-{[4-(4-morpholinyl)-1-piperidinyl]methyl)-N-(1-phenylcyclopropyl)-2-[3-(trifluoromethylphenyl]-4-quinolinecarboxamide;
3-(1,4'-bipiperidin-1'-ylmethyl)-6,8-dichloro-N-(1-phenylcyclopropyl)-2-[3-(trifluoromethylphenyl]-4-quinolinecarboxamide;
3-(1,4'-bipiperidin-1'-ylmethyl)-8-bromo-6-chloro-N-(1-phenylcyclopropyl)-2-[3-(trifluoromethylphenyl]-4-quinolinecarboxamide;
7-bromo-3-{[4-(4-morpholinyl)-1-piperidinyl]methyl)-N-(1-phenylcyclopropyl)-2-[3-(trifluoromethylphenyl]-4-quinolinecarboxamide;
8-bromo-6-chloro-3-{[4-(4-morpholinyl)-1-piperidinyl]methyl}-N-(1-phenylcyclopropyl)-2-[3-(trifluoromethyl)phenyl]-4-quinolinecarboxamide;
N-(1-phenylcyclopropyl)-3-{[4-(1-piperidinyl)cyclohexyl]methyl)-2-[3-(trifluoromethyl)phenyl]-4-quinolinecarboxamide;
3-(1,4'-bipiperidin-1'-ylmethyl)-8-(2-methylphenyl)-N-(1-phenylcyclopropyl)-2-[3-(trifluoromethyl)phenyl]-4-quinolinecarboxamide trifluoroacetate;
3-(1,4'-bipiperidin-1'-ylmethyl)-7-(2-methylphenyl)-N-(1-phenylcyclopropyl)-2-[3-(trifluoromethylphenyl]-4-quinolinecarboxamide trifluoroacetate;
6-fluoro-3-{[4-(4-morpholinyl)-1-piperidinyl]methyl}-N-(1-phenylcyclopropyl)-2-[3-(trifluoromethylphenyl]-4-quinolinecarboxamide;
3-(1,4'-bipiperidin-1'-ylmethyl)-6-fluoro-N-(1-phenylcyclopropyl)-2-(3-(trifluoromethyl)phenyl]-4-quinolinecarboxamide;
3-(1,4'-bipiperidin-1'-ylmethyl)-8-ethyl-N-(1-phenylcyclopropyl)-2-[3-(trifluoromethyl)phenyl]-4-quinolinecarboxamide;
3-(1,4'-bipiperidin-1'-ylmethyl)-7-ethyl-N-(1-phenylcyclopropyl)-2-[3-(trifluoromethyl)phenyl]-4-quinolinecarboxamide;
7-ethyl-3-{[4-(4-morpholinyl)-1-piperidinyl]methyl}-N-(1-phenylcyclopropyl)-2-[3-(trifluoromethylphenyl]-4-quinolinecarboxamide;
8-ethyl-3-{[4-(4-morpholinyl)-1-piperidinyl]methyl)-N-(1-phenylcyclopropyl)-2-[3-(trifluoromethyl)phenyl]-4-quinolinecarboxamide;
8-(Ethyloxy)-3-{[4-(4-morpholinyl)-1-piperidinyl]methyl}-N-(1-phenylcyclopropyl)-2-[3-(trifluoromethylphenyl]-4-quinolinecarboxamide;
3-(1,4²-bipiperidin-1'-ylmethyl)-8-(ethyloxy)-N-(1-phenylcyclopropyl)-2-[3-(trifluoromethyl)phenyl]-4-quinolinecarboxamide;
8-(1,4'-bipiperidin-1'-ylmethyl)-N-(1-phenylcyclopropyl)-9-(3-(trifluoromethyl)phenyl]-2,3-dihydro[1,4]dioxino[2,3-h]quinoline-7-carboxamide;
3-(1,4'-bipiperidin-1'-ylmethyl)-7,8-bis(methyloxy)-N-(1-phenylcyclopropyl)-2-[3-(trifluoromethyl)phenyl]-4-quinolinecarboxamide;
8-{[4-(4-morpholinyl)-1-piperidinyl]methyl)-N-(1-phenylcyclopropyl)-9-(3-(trifluoromethyl)phenyl]-2,3-dihydro[1,4]dioxino[2,3-h]quinoline-7-carboxamide;
7,8-bis(methyloxy)-3-{[4-(4-morpholinyl)-1-piperidinyl]methyl}-N-(1-phenylcyclopropyl)-2-[3-(trifluoromethylphenyl]-4-quinolinecarboxamide;
6-chloro-7-(methyloxy)-3-{[4-(4-morpholinyl)-1-piperidinyl]methyl}-N-(1-phenylcyclopropyl)-2-[3-(trifluoromethylphenyl]-4-quinolinecarboxamide;
6-(ethyloxy)-7-[(1-methylethyl)oxy]-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-6-(ethyloxy)-7-[(1-methylethyl)oxy]-N-(1-phenylcyclopropyl)-2-[3-(trifluoromethylphenyl]-4-quinolinecarboxamide;
3-(1,4'-bipiperidin-1'-ylmethyl)-7-(methyloxy)-N-(1-phenylcyclopropyl)-2-[3-(trifluoromethylphenyl]-4-quinolinecarboxamide;
6- chloro-7-[(1-methylethyl)oxyl-3-{[4-(4-morpholinyl) -1 - piperidinyl]methyl)-N-(1-phenylcyclopropyl)-2-[3-(trifluoromethyl)phenyl]-4-quinolinecarboxamide;
7-[(1-methylethyl)oxy)-3-{[4-(4-morpholinyl)-1-piperidinyl]methyl-N-(1-phenylcyclopropyl)-2-[3-(trifluoromethylphenyl]-4-quinolinecarboxamide;
3-(1,4²-bipiperidin-1'-ylmethyl)-7-[(1-methylethyl)oxy)-N-(1-phenylcyclopropyl)-2-[3-(trifluoromethyl)phenyl]-4-quinolinecarboxamide;
3-(1,4'-bipiperidin-1-ylmethyl)-6-chloro-7-[(1-methylethyl)oxyl-N-(1-phenylcyclopropyl)-2-[3-(trifluoromethylphenyl]-4-quinolinecarboxamide;
7-(methyloxy)-3-{[4-(4-morpholinyl)-1-piperidinyl]methyl}-N-(1-phenylcyclopropyl)-2-[3-(trifluoromethylphenyl]-4-quinolinecarboxamide;
7-[(2-methylpropyl)oxyl-3-{[4-(4-morpholinyl)-1-piperidinyl]methyl}-N-(1-phenylcyclopropyl)-2-[3-(trifluoromethylphenyl)]-4-quinolinecarboxamide;
3-(1,4'-bipiperidin-1'-ylmethyl)-7-[(2-methylpropyl)oxyl-N-(1-phenylcyclopropyl)-2-[3-(trifluoromethyl)phenyl]-4-quinolinecarboxamide;
3-(1,4'-bipiperidin-1'-ylmethyl)-N-(1-phenylcyclopropyl)-7-(propyloxy)-2-[3-(trifluoromethyl)phenyl]-4-quinolinecarboxamide;
3-{ [4-(4-morpholinyl)-1-piperidinyl]methyl}-N-(1-phenylcyclopropyl)-7-(propyloxy)-2-[3-(trifluoromethyl)phenyl]-4-quinolinecarboxamide;
3-(1,4'-bipiperidin-1'-ylmethyl)-6-(methyloxy)-N-(1-phenylcyclopropyl)-2-[3-(trifluoromethyl)phenyl]-4-quinolinecarboxamide;
6-(Methyloxy)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-7-(methylsulfonyl)-N-(1-phenylcyclopropyl)-2-[3-(trifluoromethyl)phenyl]-4-quinolinecarboxamide;
7-(methylsulfonyl)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-7-(ethylsulfonyl)-N-(1-phenylcyclopropyl)-2-[3-(trifluoromethyl)phenyl]-4-quinolinecarboxamide;
7-(ethylsulfonyl)-3-{[4-(4-morpholinyl)-1-piperidinyl] methyl)-N-(1-phenylcyclopropyl)-2-[3-(trifluoromethylphenyl]-4-quinolinecarboxamide;
3-(1,4²-bipiperidin-1'-ylmethyl)-6-(methylsulfonyl)-N-(1-phenylcyclopropyl)-2-[3-(trifluoromethyl)phenyl]-4-quinolinecarboxamide;
3-(1,4'-bipiperidin-1'-ylmethyl)-6-chloro-7-(methyloxy)-N-(1-phenylcyclopropyl)-2-[3-(trifluoromethyl)phenyl]-4-quinolinecarboxamide;
3-(1,4'-bipiperidin-1'-ylmethyl)-6-chloro-7-(ethyloxy)-N-(1-phenylcyclopropyl)-2-[3-(trifluoromethyl)phenyl]-4-quinolinecarboxamide;
6-Chloro-7-(ethyloxy)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-7-(ethyloxy)-6-(methyloxy)-N-(1-phenylcyclopropyl)-2-[3-(trifluoromethyl)phenyl]-4-quinolinecarboxamide;
3-(1,4'-bipiperidin-1'-ylmethyl)-7-[(1-methylethyl)oxy)-6-(methyloxy)-N-(1-phenylcyclopropyl)-2-[3-(trifluoromethyl)phenyl]-4-quinolinecarboxamide;
7-[(1-methylethyl)oxy]-6-(methyloxy)-3-{[4-(4-morpholinyl)-1-piperidinyl]methyl}-N-(1-phenylcyclopropyl)-2-[3-(trifluoromethyl)phenyl]-4-quinolinecarboxamide;
7-(ethyloxy)-6-(methyloxy)-3-{[4-(4-morpholinyl)-1-piperidinyl]methyl)-N-(1-phenylcyclopropyl)-2-[3-(trifluoromethyl)phenyl]-4-quinolinecarboxamide;
6,7-bis(methyloxy)-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-6,7-bis(methyloxy)-N-(1-phenylcyclopropyl)-2-[3-(trifluoromethyl)phenyl]-4-quinolinecarboxamide;
6-(Methyloxy)-3-{[4-(4-morpholinyl)-1-piperidinyl]methyl)-N-(1-phenylcyclopropyl)-7-[(2,2,2-trifluoroethyl)oxy]-2-(3-(trifluoromethyl)phenyl]-4-quinolinecarboxamide;
3-(1,4'-bipiperidin-1'-ylmethyl)-6-(methyloxy)-N-(1-phenylcyclopropyl)-7-[(2,2,2-trifluoroethyl)oxy]-2-[3-(trifluoromethyl)phenyl]-4-quinolinecarboxamide;
6-fluoro-7-[(1-methylethyl)oxyl-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-6-fluoro-7-[(1-methylethyl)oxyl-N-(1-phenylcyclopropyl)-2-[3-(trifluoromethyl)phenyl]-4-quinolinecarboxamide;
6-chloro-3-{[4-(4-morpholinyl)-1-piperidinyl)methyl}-N-(1-phenylcyclopropyl)-7-(propyloxy)-2-[3-(trifluoromethyl)phenyl]-4-quinolinecarboxamide;
3-(1,4'-bipiperidin-1'-ylmethyl)-6-chloro-N-(1-phenylcyclopropyl)-7-(propyloxy)-2-[3-(trifluoromethyl)phenyl)-4-quinolinecarboxamide;
5-bromo-7-(ethyloxy)-3-{[4-(4-morpholinyl)-1-piperidinyl]methyl)-N-(1-phenylcyclopropyl)-2-[3-(trifluoromethyl)phenyl]-4-quinolinecarboxamide;
5-bromo-7-[(1-methylethyl)oxy]-3-{[4-(4-morpholinyl)-1-piperidinyl]methyl)-N-(1-phenylcyclopropyl)-2-[3-(trifluoromethyl)phenyl]-4-quinolinecarboxamide;
7-bromo-5-(methyloxy)-3-{[4-(4-morpholinyl)-1-piperidinyl]methyl)-N-(1-phenylcyclopropyl)-2-[3-(trifluoromethyl)phenyl]-4-quinolinecarboxamide;
7-bromo-5-[(1-methylethyl)oxy]-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-6-chloro-7-[(2-methylpropyl)oxy]-N-(1-phenylcyclopropyl)-2-[3-(trifluoromethyl)phenyl]-4-quinolinecarboxamide;
6-chloro-7-[(2-methylpropyl)oxyl-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1-ylmethyl)-6-[(1-methylethyl)oxy]-N-(1-phenylcyclopropyl)-2-[3-(trifluoromethyl)phenyl]-4-quinolinecarboxamide;
6-[(1-methylethyl)oxy]-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-8-chloro-7-[(1-methylethyl)oxy]-N-(1-phenylcyclopropyl)-2-[3-(trifluoromethyl)phenyl]-4-quinolinecarboxamide;
8-chloro-7-[(1-methylethyl)oxy]-3-{[4-(4-morpholinyl)-1-piperidinyl]methyl}-N-(1-phenylcyclopropyl)-2-(3-(trifluoromethyl)phenyl]-4-quinolinecarboxamide;
3-(1,4'-bipiperidin-1'-ylmethyl)-8-chloro-7-(ethyloxy)-N-(1-phenylcyclopropyl)-2-[3-(trifluoromethylphenyl]-4-quinolinecarboxamide;
8-Chloro-7-(ethyloxy)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-8-chloro-7-(methyloxy)-N-(1-phenylcyclopropyl)-2-[3-(trifluoromethyl)phenyl]-4-quinolinecarboxamide;
8-chloro-7-(methyloxy)-3-{[4-(4-morpholinyl)-1-piperidinyl]methyl}-N-(1-phenylcyclopropyl)-2-[3-(trifluoromethylphenyl]-4-quinolinecarboxamide;
3-(1,4'-bipiperidin-1'-ylmethyl)-6-chloro-7-{[2-(methyloxy)ethyl]oxy}-N-(1-phenylcyclopropyl)-2-[3-(trifluoromethyl)phenyl]-4-quinolinecarboxamide;
6-chloro-7-{[2-(methyloxy)ethyloxy}-3-{[4-(4-morpholinyl)-1-piperidinyl)methyl)-N-(1-phenylcyclopropyl)-2-[3-(trifluoromethylphenyl]-4-quinolinecarboxamide;
3-(1,4'-bipiperidin-1'-ylmethyl)-6-cyano-7-[(1-methylethyl)oxy]-N-(1phenylcyclopropyl)-2-[3-(trifluoromethyl)phenyl]-4-quinolinecarboxamide;
6-cyano-7-[(1-methylethyl)oxyl-3-{[4-(4-morpholinyl)-1-piperidinyl]methyl}-N-(1-phenylcyclopropyl)-2-[3-(trifluoromethyl)phenyl]-4-quinolinecarboxamide;
6-methyl-7-[(1-methylethyl)oxy]-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-6-methyl-7-[(1-methylethyl)oxyl-N-(1-phenylcyclopropyl)-2-[3-(trifluoromethylphenyl]-4-quinolinecarboxamide;
3-(1,4'-bipiperidin-1'-ylmethyl)-7-[(1-methylethyl)oxy]-6-(2-oxo-1-pyrrolidinyl)-N-(1-phenylcyclopropyl)-2-[3-(trifluoromethyl)phenyl]-4-quinolinecarboxamide;
7-[(1-methylethyl)oxy]-3-{[4-(4-morpholinyl)-1-piperidinyl]methyl}-6-(2-oxo-1-pyrrolidinyl)-N-(1-phenylcyclopropyl)-2-[3-(trifluoromethyl)phenyl]-4-quinolinecarboxamide;
3-(1,4'-bipiperidin-1'-ylmethyl)-6-(4-methyl-1-piperazinyl)-N-(1-phenylcyclopropyl)-2-[3-(trifluoromethylphenyl]-4-quinolinecarboxamide;
3-(1,4'-bipiperidin-1'-ylmethyl)-6-[4-(2-methylpropyl)-1-piperazinyl]-N-(1-phenylcyclopropyl)-2-[3-(trifluoromethyl)phenyl]-4-quinolinecarboxamide;
3-(1,4'-bipiperidin-1'-ylmethyl)-6-(3-(2-methylpropyl)-1-pyrrolidinyl]-N-(1-phenylcyclopropyl)-2-[3-(trifluoromethyl)phenyl]-4-quinolinecarboxamide;
3-(1,4'-bipiperidin-1'-ylmethyl)-6-[4-(1-methylethyl)-1-piperazinyl]-N-(1-phenylcyclopropyl)-2-[3-(trifluoromethyl)phenyl]-4-quinolinecarboxamide;
3-(1,4'-bipiperidin-1'-ylmethyl)-6-[4-(2-methylpropyl)-2-oxo-1-pyrrolidinyl]-N-(1-phenylcyclopropyl)-2-[3-(trifluoromethyl)phenyl]-4-quinolinecarboxamide;
3-(1,4'-bipiperidin-1'-ylmethyl)-6-(3-(2-methylpropyl)-2-oxo-1-imidazolidinyl]-N-(1-phenylcyclopropyl)-2-[3-(trifluoromethyl)phenyl]-4-quinolinecarboxamide;
3-(1,4'-bipiperidin-1'-ylmethyl)-6-(4-methyl-2-oxo-1-pyrrolidinyl)-N-(1-phenylcyclopropyl)-2-[3-(trifluoromethylphenyl]-4-quinolinecarboxamide;
3-(1,4'-bipiperidin-1'-ylmethyl)-6-(2-oxo-1-imidazolidinyl)-N-(1-phenylcyclopropyl)-2-[3-(trifluoromethyl)phenyl]-4-quinolinecarboxamide;
3-(1,4²-bipiperidin-1-ylmethyl)-7-[(1-methylethyl)oxy]-N-(1-phenylcyclopropyl)-6-(1-pyrrolidinyl)-2-[3-(trifluoromethyl)phenyl]-4-quinolinecarboxamide;
7-[(1-methylethyl)oxyl-3-{[4-(4-morpholinyl)-1-piperidinyl]methyl-N-(1-phenylcyclopropyl)-6-(1-pyrrolidinyl)-2-[3-(trifluoromethyl)phenyl]-4-quinolinecarboxamide;
3-(1,4'-bipiperidin-1'-ylmethyl)-7-(2-methyl-1-propen-1-yl)-N-(1-phenylcyclopropyl)-2-[3-(trifluoromethyl)phenyl]-4-quinolinecarboxamide;
3-(1,4'-bipiperidin-1'-ylmethyl)-6-phenyl-N-(1-phenylcyclopropyl)-2-[3-(trifluoromethyl)phenyl]-4-quinolinecarboxamide;
6-chloro-7-(2-methylpropyl)-3-{[4-(4-morpholinyl)-1-piperidinyl]methyl}-N-(1-phenylcyclopropyl)-2-[3-(trifluoromethylphenyl]-4-quinolinecarboxamide;
3-(1,4'-bipiperidin-1'-ylmethyl)-6-chloro-7-(2-methylpropyl)-N-(1-phenylcyclopropyl)-2-[3-(trifluoromethylphenyl]-4-quinolinecarboxamide;
7-chloro-6-(methylsulfonyl)-3-{[4-(4-morpholinyl)-1-piperidinyl]methyl}-N-(1-phenylcyclopropyl)-2-[3-(trifluoromethyl)phenyl]-4-quinolinecarboxamide;
6-(methylsulfonyl)-3-{[4-(4-morpholinyl)-1-piperidinyl]methyl}-N-(1-phenylcyclopropyl)-2-[3-(trifluoromethylphenyl]-4-quinolinecarboxamide;
3-(1,4'-bipiperidin-1'-ylmethyl)-6-(ethylsulfonyl)-N-(1-phenylcyclopropyl)-2-[3-(trifluoromethyl)phenyl]-4-quinolinecarboxamide;
6-(ethylsulfonyl)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-6-[(1-methylethyl)sulfonyl]-N-(1-phenylcyclopropyl)-2-[3-(trifluoromethyl)phenyl]-4-quinolinecarboxamide;
6-[(1-methylethyl)sulfonyl]-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-7-chloro-6-(methylsulfonyl)-N-(1-phenylcyclopropyl)-2-[3-(trifluoromethyl)phenyl]-4-quinolinecarboxamide;
3-(1,4'-bipiperidin-1'-ylmethyl)-7-chloro-6-(ethylsulfonyl)-N-(1-phenylcyclopropyl)-2-[3-(trifluoromethyl)phenyl]-4-quinolinecarboxamide;
7-chloro-6-(ethylsulfonyl)-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-7-chloro-6-[(1-methylethyl)sulfonyl]-N-(1-phenylcyclopropyl)-2-[3-(trifluoromethyl)phenyl]-4-quinolinecarboxamide;
7-chloro-6-[(1-methylethyl)sulfonyl]-3-{[4-(4-morpholinyl)-1-piperidinyl]methyl}-N-(1-phenylcyclopropyl)-2-[3-(trifluoromethyl)phenyl]-4-quinolinecarboxamide;
3-(1,4'-bipiperidin-1'-ylmethyl)-6-[(1-methylethyl)sulfonyl]-7-(methyloxy)-N-(1-phenylcyclopropyl)-2-[3-(trifluoromethylphenyl]-4-quinolinecarboxamide;
6-[(1-methylethyl)sulfonyl]-7-(methyloxy)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-7-(methyloxy)-6-(methylsulfonyl)-N-(1-phenylcyclopropyl)-2-[3-(trifluoromethyl)phenyl]-4-quinolinecarboxamide;
7-(methyloxy)-6-(methylsulfonyl)-3-{[4-(4-morpholinyl)-1-piperidinyl]methyl)-N-(1-phenylcyclopropyl)-2-[3-(trifluoromethyl)phenyl)-4-quinolinecarboxamide;
3-(1,4'-bipiperidin-1'-ylmethyl)-6-(ethylsulfonyl)-7-(methyloxy)-N-(1-phenylcyclopropyl)-2-[3-(trifluoromethyl)phenyl]-4-quinolinecarboxamide;
6-(ethylsulfonyl)-7-(methyloxy)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-7-(ethyloxy)-6-(ethylsulfonyl)-N-(1-phenylcyclopropyl)-2-[3-(trifluoromethyl)phenyl]-4-quinolinecarboxamide;
7-(ethyloxy)-6-(ethylsulfonyl)-3-{[4-(4-morpholinyl)-1-piperidinyl]methyl)-N-(1-phenylcyclopropyl)-2-[3-(trifluoromethylphenyl]-4-quinolinecarboxamide;
7-(ethyloxy)-6-(ethylsulfonyl)-N-(1-phenylcyclopropyl)-3-{[4-(1-pyrrolidinyl)-1-piperidinyl]methyl)-2-[3-(trifluoromethyl)phenyl]-4-quinolinecarboxamide;
6-(ethylsulfonyl)-7-[(1-methylethyl)oxy]-3-{[4-(4-morpholinyl)-1-piperidinyl]methyl)-N-(1-phenylcyclopropyl)-2-[3-(trifluoromethylphenyl]-4-quinolinecarboxamide;
6-(ethylsulfonyl)-7-[(1-methylethyl)oxyl-N-(1-phenylcyclopropyl)-3-{[4-(1-pyrrolidinyl)-1-piperidinyl]methyl)-2-[3-(trifluoromethyl)phenyl]-4-quinolinecarboxamide;
3-(1,4'-bipiperidin-1'-ylmethyl)-6-(ethylsulfonyl)-7-[(1-methylethyl)oxy]-N-(1-phenylcyclopropyl)-2-[3-(trifluoromethylphenyl]-4-quinolinecarboxamide;
6-{[2-(methyloxy)ethyl]oxy}-3-{[4-(4-morpholinyl)-1-piperidinyl]methyl}-N-(1-phenylcyclopropyl)-2-[3-(trifluoromethylphenyl]-4-quinolinecarboxamide;
3-(1,4'-bipiperidin-1-ylmethyl)-7-(ethyloxy)-6-(methylsulfonyl)-N-(1-phenylcyclopropyl)-2-[3-(trifluoromethyl)phenyl]-4-quinolinecarboxamide;
7-(ethyloxy)-6-(methylsulfonyl)-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1-ylmethyl)-7-[(1-methylethyl)oxy]-6-(methylsulfonyl)-N-(1-phenylcyclopropyl)-2-[3-(trifluoromethyl)phenyl]-4-quinolinecarboxamide;
7-[(1-methylethyl)oxy]-6-(methylsulfonyl)-3-{[4-(4-morpholinyl)-1-piperidinyl]methyl}-N-(1-phenylcyclopropyl)-2-[3-(trifluoromethyl)phenyl]-4-quinolinecarboxamide;
3-{[4-(3,3-difluoro-1-pyrrolidinyl)-1-piperidinyl]methyl}-6-(methylsulfonyl)-N-(1-phenylcyclopropyl)-2-(3-(trifluoromethyl)phenyl]-4-quinolinecarboxamide;
3-({4-[(3S)-3-fluoro-1-pyrrolidinyl]-1-piperidinyl}methyl)-6-(methylsulfonyl)-N-(1-phenylcyclopropyl)-2-(3-(trifluoromethyl)phenyl]-4-quinolinecarboxamide;
6-(Methylsulfonyl)-N-(1-phenylcyclopropyl)-3-{[4-(1-pyrrolidinyl)-1-piperidinyl]methyl)-2-[3-(trifluoromethyl)phenyl]-4-quinolinecarboxamide;
3-[(4,4-difluoro-1,4'-bipiperidin-1-yl)methyl]-6-(methylsulfonyl)-N-(1-phenylcyclopropyl)-2-[3-(trifluoromethylphenyl]-4-quinolinecarboxamide;
6-(methylsulfonyl)-N-(1-phenylcyclopropyl)-2-[3-(trifluoromethyl)phenyl]-3-({4-[2-(trifluoromethyl)-1-pyrrolidinyl]-1-piperidinyl}methyl)-4-quinolinecarboxamide;
3-{[3-(methyloxy)-1,4'-bipiperidin-1-yl]methyl}-6-(methylsulfonyl)-N-(1-phenylcyclopropyl)-2-[3-(trifluoromethyl)phenyl]-4-quinolinecarboxamide;
3-{[(3R)-3-hydroxy-1,4'-bipiperidin-1-yl]methyl}-6-(methylsulfonyl)-N-(1-phenylcyclopropyl)-2-[3-(trifluoromethyl)phenyl]-4-quinolinecarboxamide;
3-{{4-[-(3R)-3-hydroxy-1-pyrrolidinyl]-1-piperidinyl}methyl)-6 (methylsulfonyl)-N-(1-phenylcyclopropyl)-2-[3-(trifluoromethyl)phenyl]-4-quinolinecarboxamide;
3-{[(3S)-3-hydroxy-1,4'-bipiperidin-1'-yl]methyl)-6-(methylsulfonyl)-N-(1-phenylcyclopropyl)-2-[3-(trifluoromethyl)phenyl]-4-quinolinecarboxamide;
3-{{4-[-(3S)-3-hydroxy-1-pyrrolidinyl]-1-piperidinyl}methyl)-6 (methylsulfonyl)-N-(1-phenylcyclopropyl)-2-(3-(trifluoromethyl)phenyl]-4-quinolinecarboxamide;
3-[(4-fluoro-1,4'-bipiperidin-1'-yl)methyl]-6-(methylsulfonyl)-N-(1-phenylcyclopropyl)-2-[3-(trifluoromethyl)phenyl]-4-quinolinecarboxamide;
6-(ethylsulfonyl)-7-(methyloxy)-N-(1-phenylcyclopropyl)-3-{[4-(1-pyrrolidinyl)-1-piperidinyl]methyl}-2-[3-(trifluoromethyl)phenyl]-4-quinolinecarboxamide;
6-(ethylsulfonyl)-3-[(4-fluoro-1,4'-bipiperidin-1'-yl)methyl]-7-(methyloxy)-N-(1-phenylcyclopropyl)-2-[3-(trifluoromethylphenyl]-4-quinolinecarboxamide;
3-(1,4'-bipiperidin-1'-ylmethyl)-N-(1-phenylcyclopropyl)-6-(1H-tetrazol-5-yl)-2-[3-(trifluoromethylphenyl]-4-quinolinecarboxamide;
3-(1,4'-bipiperidin-1-ylmethyl)-7-chloro-6-[(1-methylethyl)oxy]-N-(1-phenylcyclopropyl)-2-[3-(trifluoromethyl)phenyl]-4-quinolinecarboxamide;
7-(ethyloxy)-6-[(1-methylethyl)sulfonyl]-N-(1-phenylcyclopropyl)-3-{[4-(1-pyrrolidinyl)-1-piperidinyl]methyl}-2-[3-(trifluoromethylphenyl]-4-quinolinecarboxamide;
7-(ethyloxy)-6-[(1-methylethyl)sulfonyl]-3-{[4-(4-morpholinyl)-1-piperidinyl]methyl)-N-(1-phenylcyclopropyl)-2-[3-(trifluoromethyl)phenyl]-4-quinolinecarboxamide;
3-(1,4'-bipiperidin-1'-ylmethyl)-7-(ethyloxy)-6-[(1-methylethyl)sulfonyl]-N-(1-phenylcyclopropyl)-2-[3-(trifluoromethyl)phenyl]-4-quinolinecarboxamide;
3-(1,4'-bipiperidin-1'-ylmethyl)-6-[(1-methylethyl)oxy]-7-(methyloxy)-N-(1-phenylcyclopropyl)-2-[3-(trifluoromethyl)phenyl]-4-quinolinecarboxamide;
2-(3-bromophenyl)-6-(methylsulfonyl)-3-{[4-(4-morpholinyl)-1-piperidinyl]methyl-N-(1-phenylcyclopropyl)-4-quinolinecarboxamide;
3-(1,4'-bipiperidin-1'-ylmethyl)-2-(3-bromophenyl)-6-(methylsulfonyl)-N-(1-phenylcyclopropyl)-4-quinolinecarboxamide;
2-(3-bromophenyl)-6-(methylsulfonyl)-N-(1-phenylcyclopropyl)-3-{[4-(1-pyrrolidinyl)-1-piperidinyl]methyl}-4-quinolinecarboxamide;
6-[(1-methylethyl)sulfonyl]-N-(1-phenylcyclopropyl)-3-{[4-(1-pyrrolidinyl)-1-piperidinyl]methyl}-2-[3-(trifluoromethyl)phenyl]-4-quinolinecarboxamide;
6-(Methylsulfonyl)-3-{[4-(4-morpholinyl)-1-piperidinyl]methyl)-N-(1-phenylcyclopropyl)-2-[4-(trifluoromethyl)phenyl]-4-quinolinecarboxamide;
6-(methylsulfonyl)-N-(1-phenylcyclopropyl)-3-{[4-(1-pyrrolidinyl)-1-piperidinyl]methyl}-2-[4-(trifluoromethyl)phenyl]-4-quinolinecarboxamide;
3-(1,4'-bipiperidin-1'-ylmethyl)-6-(methylsulfonyl)-N-(1-phenylcyclopropyl)-2-[4-(trifluoromethyl)phenyl]-4-quinolinecarboxamide;
2-[4-(methyloxy)phenyl]-6-(methylsulfonyl)-N-(1-phenylcyclopropyl)-3-{[4-(1-pyrrolidinyl)-1-piperidinyl]methyl}-4-quinolinecarboxamide;
3-(1,4'-bipiperidin-1'-ylmethyl)-2-[4-(methyloxy)phenyl]-6-(methylsulfonyl)-N-(1-phenylcyclopropyl)-4-quinolinecarboxamide;
2-[4-(methyloxy)phenyl]-6-(methylsulfonyl)-3-{[4-(4-morpholinyl)-1-piperidinyl]methyl}-N-(1-phenylcyclopropyl)-4-quinolinecarboxamide;
2-(3,4-dichlorophenyl)-6-(methylsulfonyl)-3-{[4-(4-morpholinyl)-1-piperidinyl]methyl}-N-(1-phenylcyclopropyl)-4-quinolinecarboxamide;
3-(1,4'-bipiperidin-1'-ylmethyl)-2-(3,4-dichlorophenyl)-6-(methylsulfonyl)-N-(1-phenylcyclopropyl)-4-quinolinecarboxamide; and
2-(3,4-dichlorophenyl)-6-(methylsulfonyl)-N-(1-phenylcyclopropyl)-3-{[4-(1-pyrrolidinyl)-1-piperidinyl]methyl)-4-quinolinecarboxamide;
or a pharmaceutically acceptable salt thereof.

[5-j] In the compound of formula (3), most preferably 3-(1,4'-bipiperidin-1'-ylmethyl)-7-bromo-N-(1-phenylcyclopropyl)-2-[3-(trifluoromethyl)phenyl]-4-quinolinecarboxamide
or a pharmaceutically acceptable salt thereof.

[5-k] A compound disclosed in WO2013169396 and represented by the following formula (4).
"A compound of formula (4)": 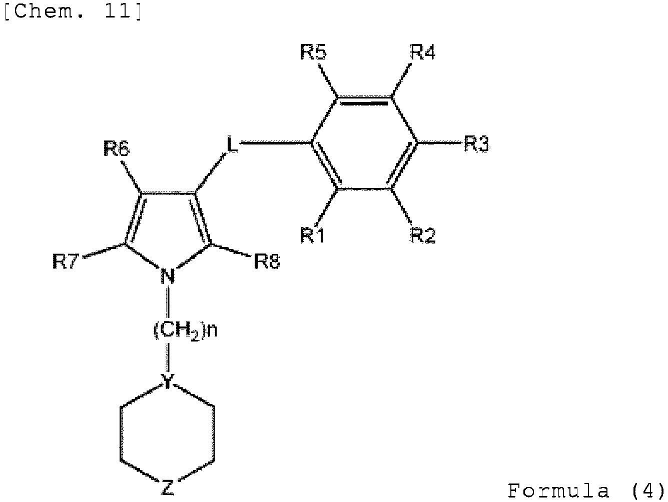
wherein L is C(=O)NR10, SO₂NR10, a C₁-C₆ alkylene, or a bond;
Y is N or CR10;
Z is O, NR10, S, SO₂ or C(R10)₂;
n is 0, 1, 2, 3, 4, 5, or 6;
R1, R2, R3, R4, and R5 are independently selected from at least one of hydro, alkyl, haloalkyl, hydroxy, alkoxy, haloalkoxy, cyano, carboxyl, carboxyalkyl or amido;
R6 and R7 are independently selected from at least one of hydro, alkyl, haloalkyl, heterocyclic or aryl, or R6 and R7 are connected to via a cyclic ring system;
R8 is hydro, alkyl, haloalkyl, heterocyclic or aryl; and
R10 is hydro, alkyl, haloalkyl, carboxyalkyl, carboxyl, alkyl methylene carbonate, methylene carbamoyl, thiophenyl or -S-carboxyalkyl;
or pharmaceutically acceptable salts thereof.

[5-l] In the compound of formula (4), more preferably the compound described in the above "compound of formula (4)", which is selected from the following:
N-(3-(trifluoromethyl)phenyl)-2-methyl-1-(3-morpholinopropyl)-5-phenyl-1H-pyrrole-3-carboxamide;
3-(2-methyl-1-(3-morpholinopropyl)-5-phenyl-1H-pyrrole-3-carboxamido)isopropyl benzoate;
2-methyl-1-(3-morpholinopropyl)-5-phenyl-N-(3-(trifluoromethoxy)phenyl)-1H-pyrrole-3-carboxamide; and
N-(3-(trifluoromethyl)phenyl)-2-methyl-1-(3-morpholinopropyl)-1H-indole-3-carboxamide;
or a pharmaceutically acceptable salt thereof.

[5-m] In the compound of formula (4), most preferably N-(3-(trifluoromethyl)phenyl)-2-methyl-1-(3-morpholinopropyl)-5-phenyl-1H-pyrrole-3-carboxamide
or a pharmaceutically acceptable salt thereof.

[5-n] Preferably, GSK3395879, GSK3527497, GSK205, GSK3491943, RN-1734, RN-1747, RN-9893, PF-05214030, or a pharmaceutically acceptable salt thereof.

[6] A use according to any one of [1] to [5], wherein the compound having TRPV4 inhibitory activity is selected from GSK2798745: (1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile), GSK2193874 (3-(1,4'-bipiperidine-1'-ylmethyl)-7-bromo-N-(1-phenylcyclopropyl)-2-[3-(trifluoromethyl)phenyl]-4-quinolinecarboxamide), HC-067047: (N-(3-(trifluoromethyl)phenyl)-2-methyl-1-(3-morpholinopropyl)-5-phenyl-1H-pyrrole-3-carboxamide), GSK3395879, GSK3527497, GSK205, GSK3491943, RN-1734, RN-1747, RN-9893, PF-05214030, (R)-1-(3'-(1-(6-(2-(3-chlorophenyl)-2-hydroxyacetyl)-4-oxo-4,5,6,7,8,9-hexahydro-3H-pyrimido[5,4-c]azepin-2-yl)cyclopropyl)-[1,1'-biphenyl]-4-yl)cyclopropane-1-carbonitrile;
(R)-2-(1-(3-(benzo[b]thiophen-3-yl)phenyl)cyclopropyl)-6-(2-hydroxy-2-(3-(trifluoromethyl)phenyl)acetyl)-3,5,6,7,8,9-hexahydro-4H-pyrimido[515,4-c]azepin-4-one;
(R)-2-(1-(3-(benzofuran-3-yl)phenyl)cyclopropyl)-6-(2-(3-chlorophenyl)-2-hydroxyacetyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(4-bromothiophen-2-yl)cyclopropyl)-6-(2-(3-chlorophenyl)-2-hydroxyacetyl)-5,6,7,8-tetrahydropyrido[4,3-d]pyrimidin-4(3H)-one;
(R) -2-(1-(5-Cyclohexylpyridin-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-5-(3-(1-(6-(2-hydroxy-2-(3-(trifluoromethyl)phenyl)acetyl)-4-oxo-4,5,6,7,8,9)-hexahydro-3H-pyrimido[5,4-c]azepin-2-yl)cyclopropyl)phenyl)nicotinonitrile;
(R)-6-(2-(2,3-difluorophenyl)-2-hydroxyacetyl)-2-(1-phenylcyclopropyl)-3,5,6,7,8,9-hexahydro-4H-pyrimido[5,4-c]azepin-4-one;
(R)-6-(2-(2,3-difluorophenyl)-2-hydroxyacetyl)-2-(1-phenylcyclopropyl)-5,6,7,8-tetrahydropyrido[4,3-d]pyrimidin-4(3H)-one;
(R)-6-(2-(3-(benzofuran-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzofuran-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzofuran-2-yl)phenyl)-2-hydroxyacetyl)-2-(1-phenylcyclopropyl)-5,6,7,8-tetrahydropyrido[4,3-d]pyrimidin-4(3H)-one;
(R)-6-(2-(3'-(tert-butyl)-[1,1'-biphenyl]-3-yl)-2-hydroxyacetyl)-2-(1-(4-isopropylthiophene-2-yl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(4-phenylthiophene-2-yl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-methyl-1H-indazol-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-methyl-1H-indazol-5-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(2-methoxypyridin-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-fluorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-chlorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-phenylthiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-phenylcyclopropyl)-3,5, 6, 7,8,9-hexahydro-4H-pyrimido[5,4-c]azepin-4-one;
(R)-6-(2-(3-chlorophenyl)-2-hydroxyacetyl)-2-(1-phenylcyclopropyl)-5,6,7,8-tetrahydropyrido[4,3-d]pyrimidine-4(3H)-one;
(R)-6-(2-(4'-(benzyloxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-Hydroxy-2-(3-(2-(trifluoromethyl)pyridin-4-yl)phenyl)acetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3-(2-(trifluoromethyl)pyridin-4-yl)phenyl)acetyl)-2-(1-(4-isopropylthiophen-2-yl)cyclopropyl)-3,5, 6, 7, 8, 9-hexahydro-4H-pyrimido[5,4-c]azepin-4-one;
(R)-6-(2-hydroxy-2-(3-(6-(trifluoromethyl)pyridin-2-yl)phenyl)acetyl)-2-(1-phenylcyclopropyl)-3,5,6,7,8,9-hexahydro-4H-pyrimido[5,4-c]azepin-4-one;
(R)-6-(2-hydroxy-2-(3-(6-(trifluoromethyl)pyridin-2-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(quinolin-3-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3'-(trifluoromethyl)-[1,1'-biphenyl]-3-yl)acetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(isothiazol-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(isothiazol-4-yl)phenyl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(prop-1-en-2-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-isopropylphenyl)cyclopropyl)-3,5,6,7,8,9-hexahydro-4H-pyrimido[5,4-c] azepin-4-one;
(R)-6-(2-hydroxy-2-(3-(trifluoromethyl)phenyl)acetyl)-2-(1-(4-isopropylthiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-isopropylthiophen-2-yl)cyclopropyl)-5,6,7,8-tetrahydropyrido[4,3-d] pyrimidin-4(3H)-one;
(R)-6-(2-hydroxy-2-(3-(trifluoromethyl)phenyl)acetyl)-2-(1-(4-isopropylthiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-isopropylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-(4'-(trifluoromethoxy)-[1,1'-biphenyl]-3-yl)acetyl)-3,5,6,7,8,9-hexahydro-4H-pyrimido[5,4-c]azepin-4-one;
2-(1-(3-chlorophenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
2-(1-(5-phenylpyridin-3-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
3'-(2-(2-(1-(3-chlorophenyl)cyclopropyl)-4-oxo-3,4,5,7,8,9-hexahydro-6H-pyrimido[5,4-c]azepine-6-yl)-2-oxoethyl)-[1,1'-biphenyl]-4-carbonitrile;
5-chloro-3'-(2-oxo-2-(4-oxo-2-(1-phenylcyclopropyl)-3,4,5,7,8,9-hexahydro-6H-pyrimido[5,4-c]azepin-6-yl)ethyl)-[1,1'-biphenyl]-3-carbonitrile;
6-(2-(2-(benzyloxy)phenyl)acetyl)-2-(1-phenylcyclopropyl)-3,5, 6, 7, 8, 9-hexahydro-4H-pyrimido[5,4-c]azepin-4-one;
6-(2-(3-(benzyloxy)phenyl)acetyl)-2-(1-phenylcyclopropyl)-3,5, 6 , 7, 8, 9-hexahydro-4H-pyrimido[5,4-c]azepin-4-one;
6-(2-(3'-(tert-butyl)-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
6-(2-(3'-chloro-5'-fluoro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
6-(2-(3'-cyclopropyl-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
6-(2-(4'-chloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2,2-difluoro-2-(3'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
6-(2,2-difluoro-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
6-(2,2-difluoro-2-(3'-isopropoxy-[1,1'-biphenyl]-3-yl)acetyl)-2-(1-phenylcyclopropyl)-3,5,6,7,8,9-hexahydro-4H-pyrimido[5,4-c]azepin-4-one; and
6-(2-hydroxy-2-(3'-(trifluoromethoxy)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
or a pharmaceutically acceptable salt thereof.

[7] The use according to any one of [1] to [4], wherein the compound having TRPV4 inhibitory activity is a polymer that is an antibody, oligonucleotide, siRNA or aptamer that binds to TRPV4 (GeneID: 59341).

[8] The use according to any one of [1] to [7], comprising combination with one or more second active agents.

[9] A pharmaceutical composition comprising a compound having TRPV4 inhibitory activity or a pharmaceutically acceptable salt thereof, which is used for preventing or treating a retinal disease accompanied with blood flow disorder or cell disorder in humans or animals.

[10] The pharmaceutical composition according to [9], wherein the retinal disease is retinal vein occlusion or wet age-related macular degeneration.

[11] The pharmaceutical composition according to [9], wherein the retinal disease is branch retinal vein occlusion or central retinal vein occlusion.

[12] The pharmaceutical composition according to [9], wherein the compound having TRPV4 inhibitory activity is the compound according to any one of [5] to [7] or a pharmaceutically acceptable salt thereof.

[13] A method for treating a retinal disease accompanied with blood flow disorder or cell disorder, which comprises administering to humans or animals an effective amount of a compound having TRPV4 inhibitory activity or a pharmaceutically acceptable salt thereof.

[14] The method according to [13], wherein the retinal disease is retinal vein occlusion or wet age-related macular degeneration.

[15] The method according to [13], wherein the compound having TRPV4 inhibitory activity is the compound according to any one of [5] to [7] or a pharmaceutically acceptable salt thereof.

[16] A compound having TRPV4 inhibitory activity or a pharmaceutically acceptable salt thereof for use in treating a retinal disease accompanied with blood flow disorder or cell disorder in humans or animals.

[17] A kit for use in preventing or treating the diseases, comprising the compound according to any one of [5] to [7] above.

[18] A kit according to [17] comprising a pharmaceutical composition according to any one of [9] to [12] above, one or more second active agents and a container.

[19] A disease marker for a retinal disease, which is a polynucleotide having at least 15 contiguous bases in the TRPV4 gene sequence shown in any of SEQ ID NOs 1 to 5 (Gene Bank ACCESSION: NM_021625, NM_147204, NM_001177433, NM_001177431 or NM_001177428) and/or complementary to the said polynucleotide.

[20] The disease marker according to [19], which is used as a probe or primer in detecting a retinal disease.

[21] A disease marker for a retinal disease, which is an antibody that recognizes the TRPV4 protein comprising of the amino acid sequences shown in SEQ ID NOs: 1 to 5 above.

[22] The disease marker according to [21], which is used as a probe in detecting a retinal disease.

[23] A method for detecting a retinal disease comprising the following steps (a), (b) and (c):
(a) a step of combining RNA prepared from a subject's biological sample or a complementary polynucleotide transcribed from the RNA with the disease marker according to [19] or [20];
(b) a step of measuring RNA derived from the biological sample that binds to the disease marker or a complementary polynucleotide transcribed from the RNA, using the above disease marker as an indicator; and
(c) a step of diagnosing the incidence of the retinal disease based on the measurement result of (b) above.

[24] The method for detecting the retinal disease according to [23], wherein diagnosing the incidence of the retinal disease in the step (c) is made by comparing the measurement results obtained for the subject with those obtained for a normal subject, using an increased amount of binding to the disease marker as an indicator.

[25] A method for detecting a retinal disease comprising the following steps (a), (b) and (c):
(a) a step of combining a protein prepared from a subject's biological sample with the disease marker according to [21] or [22];
(b) a step of measuring a protein or a partial peptide thereof derived from the biological sample that binds to the disease marker, using the above disease marker as an indicator; and
(c) a step of diagnosing the incidence of the retinal disease based on the measurement result of (b) above.

[26] The method for detecting the retinal disease according to [25], wherein diagnosing the incidence of the retinal disease in the step (c) is made by comparing the measurement results obtained for the subject with those obtained for a normal subject, using an increased amount of binding to the disease marker as an indicator.

[27] A screening method for a substance that suppresses TRPV4 gene expression, comprising the following steps (a), (b) and (c):
(a) a step of contacting a test substance with cells capable of expressing the TRPV4 gene;
(b) a step of measuring the expression level of the TRPV4 gene in cells contacted with the test substance, and comparing the said expression level with the expression level of the said gene in control cells not contacted with the test substance; and
(c) a step of selecting a test substance that reduces the expression level of the TRPV4 gene based on the comparison result of (b) above.

[28] A screening method for a substance that reduces the expression level of a TRPV4 protein, comprising the following steps (a), (b) and (c) :
(a) a step of contacting a test substance with a cell capable of expressing a TRPV4 protein or a cell fraction prepared from the said cell;
(b) a step of measuring the expression level of the TRPV4 protein in cells or cell fractions contacted with the test substance, and comparing the said expression level with the expression level of the TRPV4 protein in control cells or cell fractions not contacted with the test substance; and
(c) a step of selecting a test substance that reduces the expression level of the TRPV4 protein based on the comparison result of (b) above.

[29] A method of screening for a substance that inhibits TRPV4 activity, comprising the following steps (a), (b), and (c):
(a) a step of contacting a TRPV4 ligand with a TRPV4 protein in the presence of a test substance;
(b) a step of measuring the binding amount of the TRPV4 ligand to the TRPV4 protein in the presence of the test substance, and comparing the binding amount with the binding amount of the TRPV4 ligand to the TRPV4 protein in the absence of the test substance (control binding amount); and
(c) a step of selecting a test substance that reduces the binding amount comparing to the control binding amount based on the comparison result of (b) above.

[30] The screening method according to any one of [27] to [29], wherein the method is for searching an active ingredient of an agent for preventing or treating a retinal disease.

[31] An agent for preventing or treating a retinal disease, comprising a substance that suppresses TRPV4 gene expression as an active ingredient.

[32] The agent for preventing or treating a retinal disease according to [31], wherein the substance that suppresses TRPV4 gene expression is obtained by the screening method according to [27].

[33] An agent for preventing or treating a retinal disease, comprising a substance that suppresses the expression level of a TRPV4 protein or a substance that inhibits the activity of TRPV4 as an active ingredient.

[34] The agent for preventing or treating a retinal disease according to [33], wherein the substance that suppresses the expression level of a TRPV4 protein or the substance that inhibits the activity of TRPV4 is obtained by the screening method according to [28] or [29].

The following compounds disclosed in patent literature US63017891 (application number), PCT/JP2021/17277 (application number), WO2013012500, WO2011119704 or WO2013169396 are represented as Compounds A, B, C and D for convenience.
A TRPV4 inhibitor Compound A: (R)-6-(2-(3-chlorophenyl)-2-hydroxyacetyl)-2-(1-phenylcyclopropyl)-5,6,7,8-tetrahydropyrido[4,3-d]pyrimidin-4(3H)-one, (The English name of Compound A is (R)-6-(2-(3-chlorophenyl)-2-hydroxyacetyl)-2-(1-phenylcyclopropyl)-5,6,7,8-tetrahydropyrido[4,3-d]pyrimidin-4(3H)-one.),
Compound B (GSK2798745, CAS No. 1419609-94-1): 1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile, (The English name of Compound B is 1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile),
Compound C (GSK2193874, CAS No. 1336960-13-4): 3-(1,4'-bipiperidin-1'-ylmethyl)-7-bromo-N-(1-phenylcyclopropyl)-2-[3-(trifluoromethyl)phenyl]-4-quinolinecarboxamide, (The English name of Compound C is 3-(1,4'-bipiperidin-1'-ylmethyl)-7-bromo-N-(1-phenylcyclopropyl)-2-[3-(trifluoromethyl)phenyl]-4-quinolinecarboxamide), and
Compound D (HC-067047, CAS No. 883031-03-6): N-(3-(trifluoromethyl)phenyl)-2-methyl-1-(3-morpholinopropyl)-5-phenyl-1H-pyrrole-3-carboxamide, (The English name of Compound D is N-(3-(trifluoromethyl)phenyl)-2-methyl-1-(3-morpholinopropyl)-5-phenyl-1H-pyrrole-3-carboxamide.)

### [Brief Explanation of Drawings]

[Figure 1] Figure 1 shows the evaluation by Western blotting on TRPV4 channel expression in the pathogenesis of a mouse model of retinal vein occlusion.
   Data are expressed as mean ± standard error (N=5). *P < 0.05, **P < 0.01 (vs. Normal) Tested with Dunnett's test.
[Figure 2] Figure 2 shows histological changes after intravitreal administration of Compound A, a TRPV4 inhibitor, to a mouse model of retinal vein occlusion.
   Scale bar = 50 µm, the retinal inner nuclear layer (INL), and the retinal outer nuclear layer (ONL).
[Figure 3] Figure 3 shows the thickness of the retinal inner nuclear layer (INL) of a mouse model of retinal vein occlusion after intravitreal administration of Compound A.
   Data are expressed as mean ± standard error (N=10). ##P < 0.01 (vs. Normal), **P < 0.01, *P < 0.05 (vs. vehicle) Tested with Student's t-test.
[Figure 4] Figure 4 shows histological changes after intravitreal administration of Compounds B, C and D, which are TRPV4 inhibitors, to a mouse model of retinal vein occlusion.
   Scale bar = 50 um, the retinal inner nuclear layer (INL), and the retinal outer nuclear layer (ONL).
[Figure 5] Figure 5 shows the thickness of the retinal inner nuclear layer (INL) after intravitreal administration of Compounds B, C and D to a mouse model of retinal vein occlusion.
   Data are expressed as mean ± standard error (N=10). ##P < 0.01 (vs. Normal), **P < 0.01 (vs. vehicle) Tested with Student's t-test.
[Figure 6] Figure 6 shows the cell permeability of Compound A using human retinal microvascular endothelial cells (HRMEC).
   Data are expressed as mean ± standard error (N=4). ##P < 0.01 (vs. Control) Tested with Student's t-test, **P < 0.01 (vs. VEGF) Tested with Dunnett's test, ++P < 0.01 (vs. GSK1016790A) Tested with Student's t-test.
[Figure 7] Figure 7 shows the cell permeability of Compounds B, C and D using human retinal microvascular endothelial cells (HRMEC). Data are expressed as mean ± standard error (N=4-5). ##P < 0.01 (vs. Control) Tested with Student's t-test, **P < 0.01 (vs. VEGF) Tested with Dunnett's test.
[Figure 8] Figure 8 shows the expression of TNFα (Tumor Necrosis Factor), an inflammation-related factor, after intravitreal administration of Compound A to a mouse model of retinal vein occlusion, evaluated by Western blotting.
   Data are expressed as mean ± standard error (N=5). ##P < 0.01 (vs. Normal) Tested with Student's t-test, **P < 0.01 (vs. vehicle) Tested with Student's t-test.
[Figure 9] Figure 9 shows the retinal thickness after intravitreal administration of Compound A to a mouse model of retinal vein occlusion.
   Scale bar = 50 um, the retinal inner nuclear layer (INL), and the ret inal outer nuclear layer (ONL).
   Data are expressed as mean ± standard error (N=5). **P < 0.01 (vs. vehicle) Tested with Turkey's t-test.
[Figure 10] Figure 10 shows ocular blood flow after intravitreal admi nistration of Compound A to a mouse model of retinal vein occlusion. Data are expressed as mean ± standard error (N=10). **P < 0.01 (vs. vehicle) Tested by Student's t-test.
[Figure 11] Figure 11 shows the percentage of nonperfusion area in the retina after intravitreal administration of Compound A to a mouse model of retinal vein occlusion. Scale bar = 50 um.
   Data are expressed as mean ± standard error (N=10). **P < 0.01 (vs. vehicle) Tested with Student's t-test.

### [Description of Embodiments]

All patents, patent applications and publications, and other references cited herein, are in their entirety incorporated by reference into the present specification. The disclosures of these publications are in their entirety incorporated by reference into the present application to more fully describe the state of the art known to those skilled in the art on the date of the inventions described and claimed in the present specification.

The inventors of the present application have found that TRPV4 inhibitors not only suppress retinal edema and increase vascular permeability, which are effective in the treatment of retinal diseases, especially ocular diseases, but also surprisingly suppress inflammatory cytokines, suppress thinning of retinal layer structure, improve blood flow, and improve retinal non-perfusion, which may affect poor prognosis of visual function.

The retinal disease is more specifically a retinal disease accompanied with blood flow disorder or cell disorder, and may be a retinal disease accompanied with both blood flow disorder and cell disorder.

The retinal disease is selected from the group consisting of hypertensive retinopathy and amaurosis, which are a retinal disease accompanied with blood flow disorder; or selected from the group consisting of hereditary optic neuropathy, retinal detachment, choroidal metastases, choroidal melanoma and choroidal hemangioma, which are a retinal disease accompanied with cell disorder; or selected from the group consisting of retinal vein occlusion, retinal artery occlusion, wet age-related macular degeneration, retinitis pigmentosa, diabetic retinopathy, ischemic optic neuropathy, and glaucoma, which are retinal diseases accompanied with both blood flow disorder and cell disorder. The above specific diseases are for better understanding of the present invention and are not limited to the scope of the present invention.

The compound of the present invention is useful as a therapeutic agent that exhibits excellent effects in patients in need of treatment for a retinal disease.

A compound having TRPV4 inhibitory activity may be a naturally occurring compound or an artificially synthesized compound. Moreover, it may be a low-molecular-weight compound or a high-molecular-weight compound such as protein or nucleic acid.

Specifically, the high-molecular-weight compound includes an antibody or peptide that specifically binds to TRPV4, an antibody or peptide that specifically binds to a ligand that activates TRPV4, and the like. Antibodies can be polyclonal or monoclonal antibodies. They can also be complete antibody molecules or antibody fragments (e.g., Fab, F(Ab')2, Fab', Fv, scFv, etc.) that can bind specifically to the antigen. Human chimeric or humanized antibodies are preferred. Such antibodies include, in particular, neutralizing antibodies (anti-TRPV4 neutralizing antibodies) that have the property of inhibiting the original function or activity by binding to TRPV4 (antigen).

The high-molecular-weight compounds may be nucleic acid medicines including antisense oligonucleotides and siRNA (short interfering RNA) that suppress TRPV4 expression, aptamers that specifically bind to TRPV4, aptamers that inhibit physical or chemical stimulators that cause activation of TRPV4, and the like.

Antibodies that specifically bind to TRPV4 can be produced by known methods using TRPV4 or its fragments as antigens. Nucleotide and amino acid sequence information encoding TRPV4 of major mammals including human can be obtained from publicly known databases (DDBJ/GenBank/EMBL, *etc.*)*.* Human TRPV4 is also called transient receptor potential cation channel subfamily V member 4, and is known to correspond to nucleotide sequences such as GenBank accession numbers: NM_021625, NM_147204, NM_001177433, NM_001177431 or NM_001177428 (SEQ ID NO: 1, 2, 3, 4 or 5). Recombinant TRPV4 produced using the genetic information and known gene recombination techniques can be used as an antigen. A peptide that specifically binds to TRPV4 can be produced by a solid phase synthesis method (Fmoc method, Boc method) or a liquid phase synthesis method according to a known general peptide synthesis protocol. Pharmaceutically acceptable carriers, additives, and the like can be included.

Aptamers that bind specifically to TRPV4 can be obtained by a selection method called SELEX (systemic evolution of ligands by exponential enrichment) (Biomolecular Engineering 2007_v24_p381-403) .

Prototype aptamers obtained by SELEX are finished as medicaments by minimizing chain length, introducing modified nucleic acids, adding PEG, *etc.* (Folia Pharmacol Jpn, 2016, 147: 362-367).

The term "pharmaceutically acceptable salt", as used herein, means any acid or base of a medicament or an active metabolite or residue thereof. As is known to those of skill in the art, "salts" of the compounds of the present invention may be derived from inorganic or organic acids and bases, amino acids, and the like. Examples of inorganic acids include, but are not limited to, hydrochloric acid, hydrobromic acid, sulfuric acid, nitric acid, perchloric acid, phosphoric acid, and the like. Examples of organic acids include fumaric acid, maleic acid, glycolic acid, lactic acid, salicylic acid, succinic acid, toluene-p-sulfonic acid, tartaric acid, acetic acid, citric acid, methanesulfonic acid, ethanesulfonic acid, formic acid, benzoic acid, malonic acid, naphthalene-2-sulfonic acid, benzenesulfonic acid, and the like. In addition, when the free active ingredient contains an acidic group, examples of the base include sodium carbonate (alkali metal salts such as sodium and potassium), calcium carbonate, calcium hydroxide, magnesium carbonate, magnesium silicate, and magnesium aluminate, and the like (alkaline earth metal salts such as calcium and magnesium). Examples of amino acids include aspartic acid, glutamic acid, arginine, and the like.

Humans or animals to be prevented or treated in the present invention include, for example, humans and other mammals. Mammals include mice, rats, rabbits, hamsters, sheep, monkeys, pigs, cats, dogs, cows, horses, and the like. In addition, test organisms to which a test substance is to be contacted, ingested, or administered include human or animal individuals, tissues, cells (including cultured cells), and the like. Cells are not limited, but include cell lines derived from mammals such as humans, mice, and rats, cell lines derived from these cell lines, primary cells derived from various tissues, stem cells, ES cells, and iPS cells. Cells are preferably eye-related cells, and such cells include retinal cells (photoreceptor cells, nerve cells), pigment epithelium/choroidal cells, and the like.

The term "treatment", as used herein, means reversing, reducing, inhibiting, or preventing the progression of one or more of the diseases, disorders, or symptoms of ocular diseases described herein. It also includes alleviating symptoms and improving quality of life. The term "prevention" means suppressing the onset of symptoms.

As appreciated by those of skill in the art, "halogen" as used herein is intended to include fluoro, chloro, bromo, and iodo. Similarly, 1-6, as in C₁₋₆ is defined to identify the number as having 1, 2, 3, 4, 5, or 6. According to the definition, for example, C₁₋₆, as in C₁₋₆ alkyl is defined to identify the alkyl group as having 1, 2, 3, 4, 5, or 6 carbons. Similarly, C₂₋₆ alkenyl is defined to identify the alkenyl group as having 2, 3, 4, 5, or 6 carbons. A group which is designated as being independently substituted with substituents may be independently substituted with multiple numbers of such substituents.

The term "alkyl", as used herein, means a linear saturated monovalent hydrocarbon radical of one to six carbon atoms or a branched saturated monovalent hydrocarbon radical of three to six carbon atoms, e.g., methyl, ethyl, propyl, 2-propyl, butyl (including all isomeric forms), pentyl (including all isomeric forms), and the like.

The term "alkoxy", as used herein, means an -O-alkyl such as, but not limited to, methoxy, ethoxy, propoxy, 2-propoxy, butoxy (including all isomeric forms), and the like.

The term "cycloalkyl", as used herein, means a mono- or bi-cyclic ring such as, but not limited to, cyclopropyl, cyclobutyl, cyclopentyl, cyclohexyl, cycloheptyl, norbornyl, adamantyl groups, and the like. In this specification, preferable cycloalkyl is C₃₋₇ mono cycloalkyl, more preferable cycloalkyl is C₃₋₆ mono cycloalkyl, further more preferable cycloalkyl is C₃₋₅ mono cycloalkyl.

The term "aryl", as used herein, means unsaturated or partially saturated mono- or bi-cyclic 5 to 15-membered ring which consists of carbon atoms. Examples of such aryl include, but are not limited to, phenyl, naphthyl, indanyl, indenyl, 1,2,3,4-tetrahydronaphthyl, 1,2-dihydronaphthyl, 2,3-dihydro-1*H*-indenyl, cyclohexenyl, cyclopentenyl, (1S,4S)-bicyclo[2.2.2]oct-2-enyl, and (1R,4S)-bicyclo[2.2.1]hept-2-enyl, and the like. In this specification, preferable aryl is 6 to 10-membered unsaturated aryl, more preferable aryl is 6 to 8-membered unsaturated aryl.

The term "heteroaryl", as used herein, means unsaturated or partially saturated mono- or bi-cyclic 5 to 15-membered ring with 1-4 heteroatoms independently selected from O, N, and S, preferably unsaturated or partially saturated mono- or bi-cyclic 5 to 10-membered ring. Preferable heteroaryl, as used herein, is 5 to 6-membered monoheteroaryl, and more preferable heteroaryl is 5 to 6-membered N-containing monoheteroaryl.

Examples of such heteroaryl include, but are not limited to, thiophenyl, thiazolyl, isoxazolyl, pyrazolyl, pyrazyl, tetrazolyl, furanyl, pyrrolyl, imidazolyl, oxazolyl, isothiazolyl, triazolyl, thiadiazolyl, pyridyl, pyrimidyl, pyridazinyl, pyrazinyl, pyranyl, triazinyl, and N-oxides thereof and S-oxides thereof.

The term "heterocyclyl" as used herein includes both unsaturated and saturated heterocyclic moieties, wherein the unsaturated heterocyclic moieties (*i.e.,* "heteroaryl") include, but not limited to, benzofuranyl, benzofurazanyl, benzimidazolonyl, benzoimidazolyl, benzoisothiazolyl, benzoisoxazolyl, benzothiadiazolyl, benzothiazolyl, benzoxadiazolyl, benzoxazolonyl, benzoxazolyl, benzothiophenyl, benzotriazolyl, carbazolyl, carbolinyl, chromanyl, cinnolinyl, 2,3-dioxoindolyl, furanyl, furazanyl, furopyridyl, furopyrrolyl, imidazolyl, imidazopyrazinyl, imidazopyridinyl, imidazopyrimidinyl, imidazothiazolyl, indazolyl, indolazinyl, indolinyl, indolyl, isobenzofuranyl, isochromanyl, isoindolyl, isoquinolyl, isoxazolopyridyl, isoxazolinyl, isoxazolyl, isothiazolyl, naphthyridinyl, oxazolinyl, oxadiazolyl, oxazolyl, oxetanyl, 2-oxoindolyl, oxoisoindolyl, phthalazyl, pyrazolyl, pyrazolopyridyl, pyrazolopyrimidinyl, pyrazinyl, pyridyl, pyrimidyl, pyridazinyl, pyridopyrimidinyl, pyrrolopyridyl, pyrrolyl, quinazolinyl, quinolyl, quinoxalinyl, tetrazolopyridyl, tetrazolyl, thiadiazolyl, thiazolyl, thiophenyl, thienopyrazinyl, thienopyrazolyl, thienopyridyl, thienopyrrolyl, triazolopyrimidinyl, triazolyl, and N-oxides thereof and S-oxides thereof. In this invention, preferable heterocyclyl is 3 to 6-membered saturated mono heterocyclyl which may contain 1-4 heteroatoms selected from O, N, and S, more preferable heterocyclyl is 5 to 6-membered saturated mono heterocyclyl.

The term "alkenyl", as used herein, means a hydrocarbon radical having at least one double bond including, but not limited to, ethenyl, propenyl, 1-butenyl, 2-butenyl, and the like.

The term "alkylthio", as used herein, means a straight or branched alkylthio group, and includes, for example, a methylthio group, an ethylthio group, a propylthio group, an isopropylthio group, a n-butylthio group, a sec-butylthio group, a tert-butylthio group, a pentylthio group, an isopentylthio group, a neopentylthio group, a n-hexylthio group, an isohexylthio group, a sec-hexylthio group, and the like.

The term "haloalkylthio", as used herein, means a group wherein at least one hydrogen atom of the straight or branched alkylthio group is substituted with a halogen atom, and includes, for example, a monofluoromethylthio group, a difluoromethylthio group, a trifluoromethylthio group, a trichloromethylthio group, a tribromomethylthio group, a triiodomethylthio group, a chlorofluoromethylthio group, a pentafluoroethylthio group, a pentachloroethylthio group, a pentabromoethylthio group, a pentaiodoethylthio group, a 2,2,2-trichloroethylthio group, and the like.

Certain embodiments of the invention are used in combination with one or more second active agents for diagnosing, preventing or treating a retinal disease.

Examples can be used as "second active agents" include, but are not limited to, anti-VEGF agents (*e.g.,* aflibercept (EYLEA (registered trademark); VEGF Trap), ranibizumab (LUCENTIS (registered trademark); rhuFab V2), bevacizumab (AVASTIN (registered trademark); rhuMab-VEGF), brolucizumab (BEOVU (registered trademark); scFv), nucleic acids (*e.g.,* pegaptanib (MACUGEN (registered trademark); PEGylated RNA aptamer) and siRNA against VEGF RNA), VEGF-inhibiting fusion proteins (VEGF-Trap; see, *e.g.,* US7,087,411), small molecule kinase inhibitors of VEGF receptors (*e.g.,* sunitinib, sorafenib or pazopanib), retinal circulation improving drugs (*e.g.,* kallidinogenase, cilostazol, or ticlopidine hydrochloride salts), steroids (*e.g.,* corticosteroids, dexamethasone, triamcinolone, fluocinolone, or intravitreal steroid implants), nonsteroidal anti-inflammatory drugs (e*.g.,* NSAIDs, diclofenac, pranoprofen, bromfenac sodium or nepafenac), prostagrandins (prostaglandin F2α derivatives: prostoids such as latanoprost, travoprost, tafluprost, and bimatoprost; prostamides; prostanoids; and prostones such as isopropyl unoprostone), sympatholytics (*e.g.,* non-selective β-blockers such as timolol maleate, gelling timolol, carteolol hydrochloride, gelling carteolol; sympathetic nerve β1-selective β-blockers such as betaxolol hydrochloride; sympathetic nerve α1 blockers such as bunazosin hydrochloride; sympathetic nerve α2 blockers; and sympathetic nerve αβ blockers such as levobunolol and hydrochloride, nipradilol), sympathomimetics (non-selective stimulants; α2 agonists, e.g., epinephrine bitartrate, dipivefrine hydrochloride, and brimonidine tartrate), parasympathomimetics (cholinergics; cholinesterase inhibitors, *e.g.,* pilocarpine hydrochloride, and distigmine bromide), carbonic anhydrase inhibitors (*e.g.,* dorzolamide hydrochloride, acetazolamide, and brinzolamide), ROCK inhibitors (*e.g.,* fasudil hydrochloride), calcium antagonists (*e.g.,* lomerizine hydrochloride), EP2 agonists (*e.g.,* omidenepag isopropyl), adenosine A2a receptors agonists, blood vessel strengthening/hemostatic agents (*e.g.,* ascorbic acid, carbazochrome sodium sulfonate), vitamin B12 (*e.g.*, mecobalamin), dark adaptation improving agents (*e.g.,* helenien), calcium channel blockers (*e.g.,* nifedipine, and nilvadipine), fundus examination drug (*e.g.,* tropicamide, phenylephrine hydrochloride), and the like.

The present invention provides novel disease markers associated with TRPV4 channels regarding a retinal disease accompanied with blood flow disorder or cell disorder. Since the expression of the TRPV4 gene is elevated in ocular tissue with retinal disease accompanied with blood flow disorder or cell disorder comparing to ocular tissue in a normal state (control group), these genes and their expression products [proteins, (Poly)(oligo)peptides] or antibodies can be effectively used for the elucidation and diagnosis of a retinal disease.

The present invention provides a method of screening for drugs that are effective in preventing or treating a retinal disease accompanied with blood flow disorder or cell disorder.

The test substances to be screened include nucleic acids, antibodies, peptides, proteins, non-peptidic compounds, synthetic compounds, fermentation products, cell extracts, cell culture supernatants, plant extracts, mammalian tissue extracts and plasma, *etc.,* but are not limited to these examples. The test substances may be novel or known substances. These test substances may be in the form of a salt. The salt is composed of the test substance with a physiologically acceptable acid or base.

Method for evaluating the inhibitory function or restorative function of a test substance having TRPV4 inhibitory activity against a retinal disease accompanied with blood flow disorder or cell disorder:
The test substances may be novel or known substances.

This means a method for evaluating the status, inhibitory or restorative function of a test substance against a retinal disease accompanied with blood flow disorder or cell disorder by detecting TRPV4 gene expression or functional activity present in samples collected from test organisms contacted, ingested or administered with the test substance and comparing the results of such detection with controls.

For example, regarding the TRPV4 gene present in the sample collected from the test organism, the state of a retinal disease accompanied with blood flow disorder or cell disorder in the test organism can be evaluated by comparing the gene expression level or functional activity in a normal test organism (control, placebo, blank, solvent only, *etc.*) with that in a test organism having a retinal disease accompanied with blood flow disorder or cell disorder and then by confirming their changes (elevation or reduction) or the presence or absence of their changes.

### Samples collected from test organisms:

In the present invention, a sample collected from a test organism refers to a biological sample isolated or collected from a test organism. The sample includes body fluids such as blood, etc., parts of other tissues or organs, lysate, cell lysate, or extracts taken therefrom can be mentioned. Samples for continuous measurement are preferably cell lysates, body fluids such as blood, *etc.* When directly measuring the state of a retinal disease accompanied with blood flow disorder or cell disorder, the retina, retinal pigment epithelium, and choroid are preferable.

The test substance can be added or taken at any concentration, but it is preferable to use a concentration at which the test substance is not toxic to the test organism.

In the present invention, when the test organism is a cell, the concentration of the cell is not limited, but is preferably a concentration at which a sufficient nucleic acid concentration can be recovered during cell recovery, and the final concentration is 1.0 × 10⁵ cells/well or more, preferably, the final concentration is 5.0 × 10⁵ cells/well or more.

When selecting a substance that inhibits binding of TRPV4 and its ligand, for example, a screening method comprising a step of contacting a test substance with TRPV4 and its ligand, a step of confirming binding of TRPV4 and its ligand, and a step of selecting a test substance that inhibits the binding of TRPV4 and its ligand can be used. TRPV4 may be either a natural protein or a recombinant protein. TRPV4-expressing cells may also be used. Cannabinoids, which are endogenous ligands, and TRPV4 agonists, which are synthetic compounds, can be used as TRPV4 ligands. The activation mechanism of TRPV4 is diverse, and Ca(2+) influx is caused by activation by stimuli such as temperature, mechanical stimulation, osmotic pressure, oxidative stress, and stimulating chemicals.

The method for bringing the test substance into contact with its ligand is not particularly limited. For example, a reaction system containing TRPV4 and its ligand is prepared, and the test substance is added thereto. The contact time and temperature are not particularly limited and can be selected as appropriate. The method for assessing TRPV4 binding to its ligand is not particularly limited, and a known method for determining the level of TRPV4 binding to its ligand can be selected as appropriate. For example, ELISA, fluorescence polarization, flow cytometry, surface plasmon resonance, and the like can be preferably used. In an exemplary method using ELISA, either TRPV4 or its ligand is immobilized, the other one and the test substance are added thereto so that the reaction proceeds, and the level of TRPV4 binding to its ligand is determined with the use of appropriate primary and secondary antibodies.

When the level of TRPV4 binding to its ligand after the contact with the test substance is reduced as compared with that in the control group which is not in contact with the test substance, the test substance can be determined as a desired substance. The selection criterion for the reduction of the level of TRPV4 binding to its ligand is not particularly limited, for example, the desired substance is a substance capable of reducing the level of TRPV4 binding to its ligand to 50% or less, or 25% or less of that in the absence of contact with the test substance.

For the selection of the substance capable of inhibiting the intracellular signal transduction caused by the binding of TRPV4 to its ligand, the screening method can comprise, for example, the following steps:
adding a test substance to a culture system containing TRPV4 expressing cells and its ligand; assessing the signal transduction state downstream of TRPV4; and selecting a substance capable of inhibiting the intracellular signal transduction caused by the binding of TRPV4 to its ligand. Cannabinoids, which are endogenous ligands, and TRPV4 agonists, which are synthetic compounds, can be used as TRPV4 ligands. The activation mechanism of TRPV4 is diverse, and Ca(2+) influx is caused by activation by stimuli such as temperature, mechanical stimulation, osmotic pressure, oxidative stress, and stimulating chemicals.

TRPV4 expressing cells may be endogenous cells expressing TRPV4 or cells expressing recombinant TRPV4. For example, HEK 293 cells co-transfected with a TRPV4 expression vector and a reporter gene-linked vector downstream of a cAMP-responsive element (CRE) can be used. When TRPV4 expressing cells which a reporter gene is introduced into are used, the signal transduction state downstream of TRPV4 can be assessed by measuring the expression level of the reporter gene. The reporter gene is not particularly limited as long as it is generally used, and examples thereof include genes encoding luciferase, β-galactosidase, β-glucuronidase, chloramphenicol acetyltransferase, alkaline phosphatase, peroxidase, green fluorescent protein (GFP), and the like.

When the expression level of the reporter gene after the contact with the test substance is reduced as compared with that in the control group which is not in contact with the test substance, the test substance can be determined as a desired substance. The selection criterion for the reduction of the expression level of the reporter gene is not particularly limited, and for example, the desired substance is a substance capable of reducing the expression level of the reporter gene to 50% or less, or 25% or less of that in the absence of contact with the test substance.

In the present invention, the mode of administration for contact, ingestion, or administration include oral administration, ocular topical administration (such as eye drop administration, instillation in the conjunctival sac, intravitreal administration, subconjunctival administration and sub-Tenon's administration), intravenous administration, intraperitoneal administration, transdermal administration, *etc.,* and as needed, the present compound or composition may be formulated into a preparation suitable for such an administration mode by properly selecting and using a pharmaceutically acceptable additive. When the subject is a cell, it means adding the test substance to the culture medium.

Examples of the dosage form include, in the case of an oral preparation, a tablet, a capsule, a granule and a powder, and, in the case of a parenteral preparation, an injection, an eye drop, an eye ointment, an insert, *etc.*

Examples of the pharmaceutically acceptable carrier used in the compositions include various organic or inorganic carrier substances conventionally used as preparation materials such as excipient, disintegrant, binder, glidant, lubricant, and the like for solid preparations, and solvent, solubilizing agent, suspending agent, isotonic agent, buffering agent, soothing agent, and the like for liquid preparations. As needed, additives such as preservative, antioxidant, colorant, sweetening agent, and the like are used.

For example, in the case of a tablet, a capsule, a granule, a powder or the like, such a preparation can be prepared by properly selecting and using an excipient such as lactose, glucose, D-mannitol, anhydrous calcium hydrogen phosphate, starch or sucrose; a disintegrant such as carboxymethyl cellulose, calcium carboxymethyl cellulose, croscarmellose sodium, crosspovidone, starch, partially gelatinized starch or low-substituted hydroxypropyl cellulose; a binder such as hydroxypropyl cellulose, ethyl cellulose, gum arabic, starch, partially gelatinized starch, polyvinylpyrrolidone or polyvinyl alcohol; a lubricant such as magnesium stearate, calcium stearate, talc, hydrous silicon dioxide or a hydrogenated oil; a coating agent such as purified sucrose, hydroxypropylmethyl cellulose, hydroxypropyl cellulose, methyl cellulose, ethyl cellulose or polyvinylpyrrolidone; a preserving agent such as ethyl parahydroxybenzoate, chlorobutanol, benzyl alcohol, sodium dehydroacetate, and sorbic acid; an antioxidant agent such as sodium sulfite and ascorbic acid; a colorant such as food dye (*e.g.,* Food Red No. 2 and No. 3, and Food Yellow No. 4 and No. 5) and β-carotene; a sweetening agent such as saccharin sodium and dipotassium glycyrrhizate; a corrigent such as citric acid, aspartame, ascorbic acid or menthol; or the like as needed.

For example, in the case of a liquid formulation, such a preparation can be prepared by properly selecting and using a solvent such as purified water, ethanol, propyleneglycol, macrogol, sesame oil, corn oil, and olive oil; a solubilizing agent such as propyleneglycol, D-mannitol, benzyl benzoate, ethanol, triethanolamine, sodium carbonate, and sodium citrate; a suspending agent such as benzalkonium chloride, carmellose, hydroxypropyl cellulose, propyleneglycol, povidone, methylcellulose, and glyceryl monostearate; a soothing agent benzyl alcohol; or the like as needed.

An injection can be prepared by properly selecting and using a tonicity agent such as sodium chloride; a buffer such as sodium phosphate; a surfactant such as polyoxyethylene sorbitan monoolate; a viscosity-increasing agent such as methyl cellulose; or the like as needed.

An eye drop can be prepared by properly selecting and using a tonicity agent such as sodium chloride or concentrated glycerin; a buffer such as sodium phosphate or sodium acetate; a surfactant such as polyoxyethylene sorbitan monoolate, polyoxyl 40 stearate or polyoxyethylene hydrogenated castor oil; a stabilizer such as sodium citrate or sodium edetate; a preservative such as benzalkonium chloride or paraben; or the like as needed. The pH of the eye drop is permitted as long as it falls within the range that is acceptable as an ophthalmic preparation, but is preferably in the range of from 4 to 8, and more preferably in the range of from 5 to 7. As a pH adjusting agent, a normal pH adjusting agent, for example, sodium hydroxide and/or hydrochloric acid may be used.

An eye ointment can be prepared using a widely used base such as white petrolatum or liquid paraffin.

An insert can be prepared using a biodegradable polymer such as hydroxypropyl cellulose, hydroxypropylmethyl cellulose, carboxy vinyl polymer or polyacrylic acid, and if necessary, an excipient, a binder, a stabilizer, a pH adjusting agent or the like can be properly selected and used as appropriate.

A preparation for intraocular implant can be prepared using a biodegradable polymer such as polylactic acid, polyglycolic acid, a lactic acid-glycolic acid copolymer or hydroxypropyl cellulose, and if necessary, an excipient, a binder, a stabilizer, a pH adjusting agent or the like can be properly selected and used as appropriate.

The preferable material of the resinous container consists essentially of polyethylene. The container material may contain minor amounts of other materials than polyethylene, for example polypropylene, polyethylene terephthalate, polyvinyl chloride, acrylic resins, polystyrene, polymethyl methacrylate and nylon 6. The amount of said materials is preferably no more than about 5 to 10% of the total container material. Polyethylene is classified to several types by the density thereof, namely low density polyethylene (LDPE), medium density polyethylene (MDPE), high density polyethylene (HDPE), *etc.* and these polyethylenes are included in this invention. Preferable polyethylene is LDPE.

Containers for packaging and storing the aqueous ophthalmic solution according to the invention include all container forms suitable for user-friendly topical ophthalmic delivery. Consequently, the containers may be selected for example from the group consisting of bottles, tubes, ampoules, pipettes and fluid dispensers, in single unit dose form or in multidose form. According to a preferred embodiment of the invention, the aqueous ophthalmic solution is in a single dose or unit dose form.

The containers for the ophthalmic solution according to the invention are preferably manufactured by extrusion blow moulding method. Extrusion blow moulding gives smoother inner surface of the container comparing to injection blow moulding, which is commonly used to manufacture for example polyethylene multidose bottles. The smoother inner surface gives better chemical stability of prostaglandins in the polyethylene container comparing to polyethylene container manufactured by injection blow moulding. Furthermore, when single-dose containers are used, they are sterilized during the moulding process by heat and no additional sterilization of containers is needed, which also improves stability of prostaglandins in a single-dose container (see EP 1 825 855 and EP 1 349 580). In general, a unit dose ophthalmic container manufactured by blow moulding method has a volume of about 1 ml and about 0.2 to 0.5 ml of solution is filled. A large variety of shapes are known in such containers. Typical examples are seen in US 5,409,125 and US 6,241,124.

Although unit dose containers are preferred for the purposes of the invention, the aqueous ophthalmic solution according to the invention remains soluble, stable and bioavailable also in fluid dispensers for dispensing of minute amounts of germ-free fluid or in any other container-type wherein the aqueous ophthalmic solution is in contact with container material consisting essentially of polyethylene. Such fluid dispensers are disclosed for example in US 5,614,172.

The preservative-free aqueous ophthalmic solution according to the invention can be stored at room temperature in the above-mentioned suitable containers, including unit dose pipettes and dispensers.

A preferred embodiment according to the invention is an aqueous ophthalmic solution, which comprises 0.0001 - 0.01 % w/v of a compound, or a pharmaceutically acceptable salt thereof as an active ingredient; 0.05 - 0.5% w/v non-ionic surfactant; 0.005 - 0.2% w/v stabilizing agent; substantially no preservatives, and optionally buffering agents, pH adjusters and tonicity agents conventionally used in ophthalmic solutions, in a container consisting essentially of polyethylene.

The dose of the present compound can be properly selected depending on the dosage form, symptoms, age, body weight of a patient or the like. For example, in the case of oral administration, it can be administered in an amount of from 0.01 to 5000 mg, preferably from 0.1 to 2500 mg, particularly preferably from 0.5 to 1000 mg per day in a single dose or several divided doses. In the case of an injection, it can be administered in an amount of from 0.00001 to 2000 mg, preferably from 0.0001 to 1500 mg, particularly preferably from 0.001 to 500 mg per day in a single dose or several divided doses. In the case of an eye drop, a preparation containing the present compound at a concentration of from 0.00001 to 10% (w/v), preferably from 0.0001 to 5% (w/v), particularly preferably from 0.001 to 1% (w/v) can be instilled into the eye once or several times a day. In the case of an eye ointment, a preparation containing the present compound in an amount of from 0.0001 to 2000 mg can be applied. In the case of an insert or a preparation for intraocular implant, a preparation containing the present compound in an amount of from 0.0001 to 2000 mg can be inserted or implanted.

The present invention also relates to combining separate pharmaceutical compositions in kit form. The kit comprises a container for containing the separate compositions such as a divided bottle or a divided foil packet, however, the separate compositions may also be contained within a single, undivided container. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses are formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. The tablet or capsule can then be removed via said opening.

A kit product, prefilled syringe, which comes prefilled with desired medicament in a syringe container, can be provided.

### [EXAMPLE]

The present invention is explained in more detail in the following by referring to Reference Examples and Examples, which are not to be construed as limitative but just typical examples.

The compound of formula (1) is a TRPV4 inhibitor disclosed in US63017891 (application number) or PCT/JP2021/17277 (application number).

The compound of formula (1) can be easily prepared by a known method or a method known *per se.* For example, it can be prepared by the manufacturing method shown below. (Formula (I) in the preparation method below corresponds to formula (1) above.)

All compounds of formula (I) can be prepared by the procedures described in the general methods given below, or routine modifications thereof.

In the following general methods, descriptors (R¹, R², R³, q, r, s, Ar¹, Ar², and X) are as previously defined for the compound of the formula (I) unless otherwise stated. All starting materials in the following general syntheses may be commercially available or obtained by the conventional methods known to those skilled in the art, otherwise noted in the Intermediate synthesis part.

### General Synthesis

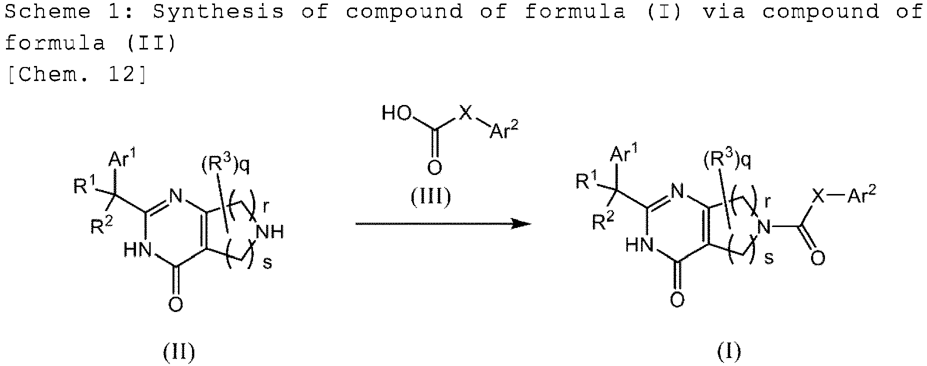

In this Scheme 1, an amide compound of formula (I) can be prepared by the coupling reaction of an amine compound of formula (II) with the acid compound or acid chloride of formula (III) in the presence or absence of a coupling reagent in an inert solvent. This reaction can be also carried out *via* activated carboxylic derivatives. Suitable coupling reagents are those typically used in peptide synthesis including, for example, not limited to, phosgene, triphosgene, ethyl chloroformate, isobutyl chloroformate, diphenyl phosphoryl azide (DPPA), diethyl phosphoryl cyanide (DEPC), carbodiimide condensing agent, imidazolium condensing agent, phosphonium condensing agent, uronium and guanidinium condensing agent, and other coupling reagents. The reaction can be carried out in the presence of a base such as, 1-hydroxybenzotriazole (HOBt), 1-hydroxy-7-azabenzotriazole (HOAt), N,N-diisopropylethylamine, N-methylmorpholine or triethylamine. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: acetone; nitromethane; DMF; NMP; sulfolane; DMSO; 2-butanone; acetonitrile; halogenated hydrocarbons, such as DCM, dichloroethane, chloroform; and ethers, such as THF and 1,4-dioxane. The reaction can take place over a wide range of temperatures. In general, it is convenient to carry out the reaction at a temperature of from -20 °C to 100 °C (°C is Celsius, same hereafter). The time required for the reaction can also vary widely, depending on many factors, notably the reaction temperature and the nature of the reagents and solvent employed. Provided that the reaction is effected under the preferred conditions outlined above, a period of from 5 minutes to 1 week. Then, the resulting acyl halide derivative can be reacted with an amine compound of formula (II) as described above to provide a compound of formula (I). A compound of racemic formula (I) is separated to their optical active form using chiral column including, for example, not limited to, Daicel Chiralpak AD-H, ASH, IA, IB, IC, Daicel Chiralcel OJ-H, OD-H, OK, OG, OA, OC and OB-H as the eluent including, for example, not limited to, n-Hexane, EtOAc, EtOH, isopropanol, acetonitrile, 1,4-dioxane, THF, MeOH, methyl tert-butyl ether and DCM, as the additive including, for example, not limited to, ethanolamine, butylamine, diethylamine, formic acid, acetic acid and trifluoroacetic acid.

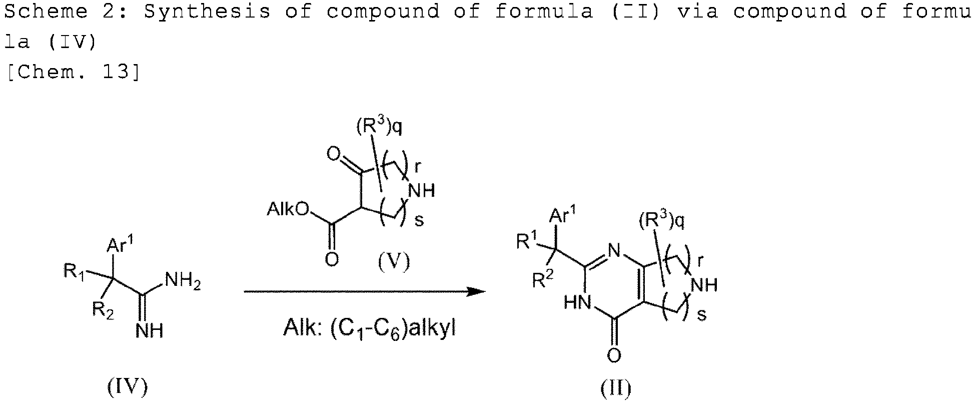

In this Scheme 2, the corresponding amine derivatives of formula (II) can be prepared by the cyclization reaction of a compound of formula (V) with an amidine compound of formula (IV) in the presence of base in an inert solvent.

In this Scheme 2, compounds of the formula (II) can be prepared by reaction of a compound of formula (V) and an amidine compound of formula (IV), for example, in the presence of a suitable base such as sodium methoxide, sodium ethoxide, potassium tert-butoxide or potassium carbonate in an organic solvent as methanol, ethanol or tert-butanol. Typically, 1.0 equivalent of amidine (IV) is reacted with 1.0 to 1.1 equivalent of a compound (V) and 1.0 to 4.5 equivalent of sodium methoxide in methanol at the range from ambient temperature to reflux condition.

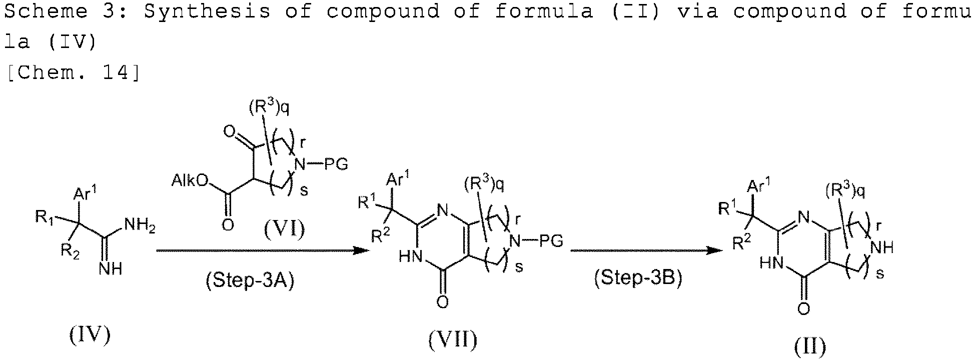

PG is an abbreviation for a suitable protecting group, such as benzyl, tert-butyl carbamoyl (Boc), benzyl carbamoyl (Cbz) or ethyl carbamoyl. Alk is (C₁₋C₆)alkyl group. In this Scheme 3, a corresponding amine derivative of formula (II) can be prepared by the cyclization reaction of an N-protected compound of formula (VI) with an amidine compound of formula (IV) in the presence of base in an inert solvent, followed by the deprotection of an N-protected compound of formula (VII).

In Step-3A, compounds of the formula (VII) can be prepared from an amidine compound of the formula (IV) and an N-protected compound of formula (VI) by the method described in Scheme 2.

In Step-3B, compounds of formula (II) can be prepared by deprotection of compounds of formula (VII) using standard methodology as described in "Protecting Groups in Organic Synthesis" by T.W.Greene and P. Wutz. More specifically, when PG is Boc, compounds of formula (VII) are typically treated with a suitable acid such as 4M to 6M hydrochloric acid or trifluoroacetic acid in a suitable solvent such as dichloromethane, methanol, ethyl acetate or 1,4-dioxane at ambient temperature for 1 to 18 hrs. When PG is benzylcarbamoyl, compounds of formula (VII) are typically reacted in a hydrogen atmosphere with a suitable palladium catalyst in a solvent such as ethanol. The compounds of formula (II) can also be prepared from compounds of formula (VII) with a suitable hydrogen transfer agent such as 1-methyl-1,4-cyclohexadiene in the presence of a suitable metal catalyst such as 10% palladium on charcoal in a solvent such as ethanol. Alternatively, compounds of formula (VII) can be treated with 48% aqueous hydrogen bromide at ambient temperature for 3 hrs.

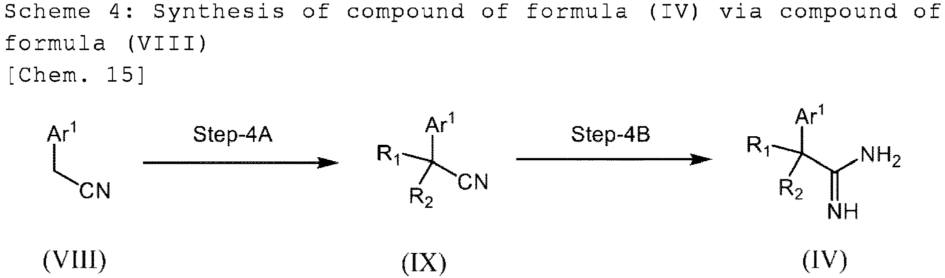

In this Scheme 4, a nitrile derivative of formula (VIII) can be converted to a compound of formula (IX) by alkylation using alkyl halide in present of an inorganic base, such as sodium hydroxide, in an inert solvent. Further, a compound of formula (IX) can be converted to an amidine derivative of formula (IV) in a single step by reaction with methylchloroaluminium amide prepared from trimethylaluminium and ammonium chloride in a suitable solvent.

In Step-4A, compounds of the formula (IX) can be prepared from a compound of the formula (VIII) by alkylation reaction under, for example, known alkylation conditions in the presence of a base in an inert solvent. A preferred base is selected from, for example, but not limited to, an alkaline earth metal hydroxide, alkoxide, carbonate, halide or hydride, such as sodium hydroxide, potassium hydroxide, sodium methoxide, potassium tert-butoxide, sodium hydride, lithium diisopropylamide or sodium bis(trimethylsilyl)amide. Examples of suitable inert aqueous, nonaqueous organic solvents include; water; ethers, such as diethyl ether, THF or 1,4-dioxane; acetone; DMF; DMSO; halogenated hydrocarbons, such as DCM, 1,2-dichloroethane or chloroform; hydrocarbons, such as hexane, heptane or toluene; or mixtures thereof. In the case of the water-organic co-solvent mixture, a preferred phase transfer catalyst is selected from tetraalkylammonium halide, such as tetrabutylammonium bromide and benzyltrialkylammonium halide, such as benzyltrimethylammonium chloride. The reaction can be carried out at a temperature of from -78 °C to 150 °C. The reaction time is, in general, from 5 minutes to 96 hrs.

In Step-4B, compounds of the formula (IV) can be prepared from a compound of the formula (IX) by the methodology described in the literature (for example, Eur. J. Med. Chem. 1981, 16, 175 or Tetrahedron Letters 1995, 36, 8761). Typically, a compound of formula (IX) is stirred in a saturated ethanolic hydrogen chloride solution and the resulting mixture is treated with a saturated ethanolic ammonia solution. Alternatively compounds of the formula (IV) can be prepared from compounds of the formula (IX) in the presence of the reagent (methylchloroaluminium) prepared from trimethylaluminium and ammonium chloride in a suitable organic solvent such as toluene at 80 °C for 18 hrs.

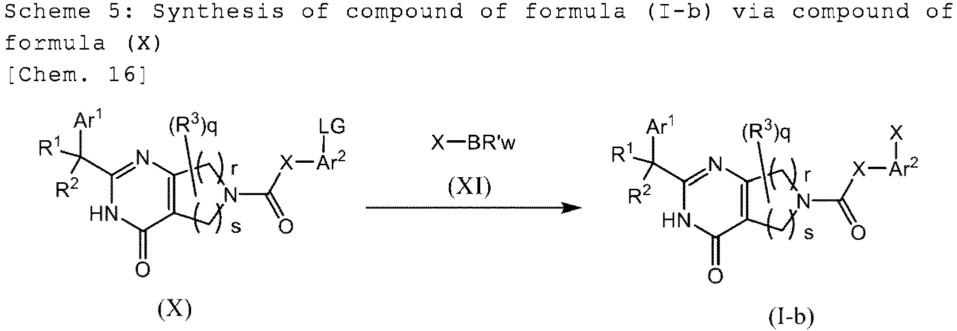

In this Scheme 5, LG (an abbreviation for Leaving Group) presents a suitable leaving group such as O-trifluoromethanesulfonate, O-tosylate, O-mesylate, iodide, bromide, or chloride and X of metal reagent (XI) is selected from, for example, but not limited to: (C₁-C₁₀) alkyl, (C₁-C₁₀) alkenyl, (C₃-C₁₀)cycloalkyl, (C₃-C₁₀)cycloalkenyl, aryl, and heteroaryl derivatives with non-substituted or substituted groups. Compounds of formula (I-b) can be prepared by the cross coupling reaction of compounds of formula (X) with a boronic (or boronic ester) compound of formula (XI) in organic solvent or water-organic co-solvent mixture under cross coupling conditions in the presence of a suitable transition metal catalyst and in the presence or absence of a base. In a representation of R'_{w}B, R' means OH, O-lower alkyl or fluorine, w is 2 or 3, and B is boron atom. As the concrete representation of substituent, B(OH)₂, B(O-lower alkyl)₂, B(lower alkyl)₂, and potassium trifluoroborate (BF₃⁻) (BF₃K) are described, but when B(O-lower alkyl)₂ may form the cyclic ring between the lower alkyl groups.

Examples of suitable transition metal catalysts include: tetrakis(triphenylphosphine)palladium(0), bis(triphenylphosphine)palladium(II) chloride, copper(0), copper(I) acetate, copper(I) bromide, copper(I) chloride, copper(I) iodide, copper(I) oxide, copper(I) trifluoromethanesulfonate, copper (II) acetate, copper(II) bromide, copper(II) chloride, copper(II) iodide, copper(II) oxide, copper(II) trifluoromethanesulfonate, palladium(II) acetate, palladium(II) chloride, bis(acetonitrile)dichloropalladium(II), bis(dibenzylideneacetone)palladium(0), tris(dibenzylideneacetone)dipalladium(0) and [1,1'-bis(diphenylphosphino)ferrocene] palladium(II) dichloride.

Examples of suitable organic solvent for the anhydrous solvent and the water-organic co-solvent mixture include the followings: THF; 1,4-dioxane; DME; DMF; acetonitrile; alcohols, such as methanol or ethanol; halogenated hydrocarbons, such as DCM, 1,2-dichloroethane, chloroform or carbon tetrachloride; and diethylether. This reaction can be carried out in the presence or absence of a base such as potassium hydroxide, sodium hydroxide, lithium hydroxide, sodium bicarbonate, sodium carbonate, potassium carbonate and potassium phosphate. This reaction can be carried out in the presence of a suitable additive agent.

Examples of such additive agents include: triphenylphosphine, tri-tert-butylphosphine, 1,1'-bis(diphenylphosphino)ferrocene, tri-2-furylphosphine, tri-o-tolylphosphine, 2-(dichlorohexylphosphino)biphenyl, triphenylarsine, tetrabutylammonium chloride, tetrabutylammonium fluoride, lithium acetate, lithium chloride, triethylamine, potassium methoxide, sodium methoxide, sodium hydroxide, cesium carbonate, tripotassium phosphate, sodium carbonate, sodium bicarbonate, and sodium iodide. The preferable reaction temperature is, in general, at a temperature of from 0 °C to 200 °C. The preferable reaction time is, in general, from 5 minutes to 96 hrs. In an alternative case, the reaction can be carried out in a microwave system in the presence of a base in an inert solvent. The reaction can be carried out at a temperature in the range of from 100 °C to 200 °C. Reaction time is, in general, from 10 minutes to 3 hrs.

The preferred conditions in the preparation of compounds are as follows:
1) Starting material (1.0 eq.), boronic acid derivative (1.0 to 3.0 eq.), palladium (II) acetate (0.2 eq.), triphenylphosphine (0.4 eq.), 1,4-dioxane-saturated sodium hydrogen carbonate aqueous solution (1:1 v/v), reaction temperature and reaction time: 100-150 °C, 1 to 6 hrs or microwave (100 to 150 °C, 10 min. to 1 hr)
2) Starting material (1.0 eq.), boronic acid derivative (1.0 to 3.0 eq.), Pd(amphos)Cl₂ (0.1 to 0.5 eq.), potassium carbonate (2.0 to 4.0 eq.), 1,4-dioxane-water (1:1 to 3:1 v/v), reaction temperature and reaction time: 80 to 150 °C, 1 to 6 hrs or microwave (100 to 150 °C, 10 min. to 1 hr)
3) Starting material (1.0 eq.), boronic acid derivative (1.0 to 3.0 eq.), Pd(PPh₃)₄ (0.1 to 0.5 eq.), potassium carbonate (2.0 to 4.0 eq.), DMF, reaction temperature and reaction time: 80 to 150 °C, 1 to 6 hrs or microwave (100 to 150 °C, 10 min. to 1 hr)

Other than a Suzuki-Miyaura cross coupling shown above, Still cross coupling reaction using trialkyltin instead of R'_{w}B substituent, and Negishi coupling reaction using zinc-halogen, wherein as a halogen, chlorine, bromine, and iodine are cited, instead of R'_{w}B substituent can be used.

The ethynyl compounds of formula (I-b) are synthesized by Sonogashira reaction from the compounds of formula (XI) and substituted ethynyl derivatives in place of compounds of formula (XI) under the above cross coupling conditions.

The cyano derivatives of formula (I-b) are synthesized by using zinc cyanide in place of compounds of formula (XI) under the above cross coupling conditions.

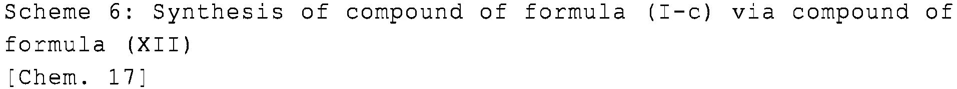

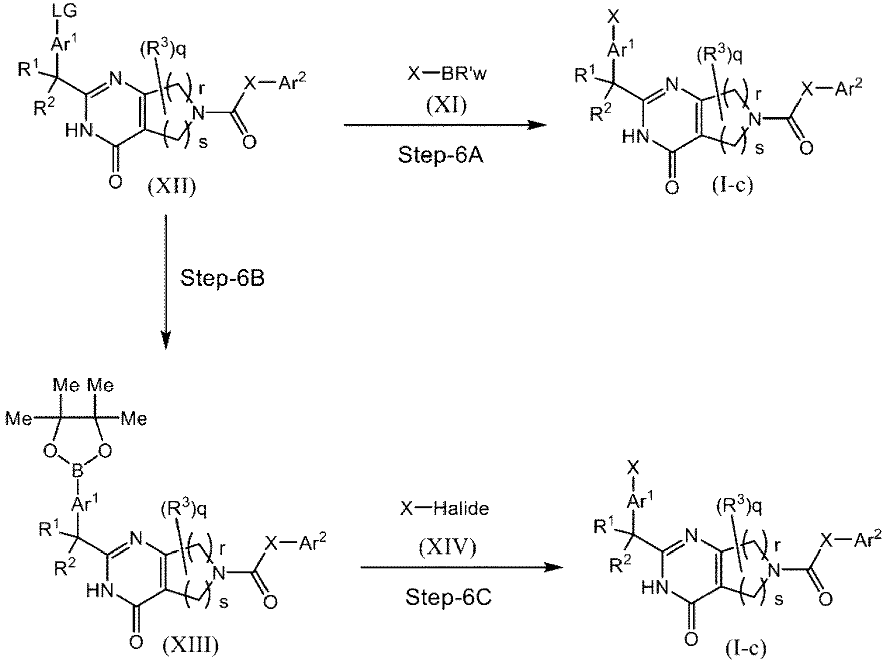

In Scheme 6, a compound of the formula (I-c) can be prepared by the cross coupling reaction of a compound of formula (XII) with a boronic (or boronic ester) compound of formula (XI) according to the general synthesis in Scheme 5. Furthermore, a compound of the formula (I-c) can also be prepared by the same cross coupling reaction from a halide compound of formula (XIV) with a boronic (or boronic ester) compound of formula (XIII) converted from the halide compound of formula (XII). Typically, the compound of the formula (XIII) can be prepared by the cross coupling reaction from a compound of the formula (XII) with bis(pinacolato)diboron in the presence of potassium acetate and [1,1'-bis(diphenylphosphino)ferrocene]dichloropalladium (II), dichloromethane adduct in a suitable solvent such as 1,4-dioxane at 80 °C for 1 to 6 hrs.

In step 6A, the ethynyl compounds of formula (I-b) are synthesized by Sonogashira reaction from the compounds of formula (XI) and substituted ethynyl derivatives in place of compounds of formula (XI) under the above cross coupling conditions.

The cyano derivatives of formula (I-b) are synthesized by using zinc cyanide in place of compounds of formula (XI) under the above cross coupling conditions.

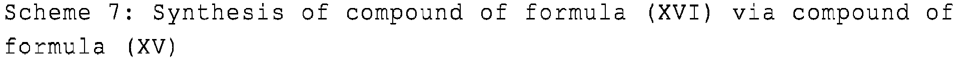

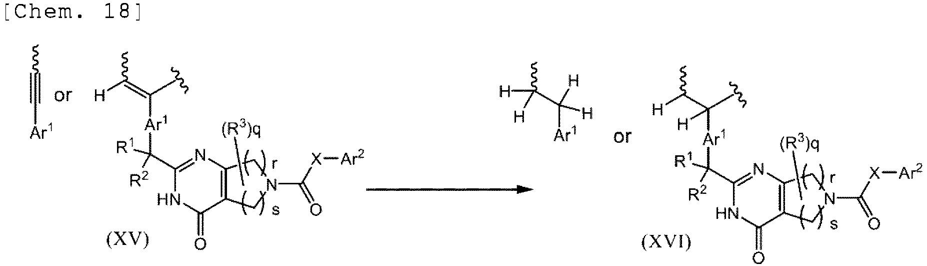

When above X moiety in formula (I-b) includes a part of unsaturated or triple bond as shown in formula (XV), compounds of formula (XV) can be converted to saturated compounds of formula (XVI) by hydrogenation reaction in the presence of hydrogen gas and a suitable metal catalyst such as 5 to10% palladium on carbon, palladium hydroxide on carbon and platinum (IV) oxide in a solvent such as methanol, ethanol and THF.

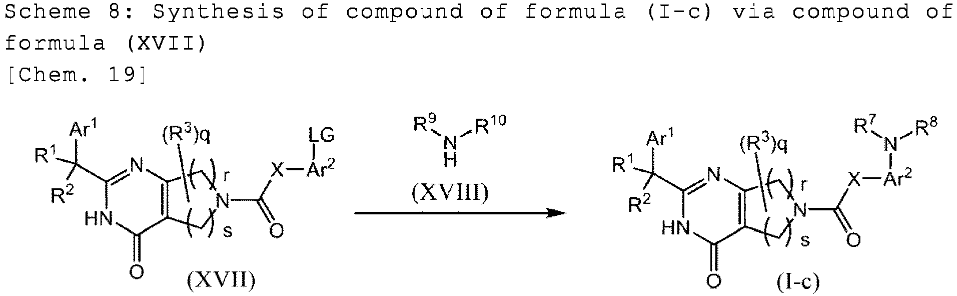

In this Scheme 8, compounds of the formula (I-c) can be prepared by substitution reaction of a compound of formula (XVII) with amine (HNR⁹R¹⁰)(XVIII). Typically, compounds of formula (I-c) may be prepared by addition of the required primary or secondary amine (HNR⁹R¹⁰)(XVIII) in a suitable solvent as tetrahydrofuran, acetonitrile, N,N-dimethylacetamide or 1-methyl-2-pyrrolidinone at either ambient or elevated temperature. A microwave oven may be used to increase reaction rates.

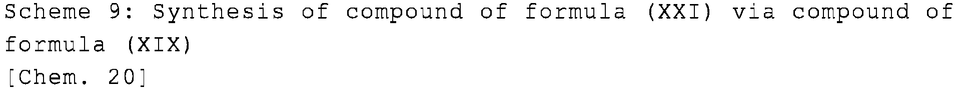

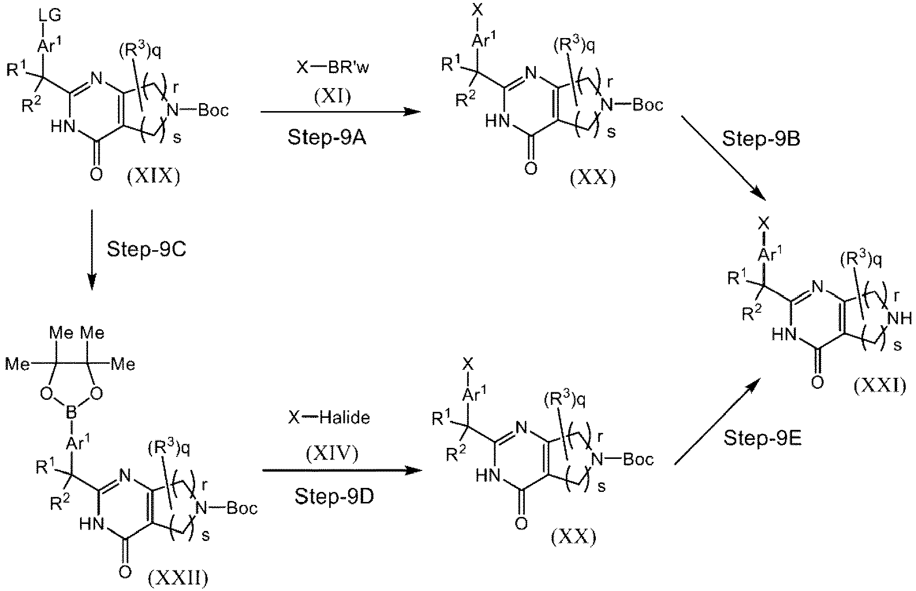

In this Scheme 9, an N-Boc derivative of the formula (XX) can be prepared according to the same general synthesis described in Scheme 5. A compound of the formula (XX) can be prepared by the cross coupling reaction of a compound of formula (XIX) with a boronic (or boronic ester) compound of formula (XI). Furthermore, a compound of the general formula (XX) can also be prepared by the same cross coupling reaction from a halide compound of formula (XIV) with a boronic ester compound of formula (XXII) converted from the compound of formula (XIX). Finally, an N-Boc compound of the general formula (XX) can be converted to an N-H compound of the general formula (XXI) according to N-Boc deprotective condition in Scheme 3.

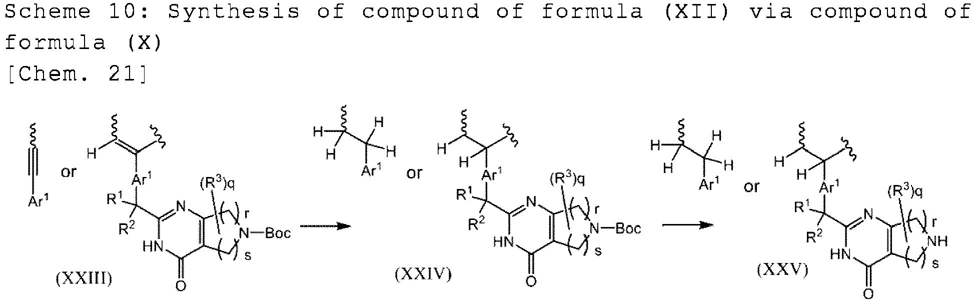

When above X moiety in formula (XX) includes a part of unsaturated or triple bond as shown in formula (XXIII), compounds of formula (XXIII) can be converted to saturated compounds of formula (XXIV) by hydrogenation reaction in the presence of hydrogen gas and a suitable metal catalyst such as 5 to10% palladium on carbon, palladium hydroxide on carbon and platinum (IV) oxide in a solvent such as methanol, ethanol and THF. Finally, an N-Boc compound of the formula (XXIV) can be converted to an N-H compound of the formula (XXV) according to N-Boc deprotective condition in Scheme 3.

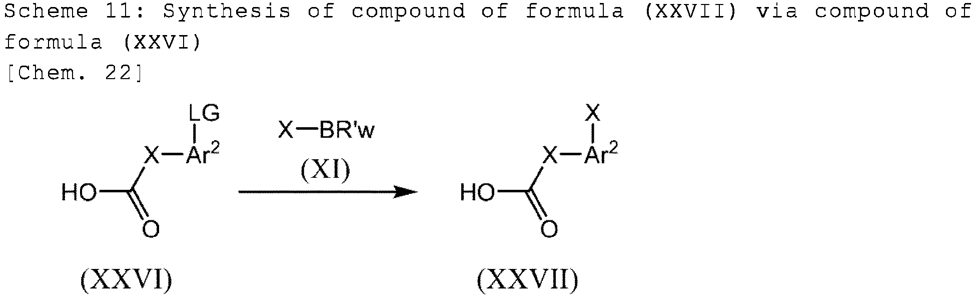

In this Scheme 11, an acid compound of the general formula (XXVII) can be prepared according to the same general synthesis described in Scheme 5.

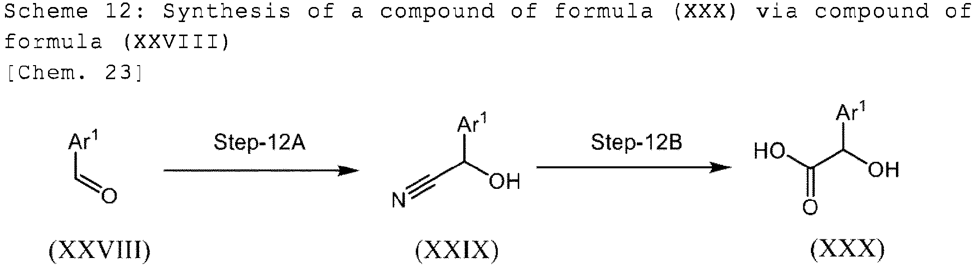

In this Scheme 12, a compound of the general formula (XXX) can be prepared by acid hydrolysis of a cyanohydrin compound of the general formula (XXIX) in step-12A. A cyanohydrin compound of the general formula (XXIX) can be prepared from a compound of the general formula (XXVIII) and trimethylsilyl cyanide in the presence of a suitable Lewis acid (e.g., titanium tetraisopropoxide) in step-12-B.

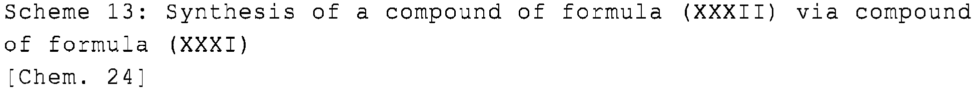

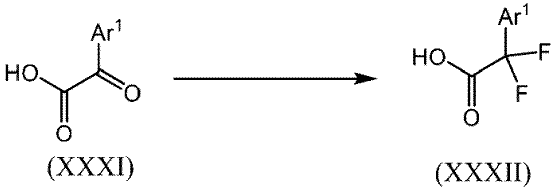

In this Scheme 13, a compound of the general formula (XXXII) can be prepared by the fluorination reaction of a compound of the general formula (XXXI) in the presence of a suitable fluorination reagent (e.g., N,N-diethylaminosulfur trifluoride).

### Compound Preparation

The synthesis of the compounds of the general formula (I) and pharmaceutically acceptable derivatives and salts thereof may be accomplished as outline above in Schemes 1 to 13. In the following description, the groups are as defined above for compounds of formulae (I) unless otherwise indicated. Starting materials are commercially available or are prepared from commercially available starting materials using methods known to those skilled in the art.

Therefore, the compound of the above formula (1) can be easily prepared according to Schemes 1 to 13, by a method known *per* se or by appropriately combining them.

Compounds of formula (2), (3) or (4) can be prepared using methods known in the art (*e.g.,* WO2013012500, WO2011119704, WO2013169396, *etc.*)*.*

Preferred examples of each compound of formula (2), (3) or (4) may be represented as Compounds B, C and D for convenience.
Compound B (GSK2798745): 1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile,
Compound C (GSK2193874): 3-(1,4'-bipiperidin-1'-ylmethyl)-7-bromo-N-(1-phenylcyclopropyl)-2-[3-(trifluoromethyl)phenyl]-4-quinolinecarboxamide, and
Compound D (HC-067047): N-(3-(trifluoromethyl)phenyl)-2-methyl-1-(3-morpholinopropyl)-5-phenyl-1H-pyrrole-3-carboxamide.

The antisense oligonucleotide 5'-CATCACCAGGATCTGCCATACTG-3' (SEQ ID NO: 6) disclosed in the literature is conveniently referred to as Compound E (Behavioral Brain Research, 2010, 208: 194-201). The siRNA 5'-UCUACCAGUACUAUGGCUUd(TT)-3' (SEQ ID NO: 7); 3'-d(TT)AGAUGGUCAUGAUACCGAA-5' (SEQ ID NO: 8) is conveniently referred to as Compound F (Gut, 2010, 59 : 481-488).

### [Example 1]

### TRPV4 inhibitory activity (in vitro):

The inhibitory activity of TRPV4 channel is measured by inhibi tion of hypotonic-induced activation in cells as changes in intracell ular calcium using an FDSS apparatus (Hamamatsu Photonics, Japan) whe rein the cells are prepared by adding the Ca (2+) -sensitive fluorescen t dye Fluo-4 (Invitrogen, USA) to cells expressing human TRPV4 channe ls, adding test compounds, and then adding a hypotonic buffer (90 mM D-mannitol and 1.26 mM CaCl₂ in distilled water) . The IC₅₀ is below 3 µM.

### [Example 2]

### Drug concentrations in ocular tissues after intravitreal administration:

A TRPV4 inhibitor or MaQaid (registered trademark) is administered intravitreally to mice, and 1 hour and/or 24 hours after administration, the mice are euthanized under anesthesia by exsanguination from the posterior vena cava and abdominal aorta, and the eyeballs are removed. The same method as above may also be used to measure the drug concentrations in rabbits.

A TRPV4 inhibitor remains in ocular tissue 1 hour and/or 24 hours after administration. Preferably, it remains at a high concentration comparing to the concentration in the eyeball tissue of MaQaid (registered trademark) administered as an index.

Examples of ocular disease models accompanied with blood flow disorder and cell disorder in the eye include the following tests.

### [Example 3]

### Pathogenesis of mouse retinal vein occlusion model:

Eight-week-old male ddY mice are anesthetized with a mixture of ketamine (120 mg/kg) and xylazine (6 mg/kg) administered intramuscularly. After that, 0.15 mL of a photosensitizer, rose bengal (20 mg/kg) is administered into the tail vein, and a laser (wavelength 532 nm) is irradiated into a vein three optic disc diameters away from the optic disc in the mouse right eye to occlude the retinal vein and form a pathological condition. Laser irradiation is performed using an attachment of Micron4 (Phoenix Research lab., CA, USA), which is a fundus imaging device, under prescribed laser irradiation conditions (wavelength 532 nm, spot size 50 µm, irradiation time 5 seconds, laser power 50 mW). The vein is occluded by 10 to 15 laser irradiations per vein.

Mice are euthanized by cervical dislocation 0.5, 1, 3, 7 days after vascular occlusion, eyeballs are removed, retinas are isolated, and then changes in TRPV4 channel expression are evaluated by Western blotting.

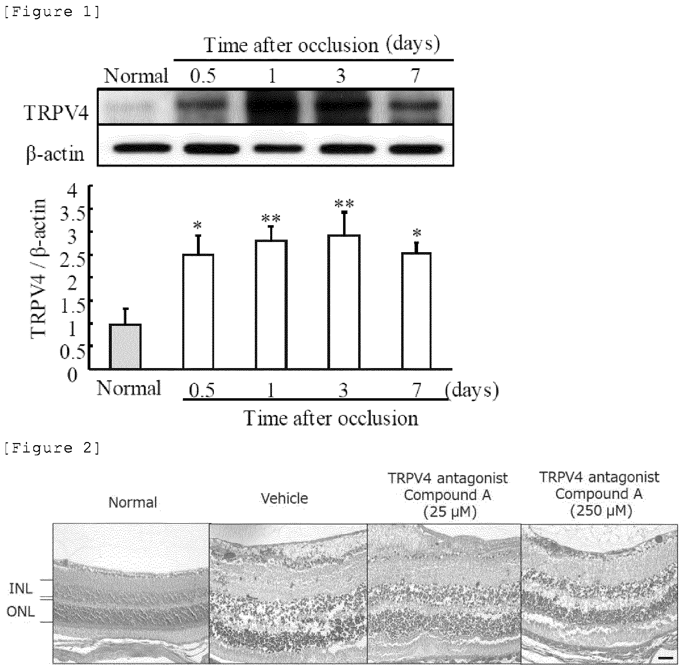

Results are shown in Figure 1. TRPV4 channel expression in the model mice of retinal vein occlusion at 0.5, 1, 3, and 7 days after vascular occlusion, continues to increase twice to three times in expression comparing to that in mice in the normal state from 0.5 to 7 days after occlusion.

### [Example 4]

### Histological evaluation for retinal vein occlusion:

Using the same method as in Example 3, the veins of the mouse eye are irradiated with a laser (wavelength 532 nm) to occlude the retinal veins.

Immediately after laser irradiation, a TRPV4 inhibitor is administered, and 24 hours after irradiation, the eyeball is removed and a retinal histopathology specimen is prepared. Hematoxylin-eosin (HE) staining is performed to measure the thickness of the retinal inner nuclear layer (INL). Other stains (e.g., TUNEL stain) may be used for analysis as needed.

Administration of a TRPV4 inhibitor is performed by intravitreal administration (local administration). The conditions of the solution for administration are investigated in advance, and the concentration for intravitreal administration is determined. However, if the conditions for systemic administration are met, systemic administration may be included in the group composition. For intravitreal administration, the dosage should be 2 µL/eye as a single dose. For systemic administration, the number of doses and dosage should be determined after a separate consideration.

The group composition is
- Normal (left eye) + Vehicle intravitreal administration group
- Pathological condition (right eye) + Vehicle intravitreal administration group
- Pathological condition (right eye) + TRPV4 inhibitor intravitreal administration (high dose) group
- Pathological condition (right eye) + TRPV4 inhibitor intravitreal administration (middle dose) group

In addition to the above group composition, one of the following groups (1) and (2) is included based on the results of preliminary studies.
- Option (1):
   Pathological condition (right eye) + TRPV4 inhibitor intravitreal administration (low dose) group
- Option (2):
   Pathological condition (right eye) + Vehicle systemic administration group
   Pathological condition (right eye) + TRPV4 inhibitor systemic administration group

In the present application, the effect may be expressed as an inhibition rate. The inhibition rate is an index showing how much the experimental group is able to inhibit the change (increase or decrease) in the numerical value by the control group, and is expressed as a percentage (%) or the like.

In a mouse retinal vein occlusion model in which retinal veins are occluded by the same method as in Example 3, the thickness of the retinal inner nuclear layer (INL) is increased due to edema caused by vascular occlusion. After a single intravitreal administration of 2 µL of a TRPV4 inhibitor (25 µM, 250 µM) or vehicle, whether the TRPV4 inhibitor restores the retinal edema is evaluated.

The inhibition rate of the increased thickness of the retinal inner nuclear layer (INL) by TRPV4 inhibitors is usually 30% or more, preferably 50% or more, more preferably 70% or more, and most preferably 90% or more, comparing to that in the vehicle group.

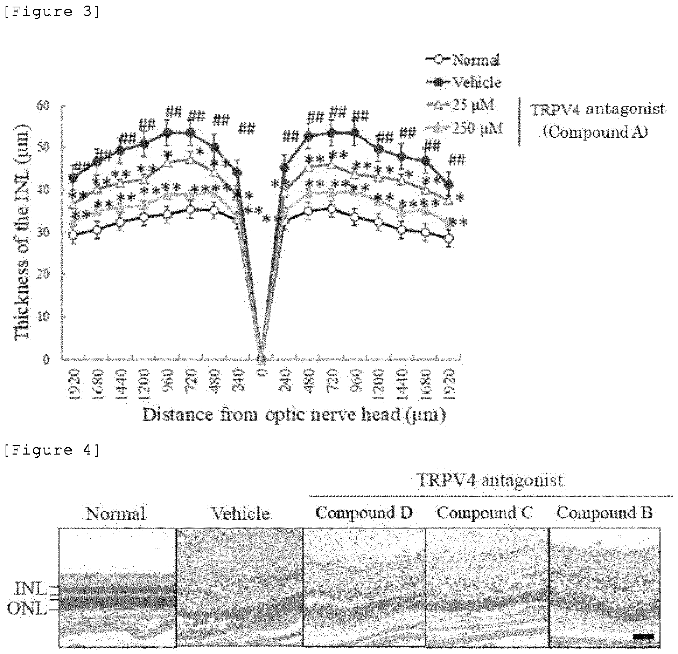

Results for the case where the TRPV4 inhibitor is Compound A: (R)-6-(2-(3-chlorophenyl)-2-hydroxyacetyl)-2-(1-phenylcyclopropyl)-5,6,7,8-tetrahydropyrido[4,3-d]pyrimidin-4(3H)-one are shown in Figures 2 and 3. A single intravitreal administration of Compound A (25 µM, 250 µM) can be confirmed to show significant inhibition of the increase in the thickness of the retinal inner nuclear layer (INL). The inhibition rate of the increase in the thickness of the retinal inner nuclear layer (INL) after 25 µM and 250 µM intravitreal administration of Compound A is 40% and 76%, respectively. The retinal edema is significantly suppressed by administration of Compound A.

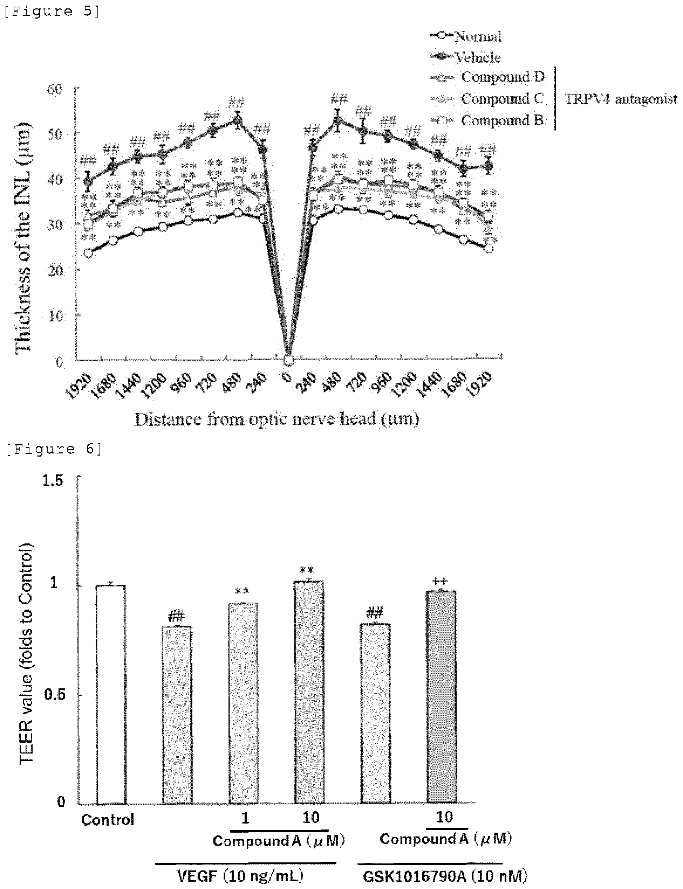

Results are shown in Figures 4 and 5 when the TRPV4 inhibitors are Compounds B, C, and D.

Each Compound B, C and D is the compound shown below. Compound B (GSK2798745): 1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile,
Compound C (GSK2193874): 3-(1,4'-bipiperidin-1'-ylmethyl)-7-bromo-N-(1-phenylcyclopropyl)-2-[3-(trifluoromethyl)phenyl]-4-quinolinecarboxamide, and
Compound D (HC-067047): N-(3-(trifluoromethyl)phenyl)-2-methyl-1-(3-morpholinopropyl)-5-phenyl-1H-pyrrole-3-carboxamide.

Compounds B, C and D are administered intravitreally at 250 µM each to a mouse retinal vein occlusion model. The rate of suppression of increased thickness of the retinal inner nuclear layer (INL) in each group is 62%, 59% and 64%, respectively, comparing to that of the vehicle group. Administration of a TRPV4 inhibitor significantly suppresses retinal edema.

### [Experiment 5]

### Evaluation of cell permeability using human retinal microvascular endothelial cells (HRMECs):

Human retinal microvascular endothelial cells (HRMECs) are seeded onto Transwell inserts at a density of 1.0 × 10⁵ cells/mL. The medium is changed every other day and the cells are cultured for 7 days. Transendothelial electrical resistance (TEER) is measured using a measurement device and cup-shaped electrode (Endohm-6; World Precision Instruments, USA). One hour after the addition of a TRPV4 inhibitor (final concentration: 1 µM, 10 µM) or buffer for evaluation to cells, VEGF (final concentration: 10 ng/mL) or GSK1016790A, a TRPV4 agonist (final concentration: 10 nM) is added, and 24 hours after the addition, transepithelial electrical resistance values (TEER values) is measured 24 hours after addition. The transepithelial electrical resistance value (TEER value) is an index for evaluating barrier functions such as tight junctions of cells.

When the TRPV4 inhibitor is Compound A, the results are shown in Figure 6. In human retinal capillary endothelial cells, the addition of VEGF or the TRPV4 agonist GSK1016790A decreases transepithelial electrical resistance (TEER values). The decrease in transepithelial electrical resistance (TEER) is considered to indicate decreased barrier function and increased cell permeability. The inhibition rate of increased vascular permeability by the TRPV4 inhibitor-added group is usually 30% or more, preferably 50% or more, more preferably 70% or more, and most preferably 90% or more, comparing to the respective increases in vascular permeability by the VEGF- or TRPV4 agonist-added groups.

The addition of Compound A (final concentration: 1 µM, 10 µM), which is a TRPV4 inhibitor, significantly suppresses the decrease in transepithelial electrical resistance. The inhibition rate of increased vascular permeability is 50% or more. Therefore, in human retinal vascular endothelial cells, the increase in vascular permeability is significantly suppressed by the addition of Compound A, which is a TRPV4 inhibitor.

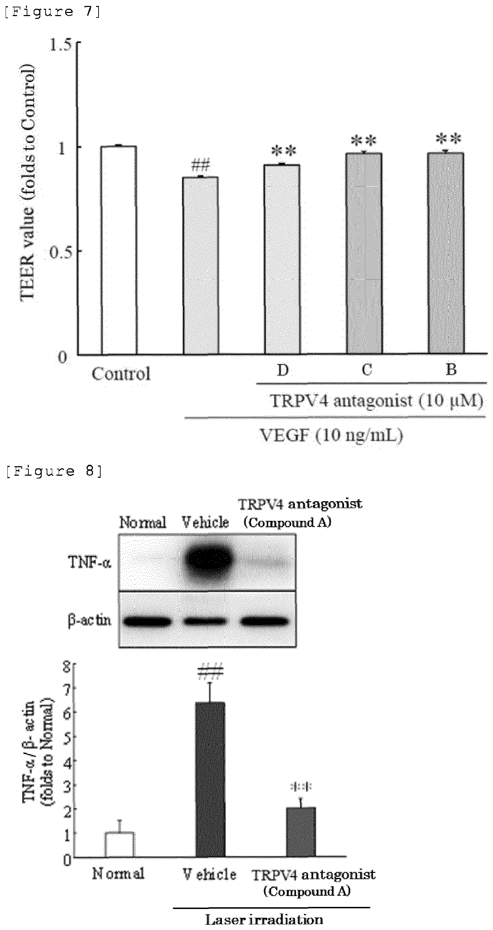

When the TRPV4 inhibitors are Compounds B, C and D, the results are shown in Figure 7. Transepithelial electrical resistance (TEER) is measured in human retinal capillary endothelial cells. Comparing to the increase in vascular permeability due to the VEGF-added group, Compounds B, C and D (final concentration: 10 µM) in the TRPV4 inhibitor-added group significantly inhibit the increase in vascular permeability, and the inhibition rate is 30% or more. Increased vascular permeability is significantly suppressed by TRPV4 inhibitors.

### [Experiment 6]

### Expression of Tumor Necrosis Factor alpha (TNFα), an inflammation-related factor:

Using the same method as in Example 3, the retinal veins of mice are occluded by laser irradiation into the veins of the eye.

Immediately after laser irradiation, 2 µL of a TRPV4 inhibitor (250 µM) is administered intravitreally. One day after vascular occlusion, the mice are euthanized by cervical dislocation, and then retinas isolated from removed eyeballs are evaluated by Western blotting.

Expression of TNFα, an inflammation-related factor (inflammatory cytokine), is increased in a mouse model of retinal vein occlusion in which retinal veins are occluded. The inhibition rate of increased TNFα expression by TRPV4 inhibitors comparing to that by the vehicle group is usually 30% or more, preferably 50% or more, more preferably 70% or more, and most preferably 90% or more.

When the TRPV4 inhibitor is Compound A, the results are shown in Figure 8. When Compound A (250 µM) is intravitreally administered to a mouse model of retinal vein occlusion immediately after vascular occlusion, the inhibition rate of the increase in TNFα expression is 50% or more. Administration of the TRPV4 inhibitor significantly suppresses the increased expression of TNFα, an inflammation-related factor.

### [Example 7]

Seven days after vascular occlusion, the retina is damaged, the retinal layer structure is thinned, the blood flow is reduced, and a retinal nonperfusion area exists. Whether the TRPV4 inhibitor restores this condition is evaluated.

### Investigation in a mouse model of retinal vein occlusion with late drug administration 7 days after vascular occlusion:

Using the same method as in Example 3, retinal veins are occluded by laser irradiation of the veins in the mouse eye.

Seven days after vascular occlusion, 2 µL of a TRPV4 inhibitor (250 µM) or vehicle is administered intravitreally. One day after administration (8 days after occlusion), the retinal layers are imaged using optical coherence tomography (OCT) and intraocular blood flow is measured using laser speckle.

After that, FITC-dextran (fluorescein-conjugated dextran) solution with a molecular weight of 2×10⁶ dissolved in PBS is injected into the tail vein at 20 mg/animal. After administration, mice are euthanized by cervical dislocation, and the eyeballs are removed and fixed in 0.1 M phosphate buffer (pH 7.4) containing 4% paraformaldehyde for 12 hours. The fixed eyeballs are separated and the retina is completely detached from the sclera. The retina is then cut into quadrants, flattened, and sealed with Fluoromount to prepare a flat-mounted retina specimen. The flat-mounted retina is then photographed using an HS all-in-one fluorescence microscope (BZ-710; Keyence, Japan).

### 1) Tissue evaluation: Suppression of retinal thinning (retinal protective effect)

The suppression rate of retinal thinning is usually 30% or more, preferably 50% or more, more preferably 70% or more, and most preferably 90% or more in the vehicle group.

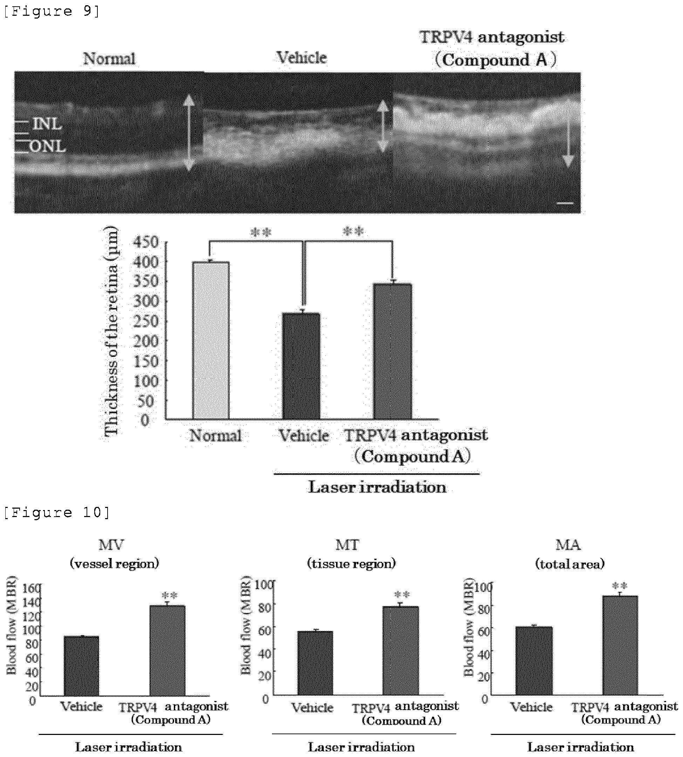

When the TRPV4 inhibitor is Compound A, the results are shown in Figure 9. Intravitreal administration of Compound A (250 µM) inhibits retinal thinning by 30% or more. Administration of the TRPV4 inhibitor significantly suppresses retinal thinning in a mouse model of retinal vein occlusion.

### 2) Evaluation of blood flow

The improvement rate of blood flow by the TRPV4 inhibitor comparing to the blood flow under pathological conditions is usually 10% or more, preferably 20% or more, more preferably 30% or more comparing to that in the vehicle group.

When the TRPV4 inhibitor is Compound A, the results are shown in Figure 10. Intravitreal administration of Compound A improves the ocular blood flow reduction rate by 30% or more. Administration of a TRPV4 inhibitor significantly ameliorated ocular blood flow reduction in a mouse model of retinal vein occlusion.

### 3) Evaluation of nonperfusion area

The suppression rate of nonperfusion areas of the retina is usually 30% or more, preferably 50% or more, more preferably 70% or more, and most preferably 90% or more comparing to that in the vehicle group.

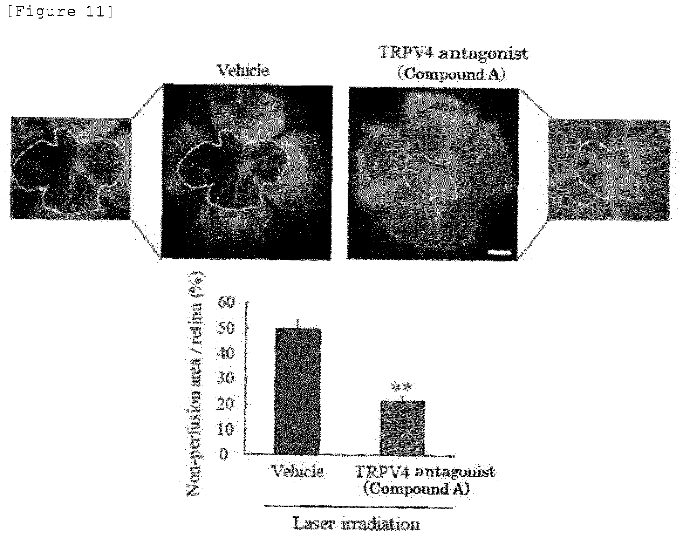

When the TRPV4 inhibitor is Compound A, the results are shown in Figure 11. Intravitreal administration of Compound A suppresses the inhibition rate of nonperfusion areas in the retina by 50% or more. Administration of TRPV4 inhibitors significantly suppresses the percentage of nonperfusion areas of the retina in the mouse model of retinal vein occlusive disease.

### [Experiment 8]

### Laser-induced choroidal neovascularization (CNV) model (wet age-related macular degeneration model):

Mydrin P ophthalmic solution (registered trademark, Santen Pharmaceutical Co., Ltd.) is dropped into the right eye of an animal (mouse, etc.) to dilate the pupil. For example, when using a male C57BL/6J mouse, under anesthesia, a laser photocoagulator (MC500, NIDEK Co., Ltd., Japan) is used to irradiate eight equally spaced laser beams on the circumference of the optic disc under predetermined laser irradiation conditions (wavelength 532 nm, spot size 50 µm, irradiation time 100 msec, laser output 120 mW).

After photocoagulation, a TRPV4 inhibitor is administered intravitreally (2 µL dose) orally/subcutaneously/intraperitoneally to the right eye. After dropping Cravit (registered trademark, Daiichi Sankyo) ophthalmic solution 0.5% into the right eye, fluorescent fundus is promptly photographed using a fundus camera.

Seven days and fourteen days after laser irradiation, the mice are anesthetized, 10-fold diluted FLUORESCITE (registered trademark, Alcon Japan) intravenous injection is administered into the tail vein, and fluorescent fundus are promptly photographed using a fundus camera.

After electroretinogram (ERG) measurements 15 days after laser irradiation, the mice are anesthetized and FITC-dextran is administered intravenously into the tail vein. After removing and fixing the eyeball from the mouse, a choroidal flat mount specimen is prepared under a microscope. The choroidal flat mount specimen is photographed using a confocal laser scanning microscope, and the photographed image is analyzed using analysis software (OLYMPUS FLUOVIEW FV1000, Japan) to calculate the area of choroidal neovascularization (CNV).

Administration of a TRPV4 inhibitor significantly reduces the CNV area comparing to the untreated group.

The present invention is not limited to the aforementioned respective embodiments and Examples. Thus, embodiments obtained by appropriately combining the technical means respectively disclosed in different embodiments are also encompassed in the technical scope of the present invention. The scope of the claims should be given the broadest interpretation consistent with the description as a whole.

### [Industrial Applicability]

The TRPV4 inhibitor of the present invention is useful for preventing or treating a retinal disease accompanied with blood flow disorder or cell disorder.

## Claims

1. A use of a compound having TRPV4 inhibitory activity or a pharmaceutically acceptable salt thereof for the manufacture of a medicament that prevents or treats a retinal disease accompanied with blood flow disorder or cell disorder in humans or animals.

2. The use according to claim 1, wherein the retinal disease is selected from the group consisting of hypertensive retinopathy and amaurosis, which are retinal diseases accompanied with blood flow disorder; selected from the group consisting of hereditary optic neuropathy, retinal detachment, choroidal metastases, choroidal melanoma and choroidal hemangioma, which are retinal diseases accompanied with cell disorder; or selected from the group consisting of retinal vein occlusion, retinal artery occlusion, wet age-related macular degeneration, retinitis pigmentosa, diabetic retinopathy, ischemic optic neuropathy, and glaucoma, which are retinal diseases accompanied with both blood flow disorder and cell disorder.

3. The use according to claim 1, wherein the retinal disease is retinal vein occlusion or wet age-related macular degeneration.

4. The use according to claim 1, wherein the retinal disease is branch retinal vein occlusion or central retinal vein occlusion.

5. The use according to claim 1, wherein the compound having TRPV4 inhibitory activity is selected from the compound represented by the following formulas (1), (2), (3), and (4); compounds disclosed in WO2013012500 represented by GSK2798745 (1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile); compounds disclosed in WO2011119704 represented by GSK2193874 (3-(1,4'-bipiperidin-1'-ylmethyl)-7-bromo-N-(1-phenylcyclopropyl)-2-[3-(trifluoromethyl)phenyl]-4-quinolinecarboxamide); compounds disclosed in WO2013169396 represented by HC-067047 (N-(3-(trifluoromethyl)phenyl)-2-methyl-1-(3-morpholinopropyl)-5-phenyl-1H-pyrrole-3-carboxamide); GSK3395879; GSK3527497; GSK205; GSK3491943; RN-1734; RN-1747; RN-9893; and PF-05214030; or a pharmaceutically acceptable salt thereof. 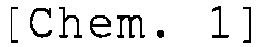 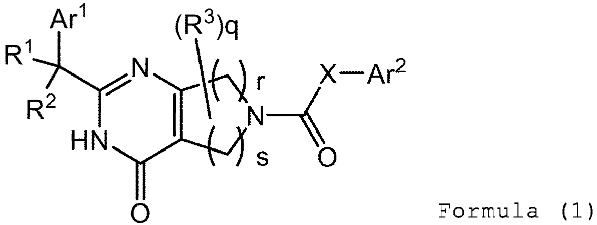 wherein
R¹ and R² are independently selected from the group consisting of: hydrogen, hydroxyl, halogen, (C₁-C₆)alkyl, (C₁-C₆)haloalkyl, (C₁-C₆)alkoxy, (C₁-C₆)haloalkoxy, (C₃-C₇)cycloalkyl, and phenyl(C₀-C₄)alkyl; wherein the said (C₃-C₇)cycloalkyl or phenyl(C₀-C₄)alkyl is optionally substituted with 1 to 4 substituents independently selected from the group consisting of: halogen, hydroxyl, (C₁-C₆)alkyl, (C₁-C₆)haloalkyl, (C₁-C₆)alkoxy, and (C₁-C₆)haloalkoxy; or alternatively R¹ and R², together with the atom to which they are attached, may form a 3 to 8 membered ring which may contain 0 to 4 heteroatoms independently selected from oxygen, sulfur, and nitrogen; wherein the said 3 to 8 membered ring is optionally substituted with 1 to 6 substituents independently selected from the group consisting of: halogen, hydroxyl, (C₁-C₆)alkyl, (C₁-C₆)haloalkyl, -SO₂(C₁-C₆)alkyl, -SO₂(C₁-C₆)haloalkyl, - C(=O)(C₁-C₆)alkyl, and -C(=O) (C₁-C₆)haloalkyl; R³ is independently selected from the group consisting of: hydrogen, fluoride, methyl, ethyl, and (C₁-C₆)haloalkyl;
X is selected from the group consisting of: a chemical bond, -N(R⁴)-, -(C(R⁵)(R⁶))ₙ-, -(C₃-C₈)cycloalkyl-, -C(R⁵)=C(R⁶)-, -C(=O)-, -CR⁴(N(R⁵)(R⁶))-, -[(C(R⁵)(R⁶))ₙO]-, -[(C(R⁵)(R⁶))ₙN(R⁴)]-, - [(C(R⁵)(R⁶))ₙS]-, and -[N(R⁴)(C(R⁵)(R⁶))ₙ]-;
R⁴ is hydrogen or (C₁-C₆) alkyl;
R⁵ and R⁶ are independently selected from the group consisting of: hydrogen, halogen, hydroxyl, (C₁-C₆)alkyl, (C₁-C₆)alkoxy, hydroxyl (C₁-C₆)alkyl, (C₁-C₆)alkoxy (C₁-C₆)alkyl, (C₁-C₆)haloalkyl, and (C₁-C₆)haloalkoxy;
when R⁵ is two or more than two, R⁵ is same or different;
when R⁶ is two or more than two, R⁶ is same or different;
n is 1, 2, 3, or 4;
q is 1, 2, 3, or 4;
r is 1, 2, 3, or 4;
s is 1, 2, 3, or 4;
Ar¹ is selected from aryl and heteroaryl which are optionally substituted with 1 to 6 substituents independently selected from the group consisting of: hydrogen, halogen, cyano, nitro, hydroxyl, -C(=O)R⁷, -C(=O)NR⁷R⁸, -NHSO₂R⁷, -SO₂NR⁷R⁸, (C₁-C₆)alkylthio-, (C₁-C₆)haloalkylthio-, (C₁-C₆)alkylsulfinyl, (C₁-C₆)alkylsulfonyl, -NR⁷R⁸, tri(C₁-C₆)alkylsilyl, (C₁-C₆)alkyl, (C₃-C₇)cycloalkyl, (C₃-C₇)cycloalkoxy, (C₁-C₆)alkoxy, (C₁-C₆)alkenyl, aryl (C₀-C₄)alkyl, heteroaryl (C₀-C₄)alkyl, aryl (C₀-C₆)alkoxy, heterocyclyl(C₀-C₄)alkyl, heterocyclyl(C₀-C₆)alkoxy, heteroaryl(C₀-C₆)alkoxy, and substituent group Q;
wherein the said (C₁-C₆)alkyl, (C₃-C₇)cycloalkyl, (C₃-C₇)cycloalkoxy, (C₁-C₆)alkoxy, (C₁-C₆)alkenyl, aryl (C₀-C₄)alkyl, heteroaryl (C₀-C₄)alkyl, aryl (C₀-C₆)alkoxy, heterocyclyl (C₀-C₄)alkyl, heterocyclyl(C₀-C₆)alkoxy, or heteroaryl(C₀-C₆)alkoxy is optionally substituted with 1 to 6 substituents independently selected from the group consisting of: halogen, hydroxyl, cyano, (C₁-C₆)alkyl, (C₃-C₇)cycloalkyl, (C₁-C₆)haloalkyl, (C₁-C₆)alkoxy, (C₁-C₆)alkoxy (C₁-C₆)alkyl, (C₁-C₆)alkoxy (C₁-C₆)alkoxy, (C₁-C₆)haloalkoxy, phenyl, - NR^{a}R^{b}, R^{a}R^{b}N(C₁-C₆)alkyl, R^{a}R^{b}N(C₁-C₆)alkoxy, -C(=O)NR^{a}R^{b}, -C(=O)R^{a}, - SO₂(C₁-C₆)alkyl, -SO₂NR^{a}R^{b}, and R^{a}R^{b}NC(=O)(C₁-C₆)alkoxy; wherein the said (C₃-C₇)cycloalkyl is optionally substituted with hydroxyl or cyano; wherein the substituent group Q is 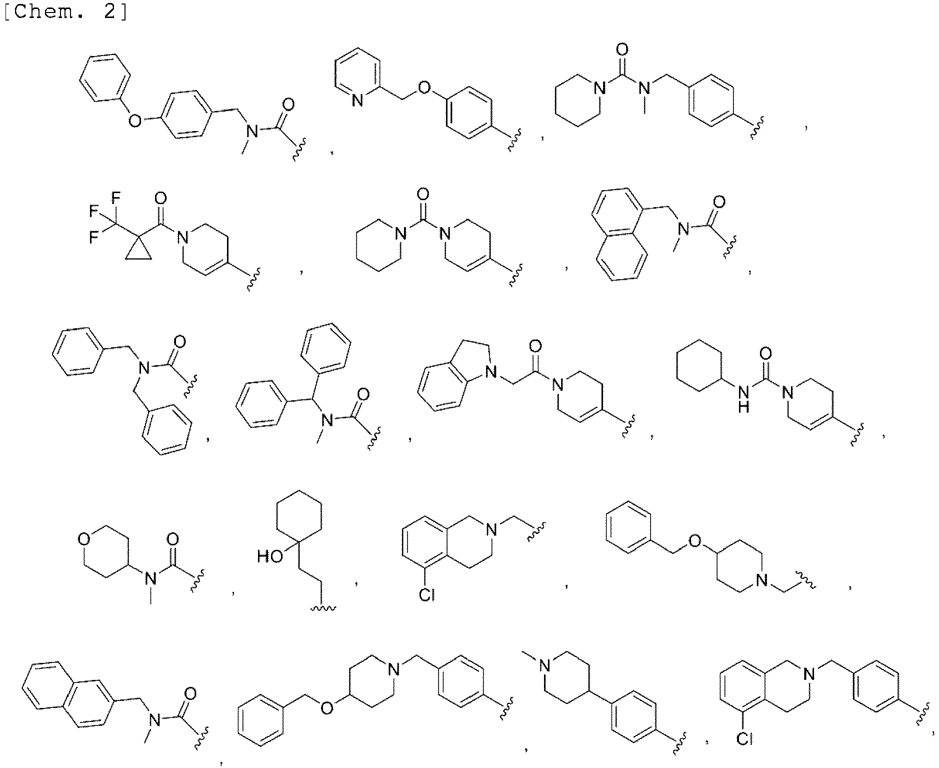 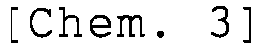 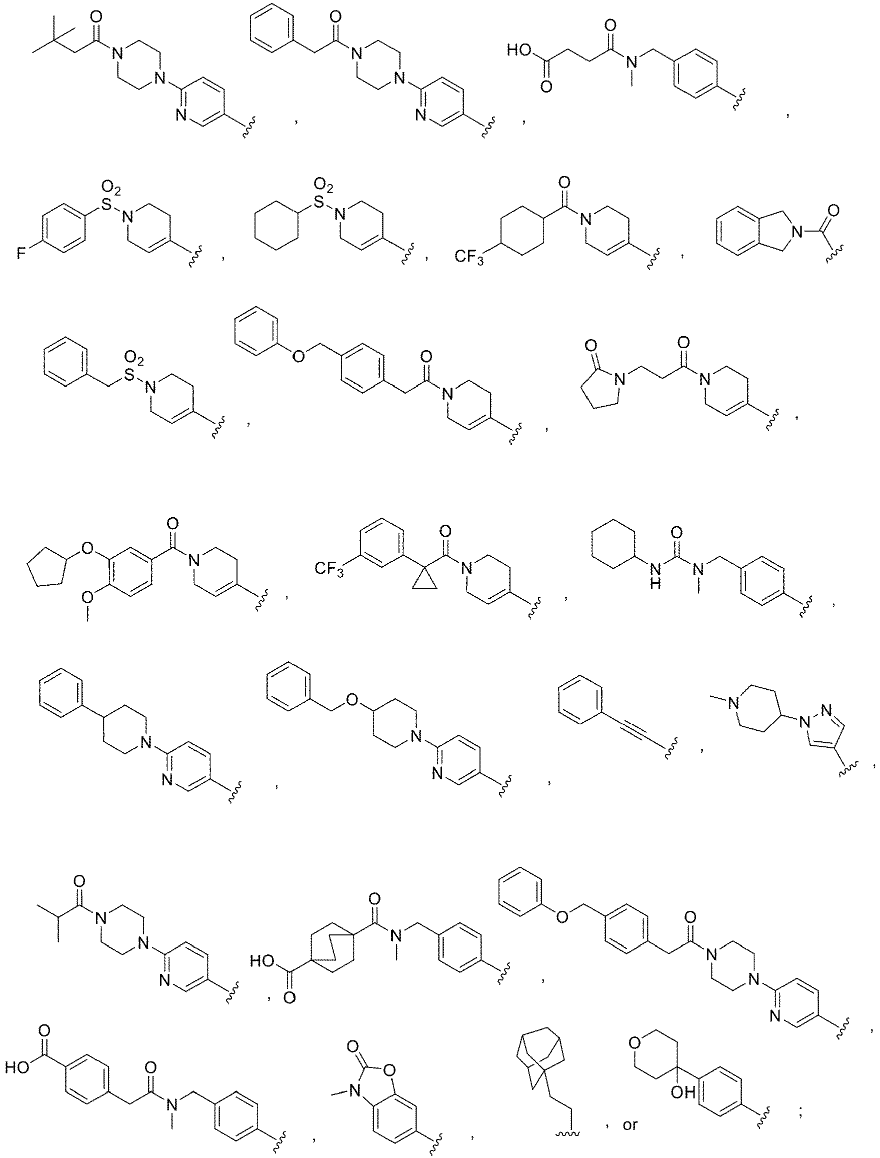 in which the substituent group Q may be optionally substituted with halogen, hydroxyl, or (C₁-C₆)alkyl;
R⁷ and R⁸ are independently selected from the group consisting of: hydrogen, hydroxyl, (C₁-C₆)alkyl, (C₁-C₆)alkoxy, (C₁-C₆)haloalkyl, (C₁-C₆)haloalkoxy, (C₃-C₇)cycloalkyl, heterocyclyl, hydroxy (C₁-C₆)alkyl, (C₁-C₆)alkoxy (C₁-C₆)alkyl, (C₁-C₆)haloalkoxy (C₁-C₆)alkyl, benzyl, H₂N(C₁-C₆)alkyl, (C₁-C₆)alkylNH(C₁-C₆)alkyl, and (C₁-C₆)alkyl]₂N(C₁-C₆)alkyl; or
R⁷ and R⁸, together with nitrogen atom to which they are attached, may form a 3 to 10 membered ring which may contain a heteroatom selected from oxygen, sulfur, and nitrogen; wherein the said 3 to 10 membered ring is optionally substituted with 1 to 6 substituents independently selected from the group consisting of: halogen, hydroxyl, oxo, (C₁-C₆)alkyl, (C₁-C₆)alkoxy, (C₁-C₆)haloalkyl, -SO₂(C₁-C₆)alkyl, -SO₂(C₁-C₆)haloalkyl, -C(=O)(C₁-C₆)alkyl, and -C (=O) (C₁-C₆)haloalkyl;
R^{a} and R^{b} are independently selected from the group consisting of: hydrogen, (C₁-C₆)alkyl, (C₁-C₆)haloalkyl, hydroxy(C₁-C₆)alkyl, (C₁-C₆)alkoxy (C₁-C₆)alkyl, (C₃-C₇)cycloalkyl, phenyl(C₀-C₆)alkyl, (C₁-C₆)haloalkoxy(C₁-C₆)alkyl, H₂N(C₁-C₆)alkyl, (C₁-C₆)alkylNH(C₁-C₆)alkyl, and [(C₁-C₆)alkyl]₂N(C₁-C₆)alkyl; or R^{a} and R^{b}, together with nitrogen atom to which they are attached, may form a 3 to 7 membered ring which may contain a heteroatom selected from oxygen, sulfur, and nitrogen; wherein the said 3 to 7 membered ring is optionally substituted with 1 to 3 substituents independently selected from (C₁-C₆)alkyl;
Ar² is selected from aryl and heteroaryl which are optionally substituted with 1 to 6 substituents independently selected from the group consisting of: hydrogen, halogen, cyano, nitro, hydroxyl, -COR⁹, -CONR⁹R¹⁰, -NHSO₂R⁹, -SO₂NR⁹R¹⁰, (C₁-C₆)alkylthio-, (C₁-C₆)alkylsulfinyl, (C₁-C₆)alkylsulfonyl, -NR⁹R¹⁰, tri(C₁-C₆)alkylsilyl, (C₁-C₆)haloalkylthio-, -SF₅, (C₁-C₆)alkyl, (C₃-C₇)cycloalkyl, (C₃-C₇)cycloalkoxy, (C₁-C₆)alkoxy, aryl (C₀-C₄)alkyl, heteroaryl(C₀-C₄)alkyl, aryl(C₀-C₆)alkoxy, phenoxy, heteroaryl(C₀-C₆)alkoxy, heterocyclyl(C₀-C₄)alkyl, and heterocyclyl(C₀-C₆)alkoxy; wherein the said (C₁-C₆)alkyl, (C₃-C₇)cycloalkyl, (C₃-C₇)cycloalkoxy, (C₁-C₆)alkoxy, aryl(C₀-C₄)alkyl, heteroaryl (C₀-C₄)alkyl, aryl (C₀-C₆)alkoxy, phenoxy, heteroaryl(C₀-C₆)alkoxy, heterocyclyl(C₀-C₄)alkyl, or heterocyclyl(C₀-C₆)alkoxy is optionally substituted with 1 to 6 substituents independently selected from the group consisting of: halogen, hydroxyl, (C₁-C₆)alkyl, (C₃-C₇)cycloalkyl (C₁-C₆)haloalkyl, (C₁-C₆)alkoxy, (C₁-C₆)alkoxy (C₁-C₆)alkoxy, (C₁-C₆)haloalkoxy, -NR^{c}R^{d}, R^{c}R^{d}N(C₁-C₆)alkyl, R^{c}R^{d}N(C₁-C₆)alkoxy, - C(=O)NR^{c}R^{d}, R^{c}R^{d}NC(=O)(C₁-C₆)alkoxy, benzyloxy, and cyano;
R⁹ and R¹⁰ are independently selected from the group consisting of: hydrogen, (C₁-C₆)alkyl, (C₁-C₆)haloalkyl, hydroxy(C₁-C₆)alkyl, (C₁-C₆)alkoxy (C₁-C₆)alkyl, (C₁-C₆)haloalkoxy (C₁-C₆)alkyl, H₂N(C₁-C₆)alkyl, (C₁-C₆)alkylNH(C₁-C₆)alkyl, and [(C₁-C₆)alkyl]₂N(C₁-C₆)alkyl; or
R⁹ and R¹⁰, together with nitrogen atom to which they are attached, may form a 3 to 10 membered ring which may contain a heteroatom selected from oxygen, sulfur, and nitrogen; wherein the said 3 to 10 membered ring is optionally substituted with 1 to 6 substituents independently selected from the group consisting of: halogen, hydroxyl, oxo, (C₁-C₆)alkyl, (C₁-C₆)alkoxy, (C₁-C₆)haloalkyl, -SO₂(C₁-C₆)alkyl, -SO₂(C₁-C₆)haloalkyl, -C(=O)(C₁-C₆)alkyl, and -C(=O)(C₁-C₆)haloalkyl; and
R^{c} and R^{d} are independently selected from the group consisting of: hydrogen, (C₁-C₆)alkyl, (C₁-C₆)haloalkyl, hydroxy(C₁-C₆)alkyl, (C₁-C₆)alkoxy (C₁-C₆)alkyl, (C₁-C₆)haloalkoxy (C₁-C₆)alkyl, H₂N(C₁-C₆)alkyl, (C₁-C₆)alkylNH(C₁-C₆)alkyl, and [(C₁-C₆)alkyl]₂N(C₁-C₆)alkyl; or
R^{c} and R^{d}, together with nitrogen atom to which they are attached, may form a 3 to 7 membered ring which may contain a heteroatom selected from oxygen, sulfur, and nitrogen; or a pharmaceutically acceptable salt thereof. 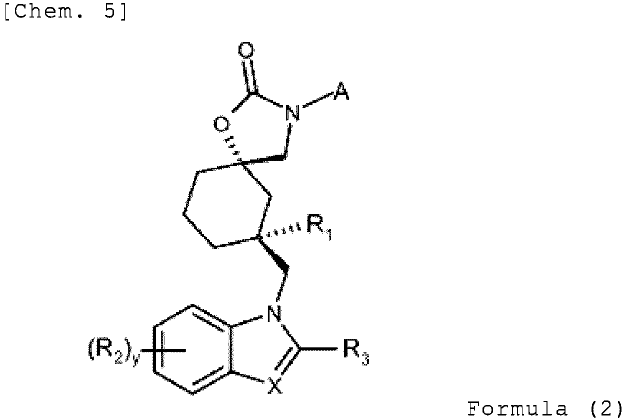 wherein:
R₁ is hydrogen, C₁₋₃alkyl, CH₂OH, CH₂-O-CH₃, CH₂OCH₂Ph, CH₂CN, CN, halo or C(O)OCH₃;
R₂ is independently hydrogen, CN, CF₃, halo, SO₂C₁₋₃alkyl, C₁₋₃alkyl or C = CH;
R₃ is hydrogen, C₁₋₂alkyl, CF₃ or OH;
R₄ is hydrogen, halo or C₁₋₃alkyl;
X is CR₄ or N;
A is (CH₂)ₙ-Het;
or A is (CH₂)ₙ-(CRₐR_{b})-(CH₂)ₘ-Het;
Rₐ is hydrogen or C₁₋₃alkyl, wherein the C₁₋₃alkyl may be further substituted with one or more halos;
R_{b} is C₁₋₃alkyl;
or Rₐ and R_{b} together with the carbon atom they are attached form a C₃₋₆cycloalkyl group;
or one of the carbon atoms in the C₃₋₆cycloalkyl group formed by Rₐ and R_{b} may be replaced with oxygen to form an oxetane, tetrahydrofuryl or tetrahydropyranyl group;
or one of the carbon atoms in the C₃₋₆cycloalkyl group formed by Rₐ and R_{b} may be replaced with nitrogen to form a pyrrolidinyl or piperidinyl group;
Het is: 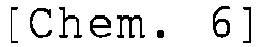 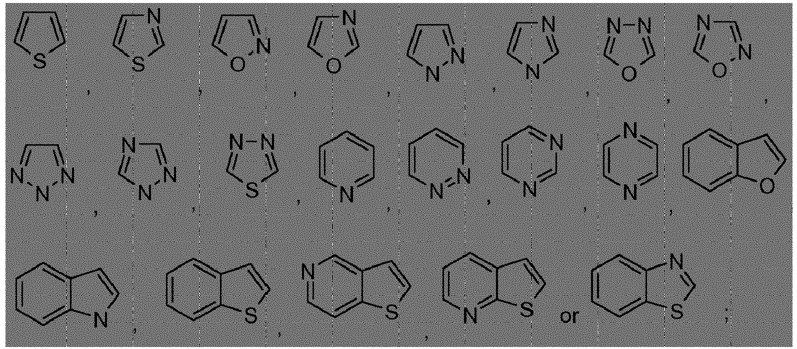
wherein Het may be substituted by one, two or three substituents chosen from: halo, C₁₋₅alkyl, CN, CH₂F, CHF₂, CF₃, C₃-₆cycloalkyl, (CH₂)ₙ-O-C₁₋₃alkyl, (CH₂)ₙ-phenyl, (CH₂)ₙ-pyridyl, pyrimidinyl, pyrazinyl, CH(CH₃)-O-C₁₋₃alkyl, CH(OH)-C₁₋₅alkyl, C(CH₃)₂-R₅, C(O)N(CH₃)ₚ, N(C₁₋₃alkyl)ₚ, NH₂, C(O)NH₂, oxetane, oxetane-CH₃, tetrahydrofuryl, tetrahydropyranyl, morpholinyl, or pyrazolyl;
wherein the phenyl, pyrazolyl, and pyridyl substituent on the Het may be further substituted by one or two substituents chosen from: halo, CN, OCH₃, C₁₋₃alkyl or CF₃;
and the C₁₋₅alkyl and C₃₋₆cycloalkyl substituent on the Het may be further substituted by CN or OH;
R₅ is CN, O-C₁₋₄alkyl, (CH₂)ₘ-OH, (CH₂)_{P}-O-C(O)-O-C₁₋₅alkyl, or O-(CH₂)ₚ-O-R₆;
R₆ is C₁₋₄alkyl or P(O)₂(CH₃)₂;
n is independently 0, 1 or 2;
m is independently 0, 1 or 2;
p is independently 1 or 2; and
y is 1, 2 or 3;
or a pharmaceutically acceptable salt thereof. 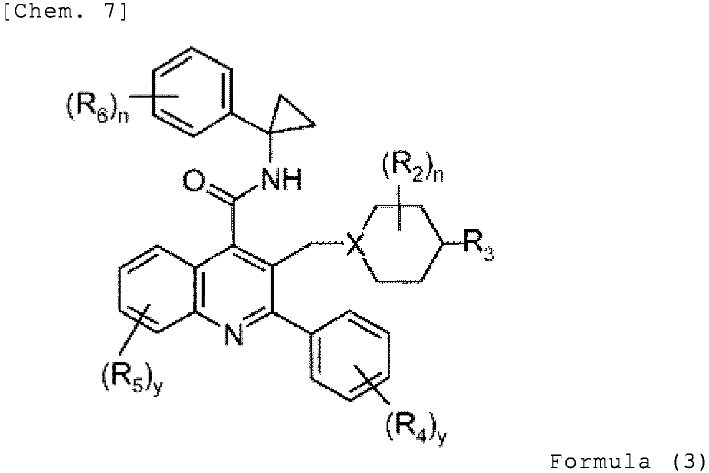
wherein:
R₁ is independently C₁₋₆ alkyl or C₃₋₆ cycloalkyl;
R₂ is independently OH, OC₁₋₄ alkyl, C₁₋₄ alkyl, CH₂OH, F, CH₂OC₁₋₄ alkyl, CF₃, or CF₂H;
R₃ is morpholinyl, piperidinyl, pyrrolidinyl, or hexahydroazepinyl, all of which may be unsubstituted or substituted by one or two R₂; or R₃ is N(C₁₋₆ alkyl)₂, wherein C₁₋₆ alkyl may be unsubstituted or substituted by OH or -OCH₃;
R₄ is independently CF₃, halo, C₁₋₃ alkyl or OC₁₋₃ alkyl;
R₅ is independently 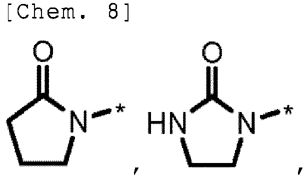
SO₂R₁, NH₂, NHSO₂R₁, NR₁SO₂R₁, C(O)NH₂, C(O)NHR₁, C(O)NHR₂, halo, cyano, CF₃, C₁₋₆ alkyl, C₂₋₄ alkenyl, pyrrolidinyl, morpholinyl, piperidinyl, phenyl, pyridyl, pyrazolyl, oxazolyl, tetrazolyl, pyrrolyl, piperazinyl, pyrimidinyl, OH, OCH₂CH₂OH, OCH₂CH₂OR₁, OCF₃, OCH₂CF₃, OCH₂CN, OR₁ or CH₂R₇;
wherein 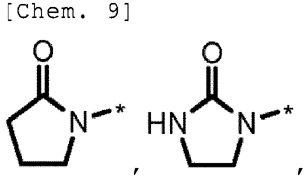
pyrrolidinyl, morpholinyl, piperidinyl, phenyl, pyridyl, pyrazolyl, oxazolyl, tetrazolyl, pyrrolyl, piperazinyl or pyrimidinyl may be unsubstituted or substituted with one or two halo, OH, OR₁ or R₁;
or two adjacent R₅ groups may be combined to form 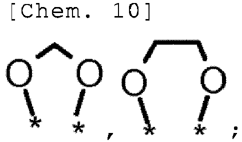
R₆ is independently halo, methyl, or OMe;
R₇ is pyrrolidinyl, morpholinyl, or piperidinyl;
n is independently 0, 1, or 2;
X is N or C;
y is independently 0, 1 or 2;
or a pharmaceutically acceptable salt thereof. 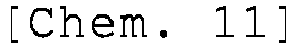 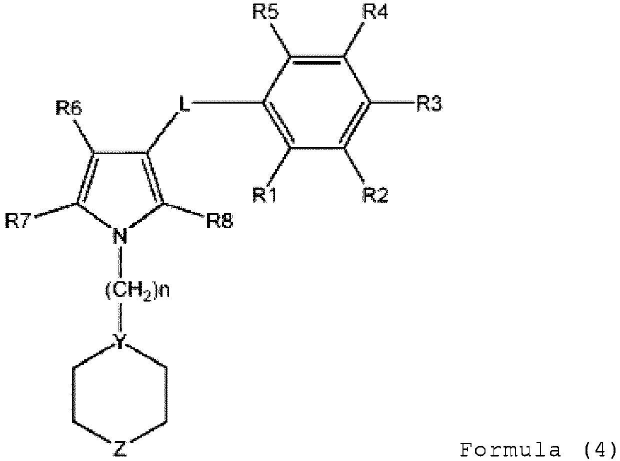
wherein L is C(=O)NR10, SO₂NR10, a C₁-C₆ alkylene, or a bond;
Y is N or CR10;
Z is O, NR10, S, SO₂ or C(R10)₂;
n is 0, 1, 2, 3, 4, 5, or 6;
R1, R2, R3, R4, and R5 are independently selected from at least one of hydro, alkyl, haloalkyl, hydroxy, alkoxy, haloalkoxy, cyano, carboxyl, carboxyalkyl or amido;
R6 and R7 are independently selected from at least one of hydro, alkyl, haloalkyl, heterocyclic or aryl, or R6 and R7 are connected to via a cyclic ring system;
R8 is hydro, alkyl, haloalkyl, heterocyclic or aryl; and
R10 is hydro, alkyl, haloalkyl, carboxyalkyl, carboxyl, alkyl methylene carbonate, methylene carbamyl, thiophenyl or -S-carboxyalkyl;
or pharmaceutically acceptable salts thereof.

6. A use according to any one of claims 1 to 5, wherein the compound having TRPV4 inhibitory activity is selected from GSK2798745 (1-(((5S,7S)-3-(5-(2-hydroxypropan-2-yl)pyrazin-2-yl)-7-methyl-2-oxo-1-oxa-3-azaspiro[4.5]decan-7-yl)methyl)-1H-benzo[d]imidazole-6-carbonitrile), GSK2193874 (3-(1,4'-bipiperidine-1'-ylmethyl)-7-bromo-N-(1-phenylcyclopropyl)-2-[3-(trifluoromethyl)phenyl]-4-quinolinecarboxamide), HC-067047 (N-(3-(trifluoromethyl)phenyl)-2-methyl-1-(3-morpholinopropyl)-5-phenyl-1H-pyrrole-3-carboxamide), GSK3395879, GSK3527497, GSK205, GSK3491943, RN-1734, RN-1747, RN-9893, PF-05214030,
(R)-1-(3'-(1-(6-(2-(3-chlorophenyl)-2-hydroxyacetyl)-4-oxo-4,5,6,7,8,9-hexahydro-3H-pyrimido[5,4-c]azepin-2-yl)cyclopropyl)-[1,1'-biphenyl]-4-yl)cyclopropane-1-carbonitrile;
(R)-2-(1-(3-(benzo[b]thiophen-3-yl)phenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(3-(benzofuran-3-yl)phenyl)cyclopropyl)-6-(2-(3-chlorophenyl)-2-hydroxyacetyl)-3,5,6,7,8,9-hexahydro-4H-pyrimido[5,4-c]azepin-4-one;
(R)-2-(1-(3-chlorophenyl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-2-(1-(4-bromothiophen-2-yl)cyclopropyl)-6-(2-(3-chlorophenyl)-2-hydroxyacetyl)-5,6,7,8-tetrahydropyride[4,3-d]pyrimidin-4(3H)-one;
(R)-2-(1-(5-Cyclohexylpyridin-3-yl)cyclopropyl)-6-(2-hydroxy-2-(3-(trifluoromethyl)phenyl)acetyl)-3,5,6,7,8,9-hexahydro-4H-pyrimido[5,4-c]azepin-4-one;
(R)-5-(3-(1-(6-(2-hydroxy-2-(3-(trifluoromethyl)phenyl)acetyl)-4-oxo-4,5,6,7,8,9)-hexahydro-3H-pyrimido[5,4-c]azepin-2-yl)cyclopropyl)phenyl)nicotinonitrile;
(R)-6-(2-(2,3-difluorophenyl)-2-hydroxyacetyl)-2-(1-phenylcyclopropyl)-3,5,6,7,8,9-hexahydro-4H-pyrimido[5,4-c]azepin-4-one;
(R)-6-(2-(2,3-difluorophenyl)-2-hydroxyacetyl)-2-(1-phenylcyclopropyl)-5,6,7,8-tetrahydropyrido[4,3-d]pyrimidin-4(3H)-one;
(R)-6-(2-(3-(benzofuran-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzofuran-2-yl)phenyl)-2-hydroxyacetyl)-2-(1-phenylcyclopropyl)-3,5,6,7,8,9-hexahydro-4H-pyrimido[5,4-c]azepin-4-one;
(R)-6-(2-(3-(benzofuran-2-yl)phenyl)-2-hydroxyacetyl)-2-(1-phenylcyclopropyl)-5,6,7,8-tetrahydropyrido[4,3-d]pyrimidin-4(3H)-one;
(R)-6-(2-(3'-(tert-butyl)-[1,1'-biphenyl]-3-yl)-2-hydroxyacetyl)-2-(1-(4-isopropylthiophene-2-yl)cyclopropyl)-3,5,6,7,8,9-hexahydro-4H-pyrimido[5,4-c]azepin-4-one;
(R)-6-(2-(3'-(tert-butyl)-[1,1'-biphenyl]-3-yl)-2-hydroxyacetyl)-2-(1-(4-phenylthiophene-2-yl)cyclopropyl)-3,5,6,7,8,9-hexahydro-4H-pyrimido[5,4-c]azepin-4-one;
(R)-6-(2-(3'-chloro-[1,1'-biphenyl]-3-yl)-2-hydroxyacetyl)-2-(1-phenylcyclopropyl)-3,5,6,7,8,9-hexahydro-4H-pyrimido[5,4-c]azepin-4-one;
(R) -6-(2-(3-chlorophenyl)-2-hydroxyacetyl)-2-(1-(3-(1-methyl-1H-indazol-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(1-methyl-1H-indazol-5-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-(2-methoxypyridin-4-yl)phenyl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(3-chlorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(3-fluorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-chlorophenyl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
(R)-6-(2-(3-chlorophenyl)-2-hydroxyacetyl)-2-(1-(4-phenylthiophen-2-yl)cyclopropyl)-5,6,7,8-tetrahydropyride[4,3-d]pyrimidin-4(3H)-one;
(R)-6-(2-(3-chlorophenyl)-2-hydroxyacetyl)-2-(1-(thiophen-2-yl)cyclopropyl)-5,6,7,8-tetrahydropyrido[4,3-d]pyrimidin-4(3H)-one;
(R)-6-(2-(3-chlorophenyl)-2-hydroxyacetyl)-2-(1-phenylcyclopropyl)-3,5,6,7,8,9-hexahydro-4H-pyrimido[5,4-c]azepin-4-one;
(R)-6-(2-(3-chlorophenyl)-2-hydroxyacetyl)-2-(1-phenylcyclopropyl)-5,6,7,8-tetrahydropyrido[4,3-d]pyrimidine-4(3H)-one;
(R)-6-(2-(4'-(benzyloxy)-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
(R)-6-(2-Hydroxy-2-(3-(2-(trifluoromethyl)pyridin-4-yl)phenyl)acetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3-(2-(trifluoromethyl)pyridin-4-yl)phenyl)acetyl)-2-(1-(4-isopropylthiophen-2-yl)cyclopropyl)-3,5,6,7,8,9-hexahydro-4H-pyrimido[5,4-c]azepin-4-one;
(R)-6-(2-hydroxy-2-(3-(6-(trifluoromethyl)pyridin-2-yl)phenyl)acetyl)-2-(1-phenylcyclopropyl)-3,5,6,7,8,9-hexahydro-4H-pyrimido[5,4-c]azepin-4-one;
(R)-6-(2-hydroxy-2-(3-(6-(trifluoromethyl)pyridin-2-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3-(quinolin-3-yl)phenyl)acetyl)-2-(1-phenylcyclopropyl)-5,6,7,8-tetrahydropyrido[4,3-d]pyrimidin-4(3H)-one;
(R)-6-(2-hydroxy-2-(3'-(trifluoromethyl)-[1,1'-biphenyl]-3-yl)acetyl)-2-(1-(3-isopropylphenyl)cyclopropyl)-3,5,6,7,8,9-hexahydro-4H-pyrimido[5,4-c]azepin-4-one;
(R)-6-(2-hydroxy-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(isothiazol-4-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(isothiazol-4-yl)phenyl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(3-(prop-1-en-2-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-(pyridin-3-yl)phenyl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(3-isopropylphenyl)cyclopropyl)-3,5,6,7,8,9-hexahydro-4H-pyrimido[5,4-c] azepin-4-one;
(R)-6-(2-hydroxy-2-(3-(trifluoromethyl)phenyl)acetyl)-2-(1-(4-isopropylthiophen-2-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(4-isopropylthiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(4-isopropylthiophen-2-yl)cyclopropyl)-5,6,7,8-tetrahydropyrido[4,3-d]pyrimidin-4(3H)-one;
(R)-6-(2-hydroxy-2-(3-(trifluoromethyl)phenyl)acetyl)-2-(1-(5-isopropylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
(R)-6-(2-hydroxy-2-(3-(trifluoromethyl)phenyl)acetyl)-2-(1-phenylcyclopropyl)-5,6,7,8-tetrahydropyrido[4,3-d]pyrimidin-4(3H)-one;
2-(1-(3-chlorophenyl)cyclopropyl)-6-(2-(4'-(trifluoromethoxy)-[1,1'-biphenyl]-3-yl)acetyl)-3,5,6,7,8,9-hexahydro-4H-pyrimido[5,4-c]azepin-4-one;
2-(1-(3-chlorophenyl)cyclopropyl)-6-(2-hydroxy-2-(3'-(trifluoromethoxy)-[1,1'-biphenyl]-3-yl)acetyl)-3,5,6,7,8,9-hexahydro-4H-pyrimido[5,4-c]azepin-4-one;
2-(1-(5-phenylpyridin-3-yl)cyclopropyl)-6-(2-(3'-(trifluoromethyl)-[1,1'-biphenyl]-3-yl)acetyl)-3,5,6,7,8,9-hexahydro-4H-pyrimido[5,4-c]azepin-4-one;
3'-(2-(2-(1-(3-chlorophenyl)cyclopropyl)-4-oxo-3,4,5,7,8,9-hexahydro-6H-pyrimido[5,4-c]azepine-6-yl)-2-oxoethyl)-[1,1'-biphenyl]-4-carbonitrile;
5-chloro-3'-(2-oxo-2-(4-oxo-2-(1-phenylcyclopropyl)-3,4,5,7,8,9-hexahydro-6H-pyrimido[5,4-c]azepin-6-yl)ethyl)-[1,1'-biphenyl]-3-carbonitrile;
6-(2-(2-(benzyloxy)phenyl)acetyl)-2-(1-phenylcyclopropyl)-3,5,6,7,8,9-hexahydro-4H-pyrimido[5,4-c]azepin-4-one;
6-(2-(3-(benzyloxy)phenyl)acetyl)-2-(1-phenylcyclopropyl)-3,5,6,7,8,9-hexahydro-4H-pyrimido[5,4-c]azepin-4-one;
6-(2-(3'-(tert-butyl)-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one;
6-(2-(3'-chloro-[1,1'-biphenyl]-3-yl)acetyl)-2-(1-phenylcyclopropyl)-3,5,6,7,8,9-hexahydro-4H-pyrimido[5,4-c]azepin-4-one;
6-(2-(3-chloro-2-fluorophenyl)-2-hydroxyacetyl)-2-(1-(4-isopropylthiophen-2-yl)cyclopropyl)-3,5,6,7,8,9-hexahydro-4H-pyrimido[5,4-c]azepin-4-one;
6-(2-(3'-chloro-5'-fluoro-[1,1'-biphenyl]-3-yl)acetyl)-2-(1-(3-chlorophenyl)cyclopropyl)-3,5,6,7,8,9-hexahydro-4H-pyrimido[5,4-c]azepin-4-one;
6-(2-(3'-cyclopropyl-[1,1'-biphenyl]-3-yl)-2,2-difluoroacetyl)-2-(1-phenylcyclopropyl)-3,5,6,7,8,9-hexahydro-4H-pyrimido[5,4-c]azepin-4-one;
6-(2-(4'-chloro-[1,1'-biphenyl]-3-yl)-2-hydroxyacetyl)-2-(1-phenylcyclopropyl)-5,6,7,8-tetrahydropyrido[4,3-d]pyrimidin-4(3H)-one;
6-(2,2-difluoro-2-(3'-(trifluoromethoxy)-[1,1'-biphenyl]-3-yl)acetyl)-2-(1-phenylcyclopropyl)-3,5,6,7,8,9-hexahydro-4H-pyrimido[5,4-c]azepin-4-one;
6-(2,2-difluoro-2-(3'-(trifluoromethyl)-[1,1'-biphenyl]-3-yl)acetyl)-2-(1-phenylcyclopropyl)-3,5,6,7,8,9-hexahydro-4H-pyrimido[5,4-c]azepin-4-one;
6-(2,2-difluoro-2-(3-(trifluoromethyl)phenyl)acetyl)-2-(1-(5-phenylpyridin-3-yl)cyclopropyl)-3,5,6,7,8,9-hexahydro-4H-pyrimido[5,4-c]azepin-4-one;
6-(2,2-difluoro-2-(3'-isopropoxy-[1,1'-biphenyl]-3-yl)acetyl)-2-(1-phenylcyclopropyl)-3,5,6,7,8,9-hexahydro-4H-pyrimido[5,4-c]azepin-4-one; and
6-(2-hydroxy-2-(3'-(trifluoromethoxy)-[1,1'-biphenyl]-3-yl)acetyl)-2-(1-(5-phenylpyridin-3-yl)cyclopropyl)-3,5,6,7,8,9-hexahydro-4H-pyrimido[5,4-c]azepin-4-one,
or a pharmaceutically acceptable salt thereof.

7. The use according to any one of claims 1 to 4, wherein the compound having TRPV4 inhibitory activity is a polymer that is an antibody, oligonucleotide, siRNA or aptamer that binds to TRPV4 (GeneID: 59341).

8. The use according to any one of claims 1 to 7, comprising combination with one or more second active agents.

9. A pharmaceutical composition comprising a compound having TRPV4 inhibitory activity or a pharmaceutically acceptable salt thereof, which is used for preventing or treating a retinal disease accompanied with blood flow disorder or cell disorder in humans or animals.

10. The pharmaceutical composition according to claim 9, wherein the retinal disease is retinal vein occlusion or wet age-related macular degeneration.

11. The pharmaceutical composition according to claim 9, wherein the retinal disease is branch retinal vein occlusion or central retinal vein occlusion.

12. The pharmaceutical composition according to claim 9, wherein the compound having TRPV4 inhibitory activity is the compound according to any one of claims 5 to 7 or a pharmaceutically acceptable salt thereof.

13. A method for treating a retinal disease accompanied with blood flow disorder or cell disorder, which comprises administering to humans or animals an effective amount of a compound having TRPV4 inhibitory activity or a pharmaceutically acceptable salt thereof.

14. The method according to claim 13, wherein the retinal disease is retinal vein occlusion or wet age-related macular degeneration.

15. The method according to claim 13, wherein the compound having TRPV4 inhibitory activity is the compound according to any one of claims 5 to 7 or a pharmaceutically acceptable salt thereof.

16. A compound having TRPV4 inhibitory activity or a pharmaceutically acceptable salt thereof for use in treating a retinal disease accompanied with blood flow disorder or cell disorder in humans or animals.

17. A kit for use in preventing or treating the diseases, comprising the compound according to any one of claims 5 to 7.

18. A kit according to claim 17 comprising a pharmaceutical composition according to any one of claims 9 to 12, one or more second active agents and a container.

19. A disease marker for a retinal disease, which is a polynucleotide having at least 15 contiguous bases in the TRPV4 gene sequence shown in any of SEQ ID NOs 1 to 5 (Gene Bank ACCESSION: NM_021625, NM_147204, NM_001177433, NM_001177431 or NM_001177428) and/or complementary to the said polynucleotide.

20. The disease marker according to claim 19, which is used as a probe or primer in detecting a retinal disease.

21. A disease marker for a retinal disease, which is an antibody that recognizes the TRPV4 protein comprising the amino acid sequences shown in SEQ ID NOs: 1 to 5.

22. The disease marker according to claim 21, which is used as a probe in detecting a retinal disease.

23. A method for detecting a retinal disease comprising the following steps (a), (b) and (c):
(a) a step of combining RNA prepared from a subject's biological sample or a complementary polynucleotide transcribed from the RNA with the disease marker according to claim 19 or claim 20;
(b) a step of measuring RNA derived from the biological sample that binds to the disease marker or a complementary polynucleotide transcribed from the RNA, using the above disease marker as an indicator; and
(c) a step of diagnosing the incidence of the retinal disease based on the measurement result of (b) above.

24. The method for detecting the retinal disease according to claim 23, wherein diagnosing the incidence of the retinal disease in the step (c) is performed by comparing the measurement results obtained for the subject with those obtained for a normal subject, using an increased amount of binding to the disease marker as an indicator.

25. A method for detecting a retinal disease comprising the following steps (a), (b) and (c):
(a) a step of combining a protein prepared from a subject's biological sample with the disease marker according to claim 21 or claim 22;
(b) a step of measuring a protein or a partial peptide thereof derived from the biological sample that binds to the disease marker, using the above disease marker as an indicator; and
(c) a step of diagnosing the incidence of the retinal disease based on the measurement result of (b) above.

26. The method for detecting the retinal disease according to claim 25, wherein diagnosing the incidence of the retinal disease in the step (c) is performed by comparing the measurement results obtained for the subject with those obtained for a normal subject, using an increased amount of binding to the disease marker as an indicator.

27. A screening method for a substance that suppresses TRPV4 gene expression, comprising the following steps (a), (b) and (c):
(a) a step of contacting a test substance with cells capable of expressing the TRPV4 gene;
(b) a step of measuring the expression level of the TRPV4 gene in cells contacted with the test substance, and comparing the said expression level with the expression level of the said gene in control cells not contacted with the test substance; and
(c) a step of selecting a test substance that reduces the expression level of the TRPV4 gene based on the comparison result of (b) above.

28. A screening method for a substance that reduces the expression level of a TRPV4 protein, comprising the following steps (a), (b) and (c):
(a) a step of contacting a test substance with a cell capable of expressing a TRPV4 protein or a cell fraction prepared from the said cell;
(b) a step of measuring the expression level of the TRPV4 protein in cells or cell fractions contacted with the test substance, and comparing the said expression level with the expression level of the TRPV4 protein in control cells or cell fractions not contacted with the test substance; and
(c) a step of selecting a test substance that reduces the expression level of the TRPV4 protein based on the comparison result of (b) above.

29. A screening method for a substance that inhibits TRPV4 activity, comprising the following steps (a), (b), and (c):
(a) a step of contacting a TRPV4 ligand with a TRPV4 protein in the presence of a test substance;
(b) a step of measuring the binding amount of the TRPV4 ligand to the TRPV4 protein in the presence of the test substance, and comparing the binding amount with a binding amount of the TRPV4 ligand to the TRPV4 protein in the absence of the test substance (control binding amount); and
(c) a step of selecting a test substance that reduces the binding amount comparing to the control binding amount based on the comparison result of (b) above.

30. The screening method according to any one of claims 27 to 29, wherein the method is for searching an active ingredient of an agent for preventing or treating a retinal disease.

31. An agent for preventing or treating a retinal disease, comprising a substance that suppresses TRPV4 gene expression as an active ingredient.

32. The agent for preventing or treating a retinal disease according to claim 31, wherein the substance that suppresses TRPV4 gene expression is obtained by the screening method according to claim 27.

33. An agent for preventing or treating a retinal disease, comprising a substance that suppresses the expression level of a TRPV4 protein or a substance that inhibits the activity of TRPV4 as an active ingredient.

34. The agent for preventing or treating a retinal disease according to claim 33, wherein the substance that suppresses the expression level of a TRPV4 protein or the substance that inhibits the activity of TRPV4 is obtained by the screening method according to claim 28 or claim 29.
